# EUROPÄISCHE PATENTSCHRIFT

(11) **EP 1 597 259 B1**
(45) Veröffentlichungstag und Bekanntmachung des Hinweises auf die Patenterteilung: **30.04.2008**
(21) Anmeldenummer: 04706145.2
(22) Anmeldetag: 29.01.2004
(51) Int. Cl.: C07D 487/04, A61K 31/519, A61P 3/04

(54) **STICKSTOFF SUBSTITUIERTE HEXAHYDRO-PYRAZINO 1,2-A PYRIMIDIN-4,7-DIONDERIVATE, VERFAHREN ZU IHRER HERSTELLUNG UND IHRE VERWENDUNG ALS ARZNEIMITTEL**
NITROGEN-SUBSTITUTED HEXAHYDROPYRAZINO 1,2-A PYRIMIDINE-4,7-DIONE DERIVATIVES, METHOD FOR THE PRODUCTION AND USE THEREOF AS MEDICAMENTS
DERIVES DE HEXAHYDRO-PYRAZINO 1,2-A PYRIMIDINE-4,7-DIONES A SUBSTITUTION AZOTE, PROCEDES DE FABRICATION ET UTILISATION EN TANT QU'AGENTS PHARMACEUTIQUES

(30) Priorität: 13.02.2003 DE 10305885; 24.10.2003 DE 10349671
(43) Veröffentlichungstag der Anmeldung: 23.11.2005
(73) Patentinhaber: Sanofi-Aventis Deutschland GmbH, 65929 Frankfurt am Main (DE)
(72) Erfinder: FLOHR, Stefanie, CH-4153 Reinach (CH); STENGELIN, Siegfried, 65817 Eppstein (DE); GOSSEL, Matthias, 65719 Hofheim (DE); KLABUNDE, Thomas, 65929 Frankfurt (DE); LENNING, Petra, 65926 Frankfurt am Main (DE); SAFAR, Pavel, Tucson, AZ 85737 (US); SPOONAMORE, James, Tucson, AZ 85737 (US); SMRCINA, Martin, Tucson, AZ 85737 (US); KLEIN, Joseph, T., Neshanic, NJ 08853 (US); MERRIMAN, Gregory, H., Phillipsburg, NJ 08865 (US); WHITELEY, Brian, K., Lebanon, NJ 08833 (US); LANTER, Carolina, Flemington, NJ 08822 (US); BORDEAU, Kenneth, J., Kintersville, PA 18930 (US); YANG, Zhaoxia, Roselle Park, NJ 07204 (US)
(86) Internationale Anmeldenummer: PCT/EP2004/000770
(87) Internationale Veröffentlichungsnummer: WO 2004/072077

(56) Entgegenhaltungen:
- WO-A-01/16135
- US-A1- 2002 065 284

## Beschreibung

Die Erfindung betrifft substituierte Hexahydro-pyrazino[1,2-a]pyrimidin-4,7-dionderivate sowie deren physiologisch verträgliche Salze.

Der Erfindung lag die Aufgabe zugrunde, Verbindungen zur Verfügung zu stellen, die eine Gewichtsreduktion bei Säugetieren bewirken und die zur Prävention und Behandlung von Adipositas geeignet sind.

Die Erfindung betrifft daher Verbindungen der Formel I, 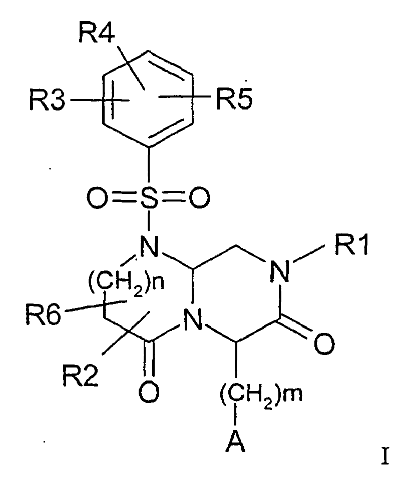 worin bedeuten
- A: 3-12 gliedriger mono-, bi- oder spirobicyclischer Ring, der ein oder mehrere Heteroatome aus der Gruppe N, O und S enthalten kann und der 3-12 gliedrige Ring weitere Substituenten wie F, Cl, Br, NO₂, CF₃, OCF₃, CN, (C₁-C₆)-Alkyl, Aryl, CON(R11)(R12), N(R13)(R14), OH, O-(C₁-C₆)-Alkyl, S-(C₁-C₆)-Alkyl, N(R15)CO(C₁-C₆)-Alkyl oder COO-(C₁-C₆)-Alkyl tragen kann;
- R11, R12, R13, R14, R15: unabhängig voneinander H, (C₁-C₆)-Alkyl, Heterocyclus;
- n: 0, 1;
- m: 0, 1, 2, 3, 4, 5, 6;
- R1: R8, (C₁-C₆)-Alkylen-R8, (C₂-C₆)-Alkenylen-R9, (SO₂)-R8 (SO₂)-(C₁-C₆)-Alkylen-R8, (SO₂)-(C₂-C₆)-Alkenylen-R9, (C=O)-R8, (C=O)-(C₁-C₆)-Alkylen-R8, (C=O)NH-R8, (C=O)-(C₂-C₆)-Akenylen-R9, (C=O)-NH- (C₁-C₆)-Alkylen-R8, (C=O)-NH- (C₂-C₆)-Alkenylen-R9, COO-R8, COO-(C₁-C₆)-Alkylen-R8, COO-(C₂-C₆)-Alkenylen-R9, Alkinylen-R9, (C₁-C₄-Alkyl)-Heterocyclus, wobei die Alkylengruppen mit F substituiert sein könne;
- R8, R9: unabhängig voneinander H, F, Cl, Br, I, OH, CF₃, Aryl, Heterocyclus, (C₃-C₈)-Cycloalkyl, wobei die Ringe oder Ringssysteme bis zu 3-fach substituiert sein können mit F, Cl, Br, I, OH, CF₃, NO₂ CN, OCF₃, O-(C₁-C₆)-Alkyl, (C₁-C₆)-Alkyl, NH₂, CON(R11)(R12), N(R13)(R14), SO₂-CH₃, COOH, COO-(C₁-C₆)-Alkyl, CONH₂;
- R2: NH₂, NO₂, N(R13)(R14), NH-SO₂-CH₃, NH-SO₂-R12, NR11-SO₂-R12, N(CO)R11, ein Stickstoffhaltiger Heterocyclus, wobei der Heterocyclus über ein Stickstoffatom gebunden ist, NHCONR11, N(C₁-C₆-Alkyl)N⁺(C₁-C₄-Alkyl)₃;
- R3, R4, R5: unabhängig voneinander H, F, Cl, Br, J, OH, CF₃, NO₂, CN, OCF₃, O-(C₁-C₆)-Alkyl, O-(C₁-C₄)-Alkoxy-(C₁-C₄)-alkyl, S-(C₁-C₆)-Alkyl, (C₁-C₆)-Alkyl, (C₂-C₆)-Alkenyl, (C₃-C₈)-Cycloalkyl, O-(C₃-C₈)-Cycloalkyl, (C₃-C₈)-Cycloalkenyl, O-(C₃-C₈)-Cycloalkenyl, (C₂-C₆)-Alkinyl, Aryl, O-Aryl (C₁-C₈)-Alkylen-Aryl, O-(C₁-C₈)-Alkylen-Aryl, S-Aryl, N((C₁-C₆)-Alkyl)₂, SO₂-CH₃, COOH, COO-(C₁-C₆)-Alkyl, CO-N((C₁-C₆)-Alkyl)₂;
- R6: H, F, Cl, Br, J, OH, CF₃, NO₂ CN, OCF₃, O-(C₁-C₆)-Alkyl, O-(C₁-C₄)-Alkoxy (C₁-C₄)-alkyl, S-(C₁-C₆)-Alkyl, (C₁-C₆)-Alkyl, (C₂-C₆)-Alkenyl, (C₃-C₈)-Cycloalkyl, O-(C₃-C₈)-Cycloalkyl, (C₃-C₈)-Cycloalkenyl, O-(C₃-C₈)-Cycloalkenyl, (C₂-C₆)-Alkinyl, (C₀-C₈)-Alkylen-Aryl, O-(C₀-C₈)-Alkylen-Aryl, S-Aryl, N((C₁-C₆)-Alkyl)₂, SO₂-CH₃, COOH, COO-(C₁-C₆)-Alkyl, CO-N((C₁-C₆)-Alkyl)₂;
sowie deren physiologisch verträglichen Salze.

Bevorzugt sind Verbindungen der Formel I der folgenden Struktur Ia 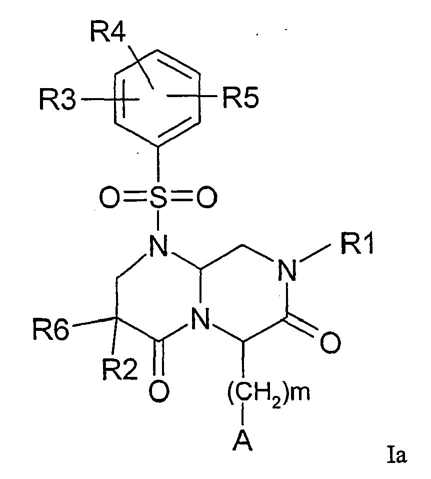 worin bedeuten
- A: 3-12 gliedriger mono-, bi- oder spirobicyclischer Ring, der ein oder mehrere Heteroatome aus der Gruppe N, O und S enthalten kann und der 3-12 gliedrige Ring weitere Substituenten wie F, Cl, Br, NO₂, CF₃, OCF₃, CN, (C₁-C₆)-Alkyl, Aryl, CON(R11)(R12), N(R13)(R14), OH, O-(C₁-C₆)-Alkyl, S-(C₁-C₆)-Alkyl, N(R15)CO(C₁-C₆)-Alkyl oder COO-(C₁-C₆)-Alkyl tragen kann;
- R11, R12, R13, R14, R15: unabhängig voneinander H, (C₁-C₆)-Alkyl, Heterocyclus;
- m: 0, 1, 2, 3, 4, 5, 6;
- R1: R8, (C₁-C₆)-Alkylen-R8, (C₂-C₆)-Alkenylen-R9, (SO₂)-R8, (SO₂)-(C₁-C₆)-Alkylen-R8, (SO₂)-(C₂-C₆)-Alkenylen-R9, (C=O)-R8, (C=O)-(C₁-C₆)-Alkylen-R8, (C=O)NH-R8, (C=O)-(C₂-C₆)-Akenylen-R9, (C=O)-NH- (C₁-C₆)-Alkylen-R8, (C=O)-NH- (C₂-C₆)-Akenylen-R9, COO-R8, COO-(C₁-C₆)-Alkylen-R8, COO-(C₂-C₆)-Akenylen-R9, Alkinylen-R9, (C₁-C₄-Alkyl)-Heterocyclus;
- R8, R9: unabhängig voneinander H, F, Cl, Br, I, OH, CF₃, Aryl, Heterocyclus, (C₃-C₈)-Cycloalkyl, wobei die Ringe oder Ringssysteme bis zu 3-fach substituiert sein können mit F, Cl, Br, I, OH, CF₃, NO₂, CN, OCF₃, O-(C₁-C₆)-Alkyl, (C₁-C₆)-Alkyl, NH₂, CON(R11)(R12), N(R13)(R14), SO₂-CH₃, COOH, COO-(C₁-C₆)-Alkyl, CONH₂;
- R2: NH₂,NO₂, N(R13)(R14), NH-SO₂-CH₃, NH-SO₂-R12, NR11-SO₂-R12, N(CO)R11, ein Stickstoffhaltiger Heterocyclus, wobei der Heterocyclus über ein Stickstoffatom gebunden ist, NHCONR11, N(C₁-C₆-Alkyl)N⁺(C₁-C₄-Alkyl)₃;
- R3, R4, R5: unabhängig voneinander H, F, Cl, Br, J, OH, CF₃, NO₂ CN, OCF₃,O-(C₁-C₆)-Alkyl, O-(C₁-C₄)-Alkoxy-(C₁-C₄)-alkyl, S-(C₁-C₆)-Alkyl, (C₁-C₆)-Alkyl, (C₂-C₆)-Alkenyl, (C₃-C₈)-Cycloalkyl, O-(C₃-C₈)-Cycloalkyl, (C₃-C₈)-Cycloalkenyl, O-(C₃-C₈)-Cycloalkenyl, (C₂-C₆)-Alkinyl, Aryl, O-Aryl (C₀-C₈)-Alkylen-Aryl, O-(C₀-C₈)-Alkylen-Aryl, S-Aryl, N((C₁-C₆)-Alkyl)₂, SO₂-CH₃, COOH, COO-(C₁-C₆)-Alkyl, CO-N((C₁-C₆)-Alkyl)₂;
- R6: H, F, Cl, Br, J, OH, CF₃, NO₂, CN, OCF₃, O-(C₁-C₆)-Alkyl, O-(C₁-C₄)-Alkoxy-(C₁-C₄)-alkyl, S-(C₁-C₆)-Alkyl, (C₁-C₆)-Alkyl, (C₂-C₆)-Alkenyl, (C₃-C₈)-Cycloalkyl, O-(C₃-C₈)-Cycloalkyl, (C₃-C₈)-Cycloalkenyl, O-(C₃-C₈)-Cycloalkenyl, (C₂-C₆)-Alkinyl, Aryl, O-Aryl, (C₁-C₈)-Alkylen-Aryl, O-(C₁-C₈)-Alkylen-Aryl, S-Aryl, N((C₁-C₆)-Alkyl)₂, SO₂-CH₃, COOH, COO-(C₁-C₆)-Alkyl, CO-N((C₁-C₆)-Alkyl)₂;
sowie deren physiologisch verträglichen Salze.

Besonders bevorzugt sind Verbindungen der Formel Ia,
worin bedeuten
- A: Aryl, wobei der Arylring substituiert sein kann mit F, Cl, Br, NO₂ CF₃, OCF₃, CN, (C₁-C₆)-Alkyl, Aryl, CON(R11)(R12), N(R13)(R14), OH, O-(C₁-C₆)-Alkyl, S-(C₁-C₆)-Alkyl, N(R15)CO(C₁-C₆)-Alkyl oder COO-(C₁-C₆)-Alkyl;
- R11, R12, R13, R14, R15: unabhängig voneinander H, (C₁-C₆)-Alkyl, Heterocyclus;
- m: 1;
- R1: R8, (C₁-C₆)-Alkylen-R8, (C₂-C₆)-Alkenylen-R9, (SO₂)-R8 (SO₂)-(C₁-C₆)-Alkylen-R8, (SO₂)-(C₂-C₆)-Alkenylen-R9, (C=O)-R8, C=O)-(C₁-C₆)-Alkylen-R8, (C=O)NH-R8, (C=O)-(C₂-C₆)-Alkenylen-R9, (C=O)-NH- (C₁-C₆)-Alkylen-R8, (C=O)-NH- (C₂-C₆)-Alkenylen-R9, COO-R8, COO-(C₁-C₆)-Alkylen-R8, COO-(C₂-C₆)-Alkenylen-R9, Alkinylen-R9, (C₁-C₄-Alkyl)-Heterocyclus;
- R8, R9: unabhängig voneinander H, F, Cl, Br, I, OH, CF₃, Aryl, Heterocyclus, (C₃-C₈)-Cycloalkyl, wobei die Ringe oder Ringssysteme bis zu 3-fach substituiert sein können mit F, Cl, Br, I, OH, CF₃, NO₂ CN, OCF₃, O-(C₁-C₆)-Allcyl, (C₁-C₆)-Alkyl, NH₂, CON(R11)(R12), N(R13)(R14), SO₂-CH₃, COOH, COO-(C₁-C₆)-Alkyl, CONH₂;
- R2: NH₂,NO₂, N(R13)(R14), NH-SO₂-CH₃, NH-SO₂-R12, NR11-SO₂-R12, N(CO)R11, ein Stickstoffhaltiger Heterocyclus, wobei der Heterocyclus über ein Stickstoffatom gebunden ist, NHCONR11, N(C₁-C₆-Allcyl)N⁺(C₁-C₄-Alkyl)₃;
- R3: H
- R4, R5: unabhängig voneinander H, F, Cl, Br, OH, CF₃, OCF₃, O-(C₁-C₆)-Alkyl, (C₁-C₆)-Alkyl;
- R6: H;
sowie deren physiologisch verträglichen Salze.

Ganz besonders bevorzugt sind Verbindungen der Formel Ia,
worin bedeuten
- A: Aryl, wobei der Arylring substituiert sein kann mit F, Cl, Br, NO₂, CF₃, OCF₃, CN, (C₁-C₆)-Alkyl, Aryl, CON(R11)(R12), N(R13)(R14), OH, O-(C₁-C₆)-Alkyl, S-(C₁-C₆)-Alkyl, N(R15)CO(C₁-C₆)-Alkyl oder COO-(C₁-C₆)-Allcyl;
- R11, R12, R13, R14, R15: unabhängig voneinander H, (C₁-C₆)-Alkyl, Heterocyclus;
- m: 1;
- R1: (C₁-C₆)-Alkyl, (C₁-C₆)-Alkylen-R8;
- R8, R9: unabhängig voneinander F, Cl, Br, I, OH, CF₃;
- R2: NH₂, NO₂ CN, N(R13)(R14), NH-SO₂-CH₃, NH-SO₂-R12, NR11-SO₂-R12, N(CO)R11, ein Stickstoffhaltiger Heterocyclus, wobei der Heterocyclus über ein Stickstoffatom gebunden ist, NHCONR11, N(C₁-C₆-Allcyl)N⁺(C₁-C₄-Alkyl)₃;
- R3: H
- R4: F, Cl, Br, OH, CF₃, OCF₃, O-(C₁-C₆)-Alkyl, (C₁-C₆)-Alkyl;
- R5: H, F, Cl, Br, OH, CF₃, OCF₃, O-(C₁-C₆)-Alkyl, (C₁-C₆)-Alkyl;
- R6: H;
sowie deren physiologisch verträglichen Salze.

Können Reste oder Substituenten mehrfach in den Verbindungen der Formel I auftreten, wie zum Beispiel CON(R11)(R12), so können sie alle unabhängig voneinander die angegebenen Bedeutungen haben und gleich oder verschieden sein.

Die Erfindung bezieht sich auf Verbindungen der Formel I, in Form ihrer Racemate, enantiomerenangereicherten Mischungen und reinen Enantiomere sowie auf ihre Diastereomere und Mischungen davon.

Die Alkyl-, Alkenyl- und Alkinylreste in den Substituenten A, R1, R2, R3, R4, R5, R6, R8, R9, R10, R11, R12, R13, R14, R15 können sowohl geradkettig, verzweigt oder optional halogeniert sein.

Unter dem Begriff "Aryl" wird eine Phenyl oder Naphthylgruppe verstanden.

Unter Heterocyclus bzw. Heterocyclischer Rest werden Ringsysteme verstanden, die außer Kohlenstoff noch Heteroatome, wie zum Beispiel Stickstoff, Sauerstoff oder Schwefel enthalten. Ferner gehören auch Ringsysteme zu dieser Definition, worin der Heterocylus bzw. der Heterocyclische Rest mit Benzolkernen kondensiert ist.

Geeignete "Heterocyclische Ringe" bzw. "Heterocyclische Reste" sind Acridinyl, Azocinyl, Benzimidazolyl, Benzofuryl, Benzothienyl, Benzothiophenyl, Benzoxazolyl, Benzthiazolyl, Benztriazolyl, Benztetrazolyl, Benzisoxazolyl, Benzisothiazolyl, Benzimidazalinyl, Carbazolyl, 4aH-Carbazolyl, Carbolinyl, Chinazolinyl, Chinolinyl, 4H-Chinolizinyl, Chinoxalinyl, Chinuclidinyl, Chromanyl, Chromenyl, Cinnolinyl, Decahydrochinolinyl, 2H,6H-1,5,2-Dithiazinyl, Dihydrofuro[2,3-b]-Tetrahydrofuran, Furyl, Furazanyl, Imidazolidinyl, Imidazolinyl, Imidazolyl, 1H-Indazolyl, Indolinyl, Indolizinyl, Indolyl, 3H-Indolyl, Isobenzofuranyl, Isochromanyl, Isoindazolyl, Isoindolinyl, Isoindolyl, Isochinolinyl (Benzimidazolyl), Isothiazolyl, Isoxazolyl, Morpholinyl, Naphthyridinyl, Octahydroisochinolinyl, Oxadiazolyl, 1,2,3-Oxadiazolyl, 1,2,4-Oxadiazolyl, 1,2,5-Oxadiazolyl, 1,3,4-Oxadiazolyl, Oxazolidinyl, Oxazolyl, Oxazolidinyl, Pyrimidinyl, Phenanthridinyl, Phenanthrolinyl, Phenazinyl, Phenothiazinyl, Phenoxathiinyl, Phenoxazinyl, Phthalazinyl, Piperazinyl, Piperidinyl, Pteridinyl, Purynyl, Pyranyl, Pyrazinyl, Pyroazolidinyl, Pyrazolinyl, Pyrazolyl, Pyridazinyl, Pyridooxazole, Pyridoimidazole, Pyridothiazole, Pyridinyl, Pyridyl, Pyrimidinyl, Pyrrolidinyl, Pyrrolinyl, 2H-Pyrrolyl, Pyrrolyl, Tetrahydrofuranyl, Tetrahydroisochinolinyl, Tetrahydrochinolinyl, 6H-1,2,5-Thiadazinyl, Thiazolyl, 1,2,3-Thiadiazolyl, 1,2,4-Thiadiazolyl, 1,2,5-Thiadiazolyl, 1,3,4-Thiadiazolyl, Thienyl, Triazolyl, Tetrazolyl und Xanthenyl.

Pyridyl steht sowohl für 2-, 3- als auch 4-Pyridyl. Thienyl steht sowohl für 2- als auch 3-Thienyl. Furyl steht sowohl für 2- als auch 3-Furyl.

Umfasst sind weiterhin die entsprechenden N-Oxide dieser Verbindungen, also z.B. 1-Oxy-2-, 3- oder 4-pyridyl.

Umfasst sind weiterhin ein oder mehrfach benzoannelierte Derivate dieser Heterozyklen.

Die Heterocyclischen Ringe bzw. Heterocyclische Reste können ein oder mehrfach mit geeigneten Gruppen substituiert sein, wie z.B.: F, Cl, Br, I, CF₃, NO₂ N₃, CN, COOH, COO(C₁-C₆)Alkyl, CONH₂, CONH(C₁-C₆)Alkyl, CON[(C₁-C₆)Alkyl]₂, (C₁-C₆)-Alkyl, (C₂-C₆)-Alkenyl, (C₂-C₆)-Alkinyl, O-(C₁-C₆)-Alkyl, wobei in den Alkylresten ein, mehrere, oder alle Wasserstoff(e) durch Fluor ersetzt sein können;
PO₃H₂, SO₃H, SO₂-NH₂ SO₂NH(C₁-C₆)-Alkyl, SO₂N[(C₁-C₆)-Alkyl]₂ , S-(C₁-C₆)-Alkyl, S-(CH₂)ₙ-Phenyl, SO-(C₁-C₆)-Alkyl, SO-(CH₂)ₙ-Phenyl, SO₂-(C₁-C₆)-Alkyl, SO₂-(CH₂)ₙ-Phenyl, wobei n = 0-6 sein kann und der Phenylrest bis zu zweifach mit F, Cl, Br, OH, CF₃, NO₂, CN, OCF₃, O-(C₁-C₆)-Alkyl, (C₁-C₆)-Alkyl, NH₂ substituiert sein kann;
C(NH)(NH₂), NH₂, NH-(C₁-C₆)-Alkyl, N((C₁-C₆)-Alkyl)₂, NH(C₁-C₇)-Acyl, Phenyl, O-(CH₂)ₙ-Phenyl, wobei n = 0-6 sein kann, wobei der Phenylring ein bis 3-fach substituiert sein kann mit F, Cl, Br, I, OH, CF₃, NO₂, CN, OCF₃, O-(C₁-C₆)-Alkyl, (C₁-C₆)-Alkyl, NH₂, NH(C₁-C₆)-Alkyl, N((C₁-C₆)-Alkyl)₂, SO₂-CH₃, COOH, COO-(C₁-C₆)-Alkyl, CONH₂.

Pharmazeutisch verträgliche Salze sind aufgrund ihrer höheren Wasserlöslichkeit gegenüber den Ausgangs- bzw. Basisverbindungen besonders geeignet für medizinische Anwendungen. Diese Salze müssen ein pharmazeutisch verträgliches Anion oder Kation aufweisen. Geeignete pharmazeutisch verträgliche Säureadditionssalze der erfindungsgemäßen Verbindungen sind Salze anorganischer Säuren, wie Salzsäure, Bromwasserstoff-, Phosphor-, Metaphosphor-, Salpeter-, Sulfon- und Schwefelsäure sowie organischer Säuren, wie z.B. Essigsäure, Benzolsulfon-, Benzoe-, Zitronen-, Ethansulfon-, Fumar-, Glucon-, Glykol-, Isethion-, Milch-, Lactobion-, Malein-, Äpfel-, Methansulfon-, Bernstein-, p-Toluolsulfon-, Wein- und Trifluoressigsäure. Für medizinische Zwecke wird-in besonders bevorzugter Weise das Chlorsalz verwendet. Geeignete pharmazeutisch verträgliche basische Salze sind Ammoniumsalze, Alkalimetallsalze (wie Natrium- und Kaliumsalze) und Erdalkalisalze (wie Magnesium- und Calciumsalze).

Salze mit einem nicht pharmazeutisch verträglichen Anion gehören ebenfalls in den Rahmen der Erfindung als nützliche Zwischenprodukte für die Herstellung oder Reinigung pharmazeutisch verträglicher Salze und/oder für die Verwendung in nicht-therapeutischen, zum Beispiel in-vitro-Anwendungen.

Der hier verwendete Begriff "physiologisch funktionelles Derivat" bezeichnet jedes physiologisch verträgliche Derivat einer erfindungsgemäßen Verbindung der Formel I, z.B. einen Ester, der bei Verabreichung an einen Säuger, wie z.B. den Menschen, in der Lage ist, (direkt oder indirekt) eine Verbindung der Formel I oder einen aktiven Metaboliten hiervon zu bilden.

Zu den physiologisch funktionellen Derivaten zählen auch Prodrugs der erfindungsgemäßen Verbindungen. Solche Prodrugs können in vivo zu einer erfindungsgemäßen Verbindung metabolisiert werden. Diese Prodrugs können selbst wirksam sein oder nicht.

Die erfindungsgemäßen Verbindungen können auch in verschiedenen polymorphen Formen vorliegen, z.B. als amorphe und kristalline polymorphe Formen. Alle polymorphen Formen der erfindungsgemäßen Verbindungen gehören in den Rahmen der Erfindung und sind ein weiterer Aspekt der Erfindung.

Nachfolgend beziehen sich alle Verweise auf "Verbindung(en) gemäß Formel (I)" auf Verbindung(en) der Formel (I) wie vorstehend beschrieben, sowie ihre Salze, Solvate und physiologisch funktionellen Derivate wie hierin beschrieben.

Die Menge einer Verbindung gemäß Formel (I), die erforderlich ist, um den gewünschten biologischen Effekt zu erreichen, ist abhängig von einer Reihe von Faktoren, z.B. der gewählten spezifischen Verbindung, der beabsichtigten Verwendung, der Art der Verabreichung und dem klinischen Zustand des Patienten. Im allgemeinen liegt die Tagesdosis im Bereich von 0,3 mg bis 100 mg (typischerweise von 3 mg bis 50 mg) pro Tag pro Kilogramm Körpergewicht, z.B. 3-10 mg/kg/Tag. Eine intravenöse Dosis kann z.B. im Bereich von 0,3 mg bis 1,0 mg/kg liegen, die geeigneterweise als Infusion von 10 ng bis 100 ng pro Kilogramm pro Minute verabreicht werden kann. Geeignete Infusionslösungen für diese Zwecke können z.B. von 0,1 ng bis 10 mg, typischerweise von 1 ng bis 10 mg pro Milliliter, enthalten. Einzeldosen können z.B. von 1 mg bis 10 g des Wirkstoffs enthalten. Somit können Ampullen für Injektionen beispielsweise von 1 mg bis 100 mg, und oral verabreichbare Einzeldosisformulierungen, wie zum Beispiel Tabletten oder Kapseln, können beispielsweise von 1,0 bis 1000 mg, typischerweise von 10 bis 600 mg enthalten. Im Falle pharmazeutisch verträglicher Salze beziehen sich die vorgenannten Gewichtsangaben auf das Gewicht der dem Salz zugrunde liegenden freien Verbindung. Zur Prophylaxe oder Therapie der oben genannten Zustände können die Verbindungen gemäß Formel (I) selbst als Verbindung verwendet werden, vorzugsweise liegen sie jedoch mit einem verträglichen Träger in Form einer pharmazeutischen Zusammensetzung vor. Der Träger muß natürlich verträglich sein, in dem Sinne, daß er mit den anderen Bestandteilen der Zusammensetzung kompatibel ist und nicht gesundheitsschädlich für den Patienten ist. Der Träger kann ein Feststoff oder eine Flüssigkeit oder beides sein und wird vorzugsweise mit der Verbindung als Einzeldosis formuliert, beispielsweise als Tablette, die von 0,05% bis 95 Gew.-% des Wirkstoffs enthalten kann. Weitere pharmazeutisch aktive Substanzen können ebenfalls vorhanden sein, einschließlich weiterer Verbindungen gemäß Formel (I). Die erfindungsgemäßen pharmazeutischen Zusammensetzungen können nach einer der bekannten pharmazeutischen Methoden hergestellt werden, die im wesentlichen darin bestehen, daß die Bestandteile mit pharmakologisch verträglichen Träger- und/oder Hilfsstoffen gemischt werden.

Erfindungsgemäße pharmazeutische Zusammensetzungen sind solche, die für orale, rektale, topische, perorale (z.B. sublinguale) und parenterale (z.B. subkutane, intramuskuläre, intradermale oder intravenöse) Verabreichung geeignet sind, wenngleich die geeignetste Verabreichungsweise in jedem Einzelfall von der Art und Schwere des zu behandelnden Zustandes und von der Art der jeweils verwendeten Verbindung gemäß Formel (I) abhängig ist. Auch dragierte Formulierungen und dragierte Retardformulierungen gehören in den Rahmen der Erfindung. Bevorzugt sind säure- und magensaftresistente Formulierungen. Geeignete magensaftresistente Beschichtungen umfassen Celluloseacetatphthalat, Polyvinylacetatphthalat, Hydroxypropylmethylcellulosephthalat und anionische Polymere von Methacrylsäure und Methacrylsäuremethylester.

Geeignete pharmazeutische Verbindungen für die orale Verabreichung können in separaten Einheiten vorliegen, wie zum Beispiel Kapseln, Oblatenkapseln, Lutschtabletten oder Tabletten, die jeweils eine bestimmte Menge der Verbindung gemäß Formel (I) enthalten; als Pulver oder Granulate; als Lösung oder Suspension in einer wäßrigen oder nicht-wäßrigen Flüssigkeit; oder als eine Öl-in-Wasser- oder Wasser-in Öl-Emulsion. Diese Zusammensetzungen können, wie bereits erwähnt, nach jeder geeigneten pharmazeutischen Methode zubereitet werden, die einen Schritt umfaßt, bei dem der Wirkstoff und der Träger (der aus einem oder mehreren zusätzlichen Bestandteilen bestehen kann) in Kontakt gebracht werden. Im allgemeinen werden die Zusammensetzungen durch gleichmäßiges und homogenes Vermischen des Wirkstoffs mit einem flüssigen und/oder feinverteilten festen Träger hergestellt, wonach das Produkt, falls erforderlich, geformt wird. So kann beispielsweise eine Tablette hergestellt werden, indem ein Pulver oder Granulat der Verbindung verpreßt oder geformt wird, gegebenenfalls mit einem oder mehreren zusätzlichen Bestandteilen. Gepreßte Tabletten können durch Tablettieren der Verbindung in frei fließender Form, wie beispielsweise einem Pulver oder Granulat, gegebenenfalls gemischt mit einem Bindemittel, Gleitmittel, inertem Verdünner und/oder einem (mehreren) oberflächenaktiven/dispergierenden Mittel in einer geeigneten Maschine hergestellt werden. Geformte Tabletten können durch Formen der pulverförmigen, mit einem inerten flüssigen Verdünnungsmittel befeuchteten Verbindung in einer geeigneten Maschine hergestellt werden.

Pharmazeutische Zusammensetzungen, die für eine perorale (sublinguale) Verabreichung geeignet sind, umfassen Lutschtabletten, die eine Verbindung gemäß Formel (I) mit einem Geschmacksstoff enthalten, üblicherweise Saccharose und Gummi arabicum oder Tragant, und Pastillen, die die Verbindung in einer inerten Basis wie Gelatine und Glycerin oder Saccharose und Gummi arabicum umfassen.

Geeignete pharmazeutische Zusammensetzungen für die parenterale Verabreichung umfassen vorzugsweise sterile wäßrige Zubereitungen einer Verbindung gemäß Formel (I), die vorzugsweise isotonisch mit dem Blut des vorgesehenen Empfängers sind. Diese Zubereitungen werden vorzugsweise intravenös verabreicht, wenngleich die Verabreichung auch subkutan, intramuskulär oder intradermal als Injektion erfolgen kann. Diese Zubereitungen können vorzugsweise hergestellt werden, indem die Verbindung mit Wasser gemischt wird und die erhaltene Lösung steril und mit dem Blut isotonisch gemacht wird. Injizierbare erfindungsgemäße Zusammensetzungen enthalten im allgemeinen von 0,1 bis 5 Gew.-% der aktiven Verbindung.

Geeignete pharmazeutische Zusammensetzungen für die rektale Verabreichung liegen vorzugsweise als Einzeldosis-Zäpfchen vor. Diese können hergestellt werden, indem man eine Verbindung gemäß Formel (I) mit einem oder mehreren herkömmLichen festen Trägem, beispielsweise Kakaobutter, mischt und das entstehende Gemisch in Form bringt.

Geeignete pharmazeutische Zusammensetzungen für die topische Anwendung auf der Haut liegen vorzugsweise als Salbe, Creme, Lotion, Paste, Spray, Aerosol oder Öl vor. Als Träger können Vaseline, Lanolin, Polyethylenglycole, Alkohole und Kombinationen von zwei oder mehreren dieser Substanzen verwendet werden. Der Wirkstoff ist im allgemeinen in einer Konzentration von 0,1 bis 15 Gew.-% der Zusammensetzung vorhanden, beispielsweise von 0,5 bis 2%.

Auch eine transdermale Verabreichung ist möglich. Geeignete pharmazeutische Zusammensetzungen für transdermale Anwendungen können als einzelne Pflaster vorliegen, die für einen langzeitigen engen Kontakt mit der Epidermis des Patienten geeignet sind. Solche Pflaster enthalten geeigneterweise den Wirkstoff in einer gegebenenfalls gepufferten wäßrigen Lösung, gelöst und/oder dispergiert in einem Haftmittel oder dispergiert in einem Polymer. Eine geeignete Wirkstoff-Konzentration beträgt ca. 1% bis 35%, vorzugsweise ca. 3% bis 15%. Als eine besondere Möglichkeit kann der Wirkstoff, wie beispielsweise in Pharmaceutical Research, 2(6): 318 (1986) beschrieben, durch Elektrotransport oder Iontophorese freigesetzt werden.

Die Verbindungen der Formel I zeichnen sich durch günstige Wirkungen auf den Fettstoffwechsel aus, insbesondere sind sie zur Gewichtsreduktion und nach erfolgter Gewichtsreduktion zum Erhalt eines reduzierten Gewichtes bei Säugetieren und als Anorektika geeignet. Die Verbindungen zeichnen sich sich durch ihre geringe Toxizität und ihre geringen Nebenwirkungen aus.

Die Verbindungen können allein oder in Kombination mit weiteren gewichtsreduzierenden oder anorektischen Wirkstoffen eingesetzt werden. Solche weiteren anorektischen Wirkstoffe werden z.B. in der Roten Liste, Kapitel 01 unter Abmageruncsmittel/Appetitzügler genannt und können auch solche Wirkstoffe beinhalten, die den Energieumsatz des Organismus erhöhen und damit zu einer Gewichtsreduktion führen oder auch solche, welche den allgemeinen Metabolismus des Organismus so beeinflussen, dass eine erhöhte Kalorienzufuhr nicht zu einer Vergrößerung der Fettdepots und eine normale Kalorienzufuhr zu einer Verringerung der Fettdepots des Organismus führt. Die Verbindungen eignen sich zur Prophylaxe sowie insbesondere zur Behandlung von Übergewicht oder Adipositas. Die Verbindungen eignen sich weiterhin zur Prophylaxe sowie insbesondere zur Behandlung von Typ II Diabetes, der Arteriosklerose sowie zur Normalisierung des Lipidstoffwechsels und zur Behandlung des Bluthochdrucks. Die Verbindungen wirken als Melanocortin-Rezeptor Agonisten und eignen sich auch zur Behandlung von Störungen des Empfindens und anderer psychiatrischen Indikationen, wie zum Beispiel Depressionen, Angstzuständen, Angstneurosen, Schizophrenie sowie zur Behandlung von Störungen assoziiert mit dem zirkadianen Rhythmus und zur Behandlung von Drogenmissbrauch.

Weiterhin eignen sie sich zur Behandlung von Krebs, Arthritis, Schlafstörungen, Schlaf Apnoe, weiblicher und männlicher Sexualstörungen, Entzündungen, Akne, Pigmentierung der Haut, des metabolischen Syndroms, Störungen des Steroidstoffwechsels, Hautkrankheiten, Psoriasis, Mykosen, neurodegenerativer Krankheiten und Alzheimer-Krankheit.

Bei einem weiteren Aspekt der Erfindung können die Verbindungen der Formel I in Kombination mit einer oder mehreren weiteren pharmakologisch wirksamen Substanzen verabreicht werden, die beispielsweise ausgewählt sind aus Antidiabetika, Antiadiposita, blutdrucksenkenden Wirkstoffen, Lipidsenkern und Wirkstoffen zur Behandlung und/oder Prävention von Komplikationen, die von Diabetes verursacht werden oder mit Diabetes assoziiert sind.

Geeignete Antidiabetika umfassen Insuline, Amylin, GLP-1- und GLP-2-Derivate wie z.B. diejenigen die in WO 98/08871 von Novo Nordisk A/S offenbart wurden, sowie oral wirksame hypoglykämische Wirkstoffe.

Die oral wirksamen hypoglykämischen Wirkstoffe umfassen vorzugsweise Sulphonylhamstoffe, Biguanidine, Meglitinide, Oxadiazolidindione, Thiazolidindione, Glukosidase-Inhibitoren, Glukagon-Rezeptor-Antagonisten, GLP-1-Agonisten, Kaliumkanalöffner wie z.B. diejenigen, die in WO 97/26265 und WO 99/03861 von Novo Nordisk A/S offenbart wurden, Insulin-Sensitizer, Aktivatoren der Insulin Rezeptor Kinase, Inhibitoren von Leberenzymen, die an der Stimulation der Glukoneogenese und/oder Glykogenolyse beteiligt sind, z.B. Inhibitoren der Glycogenphosphorylase, Modulatoren der Glukoseaufnahme und Glukoseausscheidung, den Fettstoffwechsel verändernde Verbindungen wie antihyperlipidämische Wirkstoffe und antilipidämische Wirkstoffe, z.B. HMGCoA-Reduktase-Inhibitoren, Inhibitoren des Cholesteroltransports/der Cholesterolaufnahme, Inhibitoren der Gallensäurerückresorption oder Inhibitoren des mikrosomalen Triglycerid-Transfer Proteins (MTP), Verbindungen, die die Nahrungsmitteleinnahme verringern, PPAR-und RXR-Agonisten und Wirkstoffe, die auf den ATP-abhängigen Kaliumkanal der Betazellen wirken.

Bei einer Ausführungsform der Erfindung werden die vorliegenden Verbindungen in Kombination mit Insulin verabreicht.

Bei einer weiteren Ausführungsform werden die vorliegenden Verbindungen in Kombination mit einem Sulphonylharnstoff wie z.B. Tolbutamid, Glibenclamid, Glimepirid, Glipizid, Gliquidon, Glisoxepid, Glibomurid oder Gliclazid verabreicht.

Bei einer anderen Ausführungsform werden die vorliegenden Verbindungen in Kombination mit einem Biguanid wie z.B. Metformin verabreicht.

Bei wieder einer anderen Ausführungsform werden die vorliegenden Verbindungen in Kombination mit einem Meglitinid wie z.B. Repaglinid verabreicht.

Bei noch einer weiteren Ausführungsform werden die vorliegenden Verbindungen in Kombination mit einem Thiazolidindion wie z.B. Troglitazon, Ciglitazon, Pioglitazon, Rosiglitazon oder den in WO 97/41097 von Dr. Reddy's Research Foundation offenbarten Verbindungen, insbesondere 5-[[4-[(3,4-Dihydro-3-methyl-4-oxo-2-chinazolinylmethoxy]-phenyl]methyl]-2,4-thiazolidindion, verabreicht.

Bei einer weiteren Ausführungsform werden die vorliegenden Verbindungen in Kombination mit einem α-Glukosidase-Inhibitor wie z.B. Miglitol oder Acarbose verabreicht.

Bei einer anderen Ausführungsform werden die vorliegenden Verbindungen in Kombination mit einem Wirkstoff verabreicht, der auf den ATP-abhängigen Kaliumkanal der Betazellen wirkt, wie z.B. Tolbutamid, Glibenclamid, Glimepirid, Glipizid, Gliclazid oder Repaglinid.

Bei noch einer anderen Ausführungsform werden die vorliegenden Verbindungen in Kombination mit einem antihyperlidämischen Wirkstoff oder einem antilipidämischen Wirkstoff wie z.B. Cholestyramin, Colestipol, Clofibrat, Fenofibrat, Gemfibrozil, Lovastatin, Pravastatin, Simvastatin, Atorvastatin, Cerivastatin, Fluvastatin, Probucol, Ezetimibe oder Dextrothyroxin verabreicht.

Bei einer weiteren Ausführungsform werden die vorliegenden Verbindungen in Kombination mit mehr als einer der vorstehend genannten Verbindungen, z.B. in Kombination mit einem Sulphonylharnstoff und Metformin, einem Sulphonylharnstoff und Acarbose, Repaglinid und Metformin, Insulin und einem Sulphonylharnstoff, Insulin und Metformin, Insulin und Troglitazon, Insulin und Lovastatin, etc. verabreicht.

Weiterhin können die erfindungsgemäßen Verbindungen in Kombination mit einem oder mehreren Antiadiposita oder appetitregulierenden Wirkstoffen verabreicht werden.

Solche Wirkstoffe können ausgewählt werden aus der Gruppe bestehend aus CART-Agonisten, NPY-Antagonisten, MCH-Antagonisten, Orexin-Antagonisten, H3-Antagonisten, TNF-Agonisten, CRF-Agonisten, CRF BP-Antagonisten, Urocortin-Agonisten, *β*3-Agonisten, MSH (Melanocyt-stimulierendes Hormon)-Agonisten, CCK-Agonisten, Serotonin-Wiederaufnahme-Inhibitoren, gemischte Serotonin- und Noradrenalin-Wiederaufnahme-Inhibitoren, 5HT-Modulatoren, MAO-Hemmer, Bombesin-Agonisten, Galanin-Antagonisten, Wachstumshormon, Wachstumshormon freisetzende Verbindungen, TRH-Agonisten, Modulatoren der Entkopplungsproteine 2 oder 3, Leptin-Agonisten, Dopamin-Agonisten (Bromcriptin, Doprexin), Lipase/Amylase-Inhibitoren, Antagonisten des Cannabinoid Rezeptors 1, Modulatoren des die Acylierung stimulierende Protein (ASP), PPAR-Modulatoren, RXR-Modulatoren, hCNTF-Mimetika oder TR-*β*-Agonisten.

Bei einer Ausführungsform der Erfindung ist das Antiadipositum Leptin oder modifiziertes Leptin.

Bei einer anderen Ausführungsform ist das Antiadipositum Dexamphetamin oder Amphetamin. Bei einer anderen Ausführungsform ist das Antiadipositum Fenfluramin oder Dexfenfluramin; Bei noch einer anderen Ausführungsform ist das Antiadipositum Sibutramin oder die mono- und bisdemethylierten Wirkmetabolite von Sibutramin.

Bei einer weiteren Ausführungsform ist das Antiadipositum Orlistat.

Bei einer anderen Ausführungsform ist das Antiadipositum Mazindol, Diethylpropion oder Phentermin.

Weiterhin können die vorliegenden Verbindungen in Kombination mit einem oder mehreren antihypertensiven Wirkstoffen verabreicht werden. Beispiele für antihypertensive Wirkstoffe sind Betablocker wie Alprenolol, Atenol, Timolol, Pindolol, Propanolol und Metoprolol, ACE (Angiotensin Converting Enzym)-Hemmer wie z.B. Benazepril, Captopril, Enalapril, Fosinopril, Lisinopril, Quinapril und Rampril, Calciumkanal-Blocker wie Nifedipin, Felodipin, Nicardipin, Isradipin, Nimodipin, Diltiazem und Verapamil, sowie Alphablocker wie Doxazosin, Urapidil, Prazosin und Terazosin. Weiterhin kann verwiesen werden auf Remington: The Science and Practice of Pharmacy, 19. Auflage, Gennaro, Hrsg., Mack Publishing Co., Easton, PA, 1995.

Es versteht sich, dass jede geeignete Kombination der erfindungsgemäßen Verbindungen mit einer oder mehreren der vorstehend genannten Verbindungen und wahlweise einer oder mehreren weiteren pharmakologisch wirksamen Substanzen als unter den Schutzbereich der vorliegenden Erfindung fallend angesehen wird.

Die Wirksamkeit der Verbindungen wurde wie folgt getestet:

### Biologisches Prüfmodell:

Die Prüfung der anorektischen Wirkung erfolgte an weiblichen NMRI Mäusen. Nach 24stündigem Futterentzug wurde über eine Schlundsonde das Testpräparat verabreicht. In Einzelhaltung und bei freiem Zugang zu Trinkwasser wurde den Tieren 30 Minuten nach Präparatgabe Kondensmilch angeboten. Der Kondensmilchverbrauch wurde halbstündlich 7 Stunden lang bestimmt und das Allgemeinbefinden der Tiere beobachtet. Der gemessene Milchverbrauch wurde mit den Vehikel-behandelten Kontrolltieren verglichen.

**Tabelle 1: Anorektische Wirkung, gemessen als Reduktion des kumulierten Milchkonsums behandelter im Vergleich zu Kontrolltieren.**

| Beispiel | Orale Dosis [mg/kg] | Anzahl der Tiere / Kumulierter Milchkonsum der behandelten Tiere N / [mL] | Anzahl der Tiere / Kumulierter Milchkonsum der Kontrolltiere N / [mL] | Reduktion des kumulierten Milchkonsums in % der Kontrolle |
|---|---|---|---|---|
| 5 | 50 | 5 / 2,4 | 5 / 3,40 | 31 |
| 9 | 50 | 5 / 2,06 | 5 / 3,86 | 47 |

Aus der Tabelle ist anzulesen, dass die Verbindungen der Formel I eine gute anorektische Wirkung zeigen und damit sehr gut als Antiadipositum geeignet sind.

Die nachfolgend aufgeführten Beispiele und Herstellungsmethoden dienen zur Erläuterung der Erfindung, ohne diese jedoch einzuschränken.

### Allgemeine Verfahren

Die bei der Synthese verwendeten Ausgangsmaterialien wurden von Chemikalienlieferanten wie Aldrich, Acros, Sigma, Fluka, Nova Biochem, Advanced Chemtech, Bachem, Lancaster und anderen Firmen bezogen.

Bei der Synthese wurden die funktionellen Gruppen der verwendeten Aminosäurederivate zur Vermeidung von Nebenreaktionen bei den Kopplungsschritten durch Schutzgruppen geschützt. Beispiele für geeignete Schutzgruppen und ihre Verwendung sind in The Peptides, supra, 1981 und in Bd. 9, Udenfriend und Meienhofer (Hrsg.) 1987 beschrieben (wird hier durch Bezugnahme aufgenommen).

Zur Herstellung der erfindungsgemäßen Verbindungen kamen allgemeine Methoden der Festphasensynthese zur Anwendung. Solche Methoden werden zum Beispiel von Steward und Young in Solid Phase Peptide Synthesis (Freeman & Co., San Francisco 1969) beschrieben (wird hier durch Bezugnahme aufgenommen).

Wenn nicht anders angegeben, wurden die Verbindungen mit TentaGel HL12019 Resin (Rapp Polymere, Tübingen) synthetisiert. Dieses handelsübliche Polymer enthält einen Bromacetal-Linker. Diese Art der Kopplung kann durch das von Vojkovsky, T. et al., J. Org. Chem. 1998, 63, 3162-3163, und Patek, M., Vortrag bei Combinatorial Chemistry 2000, London, 11.-14. 7. 2000 (wird hier durch Bezugnahme aufgenommen) beschriebene Verfahren in alle Arten von Hydroxy-TentaGel eingebaut werden.

Im ersten Syntheseschritt (allgemeines Syntheseschema, siehe Schema 1) wurde Amin in DMSO zum Austausch von Brom in der Bromacetalbindung bei einer hohen Temperatur verwendet. An das dabei entstehende sekundäre Amin auf dem Polymer wurde Fmocgeschützte Aminosäure gekoppelt. Die Kopplung erfolgte mittels DIC/HOAt oder HATU/DIEA, gewöhnlich in DMF. Die Kopplung wurde 16 Stunden lang bei Raumtemperatur (RT) oder 4 - 5 Stunden lang bei 55°C durchgeführt. Die Aufhebung des Schutzes der Fmoc-Gruppe erfolgte mit 50 % Piperidin in DMF (5 + 15 Minuten). Zur Bestimmung der Substitution kann die Menge freigesetztes Fmoc aus der Absorbanz der Lösung bei 302 nm nach Aufhebung des Schutzes, dem Volumen der Waschflüssigkeit und dem Gewicht des bei der Synthese eingesetzten Polymers entsprechend der Beschreibung in Krchnak, V. et al., Collect. Czech. Chem. Commun. 53 (1988) 2542 (wird hier durch Bezugnahme aufgenommen) ermittelt werden.

Die freie Aminogruppe der an die Festphase gebundenen Struktur wurde dann mit Fmoc-betaalanin (oder Fmoc-alpha-aminosäure oder substituierter Beta-Aminosäure) gekoppelt. Die Kopplung erfolgte mit N,N'-Diisopropylcarbodiimid (DIC) in Anwesenheit von HOBt, gewöhnlich in DMF. Die Vollständigkeit der Kopplung wurde durch den Ninhydrintest überwacht.

Die Aufhebung des Schutzes der Fmoc-Gruppe wurde 5 + 15 Minuten lang mit 50 % Piperidin in DMF durchgeführt. Die Menge freigesetztes Fmoc wurde aus der Absorbanz der Lösung bei 302 nm nach Aufhebung des Schutzes, dem Volumen der Waschflüssigkeit und dem Gewicht des bei der Synthese eingesetzten Polymers ermittelt.

Die freie Aminogruppe der an die Festphase gebundenen Struktur wurde dann mit bis zu 2 Äquivalenten eines geeigneten Sulfonylchlorid/DIEA in DCM oder Acetonitril sulfonyliert.

Die Vollständigkeit der Sulfonylierung wurde durch den Ninhydrintest überwacht.

Nach Abschluß des Zusammenbaus des Vorläufers der linearen Verbindung auf dem Polymer wurde die Feststoffphase nacheinander mit DMF und DCM oder THF gewaschen und im Vakuum getrocknet.

Die zyklative Abspaltung der angestrebten Verbindung erfolgte mit Ameisensäure 18 - 24 Stunden lang bei Raumtemperatur, 6 Stunden lang bei 50°C oder durch Kombination der beiden Bedingungen. Das Polymer wurde abfiltriert und mit DCM oder Ameisensäure gewaschen. Die Waschflüssigkeit wurde in die Ameisensäure-Lösung eingebracht. Die Lösung wurde abgedampft. Der Rückstand wurde in einem Gemisch aus Wasser und Acetonitril gelöst und gefriergetrocknet.

Die getrocknete Verbindung wurde durch HPLC mit einem geeigneten Gradienten von 0,1 % TFA in Wasser und Acetonitril (ACN) gereinigt. Nach dem Auffangen des Peaks, der das angestrebte synthetische Produkt enthält, wurde die Lösung der Verbindung gefriergetrocknet. Zur Bestätigung, daß die richtige Verbindung synthetisiert worden war, wurde die Verbindung einer qualitativen Bestimmung mit Elektrospray-Massenspektrum (LC/MS) und/oder einer NMR-Analyse unterzogen.

Zur Analyse mittels HPLC wurde eine Probe der Verbindung mit dem HPLC-System der Firma Beckman (bestehend aus dem Lösungsmittelzuführsystem 126, dem programmierbaren Detektormodul 166 und dem Autosampler 507e und gesteuert mit Datenstation mit Gold Nouveau-Sofware) mit einer YMC ODS-AM 4,6 x 250 mm-Säule (S-5 (5 µm), YMC, Inc. Wilmington, NC, USA) bei 230 nm analysiert. Bei dieser Einstellung wurde eine Durchlaufgeschwindigkeit von 1mL/min und ein Gradient aus Wasser/0,1 % TFA-Puffer und ACN (HPL-Qualität) als Elutionsmittel verwendet.

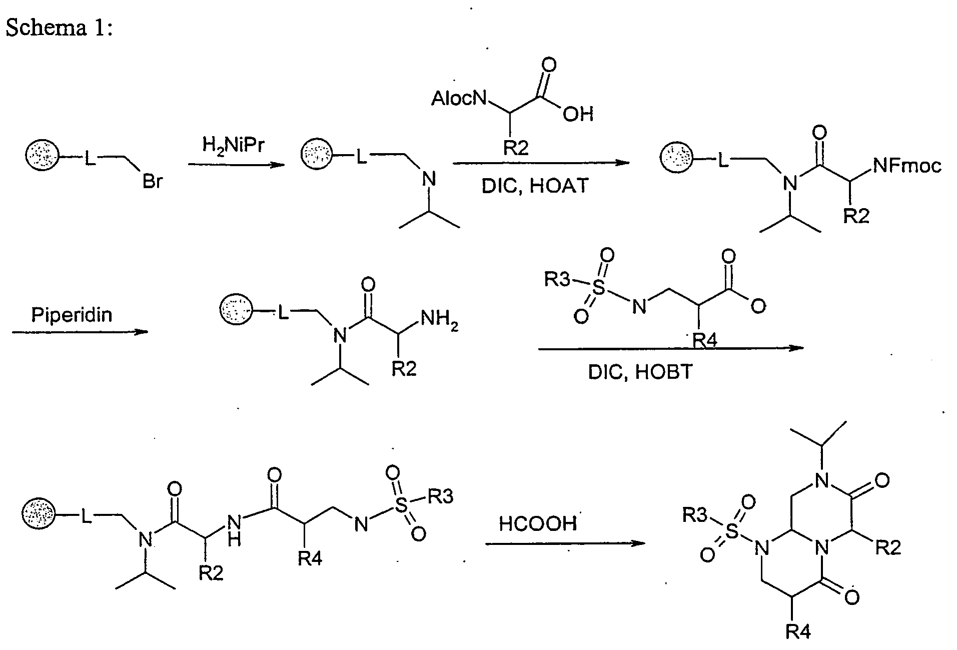

Die Verbindungen können in Analogie zur beschriebenen Synthese am Harz auch in Lösung durchgeführt werden. (Schema 2). Anstatt des funktionalisierten Harzes wird in der ersten Stufe 2-Brom-1,1-diethoxy-ethan mit einem primären Amin zur Reaktion gebracht.

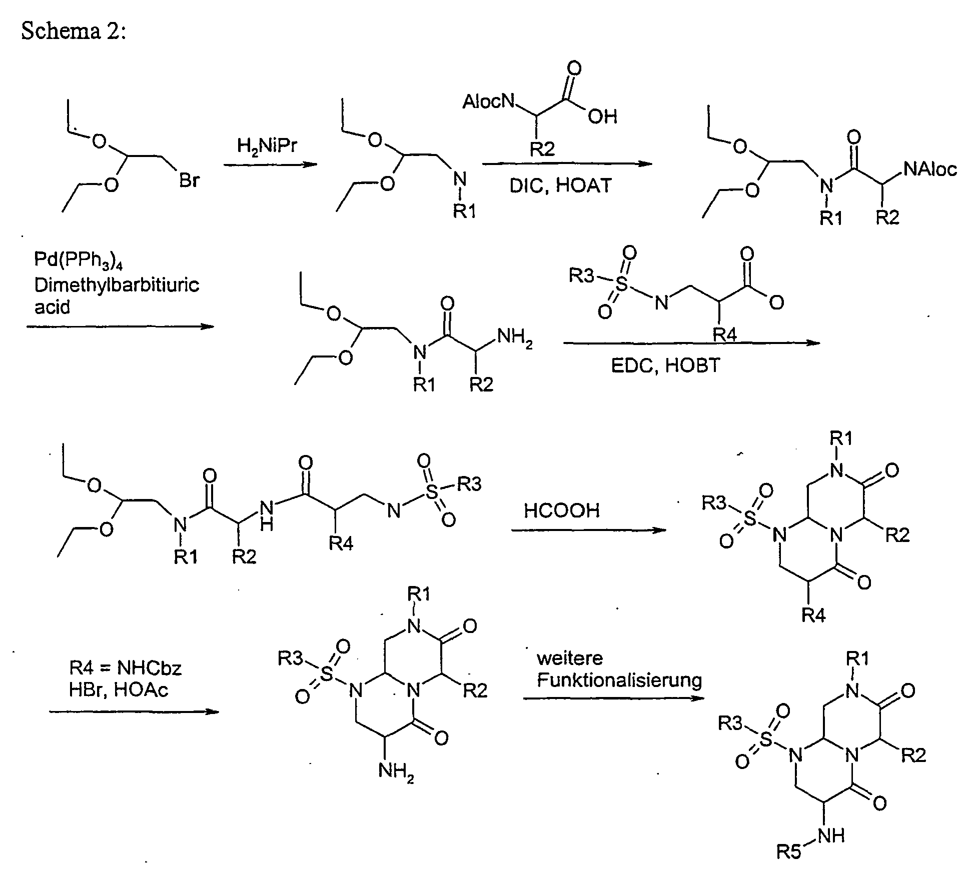

Das erhaltene Produkt wird mit der Aminosäure analog zur Festphasensynthese umgesetzt. Als Aminoschutzgruppe der Aminosäure kann anstatt der FMOC die Allyloxycarbonylschutzgruppe (Aloc) verwendet werden, die nach literaturbekannten Methoden eingeführt (Aloc-Cl, Triethylamin) und abgespalten (Pd(PPh₃)₄, Dimethylbarbitursäure)wird.

Die Aminocarbonsäure mit dem Rest R4 wird in Anwesenheit von Triethylamin mit dem Sulfonsäurechlorid zur Reaktion gebracht. Die freie Carbonsäure wird nach der Carbodiimidmethode (EDC, HOBt) oder unter Verwendung von Uroniumsalzen (HATU, HOAt) mit dem freien Amin, das durch Abspaltung der Alocgruppe erhalten wurde, gekoppelt.

Die Cyclisierung verläuft unter sauren Bedingungen und die Abspaltung der Benzyloxycarbonyl (Cbz)-gruppe wird mittels HBr in Eisessig vorgenommen. Anschließende Funktionalisierung verläuft analog, wie oben beschrieben.

Zur Reinigung des Produkts wurde eine Probe der gefriergetrockneten Rohsubstanz in einem Gemisch aus 0,1 %iger wäßriger TFA mit 10 - 50 % Acetonitril oder in Essigsäure gelöst. Die Lösung der Verbindung wurde gewöhnlich durch eine Spritze filtriert, die mit einem 0,45-µm-Filter ACRODISC 13 CR PTFE (Gelman Sciences; Ann Arbor, MI, USA) verbunden war. Ein entsprechendes Volumen der filtrierten Lösung der Verbindung wurde in eine halbpräparative C 18-Säule (YMC ODS-AM, S-5 (5 µm), 20 x 150 mm, YMC, Inc., Wilmington, NC, USA) eingespritzt. Die Durchlaufgeschwindigkeit eines Gradienten aus Wasser/0,1 % TFA-Puffer und ACN (HPL-Qualität) als Elutionsmittel wurde mittels des SYSTEM GOLD HPLC der Firma Beckman (System Gold, programmierbares Lösungsmittehnodul 126 und programmierbares Detektormodul 166, gesteuert mit SYSTEM GOLD-Software) aufrechterhalten. Die Elution der Verbindung wurde mittels UV-Detektion bei 230 oder 280 nm überwacht. Nach Identifizierung des Peaks der zu synthetisierenden Verbindung mit LC/MS wurde die Verbindung aufgefangen, gefriergetrocknet und einer biologischen Prüfung unterzogen.

Nach der Reinigung wurden Verbindungen mit basischen Gruppen als Trifluoracetate gewonnen. Hydrochloride dieser Verbindungen lassen sich durch Behandlung des Trifluoracetats der Verbindung mit einem Überschuß an HCl/Dioxan leicht darstellen. Nach dem Abdampfen der Lösungsmittel wurde das Hydrochlorid der Verbindung mit Diethylether ausgefällt und durch Filtrieren isoliert.

LC/MS erfolgte mit PE Sciex API 150EX und Sciex MassChrom-Software, ausgestattet mit einem Liquid Handler Gilson 215, zwei Flüssigkeitsmodulen Shimadzu LC-10AD, einem Detektor Shimadzu SPD-10A, einer Säule Keystone Betasil C-18 (2 x 30 mm, 3 µm, Durchlaufgeschwindigkeit des Acetonitril-/Wasser-/0,1 %TFA-Gradienten 0,7 mL/min) im ES+-Modus.

Bei der NMR-Analyse wurden die Proben in DMSO-d₆ (Aldrich) mit einem Avance DPX 300 der Firma Bruder gemessen.

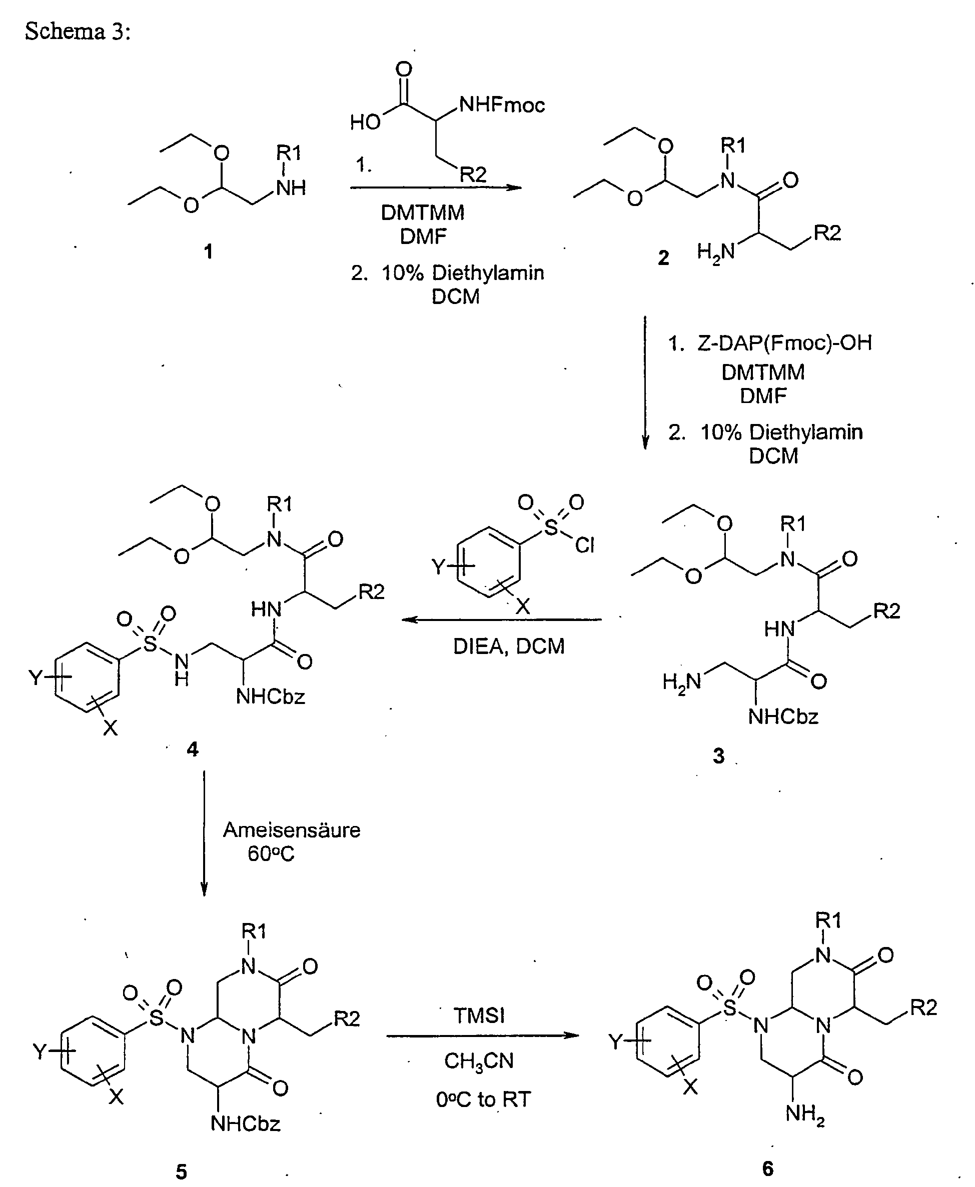

Die in Schema 3 gezeigte Synthese wurde in Analogie zu der anderen Lösungssynthese durchgeführt. Dabei wurden die Amidknüpfungen jeweils mit DMTMM als Kupplungsreagenz durchgeführt. Außerdem wurde wie in der Festphasenchemie Fmoc als Schutzgruppe eingesetzt, die mit Diethylamin wieder abgespalten wurde. Das Sulfonamid wurde nicht bereits bei der zweiten Amidknüpfung mit eingeführt, sondern nach dieser unter Verwendung von Diethylamin als Base gebildet. Die Abspaltung der Cbz-Gruppe erfolgte mit TMSI in Acetonitril. Alle weiteren Funktionalisierungen wurden analog wie oben beschrieben durchgeführt.

Die bei den Synthesen verwendeten Reagenzien und Bausteine stammen von verschiedenen Lieferanten wie Aldrich, Acros, Sigma, Fluka, Nova Biochem, Advanced Chemtech, Bachem, Lancaster, Rapp Polymere usw. Sofern nichts anderes angegeben ist, kamen bei den chemischen Analysen folgende Methoden zur Anwendung:

Flüssigkeitschromatographische/massenspektrometrische Analyse (LC/MS): Agilent 1100 LC mit Massenspektrometer-Detektor. Verwendet wurden: Waters (YMC) Combiscreen Pro C18 4,6 x 33,5 µ, 120 A, 3 Minuten lang mit 10 % Acetonitril (0,1 % Trifluoressigsäure) und 90 % Wasser (0,1 % Trifluoressigsäure) bis 0 % Acetonitril (0,1 % Trifluoressigsäure) und 100 % Wasser (0,1 % Trifluoressigsäure). 1-minütige Durchlaufzeit und anschließend 1-minütige Äquilibrierung zu den Ausgangsbedingungen. Elektrospray-Massenspek-trometrie, positiver Modus (wenn nicht anders angegeben).

Präparative LC: Halbpräparative Flüssigkeitschromatogramme wurden mit einem Gilson 215 Liquid Handler aufgezeichnet, einem für Analysen und halbpräparative Verfahren geeigneten Gerät. Mobile Phase: Wasser (0,1 % TFA) und Acetonitril (0,1 % TFA). Die Proben wurden zunächst mit analytischen Methoden untersucht. Danach kam ein entsprechendes halbpräparatives Verfahren zur Anwendung. 5 % bis 100 % Acetonitril, 12 Minuten (wenn nicht anders angegeben). Zur Anwendung kamen Waters (YMC) Combiscreen-Säulen zur Analyse, 4,6 x 50 pro C18, 5 µ, 120 A. Halbpräparative Säulen Waters Combiscreen 20 x 50,5 µ, 120 A.

Dünnschichtchromatogramme (DC) wurden mit 0;25 mm dicken, glasverstärkten Kieselgelplatten 60F-254 aufgezeichnet.

Flash-Chromatographie: Dieses Verfahren wurde nach der von Still, W. C., Kahn, M. und Mitra, A. in J. Org. Chem. 1978, 43, 2923 beschriebenen Methode durchgeführt oder an die im Handel erhältlichen Systeme wie Biotage Horizon, Isco Opix oder Companion angepaßt. Dabei kamen die in den Versuchsbeispielen angegebenen Lösungsmittelsysteme zum Einsatz:

Milcrowellensynthese: Sofern nichts anderes angegeben ist, wurden die Mikrowellenreaktionen im Personal Chemistry Creator, Optimizer oder im Synthesizer durchgeführt.

Alle berechneten Massen werden monoisotop angegeben.

### Abkürzungen

Wenn nicht anders angegeben, haben die Abkürzungen in den nachstehenden Beispielen folgende Bedeutung:
ACN = Acetonitril
Aloc = Allyloxycarbonyl
DIC = Diisopropylcarbodiimid
EDC = 1-(3-Dimethylaminopropyl)-3-ethylcarbodiimid
FMOC = 9-Fluorenylmethyloxycarbonyl
DCE = 1,2-Dichlorethan
DEA = Diethylamin
DIEA = Diisopropylethylamin
NaBH₃CN = Natriumcyanoborhydrid
DMAP = N,N'-Dimethylaminopyridin
DMF = N,N-Dimethylformamid
THF = Tetrahydrofuran
DIC = Diisopropylcarbodiimid
DMSO = Dimethylsulfoxid
DCM = Dichlormethan (auch als Methylenchlorid bezeichnet)
DMTMM = 4-(4,6-Dimethoxy[1,3,5]triazin-2-yl]-4-methyl-morpholinium chlorid
HOBt = 1-Hydroxybenzotriazol
HOAt = 1-Hydroxy-7-azabenzotriazol
HATU = Dimethylamino-([1,2,3]triazolo[4,5-b]pyridin-3-yloxy)-methylen-dimethylammoniumhexafluorphosphat
EtOAc = Ethylacetat
HOAc = Essigsäure
Et₃N = Triethylamin
HCl = Salzsäure
HBr = Bromwasserstoffsäure
HPLC = Hochleistungs-Flüssigkeitschromatographie
TEA = Triethylamin
TMSI = Trimethylsilyliodid

Die nachfolgenden Beispiele dienen zur näheren Erläuterung der Erfindung, ohne dieselbe auf in den Beispielen beschriebene Produkte und Ausführungsformen einzuschränken.

### Beispiel 1

### 3-Amino-6-(4-chlor-benzyl)-1-(5-chlor-2-methoxy-benzolsulfonyl)-8-isopropyl-hexahydropyrazino[1,2-a]pyrimidin-4,7-dion

### Struktur:

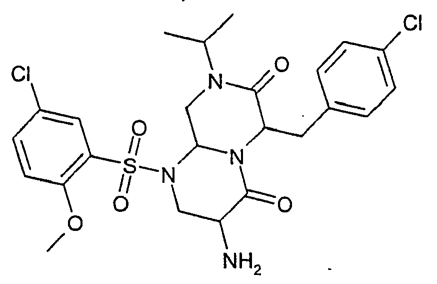

0,35 g TentaGel HL12019 (Broniacetal-Linker, S = 0,5 mMol/g, Rapp Polymere, Tübingen) wurden mit DMSO gewaschen. 20 Äquivalente 2M-Isopropylamin-Lösung in DMSO wurden zugesetzt und das Gemisch wurde 15 Stunden lang bei 60°C in einem geschlossenen Gefäß aufbewahrt. Das Polymer wurde 7mal mit DMF gewaschen.

Fmoc-(S)-4-chlorphenylalanin (3 Äquivalente) wurde mit HOAt (3 Äquivalente) und DIC (3 Äquivalente) in DMF an das sekundäre Amin auf dem Polymer gekoppelt. Die Endkonzentration betrug 0,2 - 0,3 M. Das Reaktionsgemisch wurde über Nacht bei Raumtemperatur stehengelassen. Das Polymer wurde 6mal mit DMF gewaschen. Die Fmoc-Schutzgruppe wurde mit 50 % Piperidin in DMF abgespalten (5 + 15 Minuten).

Z-Dap(Fmoc) (3 Äquivalente) wurde dann mit HOBt (3 Äquivalente) und DIC (3 Äquivalente) in DMF (Endkonzentration: ca. 0,2 M) über einen Zeitraum von mindestens 4 Stunden angekoppelt. Die Fmoc-Schutzgruppe wurde mit 50 % Piperidin in DMF abgespalten (5 + 15 Minuten).

Das Polymer wurde 5mal mit DMF und 4mal mit DCM gewaschen und mit einer Lösung von 1,5 Äquivalenten 2-Methoxy-5-chlorbenzolsulfonylchlorid und 3 Äquivalenten DIEA in DCM (Endkonzentration: 0,1 - 0,15 M) versetzt und bei Raumtemperatur 5 Stunden lang umgesetzt. Anschließend wurde es 5mal mit DMF und 5mal mit DCM gewaschen und im Vakuum getrocknet.

Zur zyklativen Abspaltung wurde das trockene Polymer mit 10 mL Ameisensäure versetzt, bei Raumtemperatur 16 Stunden lang und bei 50 - 55 °C 6 Stunden lang geschüttelt. Das Polymer wurde abfiltriert und mit DCM gewaschen. Die vereinigten Filtrate wurden im Vakuum verdampft. Der Rückstand wurde 2 Stunden lang bei Raumtemperatur mit 5 mL 37 % HBr/HOAc behandelt und im Vakuum verdampft. Das Hydrobromid des Produkts wurde durch Zugabe von Diethylether ausgefällt und abfiltriert. Die reine Titelverbindung wurde nach Reinigung mittels HPLC abgetrennt. Dabei kam das unter "Allgemeine Verfahren" beschriebene System und Verfahren zur Anwendung. MG = 554,12 (berechnet, monoisotop); Meßwert (M+H)⁺: 555,3.

### Beispiel 2

### 3-Amino-6-(4-chlor-benzyl)-1-(2,4-dichlor-benzolsulfonyl)-8-isopropyl-hexahydropyrazino[1,2-a]pyrimidin-4,7-dion

### Struktur:

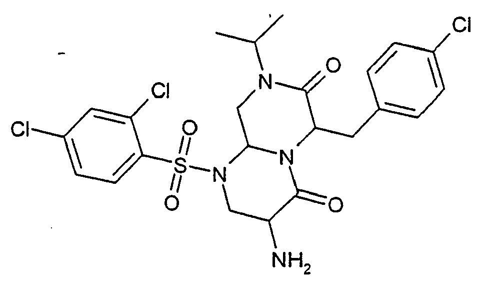

0,7 g TentaGel HL12019 (Bromacetal-Linker, S = 0,5 mMol/g, Rapp Polymere, Tübingen) wurden mit DMSO gewaschen. 20 Äquivalente 2M-Isopropylamin-Lösung in DMSO wurden zugesetzt und das Gemisch wurde 15 Stunden lang bei 60°C in einem geschlossenen Gefäß aufbewahrt. Das Polymer wurde 7mal mit DMF gewaschen.

Fmoc-4-chlorphenylalanin (3 Äquivalente) wurde mit HOAt (3 Äquivalente) und DIC (3 Äquivalente) in DMF an das sekundäre Amin auf dem Polymer gekoppelt. Die Endkonzentration betrug 0,2 - 0,3 M. Das Reaktionsgemisch wurde über Nacht bei Raumtemperatur stehengelassen. Das Polymer wurde 6mal mit DMF gewaschen. Die Fmoc-Schutzgruppe wurde mit 50 % Piperidin in DMF abgespalten (5 + 15 Minuten).

Cbz-Dap(Fmoc) (3 Äquivalente) wurde dann mit HOBt (3 Äquivalente) und DIC (3 Äquivalente) in DMF (Endkonzentration: ca. 0,2 M) über einen Zeitraum von mindestens 4 Stunden angekoppelt. Die Fmoc-Schutzgruppe wurde mit 50 % Piperidin in DMF abgespalten (5 + 15 Minuten).

Das Polymer wurde 5mal mit DMF und 4mal mit DCM gewaschen und mit einer Lösung von 1,5 Äquivalenten 2,4-Dichlorbenzolsulfonylchlorid und 3 Äquivalenten DIEA in DCM (Endkonzentration: 0,1 - 0,15 M) versetzt und bei Raumtemperatur 5 Stunden lang umgesetzt. Anschließend wurde es 5mal mit DMF und 5mal mit DCM gewaschen und im Vakuum getrocknet.

Zur zyklativen Abspaltung wurde das trockene Polymer mit 15 mL Ameisensäure versetzt, bei Raumtemperatur 16 Stunden lang und bei 50 - 55°C 6 Stunden lang geschüttelt. Das Polymer wurde abfiltriert und mit DCM gewaschen. Die vereinigten Filtrate wurden im Vakuum verdampft. Der Rückstand wurde 2 Stunden lang bei Raumtemperatur mit 10 mL

37 % HBr/HOAc behandelt und im Vakuum verdampft. Das Hydrobromid des Produkts wurde durch Zugabe von Diethylether ausgefällt und abfiltriert. Die reine Titelverbindung wurde nach Reinigung mittels HPLC abgetrennt. Dabei kam das unter "Allgemeine Verfahren" beschriebene System und Verfahren zur Anwendung. MG = 558,07 (berechnet, monoisotop); Meßwert (M+H)⁺: 559,3.

### Beispiel 3

### 6-(4-Chlor-benzyl)-1-(5-chlor-2-methoxy-benzolsulfonyl)-3-diethylamino-8-isopropylhexahydro-pyrazino[1,2-a]pyrimidin-4,7-dion

### Struktur:

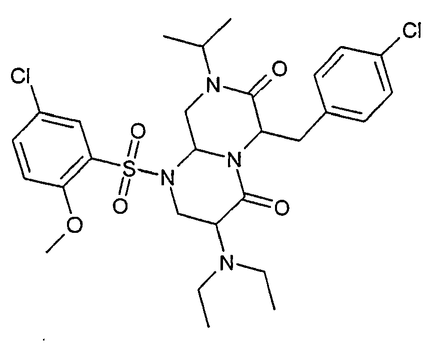

50 mg 6-(4-Chlorbenzyl)-1-(2-methoxy-5-chlorbenzolsulfonyl)-3-amino-8-isopropylhexahydro-pyrazino[1,2-a]pyrimidin-4,7-dion-hydrochlorid (Beispiel 1) wurden in 3 mL Methanol gelöst und mit 100 µl Essigsäure versetzt. Dieser Lösung wurden 100 µl Acetaldehyd und 1 mL 1M-Natriumcyanoborhydrid-Lösung in THF zugesetzt. Nach 2 Stunden wurde das Reaktionsgemisch verdampft, in 5 % Et₃N in Ethylacetat suspendiert und durch eine kleine Kieselgelsäule filtriert. Das Elutionsmittel wurde verdampft, und die Rohsubstanz wurde in einem Gemisch aus Acetonitril und Wasser gelöst und gefriergetrocknet. Die reine Titelverbindung wurde nach Reinigung mittels HPLC abgetrennt. Dabei kam das unter "Allgemeine Verfahren" beschriebene System und Verfahren zur Anwendung. MG = 610,18 (berechnet, monoisotop); Meßwert (M+H)⁺: 611,4.

### Beispiel 4

### 6-(4-Chlor-benzyl)-1-(5-chlor-2-methoxy-benzolsulfonyl)-8-isopropyl-3-isopropylaminohexahydro-pyrazino[1,2-a]pyrimidin-4,7-dion

### Struktur:

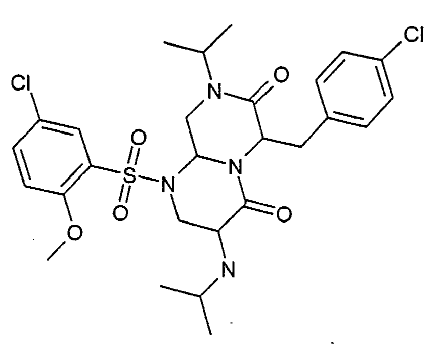

50 mg 6-(4-Chlorbenzyl)-1-(2-methoxy-5-chlorbenzolsulfonyl)-3-amino-8-isopropylhexahydro-pyrazino[1,2-a]pyrimidin-4,7-dion (Beispiel 1) wurden in 5 mL Methanol gelöst und mit 200 µl Essigsäure versetzt. Dieser Lösung wurden 400 µl Aceton und 1 mL 1M-Natriumcyanoborhydrid-Lösung in THF zugesetzt. Nach 3 Stunden wurde das Reaktionsgemisch verdampft, in 5 % Et₃N in Ethylacetat suspendiert und durch eine kleine Kieselgelsäule filtriert. Das Elutionsmittel wurde verdampft, und die Rohsubstanz wurde in einem Gemisch aus Acetonitril und Wasser gelöst und gefriergetrocknet. Die reine Titelverbindung wurde nach Reinigung mittels HPLC abgetrennt. Dabei kam das unter "Allgemeine Verfahren" beschriebene System und Verfahren zur Anwendung. MG = 596,16 (berechnet, monoisotop); Meßwert (M+H)⁺: 597,3.

### Beispiel 5

### 6-(4-Chlor-benzyl)-1-(2,4-dichlor-benzolsulfonyl)-3-dimethylamino-8-isopropyl- hexahydropyrazino[1,2-a]pyrimidin-4,7-dion

### Struktur:

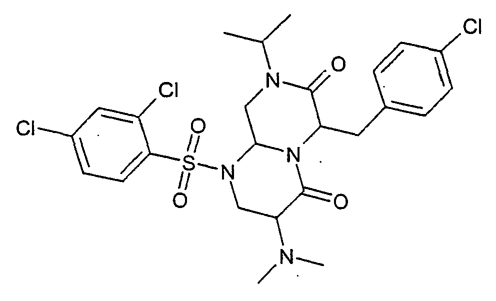

200 mg 6-(4-Chlorbenzyl)-1-(2,4-dichlorbenzolsulfonyl)-3-amino-8-isopropyl-hexahydropyrazino[1,2-a]pyrimidin-4,7-dion-hydrobromid (Beispiel 2) wurden in 15 mL Methanol gelöst und mit 600 µl Essigsäure versetzt. Dieser Lösung wurden 0,5 mL 37 %iges, wäßriges Formaldehyd und 3 mL 1M-Natriumcyanoborhydrid-Lösung in THF zugesetzt. Nach 2 Stunden wurde das Reaktionsgemisch verdampft, in 5 % Et₃N in Ethylacetat suspendiert und durch eine kleine Kieselgelsäule filtriert. Das Elutionsmittel wurde verdampft, und die Rohsubstanz wurde in einem Gemisch aus Acetonitril und Wasser gelöst und gefriergetrocknet. Die reine Titelverbindung wurde nach Reinigung mittels HPLC abgetrennt. Dabei kam das unter "Allgemeine Verfahren" beschriebene System und Verfahren zur Anwendung. MG = 586,10 (berechnet, monoisotop); Meßwert (M+H)⁺: 587,3.

### Beispiel 6

### 6-(4-Chlor-benzyl)-1-(2,4-dichlor-benzolsulfonyl)-8-isopropyl-3-pyrrol-1-yl-hexahydropyrazino[1,2-a]pyrimidin-4,7-dion

### Struktur:

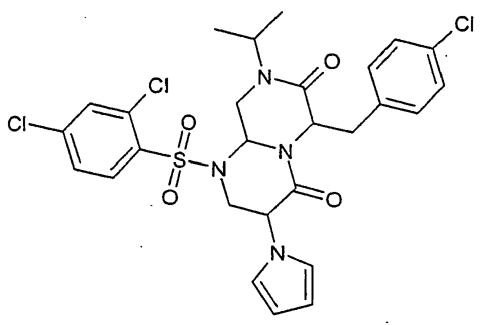

64 mg 6-(4-Chlorbenzyl)-1-(2,4-dichlorbenzolsulfonyl)-3-amino-8-isopropyl-hexahydropyrazino[1,2-a]pyrimidin-4,7-dion-hydrobromid (Beispiel 2) wurden in 1 mL Wasser suspendiert und mit 1 mL DCE versetzt. Dieses Gemisch wurde mit 2 Äquivalenten 2,5-Dimethoxytetrahydrofuran versetzt und 1 Stunde lang bei 80°C gerührt. Die DCE-Phase wurde entfernt und die wässrige Phase wurde zweimal mit DCE extrahiert. Die vereinigten Extrakte wurden verdampft, und die Rohsubstanz wurde in einem Gemisch aus Acetonitril und Wasser gelöst und gefriergetrocknet. Die reine Titelverbindung wurde nach Reinigung mittels HPLC abgetrennt. Dabei kam das unter "Allgemeine Verfahren" beschriebene System und Verfahren zur Anwendung. MG = 608,08 (berechnet, monoisotop); Meßwert (M+H)⁺: 609,4.

### Beispiel 7

### 3-Azetidin-1-yl-6-(4-chlor-benzyl)-1-(2,4-dichlor-benzolsulfonyl)-8-isopropyl- hexahydropyrazino[1,2-a]pyrimidin-4,7-dion

### Struktur:

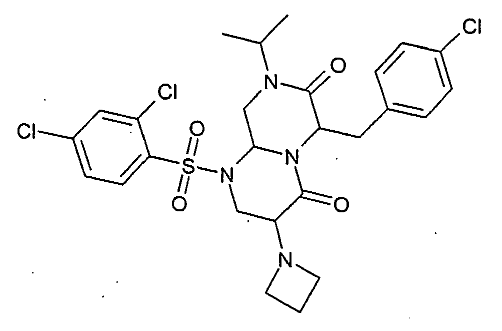

32 mg 6-(4-Chlorbenzyl)-1-(2,4-dichlorbenzolsulfonyl)-3-amino-8-isopropyl-hexahydropyrazino[1,2-a]pyrimidin-4,7-dion-hydrobromid (Beispiel 2) wurden in 1 mL Wasser suspendiert und mit 2 mL 1-Butanol versetzt. Diesem Gemisch wurden 1 mMol Kaliumcarbonat und 200 µl 1,3-Dibrompropan zugesetzt. Das Reaktionsgemisch wurde 16 Stunden lang bei 90°C gerührt und verdampft. Der Rückstand wurde in 5 % Et₃N/Ethylacetat suspendiert, filtriert und verdampft. Die reine Titelverbindung wurde nach Reinigung mittels HPLC abgetrennt. Dabei kam das unter "Allgemeine Verfahren" beschriebene System und Verfahren zur Anwendung. MG = 598,10 (berechnet, monoisotop); Meßwert (M+H)⁺: 599,3.

### Beispiel 8

### 6-(4-Chlor-benzyl)-1-(2,4-dichlor-benzolsulfonyl)-8-isopropyl-3-pyrrolidin-1-yl- hexahydropyrazino[1,2-a]pyrimidin-4,7-dion

### Struktur:

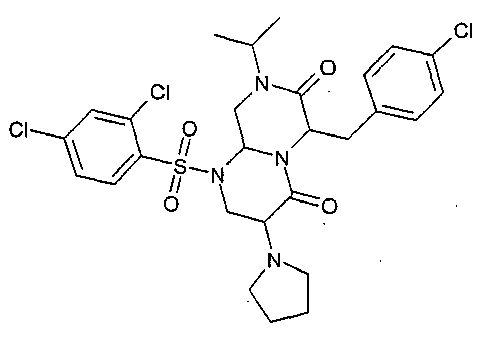

32 mg 6-(4-Chlorbenzyl)-1-(2,4-dichlorbenzolsulfonyl)-3-amino-8-isopropyl-hexahydropyrazino[1,2-a]pyrimidin-4,7-dion-hydrobromid (Beispiel 2) wurden in 1 mL Wasser suspendiert und mit 2 mL 1-Butanol versetzt. Diesem Gemisch wurden 1 mMol Kaliumcarbonat und 200 µl 1,4-Dibrombutan zugesetzt. Das Reaktionsgenüsch wurde 16 Stunden lang bei 90°C gerührt und verdampft. Der Rückstand wurde in 5 % Et₃N/Ethylacetat suspendiert, filtriert und verdampft. Die reine Titelverbindung wurde nach Reinigung mittels HPLC abgetrennt. Dabei kam das unter "Allgemeine Verfahren" beschriebene System und Verfahren zur Anwendung. MG = 612,11 (berechnet, monoisotop,); Meßwert (M+H)⁺: 613,4.

### Beispiel 9

### N-[6-(4-Chlor-benzyl)-1-(2,4-dichlor-benzolsulfonyl)-8-isopropyl-4,7-dioxo-octahydro-pyrazino[1,2-a]pyrimidin-3-yl]-methansulfonamid

### Struktur:

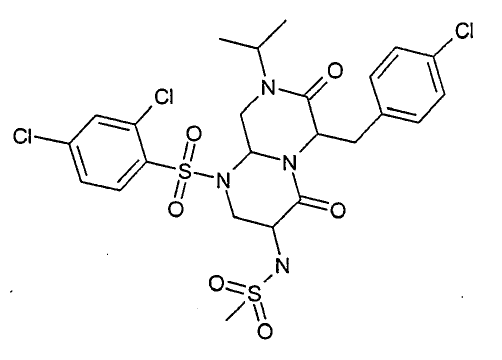

64 mg 6-(4-Chlorbenzyl)-1-(2,4-dichlorbenzolsulfonyl)-3-amino-8-isopropyl-hexahydropyrazino[1,2-a]pyrimidin-4,7-dion-hydrobromid (Beispiel 2) wurden in 3 mL DCM gelöst. Der Lösung wurden 0,5 mMol Triethylamin und 1,5 Äquivalente Methansulfonylehlorid zugesetzt. Nach 2 Stunden wurden 0,5 mL Dimethylamin in THF zugegeben, und die Lösungsmittel wurden im Vakuum verdampft. Die reine Titelverbindung wurde nach Reinigung mittels HPLC abgetrennt. Dabei kam das unter "Allgemeine Verfahren" beschriebene System und Verfahren zur Anwendung. MG = 636,04 (berechnet, monoisotop); Meßwert (M+H)⁺: 637,4.

### Beispiel 10

### 6-(4-Chlor-benzyl)-1-(5-chlor-2-methoxy-benzolsulfonyl)-3-dimethylamino-8-isopropylhexahydro-pyrazino[1,2-a]pyrimidin-4,7-dion

### Struktur:

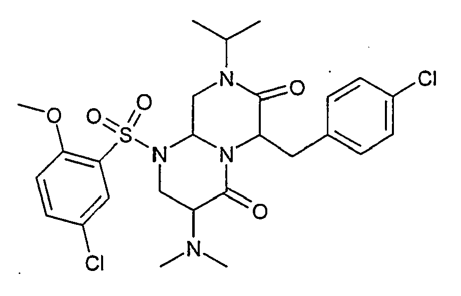

Die Verbindung in Beispiel 10 wurde nach dem in Beispiel 5 beschriebenen Verfahren, ausgehend von 6-(4-Chlorbenzyl)-1-(2-methoxy-5-chlorbenzolsulfonyl)-3-amino-8-isopropylhexahydro-pyrazino[1,2-a]pyrimidin-4,7-dion (Beispiel 1), synthetisiert.
MG = 582,15 (berechnet, monoisotop); Meßwert (M+H)⁺: 583,3.

### Beispiel 11

### 3-(Bis-cyclopropylmethyl-amino)-6-(4-chlor-benzyl)-1-(5-chlor-2-methoxy-benzolsulfonyl)-8-isopropyl-hexahydro-pyrazino[1,2-a]pyrimidin-4,7-dion

### Struktur:

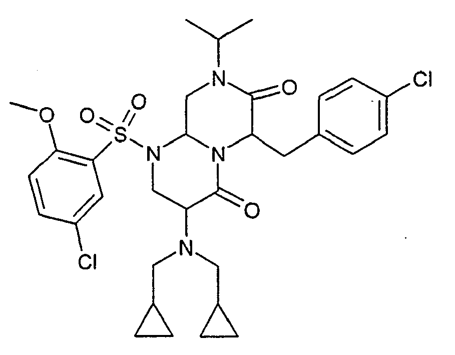

Die Verbindung in Beispiel 11 wurde nach dem in Beispiel 3 beschriebenen Verfahren unter Verwendung von 10 Äquivalenten Cyclopropancarboxaldehyd synthetisiert.
MG = 662,21 (berechnet, monoisotop); Meßwert (M+H)⁺: 663,4.

### Beispiel 12

### 6-(4-Chlor-benzyl)-1-(2,4-dichlor-benzolsulfonyl)-3-isobutylamino-8-isopropyl- hexahydropyrazino[1,2-a]pyrimidin-4,7-dion

### Struktur:

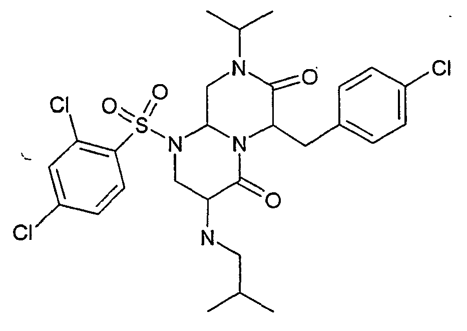

Die Verbindung in Beispiel 12 wurde nach dem in Beispiel 4 beschriebenen Verfahren unter Verwendung von 4 Äquivalenten Isobutyraldehyd synthetisiert.
MG = 614,13 (berechnet, monoisotop); Meßwert (M+H)⁺: 615,4.

### Beispiel 13

### 6-(4-Chlor-benzyl)-1-(2,4-dichlor-benzolsulfonyl)-3-diisobutylamino-8-isopropyl-hexahydropyrazino[1,2-a]pyrimidin-4,7-dion

### Struktur:

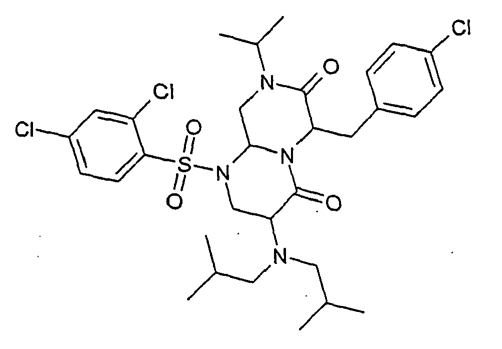

Die Verbindung in Beispiel 13 wurde nach dem in Beispiel 3 beschriebenen Verfahren unter Verwendung von 20 Äquivalenten Isobutyraldehyd synthetisiert.
MG = 670,19 (berechnet, monoisotop); Meßwert (M+H)⁺: 671,3.

### Beispiel 14

### 6-(4-Chlor-benzyl)-3-(cyclopropylmethyl-amino)-1-(2,4-dichlor-benzolsulfonyl)-8-isopropylhexahydro-pyrazino[1,2-a]pyrimidin-4,7-dion

### Struktur:

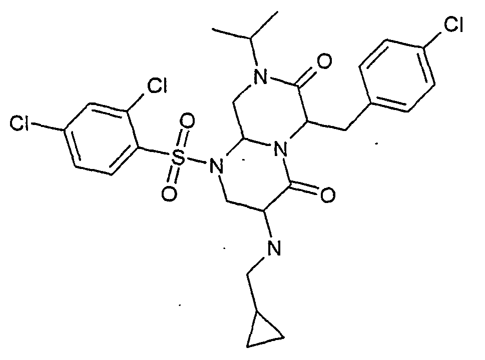

Die Verbindung in Beispiel 14 wurde nach dem in Beispiel 4 beschriebenen Verfahren unter Verwendung von 5 Äquivalenten Cyclopropylcarboxaldehyd synthetisiert.
MG = 612,11 (berechnet, monoisotop); Meßwert (M+H)⁺: 613,4.

### Beispiel 15

### 6-(4-Chlor-benzyl)-1-(2,4-dichlor-benzolsulfonyl)-3-diethylamino-8-isopropyl-hexahydropyrazino[1,2-a]pyrimidin-4,7-dion

### Struktur:

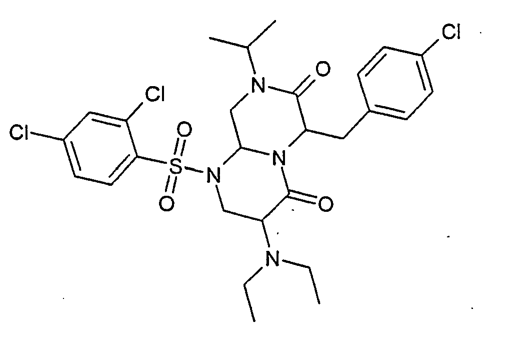

Die Verbindung in Beispiel 15 wurde nach dem in Beispiel 3 beschriebenen Verfahren, ausgehend von 6-(4-Chlorbenzyl)-1-(2,4-dichlorbenzolsulfonyl)-3-amino-8-isopropylhexahydro-pyrazino[1,2-a]pyrimidin-4,7-dion (Beispiel 2) synthetisiert.
MG = 614,13 (berechnet, monoisotop); Meßwert (M+H)⁺: 615,3.

### Beispiel 16

### 6-(4-Chlor-benzyl)-1-(2,4-dichlor-benzolsulfonyl)-8-isopropyl-3-isopropylamino-hexahydropyrazino[1,2-a]pyrimidin-4,7-dion

### Struktur:

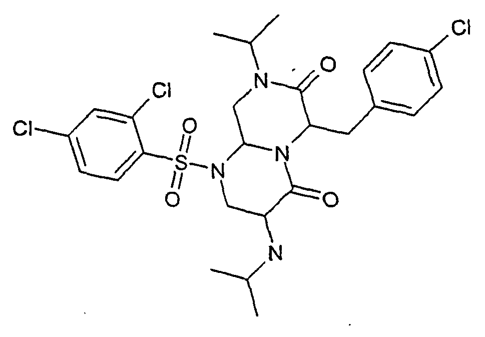

Die Verbindung in Beispiel 16 wurde nach dem in Beispiel 4 beschriebenen Verfahren, ausgehend von 6-(4-Chlorbenzyl)-1-(2,4-dichlorbenzolsulfonyl)-3-amino-8-isopropylhexahydro-pyrazino[1,2-a]pyrimidin-4,7-dion (Beispiel 2), synthetisiert.
MG = 600,11 (berechnet, monoisotop); Meßwert (M+H)⁺: 601,3.

### Beispiel 17

### 6-(4-Chlor-benzyl)-1-(2,4-dichlor-benzolsulfonyl)-8-isopropyl-3-(isopropyl-methyl- amino)-hexahydro-pyrazino[1,2-a]pyrimidin-4,7-dion

### Struktur:

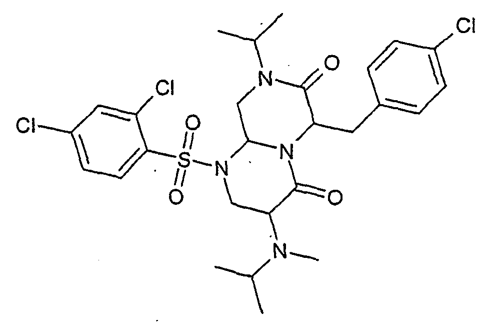

Die Verbindung in Beispiel 17 wurde nach dem in Beispiel 5 beschriebenen Verfahren, ausgehend von 6-(4-Chlorbenzyl)-1-(2,4-dichlorbenzolsulfonyl)-8-isopropyl-3-isopropylaminohexahydro-pyrazino[1,2-a]pyrimidin-4,7-dion (Beispiel 16), synthetisiert.
MG = 614,13 (berechnet, monoisotop); Meßwert (M+H)⁺:615,4.

### Beispiel 18

### 6-(4-Chlor-benzyl)-1-(5-chlor-2-methoxy-benzolsulfonyl)-8-isopropyl-3-pyrrolidin-1-ylhexahydro-pyrazino[1,2-a]pyrimidin-4,7-dion

### Struktur:

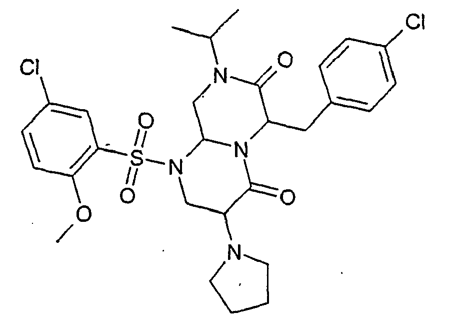

Die Verbindung in Beispiel 18 wurde nach dem in Beispiel 8 beschriebenen Verfahren, ausgehend von 3-Amino-6-(4-Chlorbenzyl)-1-(5-chlor-2-methoxy-benzolsulfonyl)-8-isopropylhexahydro-pyrazino[1,2-a]pyrimidin-4,7-dion (Beispiel 1), synthetisiert.
MG = 608,16 (berechnet, monoisotop); Meßwert (M+H)⁺: 609,4.

### Beispiel 19

### {4-[6-(4-Chlor-benzyl)-1-(2,4-dichlor-benzolsulfonyl)-8-isopropyl-4,7-dioxo-octahydro-pyrazino[1,2-a]pyrimidin-3-ylamino]-butyl}-triethyl-ammonium

### Struktur:

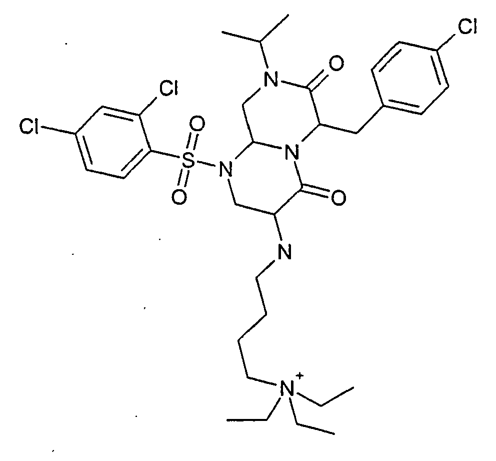

32 mg 6-(4-Chlorbenzyl)-1-(2,4-dichlorbenzolsulfonyl)-3-amino-8-isopropyl-hexahydropyrazino[l,2-a]pyrimidin-4,7-dion-hydrobromid (Beispiel 2) in 1mL Dichlorethan wurden mit 200 µl Triethylamin und 100 µl 1,4-Dibrombutan behandelt. Das Reaktionsgemisch wurde 60 Stunden lang bei 60 °C aufbewahrt. Der Niederschlag wurde abfiltriert, und das Filtrat wurde im Vakuum verdampft. Das reine Titelverbindungs-Trifluoracetat wurde nach Reinigung des Filterrückstands mittels HPLC abgetrennt. Dabei kam das unter "Allgemeine Verfahren" beschriebene System und Verfahren zur Anwendung.
MG = 714,24 (berechnet, monoisotop); Meßwert (M): 714,4.

### Beispiel 20

### (3-[6-(4-Chlor-benzyl)-1-(2,4-dichlor-benzolsulfonyl)-8-isopropyl-4,7-dioxo-octahydro-pyrazino[1,2-a]pyrimidin-3-ylamino]-propyl}-triethyl-ammonium

### Struktur:

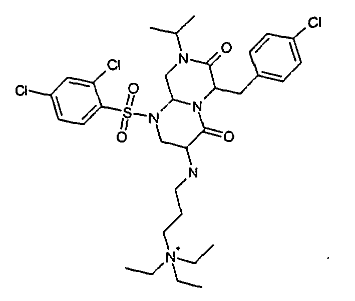

32 mg 6-(4-Chlorbenzyl)-1-(2,4-dichlorbenzolsulfonyl)-3-amino-8-isopropyl-hexahydropyräzino[1,2-a]pyrimidin-4,7-dion-hydrobromid (Beispiel 2) in 1mL Dichlorethan wurden mit 200 µl Triethylamin und 100 µl 1,3-Dibrompropan behandelt. Das Reaktionsgemisch wurde 60 Stunden lang bei 60°C aufbewahrt. Der Niederschlag wurde abfiltriert, und das Filtrat wurde im Vakuum verdampft. Das reine Titelverbindungs-Trifluoracetat wurde nach Reinigung des Filterrückstands mittels HPLC abgetrennt. Dabei kam das unter "Allgemeine Verfahren" beschriebene System und Verfahren zur Anwendung.
MG = 700,23 (berechnet, monoisotop); Messwert: 700,4.

### Beispiel 21

### N-[6-(4-Chlor-benzyl)-1-(5-chlor-2-methoxy-benzolsulfonyl)-8-isopropyl-4,7-dioxo-octahydro-pyrazino[1,2-a]pyrimidin-3-yl]-acetamid

### Struktur:

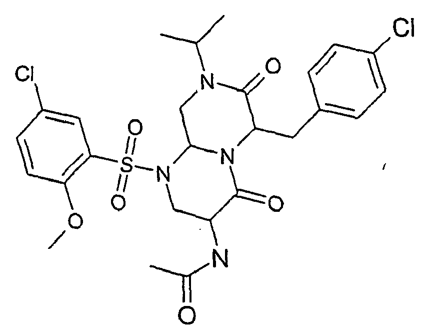

a) 2-Allyloxycarbonylamino-3-(4-chlor-phenyl)-propionsäure
   Ausgehend von 10 g 4-Chlorphenylalanin und 8 mL Chlorameisensäureallylester wird das Produkt nach literaturbekannten Vorschriften (Et₃N, Methanol) erhalten.
   MG = 283.71 (berechnet, monoisotop); Meßwert (M+H)⁺: 284.1.
b) {2-(4-Chlor-phenyl)-1-[(2,2-diethoxy-ethyl)-isopropyl-carbamoyl]-ethyl}-carbaminsäureallylester
   Zu einer Lösung von 5.7 g 2-Allyloxycarbonylamino-3-(4-chlor-phenyl)-propionsäure, 3.5 g (2,2-Diethoxy-ethyl)-isopropyl-amin, 6.8 g HOAt in 30 mL DMF wird 7.8 mL DIC getropft und 12 h gerührt. Die Reaktionslösung wird unter vermindertem Druck eingeengt und über Flashchromatographie an Kieselgel mit dem Eluent Essigsäureethylester/n-Heptan =1/3 gereinigt. Man erhält das gewünschte Produkt mit MG = 440.97 (berechnet, monoisotop); Meßwert (M+H)⁺: 441.15
c) 2-Amino-3-(4-chlor-phenyl)-N-(2,2-diethoxy-ethyl)-N-isopropyl-propionamid
   Zu einer Lösung aus 13.2 g {2-(4-Chlor-phenyl)-1-[(2,2-diethoxy-ethyl)-isopropyl-carbamoyl]-ethyl}-allylcarbamat , 18.9 g Dimethylbarbitursäure in 140 mL Methylenchlorid wird unter Argonschutzgasatmosphäre 10 mg Palladiumtetrakistriphenylphosphin gegeben und 12 h gerührt. Die Reaktionslösung wird unter vermindertem Druck eingeengt und über Flashchromatographie an Kieselgel (Eluent Methylenchlorid, 1% Et₃N, 0-10% Methanol) gereinigt. Man erhält das gewünschte Produkt mit MG = 356.90 (berechnet, monoisotop); Meßwert (M-C₂H₆O+H)⁺: 311.10
d) 2-Benzyloxycarbonylamino-3-(5-chlor-2-methoxy-benzolsulfonylamino)-propionsäure
   Zu einer Lösung von 2.3 g 3-Amino-2-benzyloxycarbonylamino-propionsäure in 20 mL IN NaOH-Lösung wird eine Lösung aus 3.8 g 5-Chlor-2-methoxy-benzolsulfonylchlorid in 5 mL Dioxan getropft. Unter pH-Kontrolle (pH>7) lässt man 12 h rühren, senkt den pH unter 7 durch Zugabe von Zitronensäure und extrahiert die Reaktionslösung anschließend mit Methylenchlorid. Die organische Phase wird über Magnesiumsulfat getrocknet, unter vermindertem Druck eingeengt und ohne weitere Reinigung im nächsten Reaktionsschritt eingesetzt.
   Produkt mit MG = 442.06 (berechnet, monoisotop); Meßwert: (M+H)⁺: 442.95
e) (2-(5-Chlor-2-methoxy-benzolsulfonylamino)-1-{2-(4-chlor-phenyl)-1-[(2,2-diethoxyethyl)-isopropyl-carbamoyl]-ethylcarbamoyl}-ethyl)-carbamatsäure benzyl ester
   Zu einer Lösung aus 124 mg 2-Benzyloxycarbonylamino-3-(5-chlor-2-methoxy-benzolsulfonylamino)-propionsäure in 1 mL DMF werden 52 mg EDC, 45 mg HOBt und 100 µl N-Ethylmorpholin gegeben. Dazu tropft man eine Lösung aus 100 mg 2-Amino-3-(4-chlor-phenyl)-N-(2,2-diethoxy-ethyl)-N-isopropyl-propionamid in 1 mL DMF und lässt 12 h rühren. Die Reaktionslösung wird filtriert mit Essigsäureethylester versetzt und anschließend mit 5% wässriger Natriumhydrogencarbonatlösung und wässriger Natriumchloridlösung extrahiert. Nach Trocknung der organischen Phase mit Chromabond XTR wird unter vermindertem Druck eingeengt und der Rückstand über HPLC (Knauer Eurospher-100-10-C18, Wasser (0.1 % Trifluoressigsäure)/Acetonitril (0.1 % Trifluoressigsäure) = 80/20 -> 10/90) getrennt. Man erhält das gewünschte Produkt mit MG = 780.24 (berechnet, monoisotop); Meßwert (M-C₂H₆O+H)⁺: 735.1
f) [6-(4-Chlor-benzyl)-1-(5-chlor-2-methoxy-benzolsulfonyl)-8-isopropyl-4,7-dioxo-octahydro-pyrazino[1,2-a]pyrimidin-3-yl]-carbaminsäurebenzylester
   Eine Lösung von 218 mg (2-(5-Chlor-2-methoxy-benzolsulfonylamino)-1-{2-(4-chlorphenyl)-1-[(2,2-diethoxy- ethyl)-isopropyl-carbamoyl]-ethylcarbamoyl}-ethyl)-carbaminsäurebenzylester in 3 mL Ameisensäure wird 12 h bei Raumtemperatur und anschließend 5 h bei 55°C gerührt. Die Reaktionslösung wird unter vermindertem Druck eingeengt und der Rückstand über HPLC (Knauer Eurospher-100-10-C18, Wasser (0.1 % Trifluoressigsäure)/Acetonitril (0.1% Trifluoressigsäure) = 80/20 -> 10/90) getrennt. Man erhält das gewünschte Produkt mit MG = 688,15 (berechnet, monoisotop); Meßwert (M+H)⁺:689.41
g) 3-Amino-6-(4-chlor-benzyl)-1-(5-chlor-2-methoxy-benzolsulfonyl)-8-isopropylhexahydro- pyrazino[1,2-a]pyrimidin-4,7-dion
   Eine Lösung aus 79 mg [6-(4-Chlor-benzyl)-1-(5-chlor-2-methoxy-benzolsulfonyl)-8-isopropyl-4,7-dioxo- octahydro-pyrazino[1,2-a]pyrimidin-3-yl]-carbaminsäurebenzylester in 2 mL einer 33%Lösung aus HBr in Eisessig wird 2 h gerührt. Die Reaktionslösung wird mit wässriger Natriumcarbonatlösung versetzt und mit Essigsäureethylester extrahiert. Die organische Phase wird über Magnesiumcarbonat getrocknet und unter vermindertem Druck eingeengt und der Rückstand über HPLC (Knauer Eurospher-100-10-C18, Wasser (0.1 % Trifluoressigsäure)/Acetonitril (0.1 % Trifluoressigsäure) = 80/20 -> 10/90) getrennt. Man erhält das gewünschte Produkt mit MG = 554,12 (berechnet, monoisotop); Meßwert (M+H)⁺: 555.12
h) N-[6-(4-Chlor-benzyl)-1-(5-chlor-2-methoxy-benzolsulfonyl)-8-isopropyl-4,7-dioxo-octahydro-pyrazino[1,2-a]pyrimidin-3-yl]-acetamid

Eine Lösung aus 7 mg 3-Amino-6-(4-chlor-benzyl)-1-(5-chlor-2-methoxy-benzolsulfonyl)-8-isopropyl-hexahydro- pyrazino[1,2-a]pyrimidin-4,7-dion, 2.4 µl Essigsäureanhydrid; 0.5 mg DMAP in 1.5 mL Pyridin wird 12 h gerührt. Die Reaktionslösung wird eingeengt und durch Flashchromatographie an Kieselgel mit dem Eluent Methylenchlorid mit einem Gradient von 0 -10% Methanol gereinigt.
Man erhält das gewünschte Produkt mit MG = 596,13 (berechnet, monoisotop); Meßwert (M+H)⁺: 597.13

### Beispiel 22

### Cyclopropancarbonsäure- [6-(4-chlor-benzyl)-1-(5-chlor-2-methoxy-benzolsulfonyl)-8-isopropyl-4,7-dioxo-octahydro-pyrazino[1,2-a]pyrimidin-3-yl]- amid

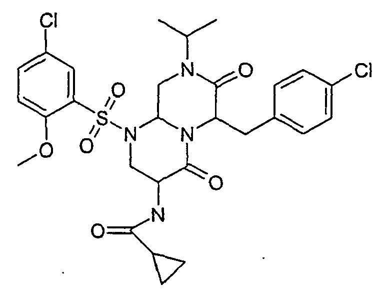

Zu einer Lösung von 3-Amino-6-(4-chlor-benzyl)-1-(5-chlor-2-methoxy-benzolsulfonyl)-8-isopropyl-hexahydro-pyrazino[1,2-a]pyrimidin-4,7-dion (56 mg, 0.10 mmol) in 1 mL Dichlormethan und 26 mg (0.20 mmol) Diisopropylethylamin werden 11 mg (0.105 mmol) Cyclopropancarbonylchlorid gegeben. Nach zwei Stunden rühren bei Raumtemperatur wird die Reaktionsmischung chromatographisch an 1 g Kieselgel (Eluent EtOAc/DCM; Gradient 0-20%) gereinigt. Es werden 42 mg des gewünschten Produktes erhalten. MW = 622 (berechnet, monoisotop), Meßwert (M+H)⁺ = 623.10

### Beispiel 23

### Cyclobutancarbonsäure- [6-(4-chlor-benzyl)-1-(5-chlor-2-methoxy-benzolsulfonyl)-8-isopropyl-4,7-dioxo-octahydro-pyrazino[1,2-a]pyrimidin-3-yl]- amid

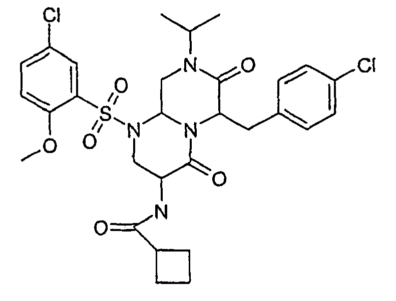

Die Synthese erfolgte analog zu Beispiel 22 unter Verwendung von Cyclobutancarbonylchlorid. Man erhält das gewünschte Produkt mit MG = 636,16 (berechnet, monoisotop); Messwert (M+H)⁺: 637.11

### Beispiel 24

### Cyclopentancarbonsäure- [6-(4-chlor-benzyl)-1-(5-chlor-2-methoxy-benzolsulfonyl)-8-isopropyl-4,7-dioxo-octahydro-pyrazino[1,2-a]pyrimidin-3-yl]-amid

### Struktur:

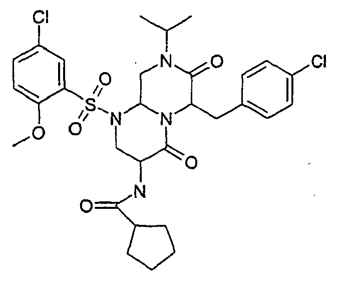

Die Synthese erfolgte analog zu Beispiel 22 unter Verwendung von Cyclopentancarbonylchlorid. Man erhält das gewünschte Produkt mit MG =650,17 (berechnet, monoisotop); Messwert (M+H)⁺: 651.12

### Beispiel 25

### 4-Dimethylamino N-[6-(4-Chlor-benzyl)-1-(5-chlor-2-methoxy-benzolsulfonyl)-8-isopropyl-4,7-dioxo- octahydro-pyrazino[1,2-a]pyrimidin-3-yl]-benzamid

### Struktur:

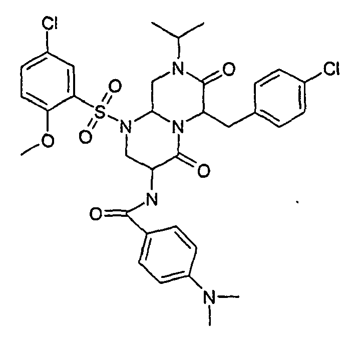

Die Synthese erfolgte analog zu Beispiel 22 unter Verwendung von 4-Dimethylaminobenzoylchlorid. Man erhält das gewünschte Produkt mit MG = 701,18 (berechnet, monoisotop); Messwert (M+H)⁺: 702.12

### Beispiel 26

### N-[6-(4-Chlor-benzyl)-1-(5-chlor-2-methoxy-benzolsulfonyl)-8-isopropyl-4,7-dioxo-octahydro-pyrazino[1,2-a]pyrimidin-3-yl]-methansulfonamid

### Struktur:

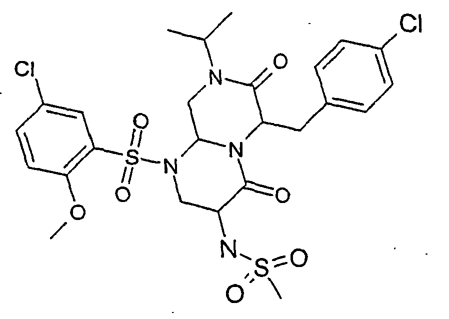

Zu einer Lösung aus 5 mg 3-Amino-6-(4-chlor-benzyl)-1-(5-chlor-2-methoxy-benzolsulfonyl)-8-isopropyl-hexahydro- pyrazino[1,2-a]pyrimidin-4,7-dion in 1 mL Methylenchlorid und 3 µl Et₃N wird bei 0°C 1.8 µl Mesylchlorid getropft. Es wird 2 h gerührt und anschließend mit wässriger Natriumchloridlösung gewaschen. Die organische Phase wird über Magnesiumsulfat getrocknet und unter vermindertem Druck eingeengt.

Man erhält als Rückstand das gewünschte Produkt mit MG = 632,09 (berechnet, monoisotop); Meßwert (M+H)⁺: 633.10

### Beispiel 27

### N- [6-(4-chlor-benzyl)-1-(5-chlor-2-methoxy-benzolsulfonyl)-8- isopropyl-4,7-dioxo-octahydro-pyrazino[1,2-a]pyrimidin-3-yl]-cyclopropansulfonamid

### Struktur:

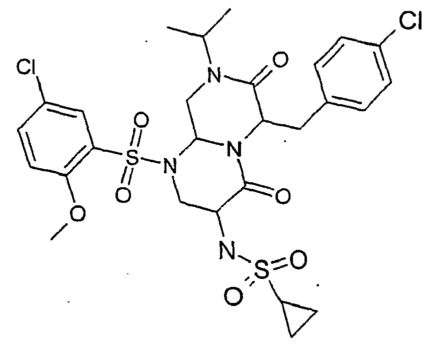

Die Synthese erfolgte analog zu Beispiel 26 unter Verwendung von Cyclopropansulfonylchlorid. Man erhält das gewünschte Produkt mit MG = 658,11 (berechnet, monoisotop); Messwert (M+H)⁺:659.10

### Beispiel 28

### 1-tert-Butyl-3-[6-(4-chlor-benzyl)-1-(5-chlor-2-methoxy-benzolsulfonyl)-8-isopropyl- 4,7-dioxo-octahydro-pyrazino[1,2-a]pyrimidin-3-yl]-harnstoff

### Struktur:

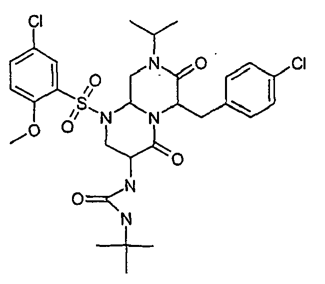

Eine Lösung aus 30 mg 3-Amino-6-(4-chlor-benzyl)-1-(5-chlor-2-methoxy-benzolsulfonyl)-8-isopropyl-hexahydro- pyrazino[1,2-a]pyrimidin-4,7-dion und 7,6 µl Et₃N in 500 µl Dioxan wird mit 5,4 mg tert-Butylisocyanat versetzt Die Lösung wird 6h auf 50 °C erwärmt. Das Lösungsmittel wird im Vakuum entfernt. Der Rückstand wird über HPLC (Waters-Xterra™ MS C18, 5 µm, Wasser (0.1 % Trifluoressigsäure)/Acetonitril (0.1% Trifluoressigsäure) = 80/20 -> 10/90) getrennt.

Man erhält das gewünschte Produkt mit MG = 653,18 (berechnet, monoisotop); Meßwert (M+H)⁺: 654.13

### Beispiel 29

### Struktur:

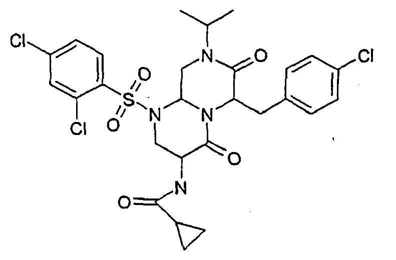

### Methode A:

a) 2-Benzyloxycarbonylamino-3-(2,4-dichlor-benzolsulfonylamino)-propionsäure
   Die Synthese erfolgt analog zu Beispiel 21d) ausgehend von 2,4-Dichlor-benzolsulfonyl chlorid. Man erhält das gewünschte Produkt mit MG = 446,01 (berechnet, monoisotop); Meßwert (M+H-CO₂)⁺: 403,00.
b) N-{2-(4-Chlor-phenyl)-1-[(2,2-diethoxy-ethyl)-isopropyl-carbamoyl]-ethyl}-3-(2,4-dichlorbenzolsulfonylamino)-2-methansulfonylamino-propionamid
   Die Synthese erfolgt analog zu Beispiel 21e) ausgehend von 2-Benzyloxycarbonylamino-3-(2,4-dichlor-benzolsulfonylamino)-propionsäure. Man erhält das gewünschte Produkt mit MG = 784,186 (berechnet, monoisotop); Meßwert (M-CO₂+H)⁺: 741,10
c) [6-(4-Chlor-benzyl)-1-(2,4-dichlor-benzolsulfonyl)-8-isopropyl-4,7-dioxo-octahydro-pyrazino[1,2-a]pyrimidin-3-yl]-carbaminsäurebenzylester
   Die Synthese erfolgt analog zu Beispiel 21f) ausgehend von N-{2-(4-Chlor-phenyl)-1-[(2,2-diethoxy-ethyl)-isopropyl-carbamoyl]-ethyl}-3-(2,4 - dichlor-benzolsulfonylamino)-2-methansulfonylamino-propionamid. Man erhält das gewünschte Produkt mit MG = 692,10 (berechnet, monoisotop); Meßwert (M+H)⁺: 693,05
d) 3-Amino-6-(4-chlor-benzyl)-1-(2,4-dichlor-benzolsulfonyl)-8-isopropyl-hexahydropyrazino[1,2-a]pyrimidin-4,7-dion
   Die Synthese erfolgt analog Bsp. 21g) ausgehend von [6-(4-Chlor-benzyl)-1-(2,4-dichlor-benzolsulfonyl)-8-isopropyl-4,7-dioxo-octahydro- pyrazino[1,2-a]pyrimidin-3-yl]-carbaminsäurebenzylester. Man erhält das gewünschte Produkt mit MG = 558,07 (berechnet, monoisotop); Meßwert (M+H)⁺: 559,10
e) Cyclopropancarbonsäure-[6-(4-chlor-benzyl)-1-(2,4-dichlor-benzolsulfonyl)-8- isopropyl-4,7-dioxo-octahydro-pyrazino[1,2-a]pyrimidin-3-yl]- amid.
   Eine Lösung von 15 mg 3-Amino-6-(4-chlor-benzyl)-1-(2,4-dichlor-benzolsulfonyl)-8-isopropyl-hexahydro- pyrazino[1,2-a]pyrimidin-4,7-dion, 3,2 mg Cyclopropancarbonsäure in 110 µl DMF wird auf 0°C gekühlt und mit 11,2 mg HATU, 4,1 mg HOAt und 11,6 µl Et₃N versetzt. Die Lösung wird 10 min bei 0 °C gerührt und anschließend 4 h bei Raumtemperatur. Das Lösungsmittel wird im Vakuum entfernt. Anschließend wird der Rückstand in Essigester und Wasser aufgenommen. Die wässrige Phase zweimal mit Essigester extrahiert. Die vereinigten organischen Phasen über Na₂SO4 getrocknet und das Lösungsmittel im Vakuum entfernt. Das Rohprodukt wird über HPLC (Waters-Xterra™ MS C18, 5 µm, Wasser (0.1 % Trifluoressigsäure)/Acetonitril (0.1 % Trifluoressigsäure) = 80/20 -> 10/90) getrennt.
   Man erhält das gewünschte Produkt mit MG = 626,09 (berechnet, monoisotop); Meßwert (M+H)⁺: 627.13

### Methode B

a) {2-(4-Chlor-phenyl)-1-[(2,2-diethoxy-ethyl)-isopropyl-carbamoyl]-ethyl}-carbaminsäure 9H-fluoren-9-ylmethylester
   Zu einer Lösung von 505 mg (1.2 mmol) N-Fmoc-4-Cl-Phe-OH in 2 mL DMF werden 210 mg (1.2 mmol) (2,2-Diethoxy-ethyl)-isopropyl-amine and 376 mg (1.20 mmol) DMTMM gegeben. Die Reaktionsmischung wird über Nacht bei Raumtemperatur gerührt. Anschließend wird sie mit 40 mL Diethylether extrahiert und mit 10 mL Wasser gewaschen. Die vereinigten organischen Phasen werden über MgSO₄ getrocknet und im Vakuum aufkonzentriert. Das Rohprodukt wird chromatographisch an 10g SiO₂ (Eluent DCM gefolgt von 20%EtOAc/DCM) gereinigt. Es werden 530 mg des gewünschten Produktes als Öl erhalten. MG = 578.26 (berechnet, monoisotop); Meßwert (M+H)⁺: 579
b) 2-Amino-3-(4-chlor-phenyl)-N-(2,2-diethoxy-ethyl)-N-isopropyl-propionamid
   Eine Lösung aus 530 mg (0.915 mmol) 3-(4-Chlor-phenyl)-N-(2,2-diethoxy-ethyl)-N-isopropyl-2-methyl-propionamid in 15 mL einer 20% Diethylamin DCM Lösung wird bei Raumtemperatur über Nacht gerührt. Die Reaktionsmischung wird im Vakuum aufkonzentriert und chromatographisch an 5 g SiO₂ (Eluent DCM gefolgt von 20% EtOAc/DCM gefolgt von 20% MeOH/DCM) gereinigt. Es werden 320 mg des gewünschten Produktes als Öl erhalten. MG= 356.19 (berechnet, monoisotop); Meßwert (M+H)⁺ = 357
c) (2-Benzyloxycarbonylamino-2-{2-(4-chlor-phenyl)-1-[(2,2-diethoxy-ethyl)-isopropylcarbamoyl]-ethylcarbamoyl}-ethyl)-carbaminsäure 9H-fluoren-9-ylmethyl ester
   Z-Dap-Fmoc-OH wurde mit 2-Amino-3-(4-chlor-phenyl)-N-(2,2-diethoxy-ethyl)-N-isopropylpropionamid unter denselben Bedingungen wie unter a) beschrieben gekuppelt. Man erhält das gewünschte Produkt mit MG= 798.34 (berechnet, monoisotop); Meßwert (M+Na)⁺ = 821.43
d) (2-Amino-1-{2-(4-chlor-phenyl)-1-[(2,2-diethoxy-ethyl)-isopropyl-carbamoyl]-ethylcarbamoyl}-ethyl)-carbaminsäurebenzylester
   Von (2-Benzyloxycarbonylamino-2-{2-(4-chlor-phenyl)-1-[(2,2-diethoxy-ethyl)-isopropylcarbamoyl]-ethylcarbamoyl}-ethyl)-carbaminsäure 9H-fluoren-9-ylmethyl ester wurde die Fmoc-Schutzgruppe unter Verwendung von Diethylamin abgespalten, wobei die Methode wie unter b) beschrieben, genutzt wurde. Man erhält das gewünschte Produkt mit MG= 576.27 (berechnet, monoisotop); Meßwert (M+H)⁺ = 577.22
e) [1-{2-(4-Chlor-phenyl)-1-[(2,2-diethoxy-ethyl)-isopropyl-carbamoyl]-ethylcarbamoyl}-2-(2,4-dichlor-benzolsulfonylamino)-ethyl]-carbaminsäurebenzylester
   Zu einer Lösung von 3.2 g (5.54 mmol) (2-Amino-1-{2-(4-chlor-phenyl)-1-[(2,2-diethoxyethyl)-isopropyl-carbamoyl]-ethylcarbamoyl}-ethyl)-carbaminsäurebenzylester in 75 mL DCM werden 1.94 mL (11.09 mmol) DIEA und 1.5 g (6.1 mmol) 2,4-Dichlorphenylsulfonylchlorid gegeben. Die Lösung wird über Nacht bei Raumtemperatur gerührt. Anschließend wird die Lösung im Vakuum aufkonzentriert und der Rückstand säulenchromatographisch an 100 g SiO₂ (Eluent DCM gefolgt von 20% EtOAc/DCM) gereinigt. Es werden 2.78 g des gewünschten Produktes als farbloser Schaum erhalten. MG= 784.19 (berechnet, monoisotop); Meßwert (M+Na)⁺ = 807.24
f) [6-(4-Chlor-benzyl)-1-(2,4-dichlor-benzolsulfonyl)-8-isopropyl-4,7-dioxo-octahydro-pyrazino[1,2-a]pyrimidin-3-yl]-carbaminsäurebenzylester
   Eine Lösung von 2.74 g (3.49 mmol) [1-{2-(4-Chlor-phenyl)-1-[(2,2-diethoxy-ethyl)-isopropyl-carbamoyl]-ethylcarbamoyl}-2-(2,4-dichlor-benzolsulfonylamino)-ethyl]-carbaminsäurebenzylester in 45 mL Ameisensäure wurde über 6 h auf 60°C erwärmt. Anschließend wurde die Reaktionsmischung im Vakuum aufkonzentriert und der Rückstand chromatographisch an 40 g SiO₂ (Eluent DCM gefolgt von 20% EtOAc/DCM) gereinigt. Es werden 2.25 g der cyclisierten Verbindung als farbloser Feststoff erhalten. LC/MS MG= 692.1 (berechnet, monoisotop); Messwert (M+H)⁺ = 693
g) 3-Amino-6-(4-chlor-benzyl)-1-(2,4-dichlor-benzolsulfonyl)-8-isopropyl-hexahydropyrazino[1,2-a]pyrimidin-4,7-dion
   Zu einer Lösung von 250 mg (0.36 mmol) [6-(4-Chlor-benzyl)-1-(2,4-dichlor-benzolsulfonyl)-8-isopropyl-4,7-dioxo-octahydro-pyrazino[1,2-a]pyrimidin-3-yl]-carbaminsäurebenzylester in 10 mL CH₃CN werden bei 0°C 206 mL (1.44 mmol) Trimethylsilyl iodid (TMSI) zugegeben. Man läßt die Reaktionslösung auf Raumtemperatur kommen und rührt bei dieser Temperatur 2 h nach. Zu der Reaktionslösung werden 5 mL MeOH gegeben und anschließend die Lösung im Vakuum eingeengt. Der Rückstand wird über eine 5g SCX Kartusche gereinigt (elutiert mit MeOH gefolgt von 3N NH₃/MeOH). Es werden 185 mg der gewünschten Verbindung als weißes Pulver erhalten. LC/MS 558,07(berechnet, monoisotop); Meßwert (M+H)⁺: 559,10
g) Cyclopropancarbonsäure-[6-(4-chlor-benzyl)-1-(2,4-dichlor-benzolsulfonyl)-8- isopropyl-4,7-dioxo-octahydro-pyrazino[1,2-a]pyrimidin-3-yl]- amid.
   Die Synthese erfolgte analog zu Beispiel 22.

### Beispiel 30

### Cyclobutancarbonsäure-[6-(4-chlor-benzyl)-1-(2,4-dichlor-benzolsulfonyl)-8- isopropyl-4,7-dioxo-octahydro-pyrazino[1,2-a]pyrimidin-3-yl]- amid

### Struktur:

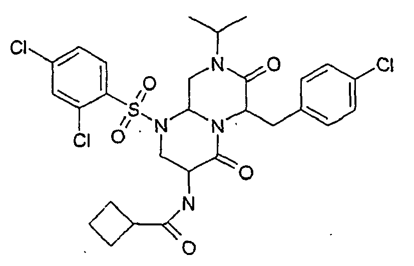

Die Synthese erfolgte analog zu Beispiel 22 unter Verwendung von Cyclobutancarbonylchlorid und 3-Amino-6-(4-chlor-benzyl)-1-(2,4-dichlor-benzolsulfonyl)-8-isopropyl-hexahydropyrazino[1,2-a]pyrimidin-4,7-dion. Man erhält das gewünschte Produkt mit MG = 640.11 (berechnet, monoisotop); Messwert (M+H)⁺: 641.09

### Beispiel 31

### N-[6-(4-chlor-benzyl)-1-(2,4-dichlor-benzolsulfonyl)-8- isopropyl-4,7-dioxo-octahydro-pyrazino[1,2-a]pyrimidin-3-yl]-cyclopentancarbonsäureamid

### Struktur:

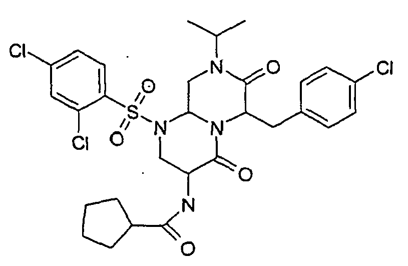

Die Synthese erfolgte analog zu Beispiel 22 unter Verwendung von Cyclopentancarbonylchlorid und 3-Amino-6-(4-chlor-benzyl)-1-(2,4-dichlor-benzolsulfonyl)-8-isopropyl-hexahydro- pyrazino[1,2-a]pyrimidin-4,7-dion. Man erhält das gewünschte Produkt mit MG = 654,12 (berechnet, monoisotop); Messwert (M+H)⁺: 655.1

### Beispiel 32

### Cyclohexanearbonsäure-[6-(4-chlor-benzyl)-1-(2,4-dichlor-benzolsulfonyl)-8-isopropyl-4,7-dioxo-octahydro-pyrazino[1,2-a]pyrimidin-3-yl]-amid

### Struktur:

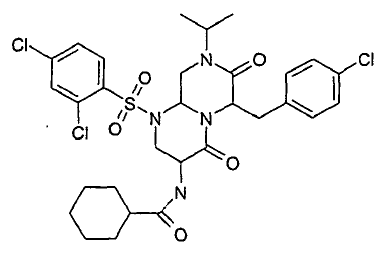

Die Synthese erfolgte analog zu Beispiel 22 unter Verwendung von Cyclohexancarbonylchlorid und 3-Amino-6-(4-chlor-benzyl)-1-(2,4-dichlor-benzolsulfonyl)-8-isopropyl-hexahydropyrazino[1,2-a]pyrimidin-4,7-dion.. Man erhält das gewünschte Produkt mit MG = 668,14 (berechnet, monoisotop); Messwert (M+H)⁺: 669.11

### Beispiel 33

### N-[6-(4-Chlor-benzyl)-1-(2,4-dichlor-benzolsulfonyl)-8-isopropyl-4,7-dioxo-octahydro-pyrazino[1,2-a]pyrimidin-3-yl]-2,2,2-trifluor-acetamid

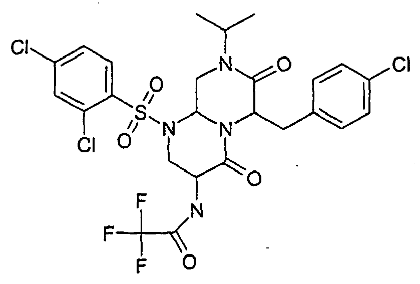

40 mg (0,071 mmol) 3-Amino-6-(4-chlorbenzyl)-1-(2,4-dichlorbenzolsulfonyl)-8-isopropylhexahydropyrazino[1,2-a]pyrimidin-4,7-dion wurden in 1,5 mL DCM gelöst und mit 0,024 mL (0,17 mmol) Trifluoressigsäureanhydrid und 0,075 mL (0,43 mmol) DIEA versetzt. Das Reaktionsgemisch wurde 1 Stunde lang bei 50°C gerührt. Nach Zugabe von 1 mL Wasser wurde die organische Phase isoliert, getrocknet (MgSO₄) und im Vakuum eingeengt. Die Reinigung mittels Flash-Chromatographie an 4 g SiO₂ (Elution mit MeOH/DCM, Gradient 0 - 4 %) ergab 21 mg Substanz. MG (berechnet, monoisotop) = 654,05; MG (Meßwert) = 655,06.

### Beispiel 34

### Cyclopropancarbonsäure [6-(4-chlor-benzyl)-1-(2,4-dichlor-benzolsulfonyl)-8- isopropyl-4,7-dioxo-octahydro-pyrazino[1,2-a]pyrimidin-3-yl]-isopropyl-amid

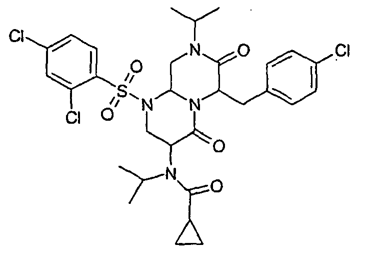

0,2 g (0,36 mmol) 3-Amino-6-(4-chlorbenzyl)-1-(2,4-dichlorbenzolsulfonyl)-8-isopropylhexahydropyrazino[1,2-a]pyrimidin-4,7-dion wurden in 12 mL MeOH gelöst und mit 1,6 mL Aceton und anschließend mit 0,8 mL CH₃COOH und 4 mL NaCNBH₃ (1,0M in THF) versetzt. Nach 1 Stunde wurde im Vakuum eingeengt, mit 5 mL EtOAc versetzt und mit 5 mL Wasser gewaschen. Die organische Phase wurde isoliert, getrocknet (MSO₄) und im Vakuum eingeengt. Eine Flash-Chromatographie der Rohsubstanz an 20 g SiO₂ (Elution mit EtOAc/DCM, Gradient 0 - 50 %) ergab 182 mg des Zwischenprodukts als Öl, das für den darauffolgenden Kopplungsschritt verwendet wurde. 30 mg (0,05 mmol) des Zwischenprodukts wurden in 1 mL DCM mit 0,026 mL (0,149 mmol) DIEA und 0,0055 mL (0,0598 mmol) Cyclopropancarbonylchlorid versetzt und über Nacht bei Raumtemperatur gerührt. Nach Zugabe von 2 mL Wasser wurde die organische Phase isoliert, getrocknet (MgSO₄) und im Vakuum eingeengt. Die Rohsubstanz wurde an 4 g SiO₂ chromatographiert (Elution mit MeOH/DCM, Gradient 0 - 5 %). Man erhielt 21,4 mg der gewünschten Verbindung. MG (berechnet, monoisotop) = 668.14; MG (Meßwert) (M⁺H) = 669.

### Beispiel 35

### Cyclopentancarbonsäure [6-(4-chlor-benzyl)-1-(2,4-dichlor-benzolsulfonyl)-8- isopropyl-4,7-dioxo-octahydro-pyrazino[1,2-a]pyrimidin-3-yl]-cyclohexyl-amid

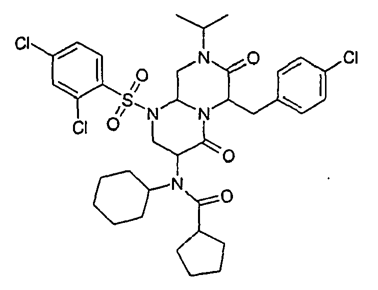

Beispiel 35 wurde analog dem Beispiel 34 unter Verwendung von Cyclohexanon bei der reduktiven Aminierung synthetisiert. Zur Amidknüpfung wurde Cyclopentancarbonylchlorid verwendet. Man erhält das gewünschte Produkts mit MG=736.20 (berechnet, monoisotop), Messwert: (M+H)⁺ = 737.15

### Beispiel 36

### 1-Methyl-piperidin-4-carbonsäure [6-(4-chlor-benzyl)-1-(2,4-dichlor- benzolsulfonyl)-8-isopropyl-4,7-dioxo-octahydro-pyrazino[1,2-a]pyrimidin-3-yl]- amid

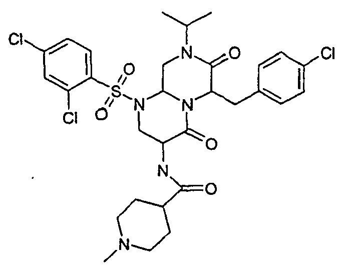

Die Synthese erfolgte analog zu Beispiel 29e unter Verwendung von 1-Methyl-piperidin-4-carbonsäure hydrochlorid. Man erhält das gewünschte Produkt mit MG = 683,15 (berechnet, monoisotop); Messwert (M+H)⁺: 684.38

### Beispiel 37

### Piperidin-4-carbonsäure [6-(4-chlor-benzyl)-1-(2,4-dichlor-benzolsulfonyl)-8-isopropyl-4,7-dioxo-octahydro-pyrazino[1,2-a]pyrimidin-3-yl]- amid

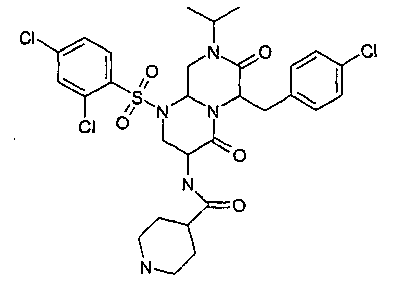

Ein Gemisch aus 100 mg (0,179 mmol) 3-Amino-6-(4-chlorbenzyl)-1-(2,4-dichlorbenzolsulfonyl)-8-isopropylhexahydropyrazino[1,2-a]pyrimidin-4,7-dion, 2 mL DCM, 23 mg (0,178 mmol) DIEA und 51 mg (0,181 mmol) Benzyl-4-(chlorcarbonyl)tetrahydro-1(2H)-pyridincarboxylat wurde bei Raumtemperatur 1 Stunde lang gerührt. Das Gemisch wurde einer Flash-Chromatographie an 2 g SiO₂ unterzogen (Elution mit EtOAc/DCM, Gradient 0 - 20 %). Man erhielt 119 mg des Zwischenprodukts 4-[6-(4-Chlorbenzyl)-1-(2,4-dichlorbenzolsulfonyl)-8-isopropyl-4,7-dioxooctahydropyrazino[1,2-a]pyrimidin-3-yl-carbamoyl]-piperidin-1-carbonsäurebenzylester. Die bei der LC/MS erzielten Ergebnisse (erwartetes, monoisotopes MG = 803, Meßwert (M⁺H) = 804) stimmten mit der Struktur überein.

Einer Lösung von 104 mg (0,129 mmol) 4-[6-(4-Chlorbenzyl)-1-(2,4-dichlorbenzolsulfonyl)-8-isopropyl-4,7-dioxooctahydropyrazino[1,2-a]pyrimidm-3-ylcarbamoyl]-piperidin-1-carbonsäurebenzylester in 2,5 mL Acetonitril wurden bei Raumtemperatur unter Rühren 103 mg (0,517 mmol) Iodtrimethylsilan zugesetzt. Nach 2 Stunden wurde das Gemisch eingeengt. Der Rückstand wurde auf einer Ionenaustauscherpatrone Varian Bond-Elut SCX gereinigt (Elution mit Methanol, anschließend mit 3N-NH₃ in Methanol). Das gereinigte Amin wurde in das Chlorhydratsalz umgewandelt. Man erhielt 75 mg der gewünschten Verbindung. LC/MS (erwartetes, monoisotopes MG = 669.13; Meßwert (M⁺H) = 670)

### Beispiel 38

### Morpholin-4-carbonsäure [6-(4-chlor-benzyl)-1-(2,4-dichlor-benzolsulfonyl)-8- isopropyl-4,7-dioxo-octahydro-pyrazino[1,2-a]pyrimidin-3-yl]-amid

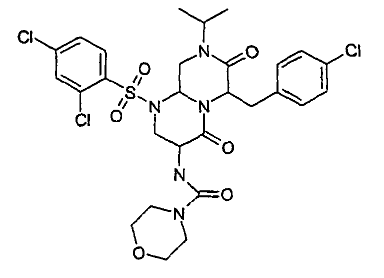

Eine Lösung von 50 mg (0,089 mmol) des Amins 3-Amino-6-(4-chlorbenzyl)-1-(2,4-dichlorbenzolsulfonyl)-8-isopropylhexahydropyrazino[1,2-a]pyrimidin-4,7-dion in 1,8 mL DCM/THF (1 : 1) wurde mit 47 µl (0,268 mmol) Hünig-Base versetzt. Dem Reaktionsgemisch wurden dann 80 mg (0,268 mmol) Triphosgen langsam zugesetzt. Das Gemisch wurde über Nacht bei Raumtemperatur gerührt. Dann wurde das Reaktionsmischung mit EtOAc verdünnt, mit H₂O gewaschen, über MgSO₄ getrocknet und im Vakuum eingeengt. Dieses Zwischenprodukt wurde in 1 mL THF gelöst und mit 31 µl (0,178 mmol) Hünig-Base sowie 12 mg (0,134 mmol) Morpholin versetzt. Das Reaktionsgemisch wurde bei Raumtemperatur über Nacht gerührt, mit EtOAc verdünnt, mit H₂O gewaschen, über MgSO₄ getrocknet und eingeengt. Der Rückstand wurde an 4 g SiO₂ chromatographiert (Elution mit MeOH/DCM, Gradient 1 - 8 %). Man erhielt 36 mg der gewünschten Substanz als farblosen Feststoff. LC/MS (erwartetes MG = 671.11; Meßwert (M⁺H) = 672).

### Beispiel 39

### Piperidin-2-carbonsäure [6-(4-chlor-benzyl)-1-(2,4-dichlor-benzolsulfonyl)-8- isopropyl-4,7-dioxo-octahydro-pyrazino[1,2-a]pyrimidin-3-yl]-amid

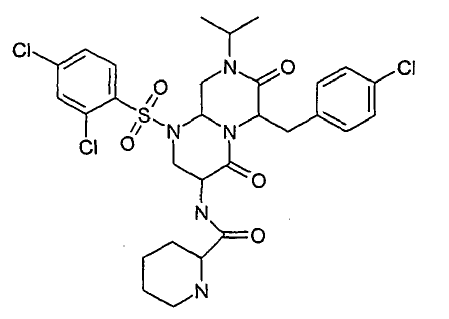

Eine Lösung von 0,05 g (0,089 mmol) 3-Amino-6-(4-chlorbenzyl)-1-(2,4-dichlorbenzolsulfonyl)-8-isopropylhexahydropyrazino[1,2-a]pyrimidin-4,7-dion in 2 mL DMF wurde mit 24,7 mg (0,089 mmol) 4-(4,6-Dimethoxy[1,3,5]triazin-2-yl)-4-methylmorpholinchlorid x H₂O (DMTMM) und anschließend mit 31,3 mg (0,089 mmol) Fmoc-Pip-OH versetzt. Das Reaktionsgemisch wurde über Nacht bei Raumtemperatur gerührt. Dann wurden 8 mL Diethylether und 5 mL Wasser zugegeben. Die organische Phase wurde isoliert, erneut mit 5 mL Wasser gewaschen, getrocknet (MgSO₄) und im Vakuum eingeengt. Die Reinigung erfolgte mittels Flash-Chromatographie an 4 g SiO₂ (MeOH/DCM als Eluentensystem, Gradient 0 - 2 %) und ergab 82 mg des FMOC-geschützten Zwischenprodukts in Form eines weißen Feststoffs. Mittels LC/MS konnte gezeigt werden, daß es sich um das gewünschte Zwischenprodukt handelte. Eine Lösung von 70 mg (0,078 mmol) des FMOC-Zwischenprodukts in 2 mL 10%igem Diethylamin in DCM wurde über Nacht bei Raumtemperatur gerührt. Das Reaktionsgemisch wurde im Vakuum eingeengt und auf der Gilson-Apparatur C18 mittels präparativer HPLC gereinigt (CH₃CN (0,1 % TFA)/H₂O (0,1 % TFA), Gradient 5 - 100 %). Die gewünschten Fraktionen wurden im Vakuum eingeengt. Man erhielt 33,9 mg des entsprechenden TFA-Salzes der gewünschten Verbindung. LC/MS MG (berechnet, monoisotop) = 669.13; Meßwert (M⁺H) = 670,11.

### Beispiel 40

### Pyrrolidin-2-carbonsäure [6-(4-chlor-benzyl)-1-(2,4-dichlor-benzolsulfonyl)-8- isopropyl-4,7-dioxo-octahydro-pyrazino[1,2-a]pyrimidin-3-yl]-amid

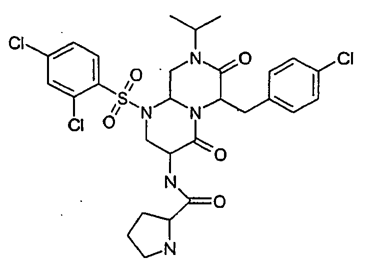

Ein Gemisch aus 50 mg (0,089 mmol) 3-Amino-6-(4-chlorbenzyl)-1-(2,4-dichlorbenzolsulfonyl)-8-isopropylhexahydropyrazino[1,2-a]pyrimidin-4,7-dion, 1 mL DCM, 23 mg (0,178 mmol) DIEA und 32 mg (0,090 mol) Fmoc-Pro-Cl wurde bei Raumtemperatur 1 Stunde lang gerührt. Das Gemisch wurde an 2 g SiO₂ chromatographisch gereinigt (Elution mit EtOAc/DCM, Gradient 0 - 20 %). Man erhielt 72 mg des Zwischenprodukts 2-[6-(4-Chlorbenzyl)-1-(2,4-dichlorbenzolsulfonyl)-8-isopropyl-4,7-dioxooctahydropyrazino[1,2-a]pyrimidin-3-ylcarbamoyl]-pyrrolidin-1-carbonsäure-9H-fluoren-9-ylmethylester.
LC/MS:(berechnet, monoisotop MG = 877, Meßwert (M⁺H) = 878)

Ein Gemisch aus 60 mg (0,068 mmol) 2-[6-(4-Chlorbenzyl)-1-(2,4-dichlorbenzolsulfonyl)-8-isopropyl-4,7-dioxooctahydropyrazino[1,2-a]pyrimidin-3-ylcarbamoyl]-pyrrolidin-1-carbonsäure-9H-fluoren-9-ylmethylester und 2 mL 15%iges Diethylamin in DCM wurde über Nacht bei Raumtemperatur gerührt. Das Gemisch wurde eingeengt und einer Flash-Chromatographie an 2 g SiO₂ unterzogen (Elution mit DCM, EtOAc und anschließend mit 10 % Methanol/EtOAc). Das Amin wurde in das Chlorhydratsalz umgewandelt. Man erhielt 30 mg Feststoff. Dieser wurde mittels präparativer Reverse-Phase(RP) HPLC weiter gereinigt. Man erhielt 17 mg der gewünschten Verbindung. LC/MS (berechnet, monoisotop MG = 655.12; Meßwert (M⁺H) = 656)

### Beispiel 41

### 1-Methyl-pyrrolidin-2-carbonsäure [6-(4-chlor-benzyl)-1-(2,4-dichlor- benzolsulfonyl)-8-isopropyl-4,7-dioxo-octahydro-pyrazino[1,2-a]pyrimidin-3-yl]- amid

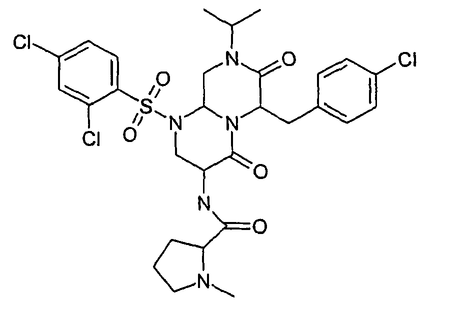

Einer Lösung von 93 mg (0,142 mmol) des Amins aus Beispiel 40 in 7,5 mL Methanol wurden 0,3 mL (5,2 mmol) Essigsäure, 0,25 mL (3,33 mmol) Formaldehyd (37 % in Wasser) und 1,5 mL (1,5 mmol) Natriumcyanborhydrid (1M in THF) zugesetzt. Das Reaktionsgemisch wurde 2 Stunden lang bei Raumtemperatur gerührt und im Vakuum eingeengt. Der Rückstand wurde mit 10 mL D CM verdünnt, mit 7N-NH₃ in Methanol basisch gestellt, filtriert und eingeengt. Dieser Rückstand wurde durch präparative RP-HPLC gereinigt. Man erhielt 50 mg der gewünschten Verbindung. LC/MS (berechnet, monoisotop MG = 669; Meßwert (M⁺H) = 670)

### Beispiel 42

### Azetidin-3-carbonsäure [6-(4-chlor-benzyl)-1-(2,4-dichlor-benzolsulfonyl)-8- isopropyl-4,7-dioxo-octahydro-pyrazino[1,2-a]pyrimidin-3-yl]-amid

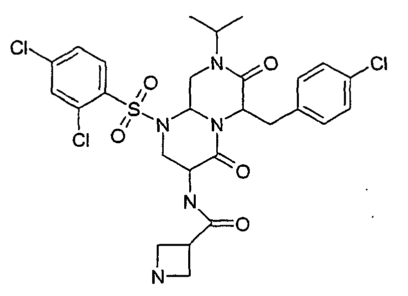

Eine Lösung von 0,1 g (0,179 mmol) des Amins 3-Amino-6-(4-chlorbenzyl)-1-(2,4-dichlorbenzolsulfonyl)-8-isopropylhexahydropyrazino[1,2-a]pyrimidin-4,7-dion in 5 mL DMF wurde mit 49,5 mg (0,179 mmol) 4-(4,6-Dimethoxy[1,3,5]triazin-2-yl)-4-methylmorpholinchlorid x H₂O (DMTMM) und anschließend mit 58 mg (0,179 mmol) 1-Fmoc-azetidin-3-carbonsäure versetzt. Das Reaktionsgemisch wurde über Nacht bei Raumtemperatur gerührt. Dann wurden 50 mL Diethylether und 50 mL Wasser zugegeben. Die organische Phase wurde isoliert, 2mal mit je 50 mL Wasser gewaschen, getrocknet (MgSO₄) und im Vakuum eingeengt. Die Reinigung mittels Flash-Chromatographie an 20 g SiO₂ (MeOH/DCM als Eluentensystem, Gradient 0 - 10 %) ergab 117 mg des FMOC-geschützten Zwischenprodukts in Form eines weißen Feststoffs. Dieser wurde im nächsten Schritt verwendet. Eine Lösung von 50 mg (0,058 mmol) des FMOC-Zwischenprodukts in 1,5 mL 10%igem Diethylamin in DCM wurde über Nacht bei Raumtemperatur gerührt. Das Reaktionsgemisch wurde im Vakuum eingeengt und auf der Gilson-Apparatur C18 mittels präparativer HPLC gereinigt (CH₃CN (0,1 % TFA)/H₂O (0,1 % TFA)). Die Fraktionen wurden im Vakuum eingeengt. Man erhält 21,6 mg des TFA-Salzes des gewünschten Produktes. MG (berechnet, monoisotop) = 641.11; Meßwert (M⁺H) = 641,99.

### Beispiel 43

### N-[6-(4-Chlor-benzyl)-1-(2,4-dichlor-benzolsulfonyl)-8-isopropyl-4,7-dioxo-octahydro-pyrazino[1,2-a]pyrimidin-3-yl]-N',N'-dimethyl-succinamide

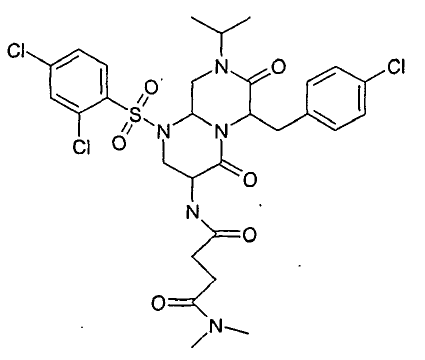

Die Synthese erfolgte analog zu Beispiel 29e unter Verwendung von N,N-Dimethylsuccinamidsäure. Man erhält das gewünschte Produkt mit MG = 685.13 (berechnet, monoisotop); Messwert (M+H)⁺: 686.30

### Beispiel 44

### Cyclohexancarbonsäure [6-(4-chlor-benzyl)-1-(2,4-dichlor-benzolsulfonyl)-8- isopropyl-4,7-dioxo-octahydro-pyrazino[1,2-a]pyrimidin-3-yl]-isopropyl-amid

### Struktur:

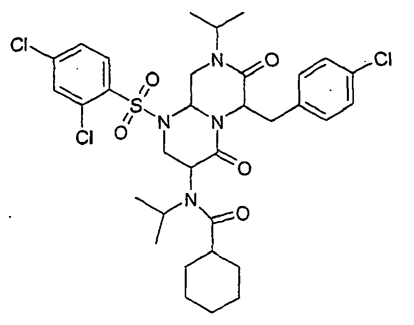

Beispiel 44 wurde analog zu Beispiel 34 unter Verwendung von Cyclohexancarbonyl chlorid im Amidknüpfungsschritt synthetisiert. Man erhält das gewünschte Produkt mit MG = 710.19 (berechnet, monoisotop); Messwert (M+H)⁺: 711.18

### Beispiel 45

### Cyclopentancarbonsäure [6-(4-chlor-benzyl)-1-(2,4-dichlor-benzolsulfonyl)-8- isopropyl-4,7-dioxo-octahydro-pyrazino[1,2-a]pyrimidm-3-yl]-isopropyl-amid

### Struktur:

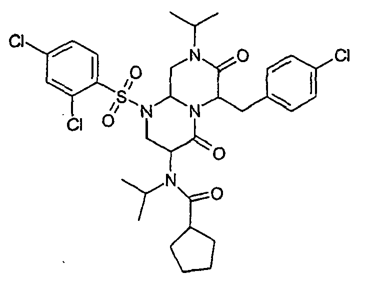

Beispiel 45 wurde analog zu Beispiel 34 unter Verwendung von Cyclopentan carbonylchlorid im Amidknüpfungsschritt synthetisiert. Man erhält das gewünschte Produkt mit MG = 696.17 (berechnet, monoisotop); Messwert (M+H)⁺: 697.17

### Beispiel 46

### Piperidin-4-carbonsäure [6-(4-chlor-benzyl)-1-(2,4-dichlor-benzolsulfonyl)-8- isopropyl-4,7-dioxo-octahydro-pyrazino[1,2-a]pyrimidin-3-yl]-isopropyl-amid

### Struktur:

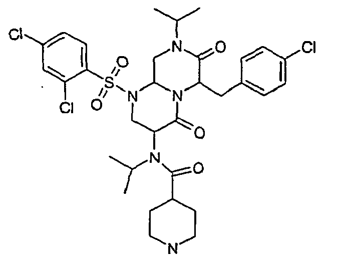

Im Beispiel 46 erfolgte die Synthese nach den unter 34 beschriebenen Verfahren. 4-Chlorcarbonylpiperidin-1-carbonsäurebenzylester kam im Kupplungsschritt zur Anwendung und ergab die Cbz-geschützte Verbindung. Zur Abspaltung der Cbz-Schutzgruppe wurden bei 0°C 0,0283 mL (0,199 mmol) TMSI und 2 mL CH₃CN zugesetzt. Das Reak-tionsgemisch wurde 3 Tage lang gerührt, im Vakuum eingeengt und durch eine mit 1 g SCX gepackte und mit MeOH angefeuchtete Säule eluiert. Die Verunreinigungen wurden mit MeOH eluiert. Durch Elution mit 2N-NH₃ in MeOH wurde das gewünschte Amin gewonnen. Dann wurde im Vakuum eingeengt und mit 0,1 mL 1,0M-HCl in Diethylether versetzt. Das dabei entstandene Salz wurde zermahlen, 4mal mit je 2 mL Diethylether gewaschen und eingeengt. Man erhielt 0,025 g der gewünschten Verbindung.. LC/MS: MG (berechnet, monoisotop) = 711.18; Meßwert (M⁺H) = 712,11.

### Beispiel 47

### N-[6-(4-Chlor-benzyl)-1-(2,4-dichlor-benzolsulfonyl)-8-isopropyl-4,7-dioxo-octahydro-pyrazino [1,2-a]pyrimidin-3-yl]-4-dimethylamino-benzamid

### Struktur:

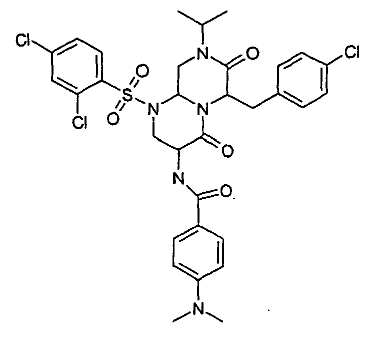

Die Synthese erfolgte analog zu Beispiel 29e unter Verwendung von 4-Dimethylaminobenzoesäure. Man erhält das gewünschte Produkt mit MG = 705.13 (berechnet, monoisotop); Messwert (M+H)⁺: 706.21

### Beispiel 48

### N-[6-(4-Chlor-benzyl)-1-(2,4-dichlor-benzolsulfonyl)-8-isopropyl-4,7-dioxo-octahydro-pyrazino[1,2-a]pyrimidin-3-yl]-2-pyridin-3-yl-acetamid

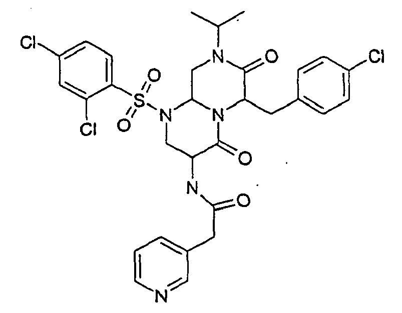

Die Synthese erfolgte analog zu Beispiel 29e unter Verwendung von 3-Pyridylessigsäure. Man erhält das gewünschte Produkt mit MG = 677.10 (berechnet, monoisotop); Messwert (M+H)⁺: 678.07

### Beispiel 49

### Pyridin-2-carbonsäure [6-(4-chlor-benzyl)-1-(2,4-dichlor-benzolsulfonyl)-8- isopropyl-4,7-dioxo-octahydro-pyrazino[1,2-a]pyrimidin-3-yl]-amid

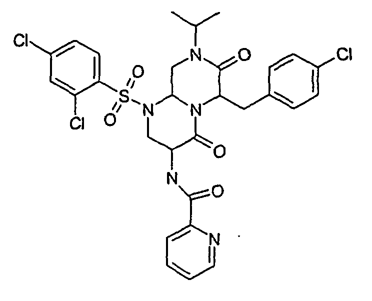

Die Synthese erfolgte analog zu Beispiel 29e unter Verwendung von Picolinsäure. Man erhält das gewünschte Produkt mit MG = 663.09 (berechnet, monoisotop); Messwert (M+H)⁺: 664.22

### Beispiel 50

### N-[6-(4-Chlor-benzyl)-1-(2,4-dichlor-benzolsulfonyl)-8-isopropyl-4,7-dioxo-octahydro-pyrazino[1,2-a]pyrimidin-3-yl]-nicotinamid

### Struktur:

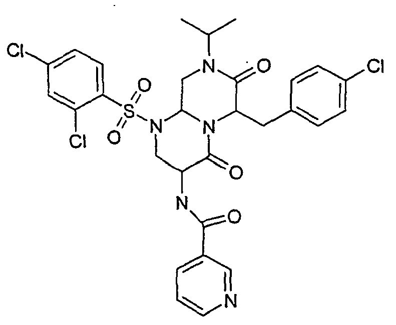

Die Synthese erfolgte analog zu Beispiel 22 unter Verwendung von Nicotinoylchlorid *HCl und 3-Amino-6-(4-chlor-benzyl)-1-(2,4-dichlor-benzolsulfonyl)-8-isopropyl-hexahydropyrazino[1,2-a]pyrimidin-4,7-dion. Man erhält das gewünschte Produkt mit MG = 663.09 (berechnet, monoisotop); Messwert (M+H)⁺: 664.11

### Beispiel 51

### N-[6-(4-Chlor-benzyl)-1-(2,4-dichlor-benzolsulfonyl)-8-isopropyl-4,7-dioxo-octahydro-pyrazino[1,2-a]pyrimidin-3-yl]-6-trifluormethyl-nicotinamid

### Struktur:

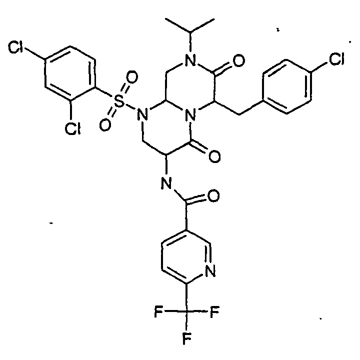

Die Synthese erfolgte analog zu Beispiel 22 unter Verwendung von 6-(Trifluormethyl)-nicotinoylchlorid und 3-Amino-6-(4-chlor-benzyl)-1-(2,4-dichlor-benzolsulfonyl)-8-isopropylhexahydro- pyrazino[1,2-a]pyrimidin-4,7-dion. Man erhält das gewünschte Produkt mit MG = 731.08 (berechnet, monoisotop); Messwert (M+H)⁺: 732.01

### Beispiel 52

### N-[6-(4-Chlor-benzyl)-1-(2,4-dichlor-benzolsulfonyl)-8-isopropyl-4,7-dioxo-octahydro-pyrazino[1,2-a]pyrimidin-3-yl]-4-trifluormethyl-nicotinamid

### Struktur:

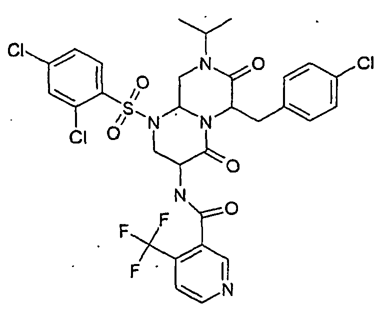

Die Synthese erfolgte analog zu Beispiel 29e unter Verwendung von 4-(Trifluormethyl)-nicotinsäure und 3-Amino-6-(4-chlor-benzyl)-1-(2,4-dichlor-benzolsulfonyl)-8-isopropylhexahydro- pyrazino[1,2-a]pyrimidin-4,7-dion. Man erhält das gewünschte Produkt mit MG = 731.08 (berechnet, monoisotop); Messwert (M+H)⁺: 732.07

### Beispiel 53

### 5-Methyl-pyrazin-2-carbonsäure [6-(4-chlor-benzyl)-1-(2,4-dichlor-benzolsulfonyl)- 8-isopropyl-4,7-dioxo-octahydro-pyrazino[1,2-a]pyrimidin-3-yl]-amid

### Struktur:

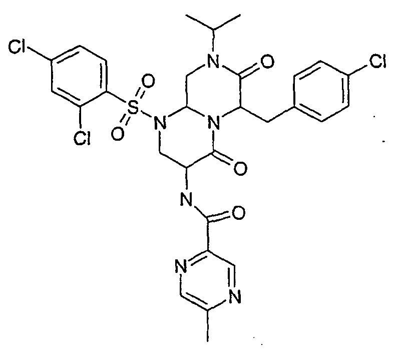

Die Synthese erfolgte analog zu Beispiel 29e unter Verwendung von 5-Methylpyrazin-2-carbonsäure und 3-Amino-6-(4-chlor-benzyl)-1-(2,4-dichlor-benzolsulfonyl)-8-isopropylhexahydro- pyrazino[1,2-a]pyrimidin-4,7-dion. Man erhält das gewünschte Produkt mit MG = 678.10 (berechnet, monoisotop); Messwert (M+H)⁺: 679.06

### Beispiel 54

### Pyrazin-2-carbonsäure [6-(4-chlor-benzyl)-1-(2,4-dichlor-benzolsulfonyl)- 8-isopropyl-4,7-dioxo-octahydro-pyrazino[1,2-a]pyrimidin-3-yl]-amid

### Struktur:

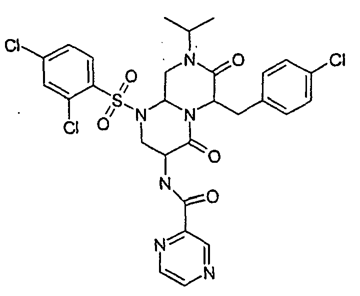

Die Synthese erfolgte analog zu Beispiel 22 unter Verwendung von Pyrazin-2-carbonylchlorid und 3-Amino-6-(4-chlor-benzyl)-1-(2,4-dichlor-benzolsulfonyl)-8-isopropyl-hexahydropyrazino[1,2-a]pyrimidin-4,7-dion. Man erhält das gewünschte Produkt mit MG = 664.08 (berechnet, monoisotop); Messwert (M+H)⁺: 665.05

### Beispiel 55

### Quinolin-3-carbonsäure [6-(4-chlor-benzyl)-1-(2,4-dichlor-benzolsulfonyl)-8-isopropyl-4,7-dioxo-octahydro-pyrazino[1,2-a]pyrimidin-3-yl]-amid

### Struktur:

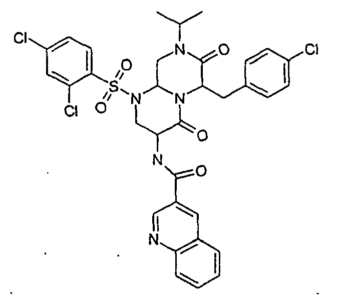

Die Synthese erfolgte analog zu Beispiel 29e unter Verwendung von 3-Quinolincarbonsäure und 3-Amino-6-(4-chlor-benzyl)-1-(2,4-dichlor-benzolsulfonyl)-8-isopropyl-hexahydropyrazino[1,2-a]pyrimidin-4,7-dion. Man erhält das gewünschte Produkt mit MG = 713.10 (berechnet, monoisotop); Messwert (M+H)⁺:714

### Beispiel 56

### 1-tert-Butyl-3-[6-(4-chlor-benzyl)-1-(2,4-dichlor-benzolsulfonyl)-8-isopropyl-4,7- dioxo-octahydro-pyrazino[1,2-a]pyrimidin-3-yl]-harnstoff

### Struktur:

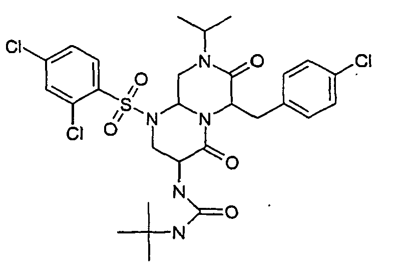

Die Synthese erfolgt analog Bsp. 28 ausgehend von 3-Amino-6-(4-chlor-benzyl)-1-(2,4-dichlor-benzolsulfonyl)-8-isopropyl-hexahydro- pyrazino[1,2-a]pyrimidin-4,7-dion. Man erhält das gewünschte Produkt mit MG = 657.13 (berechnet, monoisotop); Meßwert (M+H)⁺: 658.26

### Beispiel 57

### 1-Ethyl-3-[6-(4-chlor-benzyl)-1-(2,4-dichlor-benzolsulfonyl)-8-isopropyl-4,7- dioxo-octahydro-pyrazino[1,2-a]pyrimidin-3-yl]-harnstoff

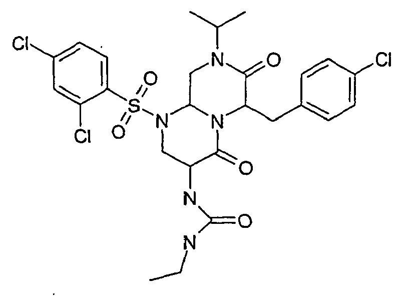

Die Synthese erfolgte analog zu Beispiel 56 unter Verwendung von Ethylisocyanat. Man erhält das gewünschte Produkt mit MG = 629.10 (berechnet, monoisotop); Messwert (M+H)⁺: 630.13

### Beispiel 58

### Cyclopropansulfonsäure [6-(4-chlor-benzyl)-1-(2,4-dichlor-benzolsulfonyl)-8- isopropyl-4,7-dioxo-octahydro-pyrazino[1,2-a]pyrimidin-3-yl]-amid

### Struktur:

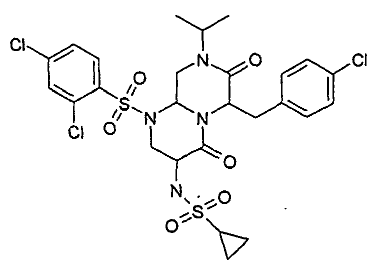

Die Synthese erfolgt analog Bsp. 27 ausgehend von 3-Amino-6-(4-chlor-benzyl)-1-(2,4-dichlor-benzolsulfonyl)-8-isopropyl-hexahydro- pyrazino[1,2-a]pyrimidin-4,7-dion. Man erhält das gewünschte Produkt mit MG = 662.06 (berechnet, monoisotop); Meßwert (M+H)⁺: 663.05.

### Beispiel 59

### 6-(4-Chlor-benzyl)-1-(2,4-dichlor-benzolsulfonyl)-8-isopropyl-3-piperidin-1-yl-hexahydropyrazino[1,2-a]pyrimidin-4,7-dion

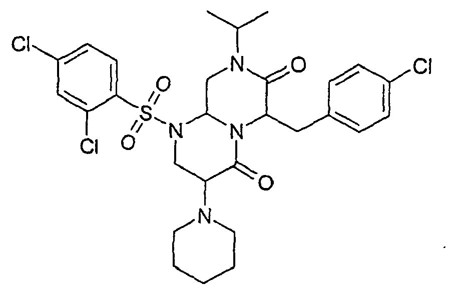

Ein Gemisch aus 50 mg (0,089 mmol) 3-Amino-6-(4-chlorbenzyl)-1-(2,4-dichlorbenzolsulfonyl)-8-isopropylhexahydropyrazino[1,2-a]pyrimidin-4,7-dion, 2,0 mL Acetonitril, 0,2 mL (0,2 mmol) 1M (H₂O)-NaHCO₃ und 83 mg (0,361 mmol) 1,5-Dibrompropan wurde im Mikrowellenofen 800 Sekunden lang bei 175°C unter Rühren erwärmt. Nach dem Erkalten wurde das Gemisch eingeengt. Der Rückstand wurde einer Flash-Chromatographie an 2 g SiO₂ unterzogen (Elution mit EtOAc/DCM, Gradient 0 - 20 %). Das gereinigte Amin wurde in das Chlorhydratsalz umgewandelt. Man erhielt 38 mg der gewünschten Verbindung: LC/MS: (MG berechnet, monoisotop = 626.13; Meßwert M+H) = 627.15)

### Beispiel 60

### 6-(4-Chlor-benzyl)-1-(2,4-dichlor-benzolsulfonyl)-8-isopropyl-3-morpholin-4-yl- hexahydro-pyrazino[1,2-a]pyrimidin-4,7-dion

### Struktur:

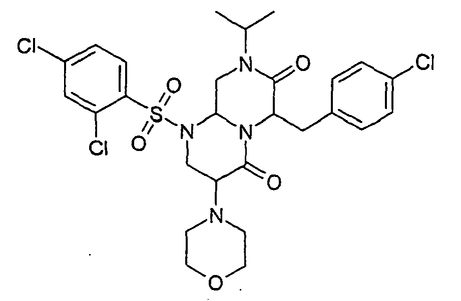

Einer Lösung von 90 mg (0,16 mmol) des Amins 3-Amino-6-(4-chlorbenzyl)-1-(2,4-dichlorbenzolsulfonyl)-8-isopropylhexahydropyrazino[1,2-a]pyrimidin-4,7-dion in 2 mL CH₃CN wurden 149 mg (0,64 mmol, 4,0 Äquiv.) 2-Bromethylether und danach 0,2 mL 1N-NaHCO₃-Lösung zugesetzt. Das Reaktionsgemisch wurde 120 Sekunden lang bei 200°C im Mikrowellenofen (Smith Creator) erwärmt. Nach dem Erkalten wurde mit DCM verdünnt und mit 1N-NaHCO₃ gewaschen. Die organische Phase wurde über MgSO₄ getrocknet und eingeengt. Der Rückstand wurde an 12 g SiO₂ chromatographiert (Elution mit MeOH/DCM, Gradient 0 - 10 %). Man erhielt 48 mg der gewünschten Substanz als weißen Feststoff. LC/MS (berechnet, monoisotopes MG = 628.18; Meßwert (M⁺H) = 629.1) stimmen mit der Struktur überein.

### Beispiel 61

### 6-(4-Chlor-benzyl)-1-(2,4-dichlor-benzolsulfonyl)-8-isopropyl-3-(4-methyl-piperazin-1-yl)-hexahydro-pyrazino[1,2-a]pyrimidin-4,7-dion

### Struktur:

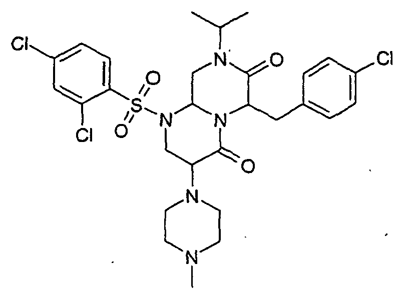

50 mg 3-Amino-6-(4-chlor-benzyl)-1-(2,4-dichlor-benzolsulfonyl)-8-isopropyl-hexahydro-pyrazino[1,2-a]pyrimidin-4,7-dion, 14,4 mg Mechlorethamin hydrochlorid, 13 mg NaHCO₃ werden in gerade soviel Ethylenglykol gelöst das eine Lösung erhalten wird. Zu dieser Lösung werden 1,6 mg NaI gegeben und die Lösung 6 h auf 110 °C erwärmt. Nach dem Abkühlen wird die Lösung mit Wasser versetzt und dreimal mit je 40 mL Essigester extrahiert. Die vereinigten organischen Phasen werden über Na₂SO₄ getrocknet und das Lösungsmittel wird im Vakuum entfernt. Der Rückstand wird über HPLC (Waters-Xterra™ MS C18, 5 µm, Wasser (0.1 % Trifluoressigsäure)/Acetonitril (0.1 % Trifluoressigsäure) = 80/20 -> 10/90) getrennt.
Man erhält das gewünschte Produkt mit MG = 641.14 (berechnet, monoisotop); Meßwert (M+H)⁺: 642.25

### Beispiel 62

### 3-(1-Benzyl-piperidin-4-ylamino)-6-(4-chlor-benzyl)-1-(2,4-dichlor-benzolsulfonyl)-8-isopropyl-hexahydro-pyrazino[1,2-a]pyrimidin-4,7-dion

### Struktur:

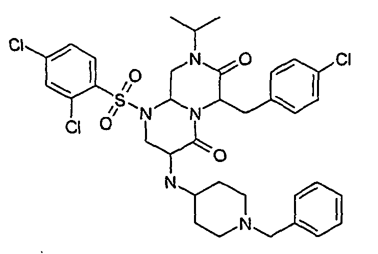

Zu einer Lösung von 100 mg (0,18 mmol) 3-Amino-6-(4-chlorbenzyl)-1-(2,4-dichlorbenzolsulfonyl)-8-isopropylhexahydropyrazino[1,2-a]pyrimidin-4,7-dion in 1, 2 mL 5 % HOAc-CH₃CN und 3 Tropfen MeOH wurden 115 mg (0,61 mmol) 1-Benzyl-4-piperidon und anschließend 130 mg (4,1 mmol/g) (Polystyrylmethyl)trimethylammonium-cyanoborhydrid gegeben. Das Reaktionsgemisch wurde 18 Stunden bei Raumtemperatur geschüttelt, filtriert und eingeengt. Man erhielt 160 mg beiges Öl. Das Rohprodukt wurde mittels Flash-Chromatographie in einer mit 4 g Kieselgel gepackten Säule isoliert (Elution mit 5 % MeOH/EtOAc). Nach Einengung der entsprechenden Fraktionen erhielt man 110 mg (85 %) der obigen Verbindung in Form eines klaren Öls. LC/MS (erwartetes, monoisotopes MG = 731.19; Meßwert (M⁺H) = 732,20)

### Beispiel 63

### 6-(4-Chlor-benzyl)-1-(2,4-dichlor-benzolsulfonyl)-8-isopropyl-3-(2-oxo-pyrrolidin-1-yl)-hexahydro-pyrazino[1,2-a]pyrimidin-4,7-dion

### Struktur:

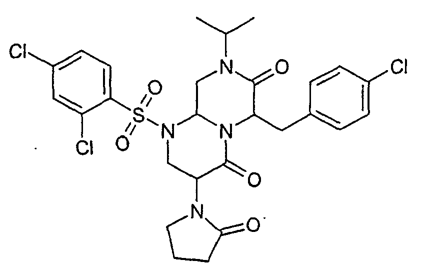

Eine Lösung von 50 mg (0,089 mmol) 3-Amino-6-(4-chlorbenzyl)-1-(2,4-dichlor-benzolsulfonyl)-8-isopropylhexahydropyrazino[1,2-a]pyrimidin-4,7-dion, 0,012 mL (0,107 mmol) 4-Chlorbutyrylchlorid und 0,031 mL DIEA in 2 mL DCM wurde über Nacht bei Raumtemperatur gerührt. Nach Zugabe von 2 mL Wasser wurde die organische Phase isoliert, mit 2 mL Kochsalzlösung gewaschen, getrocknet (MgSO₄) und im Vakuum eingeengt. Die Rohsubstanz wurde an 4 g SiO₂ chromatographiert (Elution mit MeOH/DCM, Gradient 0 - 6 %). Die eingeengten Fraktionen ergaben 45,8 mg Chlorbutyramid-Zwischenprodukt in Form eines weißen Feststoffs und wurden im folgenden Schritt verwendet. 45 mg (0,068 mmol) des in 2 mL Aceton gelösten Chloramid-Zwischenprodukts wurden mit 60 mg (0,36 mmol) Kaliumiodid und 277 mg (2 mmol) K₂CO₃ versetzt. Das Gemisch wurde im Mikrowellenofen 600 Sekunden lang auf 130°C erwärmt, danach im Vakuum eingeengt und mittels Flash-Chromatographie an 4 g SiO₂ gereinigt (Elution mit MeOH/DCM, Gradient 0 - 5 %). Man erhielt 16,2 mg weißen Feststoff. LC/MS stimmten mit der gewünschten Struktur überein. MG (berechnet, monoisotop) = 626.09 ; Meßwert (M⁺H) = 627,1.

### Beispiel 64

### 6-(4-Chlor-benzyl)-1-(2,4-dichlor-benzolsulfonyl)-8-isopropyl-3-(2-oxo-piperidin-1-yl)-hexahydro-pyrazino[1,2-a]pyrimidin-4,7-dion

### Struktur:

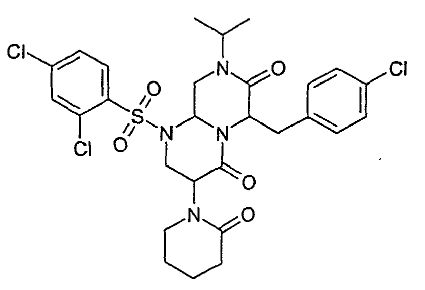

Beispiel 64 wurde analog zu Beispiel 63 unter der Verwendung von 5-Chlorpentanoyl chlorid im Amidknüpfungsschritt , synthetisiert. Man erhält das gewünschte Produkt mit MG = 640.11 (berechnet, monoisotop); Meßwert (M+H)⁺: 641.1

### Beispiel 65

### 6-(4-Chlor-benzyl)-1-(2,4-dichlor-benzolsulfonyl)-8-isopropyl-3-(2-oxo-azepan-1-yl)-hexahydro-pyrazino[1,2-a]pyrimidin-4,7-dion

### Struktur:

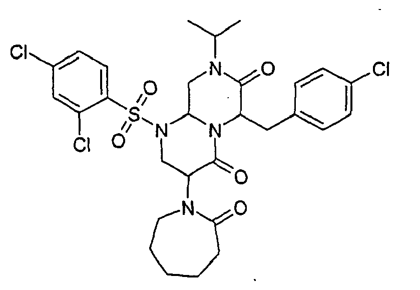

Beispiel 65 wurde analog zu Beispiel 63 unter der Verwendung von 6-Chlorhexanoyl chlorid im Amidknüpfungsschritt , synthetisiert. Man erhält das gewünschte Produkt mit MG = 654.12 (berechnet, monoisotop); Meßwert (M+H)⁺: 655.11.

### Beispiel 66

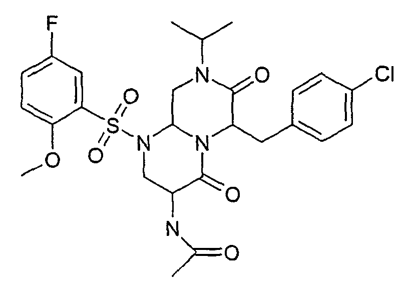

a) [6-(4-Chlor-benzyl)-1-(5-fluox-2-methoxy-benzolsulfonyl)-8-isopropyl-4,7-dioxo-octahydro-pyrazino [1,2-a]pyrimidin-3-yl]-carbaminsäurebenzylester
   Festphasenroute:
   7 g (3.64 mmol) Tentagel HL 12 019 (Rapp Polymer GMbH mit Bromacetallinker beladen) wird in einem Festphasenreaktor in 26 mL DMSO und 6 mL (70.44 mmol) Isopropylamin auf 60°C über Nacht erhitzt. Das Harz wird jeweils viermal mit DMSO, MeOH, DCM und Diethylether (30 mL jeweils) gewaschen und dann im Vakuum getrocknet. Für die Kupplung mit Fmoc(4-ClPhe)OH (4.6 g, 10.92 mmol) werden DIEA (5.7 mL, 32.76 mmol) und HATU (4.15g, 10.92 mmol) in 30 mL DMF zugegeben. Die Reaktionsmischung wird über 3 h auf 55°C erhitzt. Das Harz wird analog dem ersten Schritt gewaschen. Zum Abspalten der Fmoc Gruppe wird das Harz zweimal mit je 30 mL 20% Piperidin in DMF für jeweils 20 Minuten behandelt, danach wird es wie oben beschrieben gewaschen. Das Harz wird als nächstes mit Fmoc-ZDap(OH) (5.02g, 10.92 mmol), DIC (1.71 mL, 10.92 mmol) und HOBt (1.47 g, 10.92 mmol) in 30 mL DMF bei Raumtemperatur über Nacht umgesetzt. Es wurde analog der oben beschriebenen Prozedur gewaschen. Zu der Hälfte des Harzes (1.82 mmol) wurden 0.613 g (2.73 mmol) 5-Fluor-2-methoxy-benzolsulfonylchlorid(Butt Park 49/07-57), 0.95 mL (5.46 mmol) DIEA und 30 mL DCM gegeben. Die Reaktionslösung wurde über Nacht bei Raumtemperatur geschüttelt. Anschließend wurde das Harz wie oben beschrieben gewaschen. Danach wird das Harz mit 30 mL 99% HCOOH versetzt und über Nacht bei Raumtemperatur geschüttelt. Das Harz wird abdekantiert und zweimal mit je 20 mL HCOOH gewaschen. Die vereinigten Filtrate werden für 3 h auf 60°C erhitzt. Die Reaktionslösung wird im Vakuum aufkonzentriert und das Rohprodukt chromatographisch gereinigt (100g SiO₂, Eluent EtOAc /DCM (Gradient 0-50%)). Es werden 0.778 g des gewünschten Produktes als weißer Feststoff erhalten MG =672.18 (berechnet, monoisotop); Meßwert (M+H)⁺: 673.1
b) 3-Amino-6-(4-chlor-benzyl)-1-(5-fluor-2-methoxy-benzolsulfonyl)-8-isopropyl-hexahydropyrazino[1,2-a]pyrimidin-4,7-dion
   Die Synthese erfolgt analog zu Beispiel 22g (Methode B) ausgehend von [6-(4-Chlor-benzyl)-1-(5-fluor-2-methoxy-benzolsulfonyl)-8-isopropyl-4,7-dioxo-octahydro- pyrazino[1,2-a]pyrimidin-3-yl]-carbaminsäurebenzylester.
   Man erhält das gewünschte Produkt mit MG = 538.14 (berechnet, monoisotop); Meßwert (M+H)⁺: 539.14
c) N-[6-(4-chlor-benzyl)-1-(5-fluor-2-methoxy-benzolsulfonyl)-8- isopropyl-4,7-dioxo-octahydro-pyrazino[1,2-a]pyrimidin-3-yl]- acetamid.
   Die Synthese erfolgt analog Beispiel 21h) ausgehend von 3-Amino-6-(4-chlor-benzyl)-1-(5-fluor-2-methoxy-benzolsulfonyl)-8-isopropyl-hexahydro- pyrazino[1,2-a]pyrimidin-4,7-dion. Man erhält das gewünschte Produkt mit MG = 580.16 (berechnet, monoisotop); Meßwert (M+H)⁺: 581.13

### Beispiel 67

### Cyclopropancarbonsäure [6-(4-chlor-benzyl)-1-(5-fluor-2-methoxy-benzolsulfonyl)-8-isopropyl-4,7-dioxo-octahydro-pyrazino[1,2-a]pyrimidin-3-yl]-amid

### Struktur:

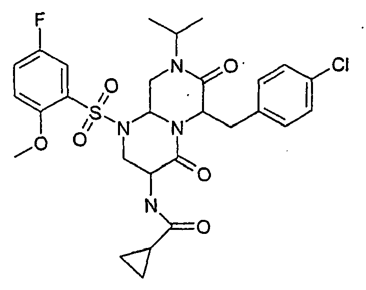

Die Synthese erfolgt analog Bsp. 22 ausgehend von 3-Amino-6-(4-chlor-benzyl)-1-(5-fluor-2-methoxy-benzolsulfonyl)-8-isopropyl-hexahydro- pyrazino[1,2-a]pyrimidin-4,7-dion. Man erhält das gewünschte Produkt mit MG = 606.17 (berechnet, monoisotop); Meßwert (M+H)⁺: 607.14

### Beispiel 68

### Cyclobutancarbonsäure [6-(4-chlor-benzyl)-1-(5-fluor-2-methoxy-benzolsulfonyl)-8- isopropyl-4,7-dioxo-octahydro-pyrazino[1,2-a]pyrimidin-3-yl]-amid

### Struktur:

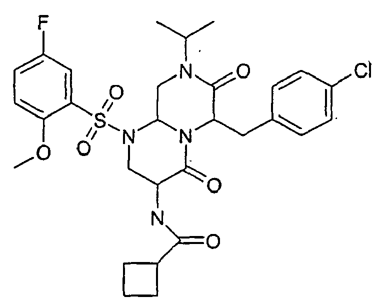

Die Synthese erfolgt analog Bsp. 30 ausgehend von 3-Amino-6-(4-chlor-benzyl)-1-(5-fluor-2-methoxy-benzolsulfonyl)-8-isopropyl-hexahydro- pyrazmo[1,2-a]pyrimidin-4,7-dion. Man erhält das gewünschte Produkt mit MG = 620.19 (berechnet, monoisotop); Meßwert (M+H)⁺: 621.16

### Beispiel 69

### Cyclopentancarbonsäure [6-(4-chlor-benzyl)-1-(5-fluor-2-methoxy-benzolsulfonyl)-8-isopropyl-4,7-dioxo-octahydro-pyrazino[1,2-a]pyrimidin-3-yl]-amid

### Struktur:

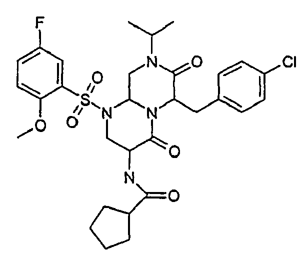

Die Synthese erfolgt analog Bsp. 31 ausgehend von 3-Amino-6-(4-chlor-benzyl)-1-(5-fluor-2-methoxy-benzolsulfonyl)-8-isopropyl-hexahydro- pyrazino[1,2-a]pyrimidin-4,7-dion. Man erhält das gewünschte Produkt mit MG = 634.20 (berechnet, monoisotop); Meßwert (M+H)⁺: 635.17

### Beispiel 70

### 1-tert-Butyl-3-[6-(4-chlor-benzyl)-1-(5-fluor-2-methoxy-benzolsulfonyl)-8-isopropyl- 4,7-dioxo-octahydro-pyrazino[1,2-a]pyrimidin-3-yl]-harnstoff

### Struktur:

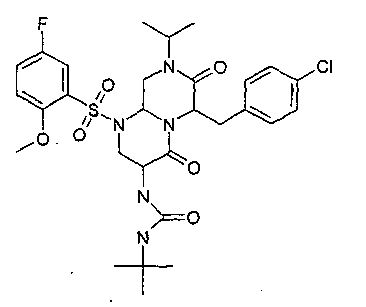

Die Synthese erfolgt analog Bsp. 28 ausgehend von 3-Amino-6-(4-chlor-benzyl)-1-(5-fluor-2-methoxy-benzolsulfonyl)-8-isopropyl-hexahydro-pyrazino[1,2-a]pyrimidin-4,7-dion. Man erhält das gewünschte Produkt mit MG = 637.31 (berechnet, monoisotop); Meßwert (M+Na)⁺: 660.17

### Beispiel 71

### N-[6-(4-Chlor-benzyl)-1-(5-fluor-2-methoxy-benzolsulfonyl)-8-isopropyl-4,7-dioxo-octahydro-pyrazino[1,2-a]pyrimidin-3-yl]-methansulfonamid

### Struktur:

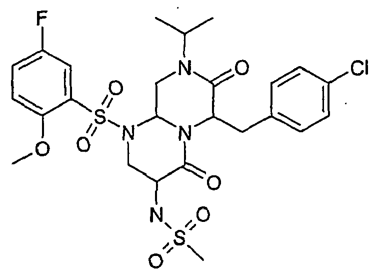

Die Synthese erfolgt analog Bsp. 26 ausgehend von 3-Amino-6-(4-chlor-benzyl)-1-(5-fluor-2-methoxy-benzolsulfonyl)-8-isopropyl-hexahydro- pyrazino[1,2-a]pyrimidin-4,7-dion. Man erhält das gewünschte Produkt mit MG = 616.12 (berechnet, monoisotop); Meßwert (M+H)⁺: 617.14.

### Beispiel 72

### 6-(4-Chlor-benzyl)-3-dimethylamino-1-(5-fluor-2-methoxy-benzolsulfonyl)-8-isopropyl-hexahydro-pyrazino[1,2-a]pyrimidin-4,7-dion

### Struktur:

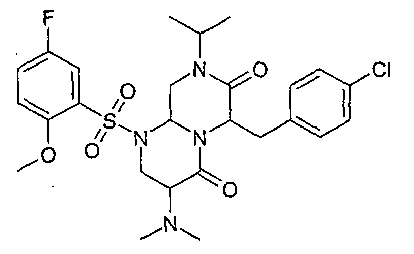

35 mg (0,065 mmol) 3-Amino-6-(4-chlorbenzyl)-1-(5-fluor-2-methoxybenzolsulfonyl)-8-isopropylhexahydropyrazino[1,2-a]pyrimidin-4,7-dion wurden in 3,5 mL MeOH gelöst. Nach Zugabe von 0,1 mL CH₃COOH, 0,1 mL Formaldehyd (37 % in Wasser) und 0,7 mL NaCNBH₃ (1,0M in THF) wurde über Nacht bei Raumtemperatur gerührt. Danach wurde das Gemisch im Vakuum eingeengt, mit 1 mL Wasser versetzt und 2mal mit je 1 mL EtOAc extrahiert. Die organische Phase wurde isoliert, getrocknet (MgSO₄) und eingeengt. Durch weitere Reinigung mittels Flash-Chromatographie an 4 g SiO₂ (Elution mit MeOH/DCM, Gradient 0 - 5 % MeOH) wurde das Amin gewonnen. Nach Zugabe von 0,2 mL 1,0M-HCl in Diethylether wurde das Amin verrieben. Der Feststoff wurde 4mal mit je 1 mL Diethylether gewaschen und im Vakuum getrocknet. Man erhielt 16,1 mg HCl-Salz der gewünschten Verbindung. LC/MS: MG (berechnet, monoisotop) = 567,08; Meßwert (M⁺H) = 567,14.

### Beispiel 73

### 3-Azetidin-1-yl-6-(4-chlor-benzyl)-1-(5-fluor-2-methoxy-benzolsulfonyl)-8-isopropyl-hexahydro-pyrazino[1,2-a]pyrimidin-4,7-dion

### Struktur:

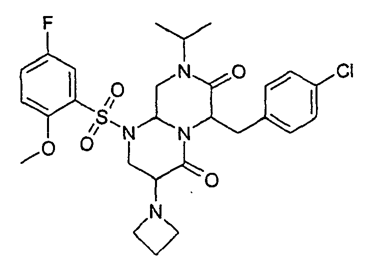

50 mg (0,093 mmol) 3-Amino-6-(4-chlorbenzyl)-1-(5-fluor-2-methoxybenzolsulfonyl)-8-isopropylhexahydropyrazino[1,2-a]pyrimidin-4,7-dion wurden in ein 2-5-mL-Mikrowellengefäß eingebracht und in 2 mL CH₃CN gelöst. Dieser Lösung wurden 0,25 mL NaHCO₃ (1,0M, in Wasser) und 0,0375 mL Dibrompropan zugesetzt. Das Reaktionsgemisch wurde 1000 Sekunden lang in einem Mikrowellenofen Personal Chemistry Smith Synthesizer auf 180°C erwärmt (hoher Absorptionsmodus mit 30 Sekunden Vorrührzeit). Dann wurde das Gemisch im Vakuum eingeengt, mit 2 mL EtOAc versetzt und mit 2 mL Wasser gewaschen. Die organische Phase wurde isoliert, getrocknet (MgSO₄) und im Vakuum eingeengt. Die Reinigung erfolgte mittels präparativer RP-HPLC auf einer GILSON-Apparatur (CH₃CN (0,1 % TFA)/H₂O (0,1 % TFA), Gradient 5 - 100 %). Man erhielt 6,9 mg TFA-Salz der gewünschten Verbindung. LC/MS: MG (berechnet, monoisotop)= 578.18; Meßwert (M⁺H) = 579,17.

### Beispiel 74

### 6-(4-Chlor-benzyl)-1-(5-fluor-2-methoxy-benzolsulfonyl)-8-isopropyl-3-pyrrolidin-1-yl-hexahydro-pyrazino[1,2-a]pyrimidin-4,7-dion

### Struktur:

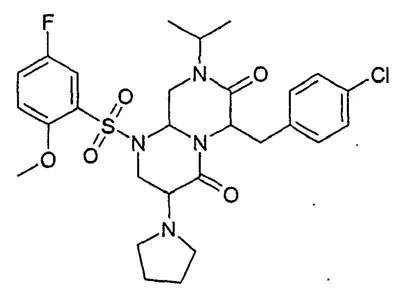

40 mg (0,074 mmol) 3-Amino-6-(4-chlorbenzyl)-1-(5-fluor-2-methoxybenzolsulfonyl)-8-isopropylhexahydropyrazino[1,2-a]pyrimidin-4,7-dion wurden in ein 2-5-mL-Mikrowellengefäß eingebracht und in 2 mL CH₃CN gelöst. Dieser Lösung wurden 0,2 mL NaHCO₃ (1,0M, in Wasser) und 0,036 mL Dibrombutan zugesetzt. Das Reaktionsgemisch wurde 800 Sekunden lang in einem Mikrowellenofen Personal Chemistry Smith Synthesizer auf 190°C erwärmt (hoher Absorptionsmodus mit 30 Sekunden Vorrührzeit). Dann wurde das Gemisch im Vakuum eingeengt, mit 1 mL EtOAc versetzt und mit 1 mL Wasser gewaschen. Die organische Phase wurde isoliert, getrocknet (MgSO₄) und im Vakuum eingeengt. Die Reinigung erfolgte mittels Flash-Chromatographie an 4 g SiO₂ (MeOH/DCM, Gradient 0 - 5 %). Die eingeengte Substanz wurde durch Zugabe von 0,2 mL 1,0M-HCl in Diethylether in das HCl-Salz umgewandelt. Der Feststoff wurde zermahlen, 4mal mit Diethylether gewaschen und im Vakuum eingeengt. Man erhielt 22,7 mg HCl-Salz der gewünschten Verbindung. LC/MS: MG (berechnet, monoisotop)= 592.19; Meßwert (M⁺H) = 593.18.

### Beispiel 75

### 6-(4-Chlor-benzyl)-1-(5-fluor-2-methoxy-benzolsulfonyl)-8-isopropyl-3-piperidin-1-yl-hexahydro-pyrazino[1,2-a]pyrimidin-4,7-dion

### Struktur:

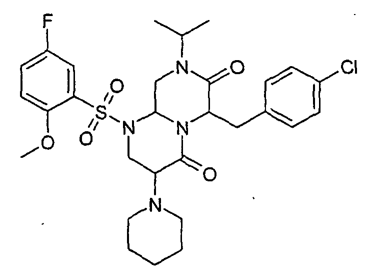

Die Synthese erfolgt analog Bsp. 59 ausgehend von 3-Amino-6-(4-chlor-benzyl)-1-(5-fluor-2-methoxy-benzolsulfonyl)-8-isopropyl-hexahydro- pyrazino[1,2-a]pyrimidin-4,7-dion. Man erhält das gewünschte Produkt mit MG = 606.21 (berechnet, monoisotop); Meßwert (M+H)⁺:607.19

### Beispiel 76

### 6-(4-Chlor-benzyl)-1-(5-fluor-2-methoxy-benzolsulfonyl)-8-isopropyl-3-morpholin-4-yl-hexahydro-pyrazino[1,2-a]pyrimidin-4,7-dion

### Struktur:

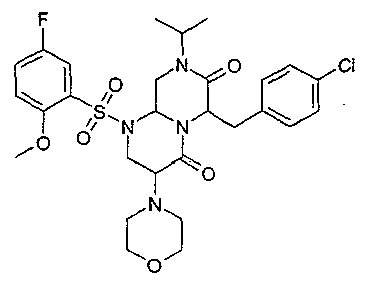

Die Synthese erfolgt analog Bsp. 60 ausgehend von 3-Amino-6-(4-chlor-benzyl)-1-(5-fluor-2-methoxy-benzolsulfonyl)-8-isopropyl-hexahydro- pyrazino[1,2-a]pyrimidin-4,7-dion. Man erhält das gewünschte Produkt mit MG = 608.19 (berechnet, monoisotop); Meßwert (M+H)⁺:609.16

### Beispiel 77

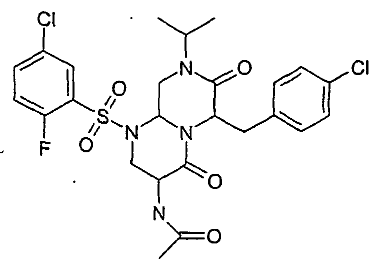

a) [6-(4-Chlor-benzyl)-1-(5-chlor-2-fluor-benzolsulfonyl)-8-isopropyl-4,7-dioxo-octahydro-pyrazino[1,2-a]pyrimidin-3-yl]-carbaminsäurebenzylester
   Die Synthese erfolgt analog zu Beispiel 66a) unter Verwendung von 5-Chlor-2-fluorbenzolsulfonylchlorid. Man erhält das gewünschte Produkt mit MG = 676.13 (berechnet, monoisotop); Meßwert (M+H)⁺: 677.1
b) 3-Amino-6-(4-chlor-benzyl)-1-(5-chlor-2-fluor-benzolsulfonyl)-8-isopropyl-hexahydropyrazino[1,2-a]pyrimidin-4,7-dion
   Die Synthese erfolgt analog Bsp. 66b) ausgehend von [6-(4-Chlor-benzyl)-1-(5-chlor-2-fluorbenzolsulfonyl)-8-isopropyl-4,7-dioxo-octahydro- pyrazino[1,2-a]pyrimidin-3-yl]-carbaminsäurebenzylester. Man erhält das gewünschte Produkt mit MG = 542.1 (berechnet, monoisotop); Meßwert (M+H)⁺: 543.1
c) N-[6-(4-chlor-benzyl)-1-(5-chlor-2-fluor-benzolsulfonyl)-8- isopropyl-4,7-dioxo-octahydro-pyrazino[1,2-a]pyrimidin-3-yl]-acetamid. Die Synthese erfolgt analog Bsp. 66c) ausgehend von 3-Amino-6-(4-chlor-benzyl)-1-(5-chlor-2-fluor-benzolsulfonyl)-8-isopropyl-hexahydropyrazino[1,2-a]pyrimidin-4,7-dion. Man erhält das gewünschte Produkt mit MG = 584.11 berechnet, monoisotop); Meßwert (M+H)⁺: 585.08

### Beispiel 78

### Cyclopropancarbonsäure [6-(4-chlor-benzyl)-1-(5-chlor-2-fluor-benzolsulfonyl)-8- isopropyl-4,7-dioxo-octahydro-pyrazino[1,2-a]pyrimidin-3-yl]-amid

### Struktur:

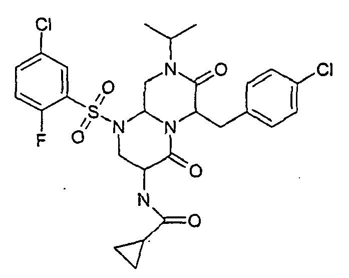

Die Synthese erfolgt analog Bsp. 22 ausgehend von 3-Amino-6-(4-chlor-benzyl)-1-(5-chlor-2-fluor-benzolsulfonyl)-8-isopropyl-hexahydro- pyrazino[1,2-a]pyrimidin-4,7-dion. Man erhält das gewünschte Produkt mit MG = 610.12 (berechnet, monoisotop); Meßwert (M+H)⁺: 611.09

### Beispiel 79

### Cyclobutancarbonsäure [6-(4-chlor-benzyl)-1-(5-chlor-2-fluor-benzolsulfonyl)-8- isopropyl-4,7-dioxo-octahydro-pyrazino[1,2-a]pyrimidin-3-yl]-amid

### Struktur:

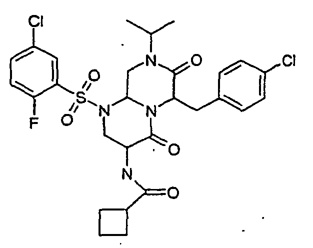

Die Synthese erfolgt analog Bsp. 30 ausgehend von 3-Amino-6-(4-chlor-benzyl)-1-(5-chlor-2-fluor-benzolsulfonyl)-8-isopropyl-hexahydro- pyrazino[1,2-a]pyrimidin-4,7-dion. Man erhält das gewünschte Produkt mit MG = 624.14 (berechnet, monoisotop); Meßwert (M+H)⁺: 625.10

### Beispiel 80

### Cyclopentancarbonsäure [6-(4-chlor-benzyl)-1-(5-chlor-2-fluor-benzolsulfonyl)-8-isopropyl-4,7-dioxo-octahydro-pyrazino[1,2-a]pyrimidin-3-yl]-amid

### Struktur:

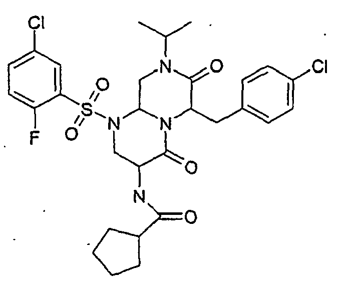

Die Synthese erfolgt analog Bsp. 31 ausgehend von 3-Amino-6-(4-chlor-benzyl)-1-(5-chlor-2-fluor-benzolsulfonyl)-8-isopropyl-hexahydro- pyrazino[1,2-a]pyrimidin-4,7-dion. Man erhält das gewünschte Produkt mit MG = 638.15 (berechnet, monoisotop); Meßwert (M+H)⁺: 639.12

### Beispiel 81

### 1-tert-Butyl-3-[6-(4-chlor-benzyl)-1-(5-chlor-2-fluor-benzolsulfonyl)-8-isopropyl- 4,7-dioxo-octahydro-pyrazino[1,2-a]pyrimidin-3-yl]-harnstoff

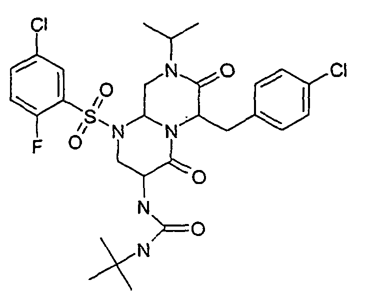

Die Synthese erfolgt analog Bsp. 28 ausgehend von 3-Amino-6-(4-chlor-benzyl)-1-(5-chlor-2-fluor-benzolsulfonyl)-8-isopropyl-hexahydro- pyrazino[1,2-a]pyrimidin-4,7-dion. Man erhält das gewünschte Produkt mit MG = 641.16 (berechnet, monoisotop); Meßwert (M+Na)⁺: 664.12

### Beispiel 82

### 6-(4-Chlor-benzyl)-1-(5-chlor-2-fluor-benzolsulfonyl)-3-dimethylamino-8-isopropylhexahydro-pyrazino[1,2-a]pyrimidin-4,7-dion

### Struktur:

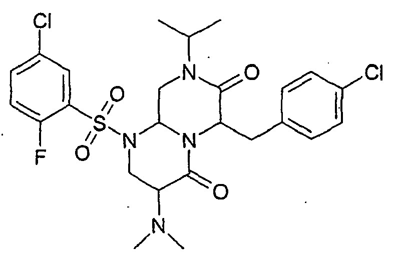

Die Synthese erfolgt analog Bsp. 72 ausgehend von 3-Amino-6-(4-chlor-benzyl)-1-(5-chlor-2-fluor-benzolsulfonyl)-8-isopropyl-hexahydro- pyrazino[1,2-a]pyrimidin-4,7-dion. Man erhält das gewünschte Produkt mit MG = 570.13 (berechnet, monoisotop); Meßwert (M+H)⁺: 571.09.

### Beispiel 83

### 3-Azetidin-1-yl-6-(4-chlor-benzyl)-1-(5-chlor-2-fluor-benzolsulfonyl)-8-isopropyl-hexahydropyrazino[1,2-a]pyrimidin-4,7-dion

### Struktur:

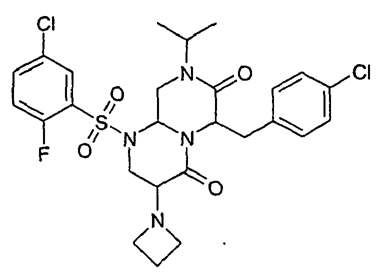

Die Synthese erfolgt analog Bsp. 73 ausgehend von 3-Amino-6-(4-chlor-benzyl)-1-(5-chlor-2-fluor-benzolsulfonyl)-8-isopropyl-hexahydro- pyrazino[1,2-a]pyrimidin-4,7-dion. Man erhält das gewünschte Produkt mit MG = 582.13 (berechnet, monoisotop); Meßwert (M+H)⁺: 583.11.

### Beispiel 84

### 6-(4-Chlor-benzyl)-1-(5-chlor-2-fluor-benzolsulfonyl)-8-isopropyl-3-pyrrolidin-1-yl-hexahydro-pyrazino[1,2-a]pyrimidin-4,7-dion

### Struktur:

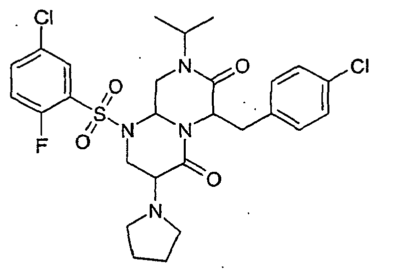

Die Synthese erfolgt analog Bsp. 74 ausgehend von 3-Amino-6-(4-chlor-benzyl)-1-(5-chlor-2-fluor-benzolsulfonyl)-8-isopropyl-hexahydro- pyrazino[1,2-a]pyrimidin-4,7-dion. Man erhält das gewünschte Produkt mit MG = 596.14 (berechnet, monoisotop); Meßwert (M+H)⁺: 597.14.

### Beispiel 85

### 6-(4-Chlor-benzyl)-1-(5-chlor-2-fluor-benzolsulfonyl)-8-isopropyl-3-piperidin-1-yl-hexahydro-pyrazino[1,2-a]pyrimidin-4,7-dion

### Struktur:

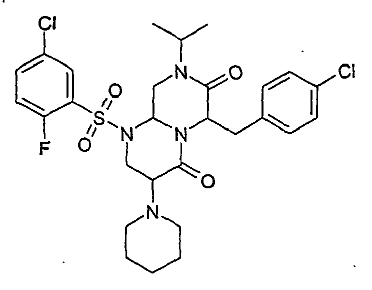

Die Synthese erfolgt analog Bsp. 59 ausgehend von 3-Amio-6-(4-chlor-benzyl)-1-(5-chlor-2-fluor-benzolsulfonyl)-8-isopropyl-hexahydro- pyrazino[1,2-a]pyrimidin-4,7-dion. Man erhält das gewünschte Produkt mit MG = 610.16 (berechnet, monoisotop); Meßwert (M+H)⁺: 611.15

### Beispiel 86

### 6-(4-Chlor-benzyl)-1-(5-chlor-2-fluor-benzolsulfonyl)-8-isopropyl-3-morpholin-4-yl-hexahydro-pyrazino[1,2-a]pyrimidin-4,7-dion

### Struktur:

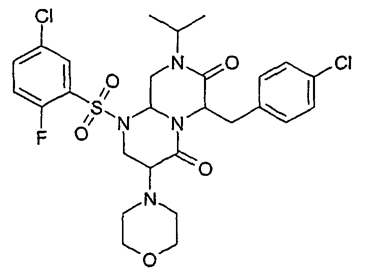

Die Synthese erfolgt analog Bsp. 60 ausgehend von 3-Amino-6-(4-chlor-benzyl)-1-(5-chlor-2-fluor-benzolsulfonyl)-8-isopropyl-hexahydro- pyrazino[1,2-a]pyrimidin-4,7-dion. Man erhält das gewünschte Produkt mit MG = 612.14 (berechnet, monoisotop); Meßwert (M+H)⁺: 613.13.

### Beispiel 87

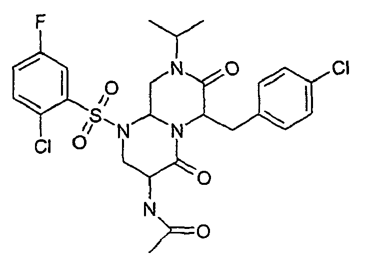

a) 2-Chlor-5-fluor-benzolsulfonylchlorid
   5 g (34,3 mmol) 2-Chlor-5-fluoranilin wurden langsam bei 0°C in eine Lösung aus 17 mL konzentrierter Salzsäurelösung und 11 mL Wasser gegeben. Das Reaktionsgemisch wurde 1 Stunde lang bei 0°C gerührt. Nach Zugabe von 2,49 g (36,1 mmol) NaNO₂ in 6 mL H₂O wurde das Gemisch 15 Minuten lang bei 0°C gerührt und dann auf eine Lösung von 692 mg (5,15 mmol) Schwefeldioxid und Kupfer(II)-chlorid in 10 mL Essigsäure gegeben. Die Reaktanten wurden 15 Minuten lang bei 0°C und danach weitere 15 Minuten bei Raumtemperatur gerührt. Das Reaktionsgemisch wurde mit EtOAc extrahiert. Die organische Phase wurde eingeengt, in EtOAc gelöst, mit 1N NaHCO₃ Lösung gewaschen, über MgSO₄ getrocknet und eingeengt. Man erhielt 7,86 g des gewünschten Sulfonylchlorids als gelbes Öl.
b) (2-(2-Chlor-5-fluor-benzolsulfonylamino)-1-{2-(4-chlor-phenyl)-1-[(2,2-diethoxy-ethyl)-isopropyl-carbamoyl]-ethylcarbamoyl}-ethyl)-carbaminsäurebenzylester
   Einer Lösung von 800 mg (1,39 mmol) des Amins (2-Amino-1-{2-(4-chlorphenyl)-1-[(2,2-diethoxyethyl)-isopropylcarbamoyl]-ethylcarbamoyl}-ethyl)-carbaminsäurebenzylester in 4 mL DCM wurde Et₃N zugesetzt. Dann wurde die wie oben beschrieben hergestellte Sulfonylchlorid-Lösung (476 mg, 2,08 mmol) in 3 mL DCM bei Raumtemperatur zugegeben. Das Reaktionsgemisch wurde 1 Stunde lang bei Raumtemperatur gerührt und mit 1N-NaHCO₃ gewaschen. Die organische Phase wurde auf MgSO₄ getrocknet und eingeengt. Der Rückstand wurde an 40 g SiO₂ chromatographiert (Elution mit 30 - 70 % EtOAc in Heptan). Man erhielt 860 mg der gewünschten Substanz als weißen Feststoff. LC/MS: MG (berechnet, monoisotop) = 769,72; Meßwert (M⁺Na) = 791.
c) [6-(4-Chlor-benzyl)-1-(2-chlor-5-fluor-benzolsulfonyl)-8-isopropyl-4,7-dioxo-octahydro-pyrazino [1,2-a]pyrimidin-3-yl]-carbaminsäurebenzylester
   Die Synthese erfolgt analog zu Beispiel 29f) (Methode B) ausgehend von N-{2-(4-Chlorphenyl)-1-[(2,2-diethoxy-ethyl)-isopropyl-carbamoyl]-ethyl}-3-(2-chlor-5-fluorbenzolsulfonylamino)-2-methanesulfonylamino-propionamid. Man erhält das gewünschte Produkt mit MG = 676.13 (berechnet, monoisotop); Meßwert (M+H)⁺: 677.16
d) 3-Amino-6-(4-chlor-benzyl)-1-(2-chlor-5-fluor-benzolsulfonyl)-8-isopropyl-hexahydropyrazino[1,2-a]pyrimidin-4,7-dion
   Die Synthese erfolgt analog Bsp. 29g) (Methode B) ausgehend von [6-(4-Chlor-benzyl)-1-(2-chlor-5-fluor-benzolsulfonyl)-8-isopropyl-4,7-dioxo-octahydro- pyrazino[1,2-a]pyrimidin-3-yl]-carbaminsäurebenzylester. Man erhält das gewünschte Produkt mit MG = 542.1 (berechnet, monoisotop); Meßwert (M+H)⁺: 543.10
e) N-[6-(4-chlor-benzyl)-1-(2-chlor-5-fluor-benzolsulfonyl)-8-isopropyl-4,7-dioxo-octahydro-pyrazino[1,2-a]pyrimidin-3-yl]- acetamid.
   Die Synthese erfolgt analog Bsp. 21h) ausgehend von 3-Amino-6-(4-chlor-benzyl)-1-(2-chlor-5-fluor-benzolsulfonyl)-8-isopropyl-hexahydro- pyrazino[1,2-a]pyrimidin-4,7-dion. Man erhält das gewünschte Produkt mit MG = 584.11 (berechnet, monoisotop); Meßwert (M+H)⁺: 585.10

### Beispiel 88

### Cyclopropancarbonsäure [6-(4-chlor-benzyl)-1-(2-chlor-5-fluor-benzolsulfonyl)-8- isopropyl-4,7-dioxo-octahydro-pyrazino[1,2-a]pyrimidin-3-yl]-amid

### Struktur:

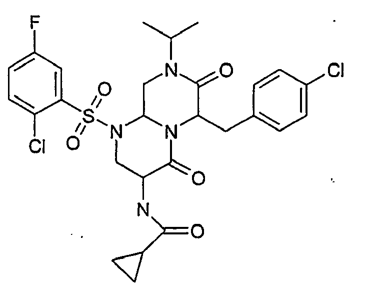

Die Synthese erfolgt analog Bsp. 22 ausgehend von 3-Amino-6-(4-chlor-benzyl)-1-(2-chlor-5-fluor-benzolsulfonyl)-8-isopropyl-hexahydro- pyrazino[1,2-a]pyrimidin-4,7-dion. Man erhält das gewünschte Produkt mit MG = 610.12 (berechnet, monoisotop); Meßwert (M+H)⁺: 611.14

### Beispiel 89

### Cyclobutancarbonsäure [6-(4-chlor-benzyl)-1-(2-chlor-5-fluor-benzolsulfonyl)-8- isopropyl-4,7-dioxo-octahydro-pyrazino[1,2-a]pyrimidin-3-yl]-amid

### Struktur:

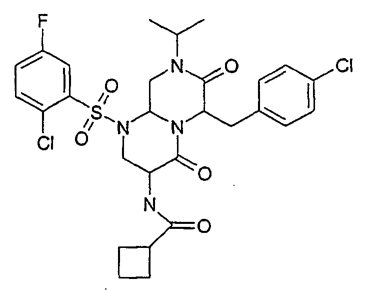

Die Synthese erfolgt analog Bsp. 30 ausgehend von 3-Amino-6-(4-chlor-benzyl)-1-(2-chlor-5-fluor-benzolsulfonyl)-8-isopropyl-hexahydro- pyrazino[1,2-a]pyrimidin-4,7-dion. Man erhält das gewünschte Produkt mit MG = 624.14 (berechnet, monoisotop); Meßwert (M+H)⁺: 625.14

### Beispiel 90

### 1-tert-Butyl-3-[6-(4-chlor-benzyl)-1-(2-chlor-5-fluor-benzolsulfonyl)-8-isopropyl- 4,7-dioxo-octahydro-pyrazino[1,2-a]pyrimidin-3-yl]-harnstoff

### Struktur:

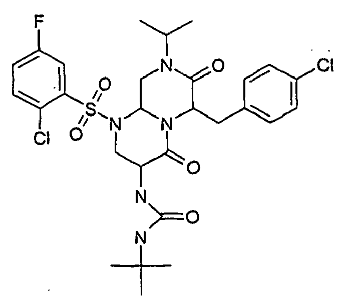

Die Synthese erfolgt analog Bsp. 28 ausgehend von 3-Amino-6-(4-chlor-benzyl)-1-(2-chlor-5-fluor-benzolsulfonyl)-8-isopropyl-hexahydro- pyrazino[1,2-a]pyrimidin-4,7-dion. Man erhält das gewünschte Produkt mit MG = 641.16 (berechnet, monoisotop); Meßwert (M+Na)⁺: 664.15

### Beispiel 91

### N-[6-(4-Chlor-benzyl)-1-(2-chlor-5-fluor-benzolsulfonyl)-8-isopropyl-4,7-dioxo- octahydro-pyrazino[1,2-a]pyrimidin-3-yl]-methansulfonamid

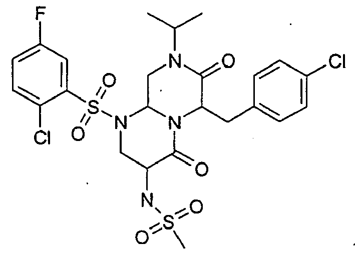

Die Synthese erfolgt analog Bsp. 26 ausgehend von 3-Amino-6-(4-chlor-benzyl)-1-(2-chlor-5-fluor-benzolsulfonyl)-8-isopropyl-hexahydro- pyrazino[1,2-a]pyrimidin-4,7-dion. Man erhält das gewünschte Produkt mit MG = 620.07 (berechnet, monoisotop); Meßwert (M+H)⁺;621.0

### Beispiel 92

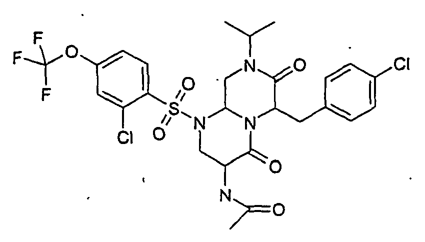

a) 2-Chlor-4-trifluormethoxy-benzolsulfonyl chlorid
   2-Chlor-4-trifluormethoxyaniline wurde mit dem entsprechenden Sulfonylchlorid nach dem selben Protokoll wie in Beispiel 87a) umgesetzt.
b) [1-{2-(4-Chlor-phenyl)-1-[(2,2-diethoxy-ethyl)-isopropyl-carbamoyl]-ethylcarbamoyl}-2-(2-chlor-4-trifluormethoxy-benzolsulfonylamino)-ethyl]-carbaminsäurebenzylester
   Die Synthese erfolgt analog zu Beispiel 87b) ausgehend von 2-Chlor-4-trifluormethoxybenzolsulfonylchlorid. Man erhält das gewünschte Produkt mit MG = 834.21 (berechnet, monoisotop); Meßwert (M+Na)⁺: 857.0
c) [6-(4-Chlor-benzyl)-1-(2-chlor-4-trifluormethoxybenzolsulfonyl)-8-isopropyl-4,7-dioxo-octahydro- pyrazino[1,2-a]pyrimidin-3-yl]-carbaminsäurebenzylester
   Die Synthese erfolgt analog zu Beispiel 29f) (Methode B) ausgehend von N-{2-(4-Chlorphenyl)-1-[(2,2-diethoxy-ethyl)-isopropyl-carbamoyl]-ethyl}-3-(2-chlor-4-trifluormethoxybenzolsulfonylamino)-2-methansulfonylamino-propionamid. Man erhält das gewünschte Produkt mit MG = 742.12 (berechnet, monoisotop); Meßwert (M+H)⁺: 743.14
d) 3-Amino-6-(4-chlor-benzyl)-1-(2-chlor-5-trifluorrnethoxy-benzolsulfonyl)-8-isopropyl-hexahydro-pyrazino[1,2-a]pyrimidin-4,7-dion
   Die Synthese erfolgt analog Bsp. 29g) (Methode B) ausgehend von [6-(4-Chlor-benzyl)-1-(2-chlor-4-trifluormethoxy-benzolsulfonyl)-8-isopropyl-4,7-dioxo-octahydro- pyrazino[1,2-a]pyrimidin-3-yl]-carbaminsäurebenzylester. Man erhält das gewünschte Produkt mit MG = 608.09 (berechnet, monoisotop); Meßwert (M+H)⁺: 609.07
e) N-[6-(4-chlor-benzyl)-1-(2-chlor-4-trifluormethoxy-benzolsulfonyl)-8- isopropyl-4,7-dioxo-octahydro-pyrazino[1,2-a]pyrimidin-3-yl]- acetamid.
   Die Synthese erfolgt analog Bsp. 21h) ausgehend von 3-Amino-6-(4-chlor-benzyl)-1-(2-chlor-4-trifluormethoxy-benzolsulfonyl)-8-isopropyl-hexahydro- pyrazino[1,2-a]pyrimidin-4,7-dion. Man erhält das gewünschte Produkt mit MG = 650.1 (berechnet, monoisotop); Meßwert (M+H)⁺: 651.09

### Beispiel 93

### Cyclopropancarbonsäure [6-(4-chlor-benzyl)-1-(2-chlor-4-trifluormethoxy-benzolsulfonyl)-8-isopropyl-4,7-dioxo-octahydro-pyrazino[1,2-a]pyrimidin-3-yl]- amid

### Struktur:

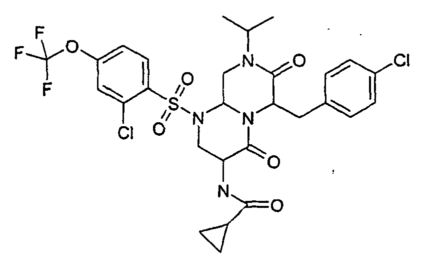

Die Synthese erfolgt analog Bsp. 22 ausgehend von 3-Amino-6-(4-chlor-benzyl)-1-(2-chlor-4-trifluormethoxy-benzolsulfonyl)-8-isopropyl-hexahydro- pyrazino[1,2-a]pyrimidin-4,7-dion. Man erhält das gewünschte Produkt mit MG = 676.11 (berechnet, monoisotop); Meßwert (M+H)⁺: 677.13

### Beispiel 94

### Cyclobutancarbonsäure [6-(4-chlor-benzyl)-1-(2-chlor-4-trifluormethoxy- berizolsulfonyl)-8-isopropyl-4,7-dioxo-octahydro-pyrazino[1,2-a]pyrimidin-3-yl]- amid

### Struktur:

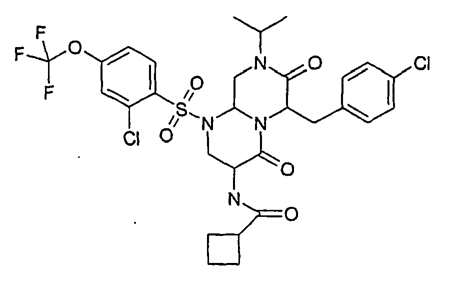

Die Synthese erfolgt analog Bsp. 30 ausgehend von 3-Amino-6-(4-chlor-benzyl)-1-(2-chlor-4-trifluormethoxy-benzolsulfonyl)-8-isopropyl-hexahydro- pyrazino[1,2-a]pyrimidin-4,7-dion. Man erhält das gewünschte Produkt mit MG = 690.13 (berechnet, monoisotop); Meßwert (M+H)⁺: 691.13

### Beispiel 95

### 1-tert-Butyl-3-[6-(4-chlor-benzyl)-1-(2-chlor-4-trifluormethoxy-benzolsulfonyl)-8- isopropyl-4,7-dioxo-octahydro-pyrazino[1,2-a]pyrimidin-3-yl]-harnstoff

### Struktur:

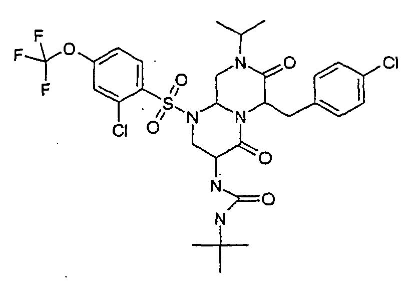

Die Synthese erfolgt analog Bsp. 28 ausgehend von 3-Amino-6-(4-chlor-benzyl)-1-(2-chlor-4-trifluormethoxy-benzolsulfonyl)-8-isopropyl-hexahydro- pyrazino[1,2-a]pyrimidin-4,7-dion. Man erhält das gewünschte Produkt mit MG =707.16 (berechnet, monoisotop); Meßwert (M+Na)⁺:730.14

### Beispiel 96

### N-[6-(4-Chlor-benzyl)-1-(2-chlor-4-trifluormethoxy-benzolsulfonyl)-8-isopropyl-4,7- dioxo-octahydro-pyrazino[1,2-a]pyrimidin-3-yl)-methansulfonamid

### Struktur:

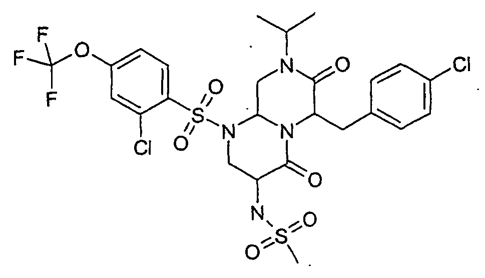

Die Synthese erfolgt analog Bsp. 26 ausgehend von 3-Amino-6-(4-chlor-benzyl)-1-(2-chlor-4-trifluonnethoxy-benzolsulfonyl)-8-isopropyl-hexahydro- pyrazino[1,2-a]pyrimidin-4,7-dion. Man erhält das gewünschte Produkt mit MG = 686.07 (berechnet, monoisotop); Meßwert (M+H)⁺:687.0

### Beispiel 97

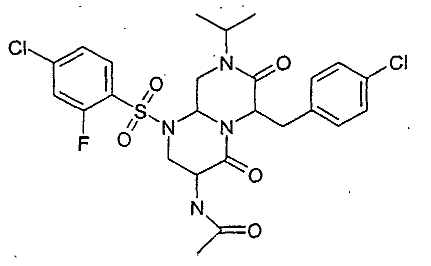

a) [1-{2-(4-Chlor-phenyl)-1-[(2,2-diethoxy-ethyl)-isopropyl-carbamoyl]-ethylcarbamoyl}-2-(4-chlor-2-fluor-benzolsulfonylamino)-ethyl]-carbaminsäurebenzylester
   Die Synthese erfolgt analog zu Beispiel 87b) ausgehend von 4-Chlor-2-fluor-benzolsulfonylchlorid. Man erhält das gewünschte Produkt mit MG = 768.22 (berechnet, monoisotop); Meßwert (M+Na)⁺: 791.0
b) [6-(4-Chlor-benzyl)-1-(4-chlor-2-fluorbenzolsulfonyl)-8-isopropyl-4,7-dioxo-octahydro-pyrazino[1,2-a]pyrimidin-3-yl]-carbaminsäurebenzylester
   Die Synthese erfolgt analog zu Beispiel 29f) (Methode B) ausgehend von N-{2-(4-Chlorphenyl)-1-[(2,2-diethoxy-ethyl)-isopropyl-carbamoyl]-ethyl} -3-(4-chlor-2-fluorbenzolsulfonylamino)-2-methansulfonylamino-propionamid. Man erhält das gewünschte Produkt mit MG = 676.13 (berechnet, monoisotop); Meßwert (M+H)⁺: 677.14
c) 3-Amino-6-(4-chlor-benzyl)-1-(4-chlor-2-fluor-benzolsulfonyl)-8-isopropyl-hexahydro-pyrazino[1,2-a]pyrimidin-4,7-dion
   Die Synthese erfolgt analog Bsp. 29g) (Methode B) ausgehend von [6-(4-Chlor-benzyl)-1-(4-chlor-2-fluor-benzolsulfonyl)-8-isopropyl-4,7-dioxo-octahydro- pyrazino[1,2-a]pyrimidin-3-yl]-carbaminsäurebenzylester. Man erhält das gewünschte Produkt mit MG = 542.1 (berechnet, monoisotop); Meßwert (M+H)⁺: 543.1
d) N-[6-(4-chlor-benzyl)-1-(4-chlor-2-fluor-benzolsulfonyl)-8- isopropyl-4,7-dioxo-octahydro-pyrazino[1,2-a]pyrimidin-3-yl]- acetamid.
   Die Synthese erfolgt analog Bsp. 21h) ausgehend von 3-Amino-6-(4-chlor-benzyl)-1-(4-chlor-2-fluor-benzolsulfonyl)-8-isopropyl-hexahydro- pyrazino[1,2-a]pyrimidin-4,7-dion. Man erhält das gewünschte Produkt mit MG = 584.11 (berechnet, monoisotop); Messwert (M+H)⁺:585.1

### Beispiel 98

### Cyclopropancarbonsäure [6-(4-chlor-benzyl)-1-(4-chlor-2-fluor-benzolsulfonyl)-8- isopropyl-4,7-dioxo-octahydro-pyrazino[1,2-a]pyrimidin-3-yl]-amid

### Struktur:

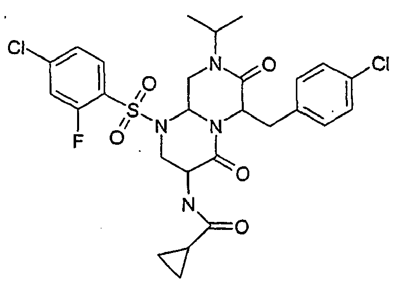

Die Synthese erfolgt analog Bsp. 29e) ausgehend von 3-Amko-6-(4-chlor-benzyl)-1-(4-chlor-2-fluor-benzolsulfonyl)-8-isopropyl-hexahydro- pyrazino[1,2-a]pyrimidin-4,7-dion. Man erhält das gewünschte Produkt mit MG = 610.12 (berechnet, monoisotop); Meßwert (M+H)⁺: 611.14

### Beispiel 99

### Cyclobutancarbonsäure [6-(4-chlor-benzyl)-1-(4-chlor-2-fluor-benzolsulfonyl)-8- isopropyl-4,7-dioxo-octahydro-pyrazino[1,2-a]pyrimidin-3-yl]-amid

### Struktur:

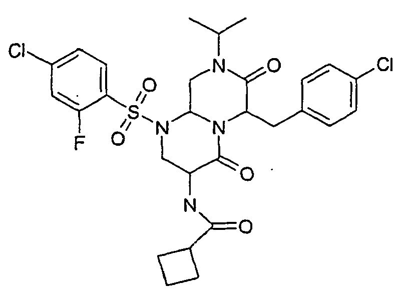

Die Synthese erfolgt analog Bsp. 30 ausgehend von 3-Amino-6-(4-chlor-benzyl)-1-(4-chlor-2-fluor-benzolsulfonyl)-8-isopropyl-hexahydro- pyrazino[1,2-a]pyrimidin-4,7-dion. Man erhält das gewünschte Produkt mit MG = 624.12 (berechnet, monoisotop); Meßwert (M+H)⁺: 625.14

### Beispiel 100

### 1-tert-Butyl-3-[6-(4-chlor-benzyl)-1-(4-chlor-2-fluor-benzolsulfonyl)-8-isopropyl- 4,7-dioxo-octahydro-pyrazino[1,2-a]pyrimidin-3-yl]-harnstoff

### Struktur:

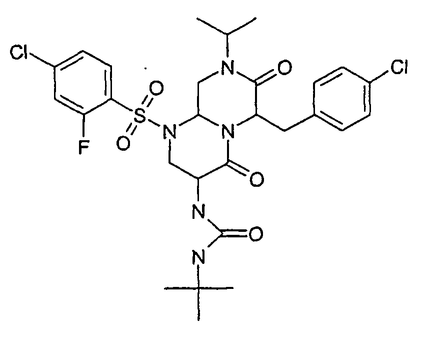

Die Synthese erfolgt analog Bsp. 28 ausgehend von 3-Amino-6-(4-chlor-benzyl)-1-(4-chlor-2-fluor-benzolsulfonyl)-8-isopropyl-hexahydro- pyrazino[1,2-a]pyrimidin-4,7-dion. Man erhält das gewünschte Produkt mit MG = 641.16 (berechnet, monoisotop); Meßwert (M+Na)⁺: 664.14

### Beispiel 101

### N-[6-(4-Chlor-benzyl)-1-(4-chlor-2-ffuor-benzolsulfonyl)-8-isopropyl-4,7-dioxo- octahydro-pyrazino[1,2-a]pyrimidin-3-yl]-methansulfonamid

### Struktur:

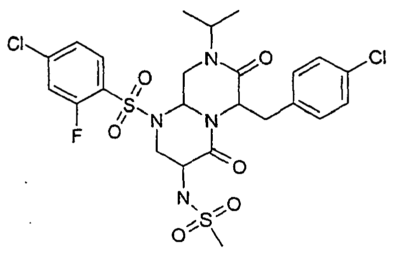

Die Synthese erfolgt analog Bsp. 26 ausgehend von 3-Amino-6-(4-chlor-benzyl)-1-(4-chlor-2-fluor-benzolsulfonyl)-8-isopropyl-hexahydro- pyrazino[1,2-a]pyrimidin-4,7-dion. Man erhält das gewünschte Produkt mit MG = 620.07 (berechnet, monoisotop); Meßwert (M+H)⁺: 621.0

### Beispiel 102

### N-[6-(4-Chlor-benzyl)-1-(2,5-dimethoxy-benzolsulfonyl)-8-isopropyl-4,7-dioxo- octahydro-pyrazino[1,2-a]pyrimidin-3-yl]-methansulfonamid

### Struktur:

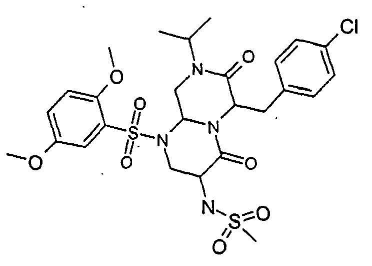

a) 2-Benzyloxycarbonylamino-3-(2,5-dimethoxy-benzolsulfonylamino)-propionsäure
   Die Synthese erfolgt analog zu Beispiel 21d) ausgehend von 2,5-Dimethoxybenzolsulfonylchlorid. Man erhält das gewünschte Produkt mit MG = 438.46 (berechnet, monoisotop); Meßwert (M+H)⁺: 439.1
b) N-{2-(4-Chlor-phenyl)-1-[(2,2-diethoxy-ethyl)-isopropyl-carbamoyl]-ethyl}-3-(2,5-dimethoxy-benzolsulfonylamino)-2-methansulfonylamino-propionamid
   Die Synthese erfolgt analog zu Beispiel 21e) ausgehend von 2-Benzyloxycarbonylamino-3-(2,5-dimethoxy-benzolsulfonylamino)-propionsäure. Man erhält das gewünschte Produkt mit MG = 777.34 (berechnet, monoisotop); Meßwert (M-C₂H₆0+H)⁺: 731.9
c) [6-(4-Chlor-benzyl)-1-(2,5-dimethoxy-benzolsulfonyl)-8-isopropyl-4,7-dioxo-octahydro- pyrazino[1,2-a]pyrimidin-3-yl]-carbaminsäurebenzylester
   Die Synthese erfolgt analog zu Beispiel 21f) ausgehend von N-{2-(4-Chlor-phenyl)-1-[(2,2-diethoxy-ethyl)-isopropyl-carbamoyl]-ethyl}-3-(2,5- dimethoxy-benzolsulfonylamino)-2-methansulfonylamino-propionamid. Man erhält das gewünschte Produkt mit MG = 684 (berechnet, monoisotop); Meßwert (M+H)⁺: 685.38
d) 3-Amino-6-(4-chlor-benzyl)-1-(2,5-dimethoxy-benzolsulfonyl)-8-isopropyl-hexahydro-pyrazino[1,2-a]pyrimidin-4,7-dion
   Die Synthese erfolgt analog Bsp. 21g) ausgehend von [6-(4-Chlor-benzyl)-1-(2,5-dimethoxy-benzolsulfonyl}-8-isopropyl-4,7-dioxo-octahydro- pyrazino[1,2-a]pyrimidin-3-yl]-carbaminsäurebenzylester. Man erhält das gewünschte Produkt mit MG = 550 (berechnet, monoisotop); Meßwert (M+H)⁺: 551.15
e) 3-Amino-6-(4-chlor-benzyl)-1-(2,5-dimethoxy-benzolsulfonyl)-8-isopropyl-hexahydro-pyrazino[1,2-a]pyrimidin-4,7-dion
   Die Synthese erfolgt analog Bsp. 26 ausgehend 3-Amino-6-(4-chlor-benzyl)-1-(2,5-dimethoxy-benzolsulfonyl)-8-isopropyl-hexahydro- pyrazino[1,2-a]pyrimidin-4,7-dion. Man erhält das gewünschte Produkt mit MG = 628.14 (berechnet, monoisotop); Meßwert (M+H)⁺: 629.15

### Beispiel 103

### N-[6-(4-Chlor-benzyl)-1-(3-fluor-benzolsulfonyl)-8-isopropyl-4,7-dioxo-octahydro-pyrazino[1,2-a]pyrimidin-3-yl]-methansulfonamid

### Struktur:

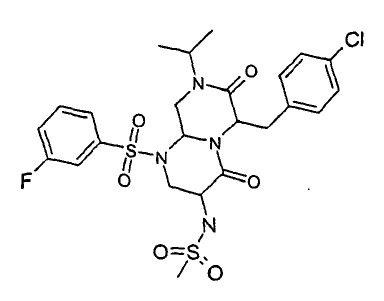

a) 2-Benzyloxycarbonylamino-3-(3-fluor-benzolsulfonylamino)-propionsäure
   Die Synthese erfolgt analog zu Beispiel 21d) ausgehend von 3-Fluor-benzolsulfonyl-chlorid. Man erhält das gewünschte Produkt mit MG = 396.40 (berechnet, monoisotop); Meßwert (M+H)⁺: 397.10
b) N-{2-(4-Chlor-phenyl)-1-[(2,2-diethoxy-ethyl)-isopropyl-carbamoyl]-ethyl}-3-(3-fluor-benzolsulfonylamino)-2-methansulfonylamino-propionamid
   Die Synthese erfolgt analog zu Beispiel 21e) ausgehend von 2-Benzyloxycarbonylamino-3-(3-fluor-benzolsulfonylamino)-propionsäure. Man erhält das gewünschte Produkt mit MG = 735.28 (berechnet, monoisotop); Meßwert (M-C₂H₆O+H)⁺: 689.7
c) [6-(4-Chlor-benzyl)-1-(3-fluor-benzolsulfonyl)-8-isopropyl-4,7-dioxo-octahydro-pyrazino[1,2-a]pyrimidin-3-yl]-carbamisäurebenzylester
   Die Synthese erfolgt analog zu Beispiel 21f) ausgehend von N-{2-(4-Chlor-phenyl)-1-[(2,2-diethoxy-ethyl)-isopropyl-carbamoyl]-ethyl}-3-(3-fluor-benzolsulfonylamino)-2-methansulfbnylamino-propionamid. Man erhält das gewünschte Produkt mit MG = 642 (berechnet, monoisotop); Meßwert (M+H)⁺: 643.31
d) 3-Amino-6-(4-chlor-benzyl)-1-(3-fluor-benzolsulfonyl)-8-isopropyl-hexahydro-pyrazino[1,2-a]pyrimidin-4,7-dion
   Die Synthese erfolgt analog Bsp. 21g) ausgehend von [6-(4-Chlor-benzyl)-1-(3-fluor-benzolsulfonyl)-8-isopropyl-4,7-dioxo-octahydro- pyrazino[1,2-a]pyrimidin-3-yl]-carbamic acid benzyl ester. Man erhält das gewünschte Produkt mit MG = 508 (berechnet, monoisotop); Meßwert (M+H)⁺: 509.15
e) 3-Amino-6-(4-chlor-benzyl)-1-(3-fluor-benzolsulfonyl)-8-isopropyl-hexahydro-pyrazino[1,2-a]pyrimidin-4,7-dion
   Die Synthese erfolgt analog Bsp. 26 ausgehend 3-Amino-6-(4-chlor-benzyl)-1-(3-fluor-benzolsulfonyl)-8-isopropyl-hexahydro- pyrazino[1,2-a]pyrimidin-4,7-dion. Man erhält das gewünschte Produkt mit MG = 586.11 (berechnet, monoisotop); Meßwert (M+H)⁺: 587.41

### Beispiel 104

### N-[1-Benzolsulfonyl-6-(4-chlor-benzyl)-8-isopropyl-4,7-dioxo-octahydro-pyrazion[1,2-a]pyrimidin-3-yl]-methansulfonamid

### Struktur:

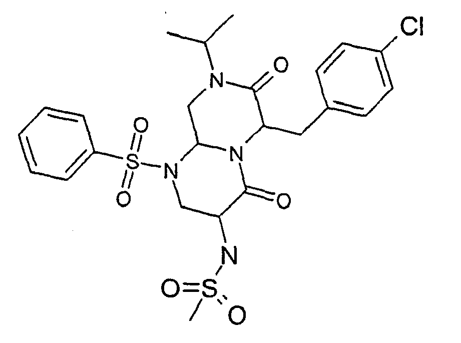

a) 2-Benzyloxycarbonylamino-3-(benzolsulfonylamino)-propionsäure
   Die Synthese erfolgt analog zu Beispiel 21d) ausgehend von Benzolsulfonylchlorid. Man erhält das gewünschte Produkt mit MG = 378.41 (berechnet, monoisotop); Meßwert (M+H)⁺: 379.10
b) N-{2-(4-Chlor-phenyl)-1-[(2,2-diethoxy-ethyl)-isopropyl-carbamoyl]-ethyl}-3-(benzolsulfonylamino)-2-methansulfonylamino-propionamid
   Die Synthese erfolgt analog zu Beispiel 21e) ausgehend von 2-Benzyloxycarbonylamino-3-(benzolsulfonylamino)-propionsäure. Man erhält das gewünschte Produkt mit MG = 717.29 (berechnet, monoisotop); Meßwert (M-C₂H₆O+H)⁺: 671.7
c) [6-(4-Chlor-benzyl)-1-(benzolsulfonyl)-8-isopropyl-4,7-dioxo-octahydro- pyrazino[1,2-a]pyrimidin-3-yl]-carbaminsäurebenzylester
   Die Synthese erfolgt analog zu Beispiel 21f) ausgehend von N-{2-(4-Chlor-phenyl)-1-[(2,2-diethoxy-ethyl)-isopropyl-carbamoyl]-ethyl; -3-(benzolsulfonylamino)-2-methansulfonylamino-propionamid. Man erhält das gewünschte Produkt mit MG = 624 (berechnet, monoisotop); Meßwert (M+H)⁺: 625.33
d) 3-Amino-6-(4-chlor-benzyl)-1-(benzolsulfonyl)-8-isopropyl-hexahydro- pyrazino[1,2-a]pyrimidin-4,7-dion
   Die Synthese erfolgt analog Bsp. 21g) ausgehend von [6-(4-Chlor-benzyl)-1-(benzolsulfonyl)-8-isopropyl-4,7-dioxo-octahydro- pyrazino[1,2-a]pyrimidin-3-yl]-carbaminsäurebenzylester. Man erhält das gewünschte Produkt mit MG = 490 (berechnet, monoisotop); Meßwert (M+H)⁺: 491.10
e) 3-Amino-6-(4-chlor-benzyl)-1-(benzolsulfonyl)-8-isopropyl-hexahydro- pyrazino[1,2-a]pyrimidin-4,7-dion
   Die Synthese erfolgt analog Bsp. 26 ausgehend 3-Amino-6-(4-chlor-benzyl)-1-(benzolsulfonyl)-8-isopropyl-hexahydro- pyrazino[1,2-a]pyrimidin-4,7-dion. Man erhält das gewünschte Produkt mit MG = 568.12 (berechnet, monoisotop); Meßwert (M+H)⁺: 569.13

### Beispiel 105

### N-[6-(4-Chlor-benzyl)-1-(2-fluor-benzolsulfonyl)-8-isopropyl-4,7-dioxo-octahydro-pyrazino[1,2-a]pyrimidin-3-yl]-methansulfonamid

### Struktur:

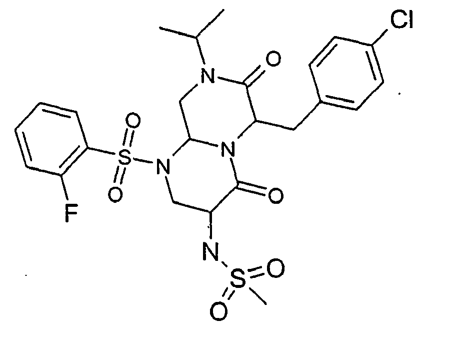

a) 2-Benzyloxycarbonylamino-3-(2-fluor-benzolsulfonylamino)-propionsäure
   Die Synthese erfolgt analog zu Beispiel 21d) ausgehend von 2-Fluorbenzolsulfonyl chlorid. Man erhält das gewünschte Produkt mit MG = 396.40 (berechnet, monoisotop); Meßwert (M+H)⁺: 397.10
b) N-{2-(4-Chlor-phenyl)-1-[(2,2-diethoxy-ethyl)-isopropyl-carbamoyl]-ethyl}-3-(2-fluor-benzolsulfonylamino)-2-methansulfonylamino-propionamid
   Die Synthese erfolgt analog zu Beispiel 21e) ausgehend von 2-Benzyloxycarbonylamino-3-(2-fluor-benzolsulfonylamino)-propionsäure. Man erhält das gewünschte Produkt mit MG = 735.28 (berechnet, monoisotop); Meßwert (M-C₂H₆O+H)⁺: 689.7
c) [6-(4-Chlor-benzyl)-1-(2-fluor-benzolsulfonyl)-8-isopropyl-4,7-dioxo-octahydro-pyrazino[1,2-a]pyrimidin-3-yl]-carbaminsäurebenzylester
   Die Synthese erfolgt analog zu Beispiel 21f) ausgehend von N-{2-(4-Chlor-phenyl)-1-[(2,2-diethoxy-ethyl)-isopropyl-carbalnoyl]-ethyl}-3-(2-fluor-benzolsulfonylamino)-2-methansulfonylamino-propionamid. Man erhält das gewünschte Produkt mit MG = 642 (berechnet, monoisotop); Meßwert (M+H)⁺: 643.31
d) 3-Amino-6-(4-chlor-benzyl)-1-(2-fluor-benzolsulfonyl)-8-isopropyl-hexahydro-pyrazino[1,2-a]pyrimidin-4,7-dion
   Die Synthese erfolgt analog Bsp. 21g) ausgehend von [6-(4-Chlor-benzyl)-1-(2-fluor-benzolsulfonyl)-8-isopropyl-4,7-dioxo-octahydro-pyrazino[1,2-a]pyrimidin-3-yl]-carbaminsäurebenzylester. Man erhält das gewünschte Produkt mit MG = 508 (berechnet, monoisotop); Meßwert (M+H)⁺: 509.15
e) 3-Amino-6-(4-chlor-benzyl}-1-(2-fluor-benzolsulfonyl)-8-isopropyl-hexahydro-pyrazino[1,2-a]pyrimidin-4,7-dion
   Die Synthese erfolgt analog Bsp. 26 ausgehend 3-Amino-6-(4-chlor-benzyl)-1-(2-fluor-benzolsulfonyl)-8-isopropyl-hexahydro-pyrazino[1,2-a]pyrimidin-4,7-dion. Man erhält das gewünschte Produkt mit MG = 586.11 (berechnet, monoisotop); Meßwert (M+H)⁺: 587.12

### Beispiel 106

### N-[6-(4-Chlor-benzyl}-1-(2,4-difluor-benzolsulfonyl)-8-isopropyl-4,7-dioxo-octahydro-pyrazino[1,2-a]pyrimidin-3-yl]-methansulfonamid

### Struktur:

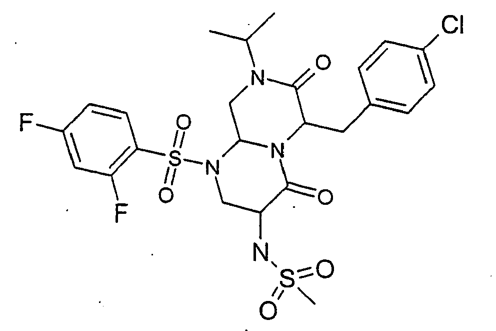

a) 2-Benzyloxycarbonylamino-3-(2,4-difluor-benzolsulfonylamino)-propionsäure
   Die Synthese erfolgt analog zu Beispiel 21d) ausgehend von 2,4-Difluor-benzolsulfonyl chlorid. Man erhält das gewünschte Produkt mit MG = 414.40 (berechnet, monoisotop); Meßwert (M+H)⁺: 415.10
b) N-{2-(4-Chlor-phenyl)-1-[(2,2-diethoxy-ethyl)-isopropyl-carbamoyl]-ethyl}-3-(2,4-difluor-benzolsulfonylamino)-2-methansulfonylamino-propionamid
   Die Synthese erfolgt analog zu Beispiel 21e) ausgehend von 2-Benzyloxycarbonylamino-3-(2,4-difluor-benzolsulfonylamino)-propionsäure. Man erhält das gewünschte Produkt mit MG = 53.27 (berechnet, monoisotop); Meßwert (M-C₂H₆O+H)⁺: 707.7
c) [6-(4-Chlor-benzyl)-1-(2,4-difluor-benzolsulfonyl)-8-isopropyl-4,7-dioxo-octahydro-pyrazino[1,2-a]pyrimidin-3-yl]-carbaminsäurebenzylester
   Die Synthese erfolgt analog zu Beispiel 21f) ausgehend von N-{2-(4-Chlor-phenyl)-1-[(2,2-diethoxy-ethyl)-isopropyl-carbamoyl]-ethyl} -3-(2,4-difluor-benzolsulfonylamino)-2-methansulfonylamino-propionamid. Man erhält das gewünschte Produkt mit MG = 660 (berechnet, monoisotop); Meßwert (M+H)⁺: 661.32
d) 3-Amino-6-(4-chlor-benzyl)-1-(2,4-difluor-benzolsulfonyl)-8-isopropyl-hexahydro-pyrazino[1,2-a]pyrimidin-4,7-dion
   Die Synthese erfolgt analog Bsp. 21g) ausgehend von [6-(4-Chlor-benzyl)-1-(2,4-difluor-benzolsulfonyl)-8-isopropyl-4,7-dioxo-octahydro- pyrazino[1,2-a]pyrimidin-3-yl]-carbaminsäurebenzylester. Man erhält das gewünschte Produkt mit MG = 526 (berechnet, monoisotop); Meßwert (M+H)⁺: 527.10
e) 3-Amino-6-(4-chlor-benzyl)-1-(2,4-difluor-benzolsulfonyl)-8-isopropyl-hexahydro-pyrazino[1,2-a]pyrimidin-4,7-dion
   Die Synthese erfolgt analog Bsp. 26 ausgehend 3-Amino-6-(4-chlor-benzyl)-1-(2,4-difluor-benzolsulfonyl)-8-isopropyl-hexahydro- pyrazino[1,2-a]pyrimidin-4,7-dion. Man erhält das gewünschte Produkt mit MG = 604.1 (berechnet, monoisotop); Meßwert (M+H)⁺: 605.11

### Beispiel 107

### N-[6-(4-Chlor-benzyl)-1-(3,4-difluor-benzolsulfonyl)-8-isopropyl-4,7-dioxo-octahydro-pyrazino[1,2-a]pyrimidin-3-yl]-methansulfonamid

### Struktur:

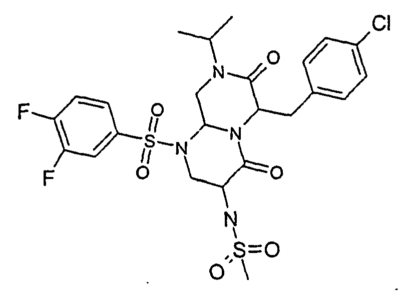

a) 2-Benzyloxycarbonylamino-3-(3,4-difluor-benzolsulfonylamino)-propionsäure
   Die Synthese erfolgt analog zu Beispiel 21d) ausgehend von 3,4-Difluor-benzolsulfonyl chlorid. Man erhält das gewünschte Produkt mit MG = 414.40 (berechnet, monoisotop); Meßwert (M+H)⁺: 415.10
b) N-{2-(4-Chlor-phenyl)-1-[(2,2-diethoxy-ethyl)-isopropyl-carbamoyl]-ethyl]-3-(3,4-difluor-benzolsulfonylamino)-2-methansulfonylamino-propionamid
   Die Synthese erfolgt analog zu Beispiel 21e) ausgehend von 2-Benzyloxycarbonylamino-3-(3,4-difluor-benzolsulfonylamino)-propionsäure. Man erhält das gewünschte Produkt mit MG = 753.27 (berechnet, monoisotop); Meßwert (M-C₂H₆O+H)⁺: 707.7
c) [6-(4-Chlor-benzyl)-1-(3,4-difluor-benzolsulfonyl)-8-isopropyl-4,7-dioxo-octahydro-pyrazino[1,2-a]pyrimidin-3-yl]-carbaminsäurebenzylester
   Die Synthese erfolgt analog zu Beispiel 21f) ausgehend von N-{2-(4-Chlor-phenyl)-1-[(2,2-diethoxy-ethyl)-isopropyl-carbamoyl]-ethyl}-3-(3,4-difluor-benzolsulfonylamino)-2-methansulfonylamino-propionamid. Man erhält das gewünschte Produkt mit MG = 660 (berechnet, monoisotop); Meßwert (M+H)⁺: 661.31
d) 3-Amino-6-(4-chlor-benzyl)-1-(3,4-difluor-benzolsulfonyl)-8-isopropyl-hexahydro-pyrazino[1,2-a]pyrimidin-4,7-dion
   Die Synthese erfolgt analog Bsp. 21g) ausgehend von [6-(4-Chlor-benzyl)-1-(3,4-difluor-benzolsulfonyl)-8-isopropyl-4,7-dioxo-octahydro- pyrazino[1,2-a]pyrinlidin-3-yl]-carbaminsäurebenzylester. Man erhält das gewünschte Produkt mit MG = 526(berechnet, monoisotop); Meßwert (M+H)⁺: 527.10
e) 3-Amino-6-(4-chlor-benzyl)-1-(3,4-difluor-benzolsulfonyl)-8-isopropyl-hexahydro-pyrazino[1,2-a]pyrimidin-4,7-dion
   Die Synthese erfolgt analog Bsp. 26 ausgehend 3-Amino-6-(4-chlor-benzyl)-1-(3,4-difluor-benzolsulfonyl)-8-isopropyl-hexahydro- pyrazmo[1,2-a]pyrimidin-4,7-dion. Man erhält das gewünschte Produkt mit MG = 604.1 (berechnet, monoisotop); Meßwert (M+H)⁺: 605.11

### Beispiel 108

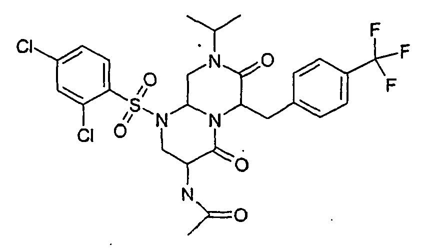

a) [1-[(2,2-Diethoxy-ethyl)-isopropyl-carbamoyl]-2-(4-trifluormethyl-phenyl)-ethyl]-carbaminsäure 9H-fluoren-9-ylmethylester
   Die Synthese erfolgt analog zu Beispiel 29a) (Methode B) ausgehend von N-Fmoc-4-CF₃-Phe-OH. Man erhält das gewünschte Produkt mit MG = 612.28 (berechnet, monoisotop); Meßwert (M+Na)⁺: 635.28
b) 2-Amino-3-(4-trifluormethyl-phenyl)-N-(2,2-diethoxy-ethyl)-N-isopropyl-propionamid
   Die Synthese erfolgt analog zu Beispiel 29b) (Methode B) ausgehend von [1-[(2,2-Diethoxyethyl)-isopropyl-carbamoyl]-2-(4-trifluormethyl-phenyl)-ethyl]- carbaminsäure 9H-fluoren-9-ylmethylester. Man erhält das gewünschte Produkt mit MG = 390.21 (berechnet, monoisotop); Meßwert (M+Na)⁺: 413.22
c) (2-Benzyloxycarbonylamino-2-{2-(4-trifluormethyl-phenyl)-1-[(2,2-diethoxy-ethyl)-isopropyl-carbamoyl]-ethylcarbamoyl}-ethyl)-carbaminsäure 9H-fluoren-9-ylmethylester
   Die Synthese erfolgte analog zu Beispiel 29c) (Methode B) ausgehend von 2-Amino-3-(4-trifluormethyl-phenyl)-N-(2,2-diethoxy-ethyl)-N-isopropyl-propionamid.. Man erhält das gewünschte Produkt mit MG = 832.37 (berechnet, monoisotop); Meßwert (M+Na)⁺: 855.0
d) (2-Amino-1-{2-(4-trifluormethyl-phenyl)-1-[(2,2-diethoxy-ethyl)-isopropyl-carbamoyl]-ethylcarbamoyl}-ethyl)-carbaminsäurebenzylester
   Die Synthese erfolgte analog zu Beispiel 29d) (Methode B) ausgehend von (2-Benzyloxycarbonylamino-2-{2-(4-trifluormethyl-phenyl)-1-[(2,2-diethoxy-ethyl)-isopropyl-carbamoyl]-ethylcarbamoyl}-ethyl)-carbaminsäure 9H-fluoren-9-ylmethylester. Man erhält das gewünschte Produkt mit MG = 610.30 (berechnet, monoisotop); Meßwert (M+Na)⁺: 611.33
e) [1-{2-(4-Trifluormethyl-phenyl)-1-[(2,2-diethoxy-ethyl)-isopropyl-carbamoyl]-ethylcarbamoyl}-2-(2,4-dichlor-benzolsulfonylamino)-ethyl]-carbaminsäurebenzylester
   Die Synthese erfolgte analog zu Beispiel 29e) (Methode B) ausgehend von (2-Amino-1-{2-(4-trifluormethyl-phenyl)-1-[(2,2-diethoxy-ethyl)-isopropyl-carbamoyl]-ethylcarbamoyl}-ethyl)-carbaminsäurebenzylester. Man erhält das gewünschte Produkt mit MG = 818.21 (berechnet, monoisotop); Meßwert (M+Na)⁺: 841.2.
f) [6-(4-Trifluormethyl-benzyl)-1-(2,4-dichlor-benzolsulfonyl)-8-isopropyl-4,7-dioxo-octahydro- pyrazino[1,2-a]pyrimidin-3-yl]-carbaminsäurebenzylester
   Die Synthese erfolgte analog zu Beispiel 29f) (Methode B) ausgehend von ) [1-{2-(4-Trifluormethyl-phenyl)-1-[(2,2-diethoxy-ethyl)-isopropyl-carbamoyl]-ethylcarbamoyl]-2-(2,4-dichlor-benzolsulfonylamino)-ethyl]-carbaminsäurebenzylester (. Man erhält das gewünschte Produkt mit MG = 726.13 (berechnet, monoisotop); Meßwert (M+H)⁺: 727.14
g) 3-Amino-6-(4-trifluormethyl-benzyl)-1-(2,4-dichlor-benzolsulfonyl)-8-isopropyl-hexahydro-pyrazino[1,2-a]pyrimidin-4,7-dion
   Die Synthese erfolgte analog zu Beispiel 29g) (Methode B) ausgehend von) [6-(4-Trifluormethyl-benzyl)-1-(2,4-dichlor-benzolsulfonyl)-8-isopropyl-4,7-dioxo-octahydro-pyrazino[1,2-a]pyrimidin-3-yl]-carbaminsäurebenzylester. Man erhält das gewünschte Produkt mit MG = 592.09 (berechnet, monoisotop); Meßwert (M+Na)⁺: 593.09
h) N-[6-(4-Trifluormethyl-benzyl)-1-(2,4-dichlor-benzolsulfonyl)-8-isopropyl-4,7-dioxo-octahydro-pyrazino[1,2-a]pyrimidin-3-yl]-acetamid
   Die Synthese erfolgt analog zu Beispiel 21h) ausgehend von 3-Amino-6-(4-trifluormethyl-benzyl)-1-(2,4-dichlor-benzolsulfonyl)-8-isopropyl-hexahydro- pyrazino[1,2-a]pyrimidin-4,7-dion. Man erhält das gewünschte Produkt mit MG = 634.10 (berechnet, monoisotop); Meßwert (M+H)⁺: 635.12

### Beispiel 109

### Cyclopropancarbonsäure [1-(2,4-dichlor-benzolsulfonyl)-8-isopropyl-4,7-dioxo-6-(4-trifluormethyl-benzyl)-octahydro-pyrazino[1,2-a]pyrimidin-3-yl]-amid

### Strukture:

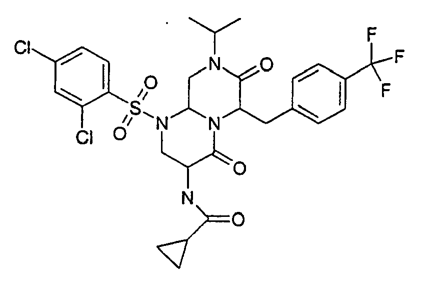

Die Synthese erfolgt analog zu Beispiel 22 ausgehend von 3-Amino-6-(4-trifluormethyl-benzyl)-1-(2,4-dichlor-benzolsulfonyl)-8-isopropyl-hexahydro- pyrazino[1,2-a]pyrimidin-4,7-dion. Man erhält das gewünschte Produkt mit MG = 660.12 (berechnet, monoisotop); Meßwert (M+H)⁺: 661.13

### Beispiel 110

### Cyclobutancarbonsäure [1-(2,4-dichlor-benzolsulfonyl)-8-isopropyl-4,7-dioxo-6-(4-trifluormethyl-benzyl)-octahydro-pyrazino[1,2-a]pyrimidin-3-yl]-amid

### Struktur:

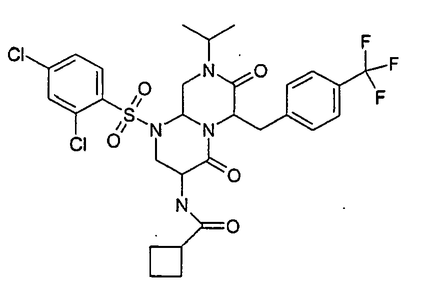

Die Synthese erfolgt analog zu Beispiel 30 ausgehend von 3-Amino-6-(4-trifluormethyl-benzyl)-1-(2,4-dichlor-benzolsulfonyl)-8-isopropyl-hexahydro- pyrazino[1,2-a]pyrimidin-4,7-dion. Man erhält das gewünschte Produkt mit MG = 674.13 (berechnet, monoisotop); Meßwert (M+H)⁺: 675.15

### Beispiel 111

### Cyclopentancarbonsäure [1-(2,4-dichlor-benzolsulfonyl)-8-isopropyl-4,7-dioxo-6-(4-trifluormethyl-benzyl)-octahydro-pyrazino[1,2-a]pyrimidin-3-yl]-amid

### Struktur:

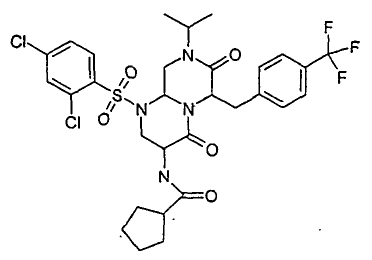

Die Synthese erfolgt analog zu Beispiel 31 ausgehend von 3-Amino-6-(4-trifluormethyl-benzyl)-1-(2,4-dichlor-benzolsulfonyl)-8-isopropyl-hexahydro- pyrazino[1,2-a]pyrimidin-4,7-dion. Man erhält das gewünschte Produkt mit MG = 688.15 (berechnet, monoisotop); Meßwert (M+H)⁺: 689.16

### Beispiel 112

### Pyrrolidin-2-carbonsäure [1-(2,4-dichlor-benzolsulfonyl)-8-isopropyl-4,7-dioxo-6- (4-trifluormethyl-benzyl)-octahydro-pyrazino[1,2-a]pyrimidin-3-yl]-amid

### Struktur:

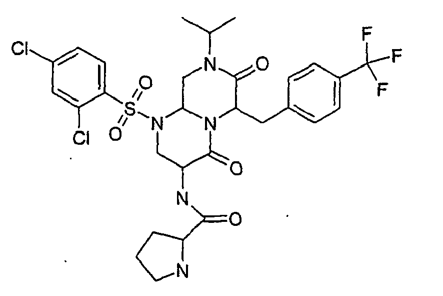

Die Synthese erfolgt analog zu Beispiel 40 ausgehend von 3-Amino-6-(4-trifluormethyl-benzyl)-1-(2,4-dichlor-benzolsulfonyl)-8-isopropyl-hexahydro- pyrazino[1,2-a]pyrimidin-4,7-dion. Man erhält das gewünschte Produkt mit MG = 689.15 (berechnet, monoisotop); Meßwert (M+H)⁺: 690.10.

### Beispiel 113

### N-[1-(2,4-Dichlor-benzolsulfonyl)-8-isopropyl-4,7-dioxo-6-(4-trifluonnethyl-benzyl)-octahydro-pyrazino[1,2-a]pyrimidin-3-yl]-4-dimethylamino-benzamid

### Struktur:

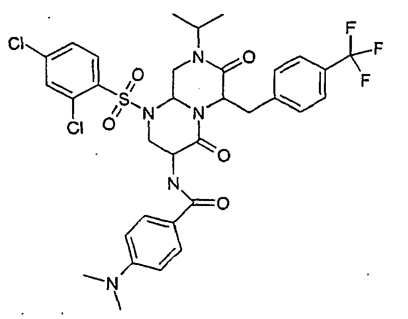

Die Synthese erfolgt analog zu Beispiel 25 ausgehend von 3-Amino-6-(4-trifluormethyl-benzyl)-1-(2,4-dichlor-benzolsulfonyl)-8-isopropyl-hexahydro- pyrazino[1,2-a]pyrimidin-4,7-dion. Man erhält das gewünschte Produkt mit MG = 739.16 (berechnet, monoisotop); Meßwert (M+H)⁺: 740.15

### Beispiel 114

### 1-tert-Butyl-3-[1-(2,4-dichlor-benzolsulfonyl)-8-isopropyl-4,7-dioxo-6-(4-trifluormethyl-benzyl)-octahydro-pyrazino[1,2-a]pyrimidin-3-yl]-harnstoff

### Struktur:

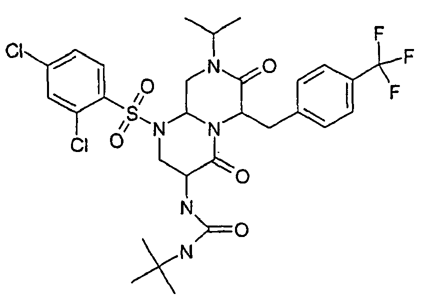

Die Synthese erfolgt analog zu Beispiel 28 ausgehend von 3-Amino-6-(4-trifluormethyl-benzyl)-1-(2,4-dichlor-benzolsulfonyl)-8-isopropyl-hexahydro- pyrazino[1,2-a]pyrimidin-4,7-dion. Man erhält das gewünschte Produkt mit MG = 691.16 (berechnet, monoisotop); Meßwert (M+H)⁺: 692.18

### Beispiel 115

### N-[1-(2,4-Dichlor-benzolsulfonyl)-8-isopropyl-4,7-dioxo-6-(4-trifluormethyl-benzyl)-octahydro-pyrazino[1,2-a]pyrimidin-3-yl]-methansulfonamid

### Struktur:

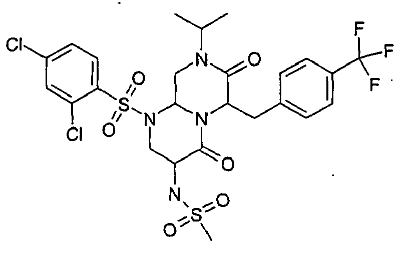

Die Synthese erfolgt analog zu Beispiel 26 ausgehend von 3-Amino-6-(4-trifluormethyl-benzyl)-1-(2,4-dichlor-benzolsulfonyl)-8-isopropyl-hexahydro- pyrazino[1,2-a]pyrimidin-4,7-dion. Man erhält das gewünschte Produkt mit MG = 670.07 (berechnet, monoisotop); Meßwert (M+H)⁺: 671.08

### Beispiel 116

### Cyclopropansulfonsäure [1-(2,4-dichlor-benzolsulfonyl)-8-isopropyl-4,7-dioxo-6-(4-trifluormethyl-benzyl)-octahydro-pyrazino[1,2-a]pyrimidin-3-yl]-amid

### Struktur:

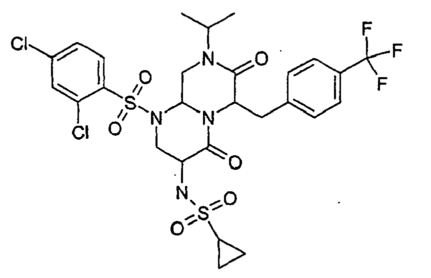

Die Synthese erfolgt analog zu Beispiel 27 ausgehend von 3-Amino-6-(4-trifluormethyl-benzyl)-1-(2,4-dichlor-benzolsulfonyl)-8-isopropyl-hexahydro- pyrazino[1,2-a]pyrimidin-4,7-dion. Man erhält das gewünschte Produkt mit MG = 696.09 (berechnet, monoisotop); Meßwert (M+H)⁺: 697.0

### Beispiel 117

### 1-(2,4-Dichlor-benzolsulfonyl)-3-dimethylamino-8-isopropyl-6-(4-trifluormethyl-benzyl)-hexahydro-pyrazino[1,2-a]pyrimidin-4,7-dion

### Struktur:

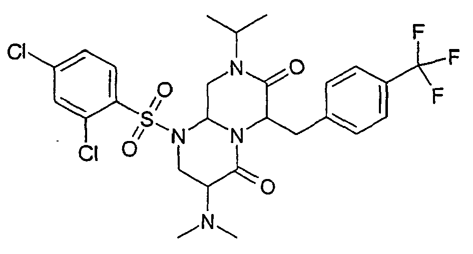

Die Synthese erfolgt analog zu Beispiel 72 ausgehend von 3-Amino-6-(4-trifluormethyl-benzyl)-1-(2,4-dichlor-benzolsulfonyl)-8-isopropyl-hexahydro- pyrazino[1,2-a]pyrimidin-4,7-dion. Man erhält das gewünschte Produkt mit MG = 620.12 (berechnet, monoisotop); Meßwert (M+H)⁺: 621.09

### Beispiel 118

### 3-Azetidin-1-yl-1-(2,4-d.ichlor-benzolsulfonyl)-8-isopropyl-6-(4-trifluormethyl-benzyl)-hexahydro-pyrazino[1,2-a]pyrimidin-4,7-dion

### Struktur:

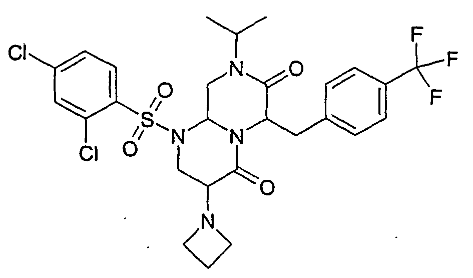

Die Synthese erfolgt analog zu Beispiel 73 ausgehend von 3-Amino-6-(4-trifluormethyl-benzyl)-1-(2,4-dichlor-benzolsulfonyl)-8-isopropyl-hexahydro- pyrazino[1,2-a]pyrimidin-4,7-dion. Man erhält das gewünschte Produkt mit MG = 632.12 (berechnet, monoisotop); Meßwert (M+H)⁺: 633.0

### Beispiel 119

### 1-(2,4-Dichlor-benzolsulfonyl)-8-isopropyl-3-pywolidin-1-yl-6-(4-trifluormethyl- benzyl)-hexahydro-pyrazino[1,2-a]pyrimidin-4,7-dion

### Struktur:

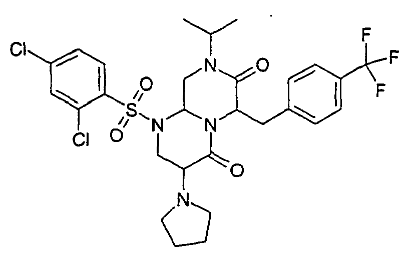

Die Synthese erfolgt analog zu Beispiel 74 ausgehend von 3-Amino-6-(4-trifluormethyl-benzyl)-1-(2,4-dichlor-benzolsulfonyl)-8-isopropyl-hexahydro- pyrazino[1,2-a]pyrimidin-4,7-dion. Man erhält das gewünschte Produkt mit MG = 646.14 (berechnet, monoisotop); Meßwert (M+H)⁺: 647.14

### Beispiel 120

### 1-(2,4-Dichlor-benzolsulfonyl)-8-isopropyl-3-piperidin-1-yl-6-(4-trifluormethyl- benzyl)-hexahydro-pyrazino[1,2-a]pyrimidin-4,7-dion

### Struktur:

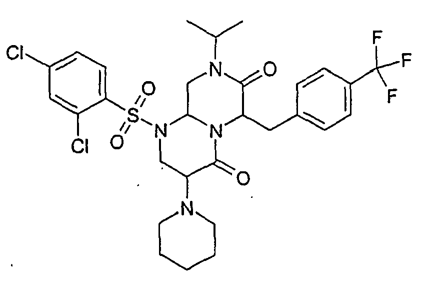

Die Synthese erfolgt analog zu Beispiel 59 ausgehend von 3-Amino-6-(4-trifluormethyl-benzyl)-1-(2,4-dichlor-benzolsulfonyl)-8-isopropyl-hexahydro- pyrazino[1,2-a]pyrimidin-4,7-dion. Man erhält das gewünschte Produkt mit MG = 660.16 (berechnet, monoisotop); Meßwert (M+H)⁺: 661.13

### Beispiel 121

### 1-(2,4-Dichlor-benzolsulfonyl)-8-isopropyl-3-morpholin-4-yl-6-(4-trifluormethyl- benzyl)-hexahydro-pyrazino[1,2-a]pyrimidin-4,7-dion

### Struktur:

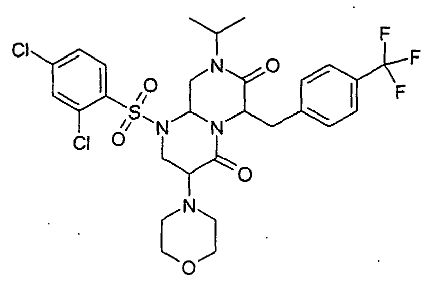

Die Synthese erfolgt analog zu Beispiel 60 ausgehend von 3-Amino-6-(4-trifluormethyl-benzyl)-1-(2,4-dichlor-benzolsulfonyl)-8-isopropyl-hexahydro- pyrazino[1,2-a]pyrimidin-4,7-dion. Man erhält das gewünschte Produkt mit MG = 662.13 (berechnet, monoisotop); Meßwert (M+H)⁺: 663.07

### Beispiel 122

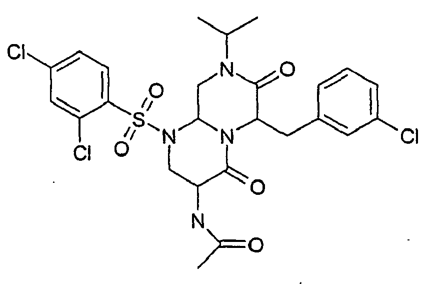

a) [1-[(2,2-Diethoxy-ethyl)-isopropyl-carbamoyl]-2-(3-chlor-phenyl)-ethyl]- carbaminsäure 9H-fluoren-9-ylmethylester
   Die Synthese erfolgt analog zu Beispiel 29a) (Methode B) ausgehend von N-Fmoc-3-CI-Phe-OH. Man erhält das gewünschte Produkt mit MG = 578.25 (berechnet, monoisotop); Meßwert (M+H)⁺: 579.27
b) 2-Amino-3-(3-chlor-phenyl)-N-(2,2-diethoxy-ethyl)-N-isopropyl-propionamid
   Die Synthese erfolgt analog zu Beispiel 29b) (Methode B) ausgehend von [1-[(2,2-Diethoxyethyl)-isopropyl-carbamoyl]-2-(3-chlor-phenyl)-ethyl]- carbaminsäure 9H-fluoren-9-ylmethylester. Man erhält das gewünschte Produkt mit MG = 356.19 (berechnet, monoisotop); Meßwert (M+Na)⁺: 379.18
c) (2-Benzyloxycarbonylamino-2-{2-(3-chlor-phenyl)-1-[(2,2-diethoxy-ethyl)-isopropyl-carbamoyl]-ethylcarbamoyl}-ethyl)-carbaminsäure 9H-fluoren-9-ylmethyl ester
   Die Synthese erfolgte analog zu Beispiel 29c) (Methode B) ausgehend von 2-Amino-3-(3-chlor-phenyl)-N-(2,2-diethoxy-ethyl)-N-isopropyl-propionamid.. Man erhält das gewünschte Produkt mit MG = 798.34 (berechnet, monoisotop); Meßwert (M+H)⁺: 799.35
d) (2-Amino-1-{2-(3-chlor-phenyl)-1-[(2,2-diethoxy-ethyl)-isopropyl-carbamoyl)-ethylcarbamoyl}-ethyl)-carbaminsäurebenzylester
   Die Synthese erfolgte analog zu Beispiel 29d) (Methode B) ausgehend von (2-Benzyloxycarbonylamino-2-{2-(3-chlor-phenyl)-1-[(2,2-diethoxy-ethyl)-isopropyl-carbamoyl]-ethylcarbamoyl}-ethyl)-carbaminsäure 9H-fluoren-9-ylmethyl ester. Man erhält das gewünschte Produkt mit MG = 576.27 (berechnet, monoisotop); Meßwert (M+H)⁺: 577.22
e) [1-{2-(3-Chlor-phenyl)-1-[(2,2-diethoxy-ethyl)-isopropyl-carbamoyl]-ethylcarbamoyl}-2-(2,4-dichlor-benzolsulfonylamino)-ethyl]-carbaminsäurebenzylester
   Die Synthese erfolgte analog zu Beispiel 29e) (Methode B) ausgehend von (2-Amino-1-{2-(3-chlor-phenyl)-1-[(2,2-diethoxy-ethyl)-isopropyl-carbamoyl]-ethylcarbamoyl}-ethyl)-carbaminsäurebenzylester. Man erhält das gewünschte Produkt mit MG = 784.19 (berechnet, monoisotop); Meßwert (M+H)⁺: 785.18.
f) [6-(3-Chlor-benzyl)-1-(2,4-dichlor-benzolsulfonyl)-8-isopropyl-4,7-dioxo-octahydro-pyrazino[1,2-a]pyrimidin-3-yl]-carbaminsäurebenzylester
   Die Synthese erfolgte analog zu Beispiel 29f) (Methode B) ausgehend von [1-{2-(3-Chlorphenyl)-1-[(2,2-diethoxy-ethyl)-isopropyl-carbamoyl]-ethylcarbamoyl}-2-(2,4-dichlor-benzolsulfonylamino)-ethyl]-carbaminsäurebenzylester. Man erhält das gewünschte Produkt mit MG = 692.10 (berechnet, monoisotop); Meßwert (M+H)⁺: 693.08
g) 3-Amino-6-(3-chlor-benzyl)-1-(2,4-dichlor-benzolsulfonyl)-8-isopropyl-hexahydro-pyrazino[1,2-a]pyrimidin-4,7-dion
   Die Synthese erfolgte analog zu Beispiel 29g) (Methode B) ausgehend von) [6-(3-Chlorbenzyl)-1-(2,4-dichlor-benzolsulfonyl)-8-isopropyl-4,7-dioxo-octahydro- pyrazino[1,2-a]pyrimidin-3-yl]-carbaminsäurebenzylester. Man erhält das gewünschte Produkt mit MG = 558.07 (berechnet, monoisotop); Meßwert (M+H)⁺: 559.07
h) N-[6-(3-Chlor-benzyl)-1-(2,4-dichlor-benzolsulfonyl)-8-isopropyl-4,7-dioxo- octahydro-pyrazino[1,2-a]pyrimidin-3-yl]-acetamid
   Die Synthese erfolgt analog zu Beispiel 21h) ausgehend von 3-Amino-6-(3-chlor-benzyl)-1-(2,4-dichlor-benzolsulfonyl)-8-isopropyl-hexahydro- pyrazino[1,2-a]pyrimidin-4,7-dion. Man erhält das gewünschte Produkt mit MG = 600.08 (berechnet, monoisotop); Meßwert (M+H)⁺: 601.07

### Beispiel 123

### Cyclopropancarbonsäure [6-(3-chlor-benzyl)-1-(2,4-dichlor-benzolsulfonyl)-8- isopropyl-4,7-dioxo-octahydro-pyrazino[1,2-a]pyrimidin-3-yl]-axnid

### Struktur:

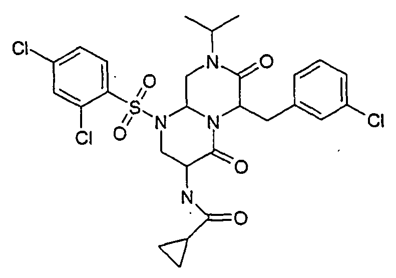

Die Synthese erfolgt analog zu Beispiel 22 ausgehend von 3-Amino-6-(3-chlor-benzyl)-1-(2,4-dichlor-benzolsulfonyl)-8-isopropyl-hexahydro- pyrazino[1,2-a]pyrimidin-4,7-dion. Man erhält das gewünschte Produkt mit MG = 626.09 (berechnet, monoisotop); Meßwert (M+H)⁺: 627.08

### Beispiel 124

### Cyclobutancarbonsäure [6-(3-chlor-benzyl)-1-(2,4-dichlor-benzolsulfonyl)-8- isopropyl-4,7-dioxo-octahydro-pyrazino[1,2-a]pyrimidin-3-yl]-amid

### Struktur:

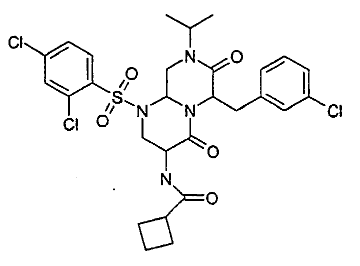

Die Synthese erfolgt analog zu Beispiel 30 ausgehend von 3-Amino-6-(3-chlor-benzyl)-1-(2,4-dichlor-benzolsulfonyl)-8-isopropyl-hexahydro- pyrazino[1,2-a]pyrimidin-4,7-dion. Man erhält das gewünschte Produkt mit MG = 640.11 (berechnet, monoisotop); Meßwert (M+H)⁺: 641.08

### Beispiel 125

### Cyclopentancarbonsäure [6-(3-chlor-benzyl)-1-(2,4-dichlor-benzolsulfonyl)-8- isopropyl-4,7-dioxo-octahydro-pyrazino[1,2-a]pyrimidin-3-yl]-amid

### Struktur:

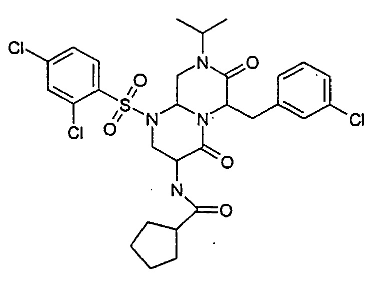

Die Synthese erfolgt analog zu Beispiel 31 ausgehend von 3-Amino-6-(3-chlor-benzyl)-1-(2,4-dichlor-benzolsulfonyl)-8-isopropyl-hexahydro- pyrazino[1,2-a]pyrimidin-4,7-dion. Man erhält das gewünschte Produkt mit MG = 654.12 (berechnet, monoisotop); Meßwert (M+H)⁺: 655.09

### Beispiel 126

### N-[6-(3-Chlor-benzyl)-1-(2,4-dichlor-benzolsulfonyl)-8-isopropyl-4,7-dioxo-octahydro-pyrazino[1,2-a]pyrimidin-3-yl]-4-dimethylamino-benzamid

### Struktur:

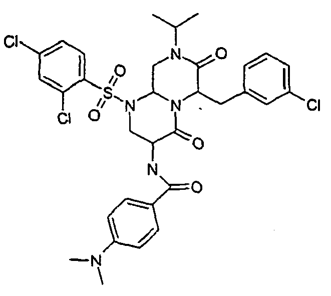

Die Synthese erfolgt analog zu Beispiel 25 ausgehend von 3-Amino-6-(3-chlor-benzyl)-1-(2,4-dichlor-benzolsulfonyl)-8-isopropyl-hexahydro- pyrazino[1,2-a]pyrimidin-4,7-dion. Man erhält das gewünschte Produkt mit MG = 705.13 (berechnet, monoisotop); Meßwert (M+H)⁺: 706.10

### Beispiel 127

### 1-tert-Butyl-3-[6-(3-chlor-benzyl)-1-(2,4-dichlor-benzolsulfonyl)-8-isopropyl-4,7- dioxo-octahydro-pyrazino[1,2-a]pyrimidin-3-yl]-harnstoff

### Struktur:

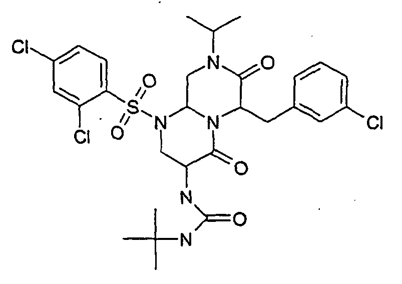

Die Synthese erfolgt analog zu Beispiel 28 ausgehend von 3-Amino-6-(3-chlor-benzyl)-1-(2,4-dichlor-benzolsulfonyl)-8-isopropyl-hexahydro- pyrazino[1,2-a]pyrimidin-4,7-dion. Man erhält das gewünschte Produkt mit MG = 657.13 (berechnet, monoisotop); Meßwert (M+H)⁺: 658.11

### Beispiel 128

### N-[6-(3-Chlor-benzyl)-1-(2,4-dichlor-benzolsulfonyl)-8-isopropyl-4,7-dioxo-octahydro-pyrazino[1,2-a]pyrimidin-3-yl]-methansulfonamid

### Struktur:

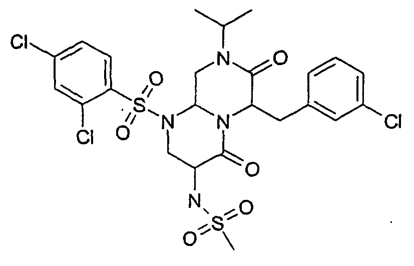

Die Synthese erfolgt analog zu Beispiel 26 ausgehend von 3-Amino-6-(3-chlor-benzyl)-1-(2,4-dichlor-benzolsulfonyl)-8-isopropyl-hexahydro- pyrazino[1,2-a]pyrimidin-4,7-dion. Man erhält das gewünschte Produkt mit MG = 636.04 (berechnet, monoisotop); Meßwert (M+H)⁺: 637.03

### Beispiel 129

### 6-(3-Chlor-benzyl)-1-(2,4-dichlor-benzolsulfonyl)-3-dimethylamino-8-isopropyl- hexahydro-pyrazino[1,2-a]pyrimidin-4,7-dion

### Struktur:

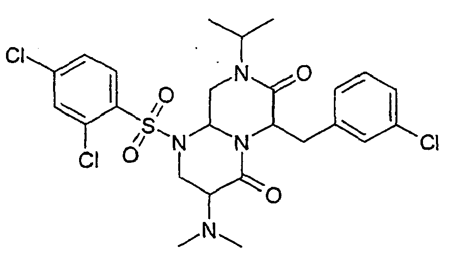

Die Synthese erfolgt analog zu Beispiel 72 ausgehend von 3-Amino-6-(3-chlor-benzyl)-1-(2,4-dichlor-benzolsulfonyl)-8-isopropyl-hexahydro- pyrazino[1,2-a]pyrimidin-4,7-dion. Man erhält das gewünschte Produkt mit MG = 586.10 (berechnet, monoisotop); Meßwert (M+H)⁺: 587.09

### Beispiel 130

### 6-(3-Chlor-benzyl)-1-(2,4-dichlor-benzolsulfonyl)-8-isopropyl-3-pyrrolidin-1-yl- hexahydropyrazino[1 ,2-a]pyrimidin-4,7-dion

### Struktur:

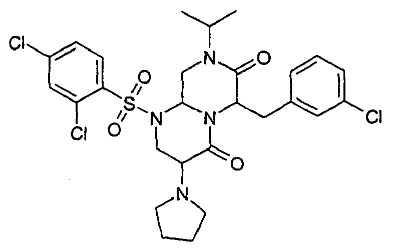

Die Synthese erfolgt analog zu Beispiel 74 ausgehend von 3-Amino-6-(3-chlor-benzyl)-1-(2,4-dichlor-benzolsulfonyl)-8-isopropyl-hexahydro- pyrazino[1,2-a]pyrimidin-4,7-dion. Man erhält das gewünschte Produkt mit MG = 612.11 (berechnet, monoisotop); Meßwert (M+H)⁺: 613.10

### Beispiel 131

### 6-(3-Chlor-benzyl)-1-(2,4-dichlor-benzolsulfonyl)-8-isopropyl-3-piperidin-1-yl- hexahydropyrazino[1,2-a]pyrimidin-4,7-dion

### Struktur:

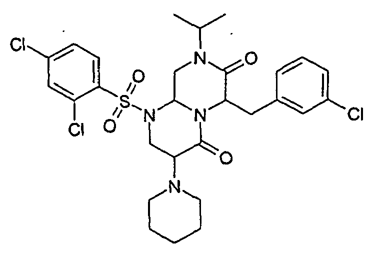

Die Synthese erfolgt analog zu Beispiel 59 ausgehend von 3-Amino-6-(3-chlor-benzyl)-1-(2,4-dichlor-benzolsulfonyl)-8-isopropyl-hexahydro- pyrazino[1,2-aapyrimidin-4,7-dion. Man erhält das gewünschte Produkt mit MG = 626.13 (berechnet, monoisotop); Meßwert (M+H)⁺: 627.13

### Beispiel 132

### 6-(3-Chlor-benzyl)-1-(2,4-dichlor-benzolsulfonyl)-8-isopropyl-3-morpholin-4-yl- hexahydropyrazino[1,2-a]pyrimidin-4,7-dion

### Struktur:

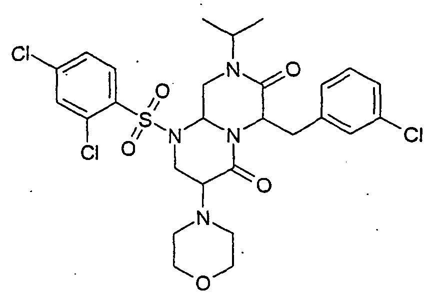

Die Synthese erfolgt analog zu Beispiel 60 ausgehend von 3-Amino-6-(3-chlor-benzyl)-1-(2,4-dichlor-benzolsulfonyl)-8-isopropyl-hexahydro- pyrazino[1,2-a]pyrimidin-4,7-dion. Man erhält das gewünschte Produkt mit MG = 628.11 (berechnet, monoisotop); Meßwert (M+H)⁺:629.09

### Beispiel 133

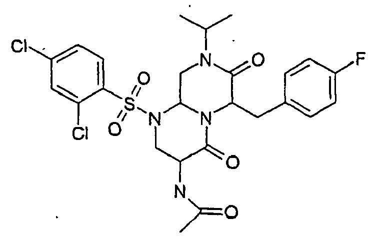

a) [1-[(2,2-Diethoxy-ethyl)-isopropyl-carbamoyl]-2-(4-fluor-phenyl)-ethyl]- carbaminsäure 9H-fluoren-9-yhnethylester
   Die Synthese erfolgt analog zu Beispiel 29a) (Methode B) ausgehend von N-Fmoc-4-F-Phe-OH. Man erhält das gewünschte Produkt mit MG = 562.28 (berechnet, monoisotop); Meßwert (M+H)⁺: 563.27
b) 2-Amino-3-(4-fluor-phenyl)-N-(2,2-diethoxy-ethyl)-N-isopropyl-propionamid
   Die Synthese erfolgt analog zu Beispiel 29b) (Methode B) ausgehend von [1-[(2,2-Diethoxyethyl)-isopropyl-carbamoyl]-2-(4-fluor-phenyl)-ethyl]- carbaminsäure 9H-fluoren-9-ylmethylester. Man erhält das gewünschte Produkt mit MG = 340.22 (berechnet, monoisotop); Meßwert (M+H)⁺: 341.20
c) (2-Benzyloxycarbonylamino-2-{2-(4-fluor-phenyl)-1-[(2,2-diethoxy-ethyl)-isopropyl-carbamoyl]-ethylcarbamoyl}-ethyl)-carbaminsäure 9H-fluoren-9-ylmethyl ester
   Die Synthese erfolgte analog zu Beispiel 29c) (Methode B) ausgehend von 2-Amino-3-(4-fluorphenyl)-N-(2,2-diethoxy-ethyl)-N-isopropyl-propionamid.. Man erhält das gewünschte Produkt mit MG = 782.37 (berechnet, monoisotop); Meßwert (M+Na)⁺: 805.37
d) (2-Amino-1-{2-(4-fluor-phenyl)-1-[(2,2-diethoxy-ethyl)-isopropyl-carbamoyl]-ethylcarbamoyl}-ethyl)-carbaminsäurebenzylester
   Die Synthese erfolgte analog zu Beispiel 29d) (Methode B) ausgehend von (2-Benzyloxycarbonylamino-2-{2-(4-fluor-phenyl)-1-[(2,2-diethoxy-ethyl)-isopropyl-carbamoyl]-ethylcarbamoyl}-ethyl)-carbaminsäure 9H-fluoren-9-ylmethyl ester. Man erhält das gewünschte Produkt mit MG = 560.30 (berechnet, monoisotop); Meßwert (M+H)⁺: 561.31
e) [1-{2-(4-Fluor-phenyl)-1-[(2,2-diethoxy-ethyl)-isopropyl-carbamoyl]-ethylcarbamoyl}-2-(2,4-dichlor-benzolsulfonylamino)-ethyl]-carbaminsäurebenzylester
   Die Synthese erfolgte analog zu Beispiel 29e) (Methode B)ausgehend von (2-Amino-1-{2-(4-fluor-phenyl)-1-[(2,2-diethoxy-ethyl)-isopropyl-carbamoyl]-ethylcarbamoyl} -ethyl)-carbaminsäurebenzylester. Man erhält das gewünschte Produkt mit MG = 768.22 (berechnet, monoisotop); Meßwert (M+Na)⁺:
f) [6-(4-Fluor-benzyl)-1-(2,4-dichlor-benzolsulfonyl)-8-isopropyl-4,7-dioxo-octahydro-pyrazino[1,2-a]pyrimidin-3-yl]-carbaminsäurebenzylester
   Die Synthese erfolgte analog zu Beispiel 29f) (Methode B) ausgehend von) [1-{2-(4-Fluorphenyl)-1-[(2,2-diethoxy-ethyl)-isopropyl-carbamoyl]-ethylcarbamoyl}-2-(2,4-dichlor-benzolsulfonylamino)-ethyl]-carbaminsäurebenzylester . Man erhält das gewünschte Produkt mit MG = 676.13 (berechnet, monoisotop); Meßwert (M+H)⁺: 677.1
g) 3-Amino-6-(4-fluor-benzyl)-1-(2,4-dichlor-benzolsulfonyl)-8-isopropyl-hexahydro-pyrazino[1,2-a]pyrimidin-4,7-dion
   Die Synthese erfolgte analog zu Beispiel 29g) (Methode B) ausgehend von ) [6-(4-Fluorbenzyl)-1-(2,4-dichlor-benzolsulfonyl)-8-isopropyl-4,7-dioxo-octahydro-pyrazino[1,2-a]pyrimidin-3-yl]-carbaminsäurebenzylester. Man erhält das gewünschte Produkt mit MG = 542.1 (berechnet, monoisotop); Meßwert (M+H)⁺: 543.12
h) N-[6-(4-Fluor-benzyl)-1-(2,4-dichlor-benzolsulfonyl)-8-isopropyl-4,7-dioxo- octahydro-pyrazino[1,2-a]pyrimidin-3-yl]-acetamid
   Die Synthese erfolgt analog zu Beispiel 21h) ausgehend von 3-Amino-6-(4-fluor-benzyl)-1-(2,4-dichlor-benzolsulfonyl)-8-isopropyl-hexahydro-pyrazino[1,2-a]pyrimidin-4,7-dion. Man erhält das gewünschte Produkt mit MG = 584.11 (berechnet, monoisotop); Meßwert (M+H)⁺: 585.08

### Beispiel 134

### Cyclopropancarbonsäure [1-(2,4-dichlor-benzolsulfonyl)-6-(4-fluor-benzyl)-8- isopropyl-4,7-dioxo-octahydro-pyrazino[1,2-a]pyrimidin-3-yl]-amid

### Struktur:

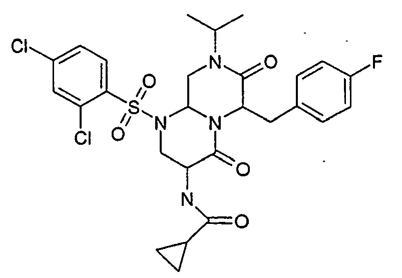

Die Synthese erfolgt analog zu Beispiel 22 ausgehend von 3-Amino-6-(4-fluor-benzyl)-1-(2,4-dichlor-benzolsulfonyl)-8-isopropyl-hexahydro- pyrazino[1,2-a]pyrimidin-4,7-dion. Man erhält das gewünschte Produkt mit MG = 610.12 (berechnet, monoisotop); Meßwert (M+H)⁺: 611.09

### Beispiel 135

### Cyclobutancarbonsäure[1-(2,4-dichlor-benzolsulfonyl)-6-(4-fluor-benzyl)-8- isopropyl-4,7-dioxo-octahydro-pyrazino[1,2-a]pyrimidin-3-yl]-amid

### Struktur:

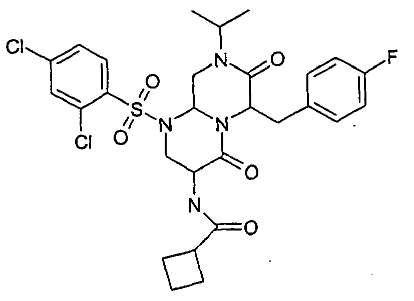

Die Synthese erfolgt analog zu Beispiel 30 ausgehend von 3-Amino-6-(4-fluor-benzyl)-1-(2,4-dichlor-benzolsulfonyl)-8-isopropyl-hexahydro- pyrazino[1,2-a]pyrimidin-4,7-dion. Man erhält das gewünschte Produkt mit MG = 624.14 (berechnet, monoisotop); Meßwert (M+H)⁺: 625.13

### Beispiel 136

### Cyclopentancarbonsäure [1-(2,4-dichlor-benzolsulfonyl)-6-(4-fluor-benzyl)-8- isopropyl-4,7-dioxo-octahydro-pyrazino[1,2-a]pyrimidin-3-yl]-amid

### Struktur:

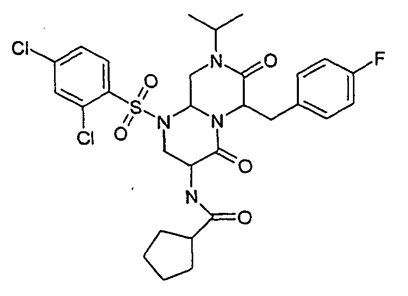

Die Synthese erfolgt analog zu Beispiel 31 ausgehend von 3-Amino-6-(4-fluor-benzyl)-1-(2,4-dichlor-benzolsulfonyl)-8-isopropyl-hexahydro- pyrazino[1,2-a]pyrimidin-4,7-dion. Man erhält das gewünschte Produkt mit MG = 638.15 (berechnet, monoisotop); Meßwert (M+H)⁺: 639.14

### Beispiel 137

### N-[1-(2,4-Dichlor-benzolsulfonyl)-6-(4-fluor-benzyl)-8-isopropyl-4,7-dioxo-octahydro-pyrazino [1,2-a]pyrimidin-3-yl]-4-dimethylamino-benzamid

### Struktur:

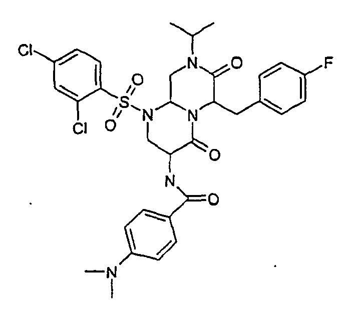

Die Synthese erfolgt analog zu Beispiel 25 ausgehend von 3-Amino-6-(4-fluor-benzyl)-1-(2,4-dichlor-benzolsulfonyl)-8-isopropyl-hexahydro- pyrazino[1,2-a]pyrimidin-4,7-dion. Man erhält das gewünschte Produkt mit MG = 689.16 (berechnet, monoisotop); Meßwert (M+H)⁺: 690.10

### Beispiel 138

### N-[1-(2,4-Dichlor-benzolsulfonyl)-6-(4-fluor-benzyl)-8-isopropyl-4,7-dioxo-octahydro-pyrazino[1,2-a]pyrimidin-3-yl]-methansulfonamid

### Struktur:

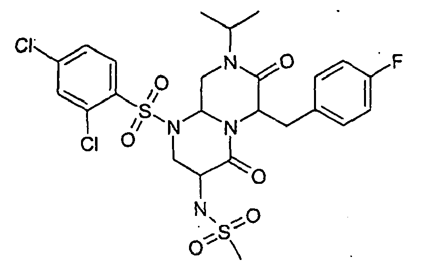

Die Synthese erfolgt analog zu Beispiel 26 ausgehend von 3-Amino-6-(4-fluor-benzyl)-1-(2,4-dichlor-benzolsulfonyl)-8-isopropyl-hexahydro- pyrazino[1,2-a]pyrimidin-4,7-dion. Man erhält das gewünschte Produkt mit MG = 620.07 (berechnet, monoisotop); Meßwert (M+H)⁺: 621.04

### Beispiel 139

### 1-(2,4-Dichlor-benzolsulfonyl)-3-dimethylamino-6-(4-fluor-benzyl)-8-isopropyl-hexahydro-pyrazino[1,2-a]pyrimidin-4,7-dion

### Struktur:

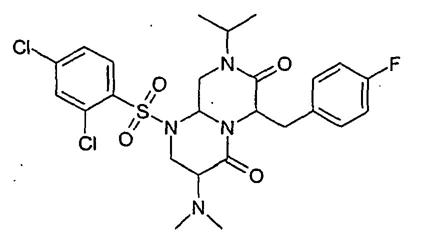

Die Synthese erfolgt analog zu Beispiel 72 ausgehend von 3-Amino-6-(4-fluor-benzyl)-1-(2,4-dichlor-benzolsulfonyl)-8-isopropyl-hexahydro- pyrazino[1,2-a]pyrimidin-4,7-dion. Man erhält das gewünschte Produkt mit MG = 570.13 (berechnet, monoisotop); Meßwert (M+H)⁺: 571.09

### Beispiel 140

### 3-Azetidin-1-yl-1-(2,4-dichlor-benzolsulfonyl)-6-(4-fluor-benzyl)-8-isopropyl- hexahydropyrazino[1,2-a]pyrimidin-4,7-dion

### Struktur:

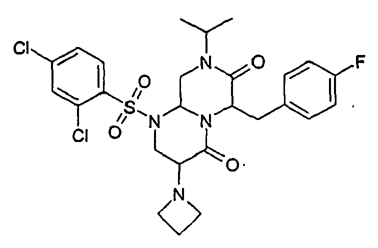

Die Synthese erfolgt analog zu Beispiel 73 ausgehend von 3-Amino-6-(4-fluor-benzyl)-1-(2,4-dichlor-benzolsulfonyl)-8-isopropyl-hexahydro- pyrazino[1,2-a]pyrimidin-4,7-dion. Man erhält das gewünschte Produkt mit MG = 582.13 (berechnet, monoisotop); Meßwert (M+H)⁺: 583.04

### Beispiel 141

### 1-(2,4-Dichlor-benzolsulfonyl)-6-(4-fluor-benzyl)-8-isopropyl-3-pyrrolidin-1-yl- hexahydropyrazino[1,2-a]pyrimidin-4,7-dion

### Struktur:

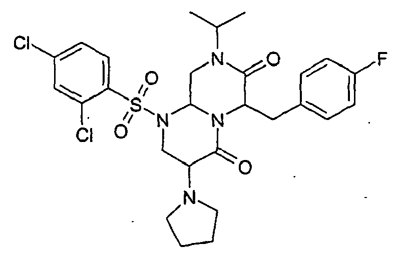

Die Synthese erfolgt analog zu Beispiel 74 ausgehend von 3-Amino-6-(4-fluor-benzyl)-1-(2,4-dichlor-benzolsulfonyl)-8-isopropyl-hexahydro- pyrazino[1,2-a]pyrimidin-4,7-dion. Man erhält das gewünschte Produkt mit MG = 596.14 (berechnet, monoisotop); Meßwert (M+H)⁺: 597.12

### Beispiel 142

### 1-(2,4-Dichlor-benzolsulfonyl)-6-(4-fluor-benzyl)-8-isopropyl-3-piperidin-1-yl-hexahydro-pyrazino[1,2-a]pyrimidin-4,7-dion

### Struktur:

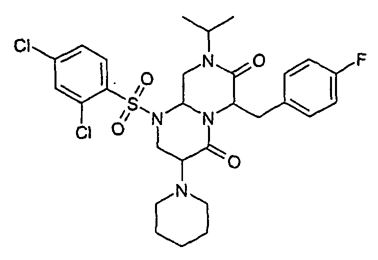

Die Synthese erfolgt analog zu Beispiel 59 ausgehend von 3-Amino-6-(4-fluor-benzyl)-1-(2,4-dichlor-benzolsulfonyl)-8-isopropyl-hexahydro- pyrazino[1,2-a]pyrimidin-4,7-dion. Man erhält das gewünschte Produkts mit MG = 610.16 (berechnet, monoisotop); Meßwert (M+H)⁺: 611.16

### Beispiel 143

### 1-(2,4-Dichlor-benzolsulfonyl)-6-(4-fluor-benzyl)-8-isopropyl-3-morpholin-4-yl-hexahydro-pyrazino[1,2-a]pyrimidin-4,7-dion

### Struktur:

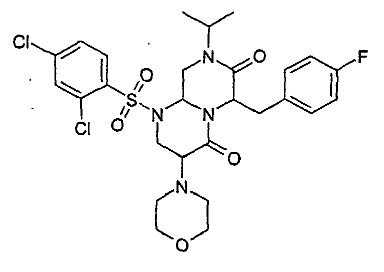

Die Synthese erfolgt analog zu Beispiel 60 ausgehend von 3-Amino-6-(4-fluor-benzyl)-1-(2,4-dichlor-benzolsulfonyl)-8-isopropyl-hexahydro- pyrazino[1,2-a]pyrimidin-4,7-dion. Man erhält das gewünschte Produkt mit MG = 612.14 (berechnet, monoisotop); Meßwert (M+H)⁺: 613.13

### Beispiel 144

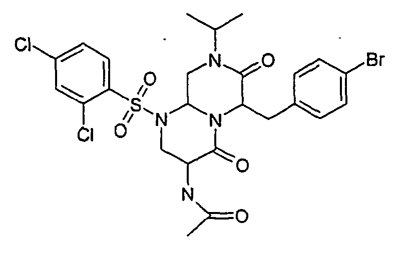

a) [1-[(2,2-Diethoxy-ethyl)-isopropyl-carbamoyl]-2-(4-brom-phenyl)-ethyl]- carbaminsäure 9H-fluoren-9-ylmethylester
   Die Synthese erfolgt analog zu Beispiel 29a) (Methode B) ausgehend von N-Fmoc-4-Br-Phe-OH. Man erhält das gewünschte Produkt mit MG = 622.20 (berechnet, monoisotop); Meßwert (M+Na)⁺: 645.30
b) 2-Amino-3-(4-brom-phenyl)-N-(2,2-diethoxy-ethyl)-N-isopropyl-propionamid
   Die Synthese erfolgt analog zu Beispiel 29b) (Methode B) ausgehend von [1-[(2,2-Diethoxyethyl)-isopropyl-carbamoyl]-2-(4-brom-phenyl)-ethyl]- carbaminsäure -9H-fluoren-9-ylmethylester. Man erhält das gewünschte Produkt mit MG = 400.14 (berechnet, monoisotop); Meßwert (M+H)⁺: 401.10
c) (2-Benzyloxycarbonylamino-2-{2-(4-brom-phenyl)-1-[(2,2-diethoxy-ethyl)-isopropyl-carbamoyl]-ethylcarbamoyl}-ethyl)-carbaminsäure 9H-fluoren-9-ylmethyl ester
   Die Synthese erfolgte analog zu Beispiel 29c) (Methode B) ausgehend von 2-Amino-3-(4-brom-phenyl)-N-(2,2-diethoxy-ethyl)-N-isopropyl-propionamid.. Man erhält das gewünschte Produkt mit MG = 842.29 (berechnet, monoisotop); Meßwert (M+Na)⁺: 865.4
d) (2-Amino-1-{2-(4-brom-phenyl)-1-[(2,2-diethoxy-ethyl)-isopropyl-carbamoyl]-ethylcarbamoyl}-ethyl)-carbaminsäurebenzylester
   Die Synthese erfolgte analog zu Beispiel 29d) (Methode B) ausgehend von (2-Benzyloxycarbonylamino-2-{2-(4-brom-phenyl)-1-[(2,2-diethoxy-ethyl)-isopropyl-carbamoyl]-ethylcarbamoyl}-ethyl)-carbaminsäure 9H-fluoren-9-ylmethyl ester. Man erhält das gewünschte Produkt mit MG = 620.22 (berechnet, monoisotop); Meßwert (M+H)⁺: 621.20
e) [1-{2-(4-Brom-phenyl)-1-[(2,2-diethoxy-ethyl)-isopropyl-carbamoyl]-ethylcarbamoyl}-2-(2,4-dichlor-benzolsulfonylamino)-ethyl]-carbaminsäurebenzylester
   Die Synthese erfolgte analog zu Beispiel 29e) (Methode B)ausgehend von (2-Amino-1-{2-(4-brom-phenyl)-1-[(2,2-diethoxy-ethyl)-isopropyl-carbamoyl]-ethylcarbamoyl}-ethyl)-carbaminsäurebenzylester. Man erhält das gewünschte Produkt mit MG = 828.14 (berechnet, monoisotop); Meßwert (M+H)⁺: 829.11.
f) [6-(4-Brom-benzyl)-1-(2,4-dichlor-benzolsulfonyl)-8-isopropyl-4,7-dioxo-octahydro-pyrazino[1,2-a]pyrimidin-3-yl]-carbaminsäurebenzylester
   Die Synthese erfolgte analog zu Beispiel 29f) (Methode B) ausgehend von) [1-{2-(4-Bromphenyl)-1-[(2,2-diethoxy-ethyl)-isopropyl-carbamoyl]-ethylcarbamoyl}-2-(2,4-dichlor-benzolsulfonylamino)-ethyl]-carbaminsäurebenzylester . Man erhält das gewünschte Produkt mit MG = 736.05 (berechnet, monoisotop); Meßwert (M+H)⁺: 737.1
g) 3-Amino-6-(4-brom-benzyl)-1-(2,4-dichlor-benzolsulfonyl)-8-isopropyl-hexahydro-pyrazino[1,2-a]pyrimidin-4,7-dion
   Die Synthese erfolgte analog zu Beispiel 29g) (Methode B) ausgehend von ) [6-(4-Brombenzyl)-1-(2,4-dichlor-benzolsulfonyl)-8-isopropyl-4,7-dioxo-octahydro- pyrazino[1,2-a]pyrimidin-3-yl]-carbaminsäurebenzylester. Man erhält das gewünschte Produkt mit MG = 602.02 (berechnet, monoisotop); Meßwert (M+H)⁺: 603.01
h) N-[6-(4-Brom-benzyl)-1-(2,4-dichlor-benzolsulfonyl)-8-isopropyl-4,7-dioxo- octahydro-pyrazino[1,2-a]pyrimidin-3-yl]-acetamid
   Die Synthese erfolgt analog zu Beispiel 21h) ausgehend von 3-Amino-6-(4-brom-benzyl)-1-(2,4-dichlor-benzolsulfonyl)-8-isopropyl-hexahydro- pyrazino[1,2-a]pyrimidin-4,7-dion. Man erhält das gewünschte Produkt mit MG = 644.03 (berechnet, monoisotop); Meßwert (M+H)⁺: 645.0

### Beispiel 145

### Cyclopropancarbonsäure [6-(4-brom-benzyl)-1-(2,4-dichlor-benzolsulfonyl)-8- isopropyl-4,7-dioxo-octahydro-pyrazino[1,2-a]pyrimidin-3-yl]-amid

### Struktur:

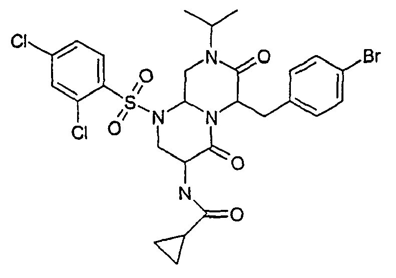

Die Synthese erfolgt analog zu Beispiel 22 ausgehend von 3-Amino-6-(4-brom-benzyl)-1-(2,4-dichlor-benzolsulfonyl)-8-isopropyl-hexahydro- pyrazino[1,2-a]pyrimidin-4,7-dion. Man erhält das gewünschte Produkt mit MG = 670.04 (berechnet, monoisotop); Meßwert (M+H)⁺: 671.06

### Beispiel 146

### Cyclobutancarbonsäure [6-(4-brom-benzyl)-1-(2,4-dichlor-benzolsulfonyl)-8-isopropyl-4,7-dioxo-octahydro-pyrazino[1,2-a]pyrimidin-3-yl]-amid

### Struktur:

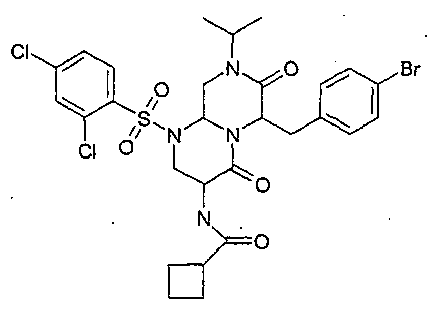

Die Synthese erfolgt analog zu Beispiel 30 ausgehend von 3-Amino-6-(4-brom-benzyl)-1-(2,4-dichlor-benzolsulfonyl)-8-isopropyl-hexahydro- pyrazino[1,2-a]pyrimidin-4,7-dion. Man erhält das gewünschte Produkt mit MG = 684.06 (berechnet, monoisotop); Meßwert (M+H)⁺: 685.04

### Beispiel 147

### Cyclopentancarbonsäure [6-(4-brom-benzyl)-1-(2,4-dichlor-benzolsulfonyl)-8- isopropyl-4,7-dioxo-octahydro-pyrazino[1,2-a]pyrimidin-3-yl]-amid

### Struktur:

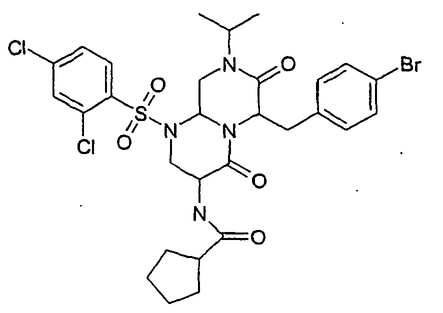

Die Synthese erfolgt analog zu Beispiel 31 ausgehend von 3-Amino-6-(4-brom-benzyl)-1-(2,4-dichlor-benzolsulfonyl)-8-isopropyl-hexahydro-pyrazino[1,2-a]pyrimidin-4,7-dion. Man erhält das gewünschte Produkt mit MG = 698.07 (berechnet, monoisotop); Meßwert (M+H)⁺: 699.1

### Beispiel 148

### Pyrrolidin-2-carbonsäure [6-(4-brom-benzyl)-1-(2,4-dichlor-benzolsulfonyl)-8- isopropyl-4,7-dioxo-octahydro-pyrazino[1,2-a]pyrimidin-3-yl]-amid

### Struktur:

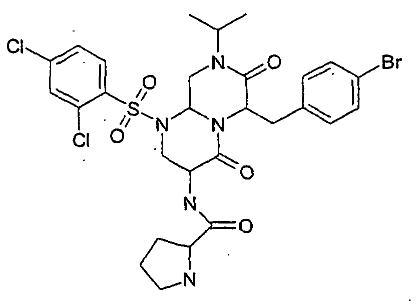

Die Synthese erfolgt analog zu Beispiel 40 ausgehend von 3-Amino-6-(4-brom-benzyl)-1-(2,4-dichlor-benzolsulfonyl)-8-isopropyl-hexahydro- pyrazino[1,2-a]pyrimidin-4,7-dion. Man erhält das gewünschte Produkt mit MG = 699.07 (berechnet, monoisotop); Meßwert (M+H)⁺: 700.2

### Beispiel 149

### N-[6-(4-Brom-benzyl)-1-(2,4-dichlor-benzolsulfonyl)-8-isopropyl-4,7-dioxo-octahydro-pyrazino[1,2-a]pyrimidin-3-yl]-4-dimethylamino-benzamid

### Struktur:

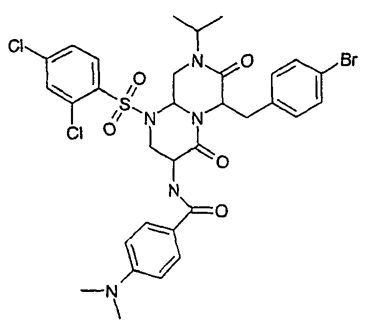

Die Synthese erfolgt analog zu Beispiel 25 ausgehend von 3-Amino-6-(4-brom-benzyl)-1-(2,4-dichlor-benzolsulfonyl)-8-isopropyl-hexahydro- pyrazino[1,2-a]pyrimidin-4,7-dion. Man erhält das gewünschte Produkt mit MG = 749.08 (berechnet, monoisotop); Meßwert (M+H)⁺: 750.10

### Beispiel 150

### N-[6-(4-Brom-benzyl)-1-(2,4-dichlor-benzolsulfonyl)-8-isopropyl-4,7-dioxo-octahydro-pyrazino[1,2-a]pyrimidin-3-yl]-nicotinamid

### Struktur:

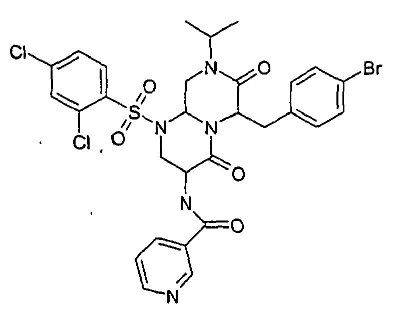

Die Synthese erfolgt analog zu Beispiel 50 ausgehend von 3-Amino-6-(4-brom-benzyl)-1-(2,4-dichlor-benzolsulfonyl)-8-isopropyl-hexahydro- pyrazino[1,2-a]pyrimidin-4,7-dion. Man erhält das gewünschte Produkt mit MG = 707.04 (berechnet, monoisotop); Meßwert (M+H)⁺: 708.07

### Beispiel 151

### 1-[6-(4-Brom-benzyl)-1-(2,4-dichlor-benzolsulfonyl)-8-isopropyl-4,7-dioxo-octahydro-pyrazino [1,2-a]pyrimidin-3-yl]-3-tert-butyl-harnstoff

### Struktur:

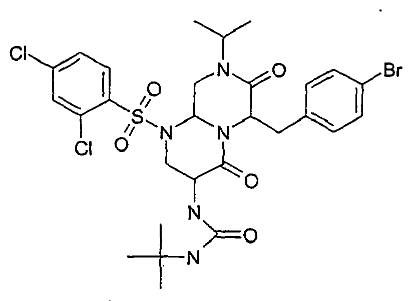

Die Synthese erfolgt analog zu Beispiel 28 ausgehend von 3-Amino-6-(4-brom-benzyl)-1-(2,4-dichlor-benzolsulfonyl)-8-isopropyl-hexahydro- pyrazino[1,2-a]pyrimidin-4,7-dion. Man erhält das gewünschte Produkt mit MG = 701.08 (berechnet, monoisotop); Meßwert (M+H)⁺: 702.0

### Beispiel 152

### N-[6-(4-Brom-benzyl)-1-(2,4-dichlor-benzolsulfonyl)-8-isopropyl-4,7-dioxo-octahydro-pyrazino[1,2-a]pyrimidin-3-yl]-methansulfonamid

### Struktur:

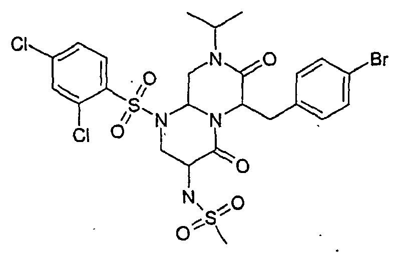

Die Synthese erfolgt analog zu Beispiel 26 ausgehend von 3-Amino-6-(4-brom-benzyl)-1-(2,4-dichlor-benzolsulfonyl)-8-isopropyl-hexahydro- pyrazino[1,2-a]pyrimidin-4,7-dion. Man erhält das gewünschte Produkt mit MG = 679.99 (berechnet, monoisotop); Meßwert (M+H)⁺: 681.0

### Beispiel 153

### Cyclopropansulfonsäure [6-(4-brom-benzyl)-1-(2,4-dichlor-benzolsulfonyl)-8- isopropyl-4,7-dioxo-octahydro-pyazino[1,2-a]pyrimidin-3-yl]-amid

### Struktur:

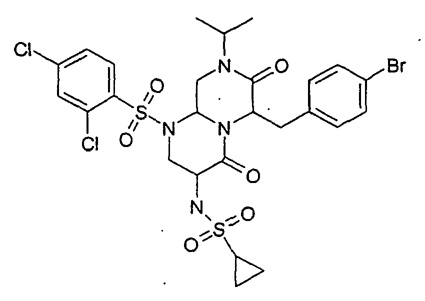

Die Synthese erfolgt analog zu Beispiel 27 ausgehend von 3-Amino-6-(4-brom-benzyl)-1-(2,4-dichlor-benzolsulfonyl)-8-isopropyl-hexahydro- pyrazino[1,2-a]pyrimidin-4,7-dion. Man erhält das gewünschte Produkt mit MG = 706.01 (berechnet, monoisotop); Meßwert (M+H)⁺: 707.01

### Beispiel 154

### 6-(4-Brom-benzyl)-1-(2,4-dichlor-benzolsulfonyl)-8-isopropyl-3-pyrrolidin-1-yl- hexahydropyrazino[1,2-a]pyrimidin-4,7-dion

### Struktur:

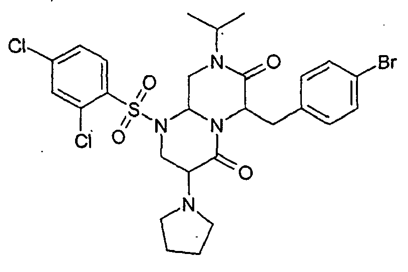

Die Synthese erfolgt analog zu Beispiel 74 ausgehend von 3-Amino-6-(4-brom-benzyl)-1-(2,4-dichlor-benzolsulfonyl)-8-isopropyl-hexahydro- pyrazino[1,2-a]pyrimidin-4,7-dion. Man erhält das gewünschte Produkt mit MG = 656.06 (berechnet, monoisotop); Meßwert (M+H)⁺: 657.06

### Beispiel 155

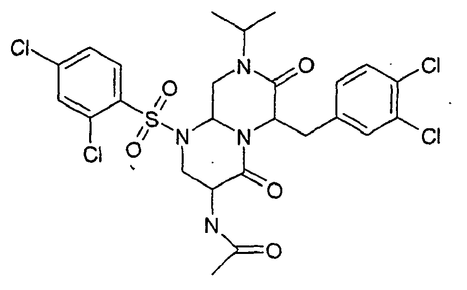

a) [1-[(2,2-Diethoxy-ethyl)-isopropyl-carbamoyl]-2-(3,4-dichlor-phenyl)-ethyl]- carbaminsäure 9H-fluoren-9-ylmethylester
   Die Synthese erfolgt analog zu Beispiel 29a) (Methode B) ausgehend von N-Fmoc-3,4-Cl₂-Phe-OH. Man erhält das gewünschte Produkt mit MG = 612.22 (berechnet, monoisotop); Meßwert (M+Na)⁺: 635.2
b) 2-Amino-3-(3,4-dichlor-phenyl)-N-(2,2-diethoxy-ethyl)-N-isopropyl-propionamid
   Die Synthese erfolgt analog zu Beispiel 29b) (Methode B)ausgehend von [1-[(2,2-Diethoxyethyl)-isopropyl-carbamoyl]-2-(3,4-dichlor-phenyl)-ethyl]- carbaminsäure 9H-fluoren-9-ylmethylester. Man erhält das gewünschte Produkt mit MG = 390.34 (berechnet, monoisotop); Meßwert (M+Na)⁺: 391.16
c) (2-Benzyloxycarbonylamino-2-{2-(3,4-dichlor-phenyl)-1-[(2,2-diethoxy-ethyl)-isopropyl-carbamoyl]-ethylcarbamoyl}-ethyl)-carbaminsäure 9H-fluoren-9-ylmethyl ester
   Die Synthese erfolgte analog zu Beispiel 29c) (Methode B) ausgehend von 2-Amino-3-(3,4-dichlor-phenyl)-N-(2,2-diethoxy-ethyl)-N-isopropyl-propionamid.. Man erhält das gewünschte Produkt mit MG = 832.30 (berechnet, monoisotop); Meßwert (M+H)⁺: 833.30
d) (2-Amino-1-{2-(3,4-dichlor-phenyl)-1-[(2,2-diethoxy-ethyl)-isopropyl-carbamoyl]-ethylcarbamoyl}-ethyl)-carbaminsäurebenzylester
   Die Synthese erfolgte analog zu Beispiel 29d) (Methode B) ausgehend von (2-Benzyloxycarbonylamino-2-{2-(3,4-dichlor-phenyl)-1-[(2,2-diethoxy-ethyl)-isopropyl-carbamoyl]-ethylcarbamoyl}-ethyl)-carbaminsäure 9H-fluoren-9-ylmethyl ester. Man erhält das gewünschte Produkt mit MG = 610.23 (berechnet, monoisotop); Meßwert (M+H)⁺: 611.27
e) [1-{2-(3,4-Dichlor-phenyl)-1-[(2,2-diethoxy-ethyl)-isopropyl-carbamoyl]-ethylcarbamoyl}-2-(2,4-dichlor-benzolsulfonylamino)-ethyl]-carbaminsäurebenzylester
   Die Synthese erfolgte analog zu Beispiel 29e) (Methode B) ausgehend von (2-Amino-1-{2-(3,4-dichlor-phenyl)-1-[(2,2-diethoxy-ethyl)-isopropyl-carbamoyl]-ethylcarbamoyl}-ethyl)-carbaminsäurebenzylester. Man erhält das gewünschte Produkt mit MG = 818.15 (berechnet, monoisotop); Meßwert (M+H)⁺: 819.20.
f) [6-(3,4-Dichlor-benzyl)-1-(2,4-dichlor-benzolsulfonyl)-8-isopropyl-4,7-dioxo-octahydro-pyrazino[1,2-a]pyrimidin-3-yl]-carbaminsäurebenzylester
   Die Synthese erfolgte analog zu Beispiel 29f) (Methode B) ausgehend von)[1-{2-(3,4-Dichlorphenyl)-1-[(2,2-diethoxy-ethyl)-isopropyl-carbamoyl]-ethylcarbamoyl}-2-(2,4-dichlor-benzolsulfonylamino)-ethyl]-carbaminsäurebenzylester . Man erhält das gewünschte Produkt mit MG = 726.06 (berechnet, monoisotop); Meßwert (M+H)⁺: 727.1
g) 3-Amino-6-(3,4-dichlor-benzyl)-1-(2,4-dichlor-benzolsulfonyl)-8-isopropyl-hexahydro-pyrazino[1,2-a]pyrimidin-4,7-dion
   Die Synthese erfolgte analog zu Beispiel 29g) (Methode B) ausgehend von)[6-(3,4-Dichlorbenzyl)-1-(2,4-dichlor-benzolsulfonyl)-8-isopropyl-4,7-dioxo-octahydro- pyrazino[1,2-a]pyrimidin-3-yl]-carbaminsäurebenzylester. Man erhält das gewünschte Produkt mit MG = 592.03 (berechnet, monoisotop); Meßwert (M+H)⁺: 593.00
h) N-[6-(3,4-Dichlor-benzyl)-1-(2,4-dichlor-benzolsulfonyl)-8-isopropyl-4,7-dioxo- octahydro-pyrazino[1,2-a]pyrimidin-3-yl]-acetamid
   Die Synthese erfolgt analog zu Beispiel 21h) ausgehend von 3-Amino-6-(3,4-dichlor-benzyl)-1-(2,4-dichlor-benzolsulfonyl)-8-isopropyl-hexahydro- pyrazino[1,2-a]pyrimidin-4,7-dion. Man erhält das gewünschte Produkt mit MG = 634.04 (berechnet, monoisotop); Meßwert (M+H)⁺: 635.0

### Beispiel 156

### Cyclopropancarbonsäure [1-(2,4-dichlor-benzolsulfonyl)-6-(3,4-dichlor-benzyl)-8- isopropyl-4,7-dioxo-octahydro-pyrazino[1,2-a]pyrimidin-3-yl]-amid

### Struktur:

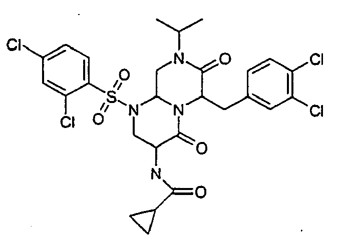

Die Synthese erfolgt analog zu Beispiel 22 ausgehend von 3-Amino-6-(3,4-dichlor-benzyl)-1-(2,4-dichlor-benzolsulfonyl)-8-isopropyl-hexahydro- pyrazino[1,2-a]pyrimidin-4,7-dion. Man erhält das gewünschte Produkt mit MG = 660.05 (berechnet, monoisotop); Meßwert (M+H)⁺: 661.0

### Beispiel 157

### Cyclobutancarbonsäure [1-(2,4-dichlor-benzolsulfonyl)-6-(3,4-dichlor-benzyl)-8- isopropyl-4,7-dioxo-octahydro-pyrazino[1,2-a]pyrimidin-3-yl]-amid

### Struktur:

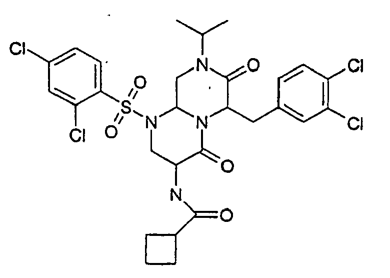

Die Synthese erfolgt analog zu Beispiel 30 ausgehend von 3-Amino-6-(3,4-dichlor-benzyl)-1-(2,4-dichlor-benzolsulfonyl)-8-isopropyl-hexahydro- pyrazino[1,2-a]pyrimidin-4,7-dion. Man erhält das gewünschte Produkt mit MG = 674.07 (berechnet, monoisotop); Meßwert (M+H)⁺: 675.05

### Beispiel 158

### Cyclopentancarbonsäure [1-(2,4-dichlor-benzolsulfonyl)-6-(3,4-dichlor-benzyl)-8-isopropyl-4,7-dioxo-octahydro-pyrazino[1,2-a]pyrimidin-3-yl]-amid

### Struktur:

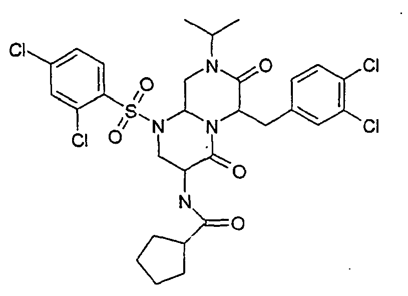

Die Synthese erfolgt analog zu Beispiel 31 ausgehend von 3-Amino-6-(3,4-dichlor-benzyl)-1-(2,4-dichlor-benzolsulfonyl)-8-isopropyl-hexahydro- pyrazino[1,2-a]pyrimidin-4,7-dion. Man erhält das gewünschte Produkt mit MG = 688.08 (berechnet, monoisotop); Meßwert (M+H)⁺: 689.09

### Beispiel 159

### N-[1-(2,4-Dichlor-benzolsulfonyl)-6-(3,4-dichlor-benzyl)-8-isopropyl-4,7-dioxo- octahydro-pyrazino[1,2-a]pyrimidin-3-yl]-4-dimethylamino-benzamid

### Struktur:

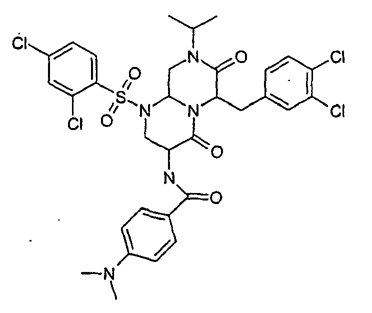

Die Synthese erfolgt analog zu Beispiel 25 ausgehend von 3-Amino-6-(3,4-dichlor-benzyl)-1-(2,4-dichlor-benzolsulfonyl)-8-isopropyl-hexahydro- pyrazino[1,2-a]pyrimidin-4,7-dion. Man erhält das gewünschte Produkt mit MG = 739,10 (berechnet, monoisotop); Meßwert (M+H)⁺: 740.0

### Beispiel 160

### 1-tert-Butyl-3-[1-(2,4-dichlor-benzolsulfonyl)-6-(3,4-dichlor-benzyl)-8-isopropyl- 4,7-dioxo-octahydro-pyrazino[1,2-a]pyrimidin-3-yl]-harnstoff

### Struktur:

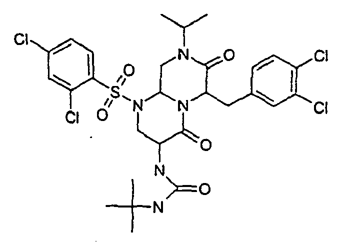

Die Synthese erfolgt analog zu Beispiel 28 ausgehend von 3-Amino-6-(3,4-dichlor-benzyl)-1-(2,4-dichlor-benzolsulfonyl)-8-isopropyl-hexahydro-pyrazino[1,2-a]pyrimidin-4,7-dion. Man erhält das gewünschte Produkt mit MG = 691.10 (berechnet, monoisotop); Meßwert (M+H)⁺: 692.0

### Beispiel 161

### N-[1-(2,4-Dichlor-benzolsulfonyl)-6-(3,4-dichlor-benzyl)-8-isopropyl-4,7-dioxo- octahydro-pyrazino[1,2-a]pyrimidin-3-yl]-methansulfonamid

### Struktur:

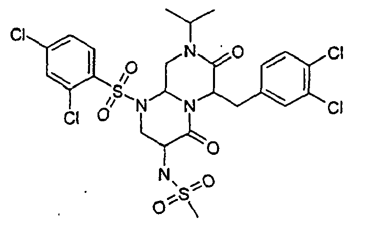

Die Synthese erfolgt analog zu Beispiel 26 ausgehend von 3-Amino-6-(3,4-dichlor-benzyl)-1-(2,4-dichlor-benzolsulfonyl)-8-isopropyl-hexahydro- pyrazino[1,2-a]pyrimidin-4,7-dion. Man erhält das gewünschte Produkt mit MG = 670.0 (berechnet, monoisotop); Meßwert (M+H)⁺: 670.98

### Beispiel 162

### 1-(2,4-Dichlor-benzolsulfonyl)-6-(3,4-dichlor-benzyl)-8-isopropyl-3-pyrrolidin-1-yl-hexahydro-pyrazino[1,2-a]pyrimidin-4,7-dion

### Struktur:

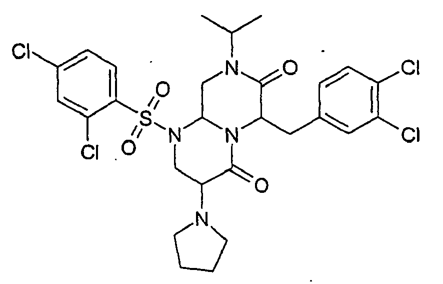

Die Synthese erfolgt analog zu Beispiel 74 ausgehend von 3-Amino-6-(3,4-dichlor-benzyl)-1-(2,4-dichlor-benzolsulfonyl)-8-isopropyl-hexahydro- pyrazino[1,2-a]pyrimidin-4,7-dion. Man erhält das gewünschte Produkt mit MG = 646.07 (berechnet, monoisotop); Meßwert (M+H)⁺: 647.05

### Beispiel 163

### 1-(2,4-Dichlor-benzolsulfonyl)-6-(3,4-dichlor-benzyl)-8-isopropyl-3-morpholin-4-yl-hexahydro-pyrazino[1,2-a]pyrimidin-4,7-dion

### Struktur:

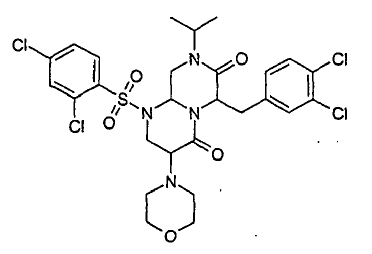

Die Synthese erfolgt analog zu Beispiel 60 ausgehend von 3-Amino-6-(3,4-dichlor-benzyl)-1-(2,4-dichlor-benzolsulfonyl)-8-isopropyl-hexahydro- pyrazino[1,2-a]pyrimindin-4,7-dion. Man erhält das gewünschte Produkt mit MG = 662.07 (berechnet, monoisotop); Meßwert (M+H)⁺: 663.06

### Beispiel 164

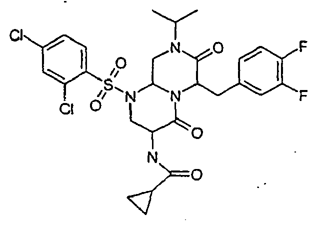

a) [1-[(2,2-Diethoxy-ethyl)-isopropyl-carbamoyl]-2-(3,4-difluor-phenyl)-ethyl]- carbaminsäure 9H-fluoren-9-ylmethylester
   Die Synthese erfolgt analog zu Beispiel 29a) (Methode B) ausgehend von N-Fmoc-3,4-F₂-Phe-OH. Man erhält das gewünschte Produkt mit MG = 580.27 (berechnet, monoisotop); Meßwert (M+Na)⁺: 603.25
b) 2-Amino-3-(3,4-difluor-phenyl)-N-(2,2-diethoxy-ethyl)-N-isopropyl-propionamid
   Die Synthese erfolgt analog zu Beispiel 29b) (Methode B) ausgehend von [1-[(2,2-Diethoxyethyl)-isopropyl-carbamoyl]-2-(3,4-difluor-phenyl)-ethyl]-carbaminsäure 9H-fluoren-9-ylmethylester. Man erhält das gewünschte Produkt mit MG = 358.21 (berechnet, monoisotop); Meßwert (M+H)⁺: 359.2
c) (2-Benzyloxycarbonylamino-2-{2-(3,4-difluor-phenyl)-1-[(2,2-diethoxy-ethyl)-isopropyl-carbamoyl]-ethylcarbamoyl}-ethyl)-carbaminsäure 9H-fluoren-9-ylmethyl ester
   Die Synthese erfolgte analog zu Beispiel 29c) (Methode B) ausgehend von 2-Amino-3-(3,4-difluor-phenyl)-N-(2,2-diethoxy-ethyl)-N-isopropyl-propionamid.. Man erhält das gewünschte Produkt mit MG = 800.36 (berechnet, monoisotop); Meßwert (M+H)⁺: 801.35
d) (2-Amino-1-{2-(3,4-difluor-phenyl)-1-[(2,2-diethoxy-ethyl)-isopropyl-carbamoyl]-ethylcarbamoyl}-ethyl)-carbaminsäurebenzylester
   Die Synthese erfolgte analog zu Beispiel 29d) (Methode B) ausgehend von (2-Benzyloxycarbonylamino-2-{2-(3,4-difluor-phenyl)-1-[(2,2-diethoxy-ethyl)-isopropyl-carbamoyl]-ethylcarbamoyl}-ethyl)-carbaminsäure 9H-fluoren-9-ylmethyl ester. Man erhält das gewünschte Produkt mit MG = 578.29 (berechnet, monoisotop); Meßwert (M+H)⁺: 579.31
e) [1-{2-(3,4-Difluor-phenyl)-1-[(2,2-diethoxy-ethyl)-isopropyl-carbamoyl]-ethylcarbamoyl}-2-(2,4-dichlor-benzolsulfonylamino)-ethyl]-carbaminsäurebenzylester
   Die Synthese erfolgte analog zu Beispiel 29e) (Methode B) ausgehend von (2-Amins-1-{2-(3,4-difluor-phenyl)-1-[(2,2-diethoxy-ethyl)-isopropyl-carbamoyl]-ethylcarbamoyl}-ethyl)-carbaminsäurebenzylester. Man erhält das gewünschte Produkt mit MG = 786.21 (berechnet, monoisotop); Meßwert (M+Na)⁺: 809.19.
f) [6-(3,4-Difluor-benzyl)-1-(2,4-dichlor-benzolsulfonyl)-8-isopropyl-4,7-dioxo-octahydro-pyrazino[1,2-a]pyrimidin-3-yl]-carbaminsäurebenzylester
   Die Synthese erfolgte analog zu Beispiel 29f) (Methode B) ausgehend von)[1-{2-(3,4-Difluorphenyl)-1-[(2,2-diethoxy-ethyl)-isopropyl-carbamoyl]-ethylcarbamoyl}-2-(2,4-dichlor-benzolsulfonylamino)-ethyl]-carbaminsäurebenzylester . Man erhält das gewünschte Produkt mit MG = 694.12 (berechnet, monoisotop); Meßwert (M+H)⁺: 695.10
g) 3-Amino-6-(3,4-difluor-benzyl)-1-(2,4-dichlor-benzolsulfonyl)-8-isopropyl-hexahydro-pyrazino[1,2-a]pyrimidin-4,7-dion
   Die Synthese erfolgte analog zu Beispiel 29g) (Methode B) ausgehend von) [6-(3,4-Difluor-benzyl)-1-(2,4-dichlor-benzolsulfonyl)-8-isopropyl-4,7-dioxo-octahydro- pyrazino[1,2-a]pyrimidin-3-yl]-carbaminsäurebenzylester. Man erhält das gewünschte Produkt mit MG = 560.09 (berechnet, monoisotop); Meßwert (M+H)⁺: 561.13
h) Cyclopropancarbonsäure [1-(2,4-dichlor-benzolsulfonyl)-6-(3,4-difluor-benzyl)-8-isopropyl-4,7-dioxo-octahydro-pyrazino[1,2-a]pyrimidin-3-yl]-amid
   Die Synthese erfolgt analog zu Beispiel 22 ausgehend von 3-Amino-6-(3,4-difluor-benzyl)-1-(2,4-dichlor-benzolsulfonyl)-8-isopropyl-hexahydro- pyrazino[1,2-a]pyrimidin-4,7-dion. Man erhält das gewünschte Produkt mit MG = 628.11 (berechnet, monoisotop); Meßwert (M+H)⁺: 629.18

### Beispiel 165

### Cyclobutancarbonsäure [1-(2,4-dichlor-benzolsulfonyl)-6-(3,4-difluor-benzyl)-8-isopropyl-4,7-dioxo-octahydro-pyrazino[1,2-a]pyrimidin-3-yl]-amid

### Struktur:

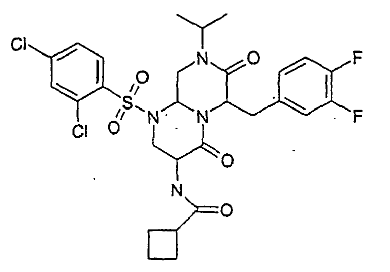

Die Synthese erfolgt analog zu Beispiel 30 ausgehend von 3-Amino-6-(3,4-difluor-benzyl)-1-(2,4-dichlor-benzolsulfonyl)-8-isopropyl-hexahydro- pyrazino[1,2-a]pyrimidin-4,7-dion. Man erhält das gewünschte Produkt mit MG = 642.13 (berechnet, monoisotop); Meßwert (M+H)⁺: 643.21

### Beispiel 166

### Cyclopentancarbonsäure [1-(2,4-dichlor-benzolsulfonyl)-6-(3,4-difluor-benzyl)-8-isopropyl-4,7-dioxo-octahydro-pyrazino[1,2-a]pyrimidin-3-yl]-amid

### Struktur:

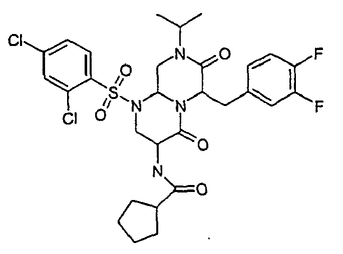

Die Synthese erfolgt analog zu Beispiel 31 ausgehend von 3-Amino-6-(3,4-difluor-benzyl)-1-(2,4-dichlor-benzolsulfonyl)-8-isopropyl-hexahydro- pyrazino[1,2-a]pyrimidin-4,7-dion. Man erhält das gewünschte Produkt mit MG = 656.14 (berechnet, monoisotop); Meßwert (M+H)⁺: 657.21

### Beispiel 167

### Cyclohexancarbonsäure [1-(2,4-dichlor-benzolsulfonyl)-6-(3,4-difluor-benzyl)-8- isopropyl-4,7-dioxo-octahydro-pyrazino[1,2-a]pyrimidin-3-yl]-amid

### Struktur:

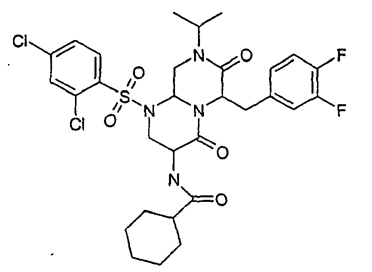

Die Synthese erfolgt analog zu Beispiel 32 ausgehend von 3-Amino-6-(3,4-difluor-benzyl)-1-(2,4-dichlor-benzolsulfonyl)-8-isopropyl-hexahydro- pyrazino[1,2-a]pyrimidin-4,7-dion. Man erhält das gewünschte Produkt mit MG = 670.16 (berechnet, monoisotop); Meßwert (M+H)⁺: 671.24

### Beispiel 168

### N-[1-(2,4-Dichlor-benzolsulfonyl)-6-(3,4-difluor-benzyl)-8-isopropyl-4,7-dioxo- octahydro-pyrazino [1,2-a]pyrimidin-3-yl]-methansulfonamid

### Struktur:

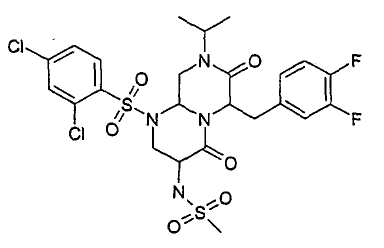

Die Synthese erfolgt analog zu Beispiel 26 ausgehend von 3-Amino-6-(3,4-difluor-benzyl)-1-(2,4-dichlor-benzolsulfonyl)-8-isopropyl-hexahydro- pyrazino[1,2-a]pyrimidin-4,7-dion. Man erhält das gewünschte Produkt mit MG = 638.06 (berechnet, monoisotop); Meßwert (M+H)⁺: 639.02

### Beispiel 169

### 1-(2,4-Dichlor-benzolsulfonyl)-6-(3,4-difluor-benzyl)-3-dimethylamino-8-isopropyl-hexahydro-pyrazino[1,2-a]pyrimidin-4,7-dion

### Struktur:

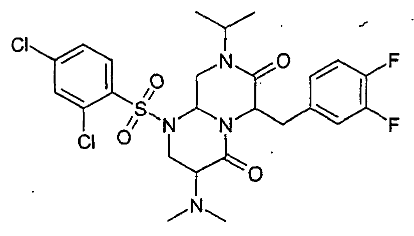

Die Synthese erfolgt analog zu Beispiel 72 ausgehend von 3-Amino-6-(3,4-difluor-benzyl)-1-(2,4-dichlor-benzolsulfonyl)-8-isopropyl-hexahydro-pyrazino[1,2-a]pyrimidin-4,7-dion. Man erhält das gewünschte Produkt mit MG = 588.12 (berechnet, monoisotop); Meßwert (M+H)⁺: 589.10

### Beispiel 170

### 1-(2,4-Dichlor-benzolsulfonyl)-6-(3,4-difluor-benzyl)-8-isopropyl-3-pyrrolidin-1-yl-hexahydro-pyrazino[1,2-a]pyrimidin-4,7-dion

### Struktur:

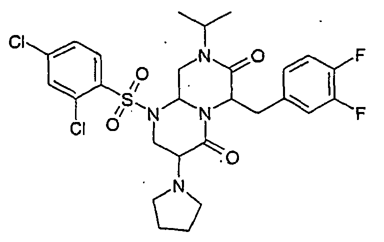

Die Synthese erfolgt analog zu Beispiel 74 ausgehend von 3-Amino-6-(3,4-difluor-benzyl)-1-(2,4-dichlor-benzolsulfonyl)-8-isopropyl-hexahydro- pyrazino[1,2-a]pyrimidin-4,7-dion. Man erhält das gewünschte Produkt mit MG = 614.13 (berechnet, monoisotop); Meßwert (M+H)⁺: 615.10

### Beispiel 171

### 1-(2,4-Dichlor-benzolsulfonyl)-6-(3,4-difluor-benzyl)-8-isopropyl-3-piperidin-1-yl- hexahydropyrazino[1,2-a]pyrimidin-4,7-dion

### Struktur:

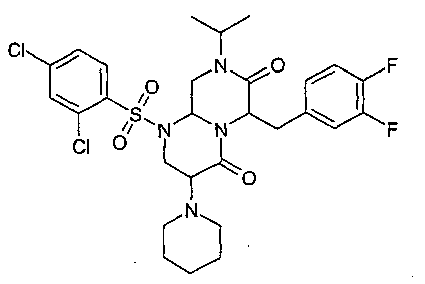

Die Synthese erfolgt analog zu Beispiel 59 ausgehend von 3-Amino-6-(3,4-difluor-benzyl)-1-(2,4-dichlor-benzolsulfonyl)-8-isopropyl-hexahydro- pyrazino[1,2-a]pyrimidin-4,7-dion. Man erhält das gewünschte Produkt mit MG = 628.15 (berechnet, monoisotop); Meßwert (M+H)⁺: 629.22

### Beispiel 172

### 1-(2,4-Dichlor-benzolsulfonyl)-6-(3,4-diffuor-benzyl)-8-isopropyl-3-morpholin-4-yl-hexahydro-pyrazino[1,2-a]pyrimidin-4,7-dion

### Struktur:

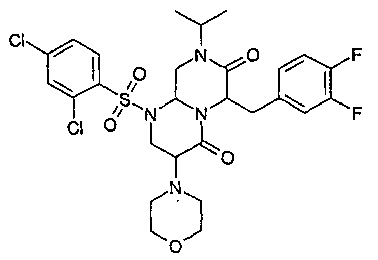

Die Synthese erfolgt analog zu Beispiel 60 ausgehend von 3-Amino-6-(3,4-difluor-benzyl)-1-(2,4-dichlor-benzolsulfonyl)-8-isopropyl-hexahydro-pyrazino[1,2-a]pyrimidin-4,7-dion. Man erhält das gewünschte Produkt mit MG = 630.13 (berechnet, monoisotop); Meßwert (M+H)⁺: 631.10

### Beispiel 173

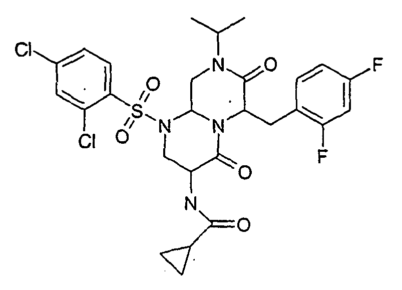

a) 2-Allyloxycarbonylamino-3-(2,4-difluor-phenyl)-propionsäure
   Die Synthese erfolgt analog zu Beispiel 21a) ausgehend von 2-Amino-3-(2,4-Difluorphenyl)propionsäure. Man erhält das gewünschte Produkt mit MG = 285.08 (berechnet, monoisotop); Meßwert (M+H)⁺: 286.05
b) {2-(2,4-Difluor-phenyl)-1-[(2,2-diethoxy-ethyl)-isopropyl-carbamoyl]-ethyl}-allylcarbamat. Die Synthese erfolgt analog zu Beispiel 21b) ausgehend von 2-Allyloxycarbonylamino-3-(2,4-Difluor-phenyl)-propionsäure Man erhält das gewünschte Produkt mit MG = 442,23 (berechnet, monoisotop); Meßwert (M+H)⁺: 443.2
c) 2-Amino-3-(2,4-difluor-phenyl)-N-(2,2-diethoxy-ethyl)-N-isopropyl-propionamid
   Die Synthese erfolgt analog zu Beispiel 21c) ausgehend von {2-(2,4-Difluor-phenyl)-1-[(2,2-diethoxy-ethyl)-isopropyl-carbamoyl]-ethyl}-carbaminsäureallylester. Man erhält das gewünschte Produkt mit MG = 358.21 (berechnet, monoisotop); Meßwert (M+H)⁺: 359.2
d) (2-(2,4-Dichlor-benzolsulfonylamino)-1-{2-(2,4-difluor-phenyl)-1-[(2,2-diethoxy- ethyl)-isopropyl-carbamoyl]-ethylcarbamoyl}-ethyl)-carbaminsäurebenzylester
   Die Synthese erfolgt analog zu Beispiel 29b) ausgehend von 2-Amino-3-(2,4-difluor-phenyl)-N-(2,2-diethoxy-ethyl)-N-isopropyl-propionamid. Man erhält das gewünschte Produkt mit MG = 786.21 (berechnet, monoisotop); Meßwert (M+H)⁺: 787,30
e) [6-(2,4-Difluor-benzyl)-1-(2,4-dichlor-benzolsulfonyl)-8-isopropyl-4,7-dioxo-octahydro-pyrazino[1,2-a]pyrimidin-3-yl]-carbaminsäurebenzylester
   Die Synthese erfolgt analog zu Beispiel 21f) ausgehend von (2-(2,4-Dichlor-benzolsulfonylamino)-1-{2-(2,4-difluor-phenyl)-1-[(2,2-diethoxy- ethyl)-isopropyl-carbamoyl]-ethylcarbamoyl}-ethyl)-carbaminsäurebenzylester. Man erhält das gewünschte Produkt mit MG = 694.12 (berechnet, monoisotop); Meßwert (M+H)⁺: 695.05
f) 3-Amino-6-(2,4-difluor-benzyl)-1-(2,4-dichlor-benzolsulfonyl)-8-isopropyl-hexahydro-pyrazino[1,2-a]pyrimidin-4,7-dion
   Die Synthese erfolgt analog zu Beispiel 21g) ausgehend von (2-(2,4-Dichlor-benzolsulfonylamino)-1-{2-(2,4-difluor-phenyl)-1-[(2,2-diethoxy- ethyl)-isopropyl-carbamoyl]-ethylcarbamoyl}-ethyl)-carbaminsäurebenzylester. Man erhält das gewünschte Produkt mit MG = 560.09 (berechnet, monoisotop); Meßwert (M+H)⁺: 561.0
g) Cyclopropancarbonsäure [1-(2,4-dichlor-benzolsulfonyl)-6-(2,4-difluor-benzyl)-8-isopropyl-4,7-dioxo-octahydro-pyrazino[1,2-a]pyrimidin-3-yl]-amid
   Die Synthese erfolgt analog zu Beispiel 29e) (Methode A) ausgehend von 3-Amino-6-(2,4-difluor-benzyl)-1-(2,4-dichlor-benzolsulfonyl)-8-isopropyl-hexahydro- pyrazino[1,2-a]pyrimidin-4,7-dion. Man erhält das gewünschte Produkt mit MG = 628.11 (berechnet, monoisotop); Meßwert (M+H)⁺: 629.31

### Beispiel 174

### 1-(2,4-Dichlor-benzolsulfonyl)-6-(2,4-difluor-benzyl)-3-dimethylamino-8-isopropyl-hexahydro-pyrazino[1,2-a]pyrimidin-4,7-dion

### Struktur:

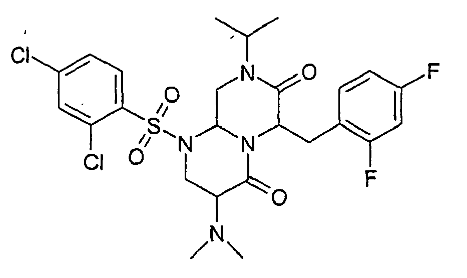

Die Synthese erfolgt analog zu Beispiel 72 ausgehend von 3-Amino-6-(2,4-diffuor-benzyl)-1-(2,4-dichlor-benzolsulfonyl)-8-isopropyl-hexahydro- pyrazino[1,2-a]pyrimidin-4,7-dion. Man erhält das gewünschte Produkt mit MG = 588.12 (berechnet, monoisotop); Meßwert (M+H)⁺: 589.07

### Beispiel 175

### 3-Azetidin-1-yl-1-(2,4-dichlor-benzolsulfonyl)-6-(2,4-difluor-benzyl)-8-isopropyl- hexahydropyrazino[1,2-a]pyrimidin-4,7-dion

### Struktur:

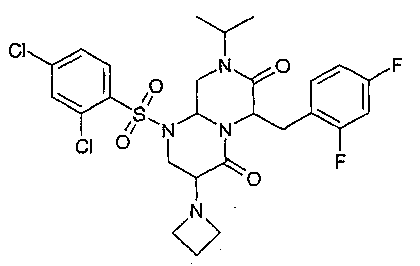

Die Synthese erfolgt analog zu Beispiel 73 ausgehend von 3-Amino-6-(2,4-difluor-benzyl)-1-(2,4-dichlor-benzolsulfonyl)-8-isopropyl-hexahydro- pyrazino[1,2-a]pyrimidin-4,7-dion. Man erhält das gewünschte Produkt mit MG = 600.12 (berechnet, monoisotop); Meßwert (M+H)⁺: 600.81

### Beispiel 176

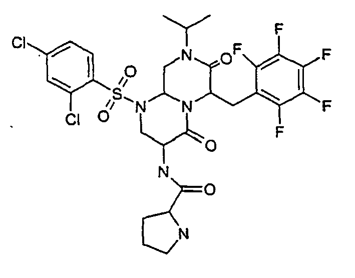

a) [1-[(2,2-Diethoxy-ethyl)-isopropyl-carbanoyl]-2-pentafluorphenyl-ethyl]- carbaminsäure 9H-fluoren-9-ylmethylester
   Die Synthese erfolgt analog zu Beispiel 29a) (Methode B) ausgehend von N-Fmoc-2,3,4,5,6-F₅-Phe-OH. Man erhält das gewünschte Produkt mit MG = 634.25 (berechnet, monoisotop); Meßwert (M+Na)⁺: 657.17
b) 2-Amino-3-pentafluorphenyl-N-(2,2-diethoxy-ethyl)-N-isopropyl-propionamid
   Die Synthese erfolgt analog zu Beispiel 29b) (Methode B) ausgehend von [1-[(2,2-Diethoxyethyl)-isopropyl-carbamoyl]-2-pentafluorphenyl-ethyl]- carbaminsäure 9H-fluoren-9-ylmethylester. Man erhält das gewünschte Produkt mit MG = 412.18 (berechnet, monoisotop); Meßwert (M+Na)⁺: 435.17
c) (2-Benzyloxycarbonylamino-2-{2-(pentafluorphenyl)-1-[(2,2-diethoxy-ethyl)-isopropyl-carbamoyl]-ethylcarbamoyl)-ethyl)-carbaminsäure 9H-fluoren-9-ylmethyl ester
   Die Synthese erfolgte analog zu Beispiel 29c) (Methode B) ausgehend von 2-Amino-3-pentafluorphenyl-N-(2,2-diethoxy-ethyl)-N-isopropyl-propionamid. . Man erhält das gewünschte Produkt mit MG = 854.33 (berechnet, monoisotop); Meßwert (M+Na)⁺: 877.41
d) (2-Amino-1-{2-pentafluorphenyl-1-[(2,2-diethoxy-ethyl)-isopropyl-carbamoyl]-ethylcarbamoyl}-ethyl)-carbaminsäurebenzylester
   Die Synthese erfolgte analog zu Beispiel 29d) (Methode B) ausgehend von (2-Benzyloxycarbonylamino-2-{2-pentafluorphenyl-1-[(2,2-diethoxy-ethyl)-isopropyl-carbamoyl]-ethylcarbamoyl}-ethyl)-carbaminsäure 9H-fluoren-9-ylmethyl ester. Man erhält das gewünschte Produkt mit MG = 632.26 (berechnet, monoisotop); Meßwert (M+Na)⁺: 655.24
e) [1-{2-Pentafluorphenyl-1-[(2,2-diethoxy-ethyl)-isopropyl-carbamoyl]-ethylcarbamoyl}-2-(2,4-dichlor-benzolsulfonylamino)-ethyl]-carbaminsäurebenzylester
   Die Synthese erfolgte analog zu Beispiel 29e) (Methode B) ausgehend von (2-Amino-1-{2-pentafluor-phenyl-1-[(2,2-diethoxy-ethyl)-isopropyl-carbamoyl]-ethylcarbamoyl}-ethyl)-carbaminsäurebenzylester. Man erhält das gewünschte Produkt mit MG = 840.18 (berechnet, monoisotop); Meßwert (M+Na)⁺: 863.18.
f) [1-(2,4-Dichlor-benzolsulfonyl)-8-isopropyl-4,7-dioxo-6-pentafluorphenylmethyl- octahydro-pyrazino[1,2-a]pyrimidin-3-yl]-carbaminsäurebenzylester
   Die Synthese erfolgt analog zu Beispiel 29f) ausgehend von (2-(2,4-Dichlor-benzolsulfonylamino)-1-{1-[(2,2-diethoxy-ethyl)-isopropyl-carbamoyl]-2-pentafluorphenyl-ethylcarbamoyl}-ethyl)-carbaminsäurebenzylester. Man erhält das gewünschte Produkt mit MG = 748.09 (berechnet, monoisotop); Meßwert (M+H)⁺: 749.15
g) 3-Amino-1-(2,4-dichlor-benzolsulfonyl)-8-isopropyl-6-pentafluorphenylmethyl-hexahydro-pyrazino[1,2-a]pyrimidin-4,7-dion
   Die Synthese erfolgt analog zu Beispiel 29g) ausgehend von [1-(2,4-Dichlor-benzolsulfonyl)-8-isopropyl-4,7-dioxo-6-pentafluorphenylmethyl- octahydro-pyrazino[1,2-a]pyrimidin-3-yl]-carbaminsäurebenzylester. Man erhält das gewünschte Produkt mit MG = 614.06 (berechnet, monoisotop); Meßwert (M+H)⁺: 615.05
h) Pyrrolidin-2-carbonsäure [1-(2,4-dichlor-benzolsulfonyl)-8-isopropyl-4,7-dioxo-6-pentafluorphenylmethyl-octahydro-pyrazino[1,2-a]pyrimidin-3-yl]-amid
   Die Synthese erfolgt analog zu Beispiel 40 ausgehend von 3-Amino-1-(2,4-dichlor-benzolsulfonyl)-8-isopropyl-6-pentafluorphenylmethyl-hexahydro-pyrazino[1,2-a]pyrimidin-4,7-dion. Man erhält das gewünschte Produkt mit MG = 711.11 (berechnet, monoisotop); Meßwert (M+H)⁺: 712.06

### Beispiel 177

### 1-(2,4-Dichlor-benzolsulfonyl)-3-dimethylamino-8-isopropyl-6-pentafluorphenylmethyl-hexahydro-pyrazino[1,2-a]pyrimidin-4,7-dion

### Struktur:

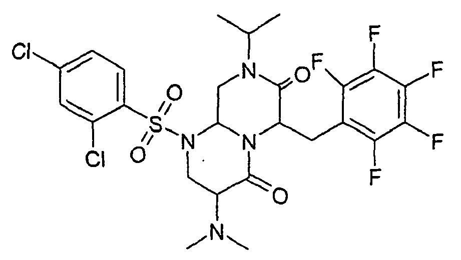

Die Synthese erfolgt analog zu Beispiel 72 ausgehend von 3-Amino-1-(2,4-dichlor-benzolsulfonyl)-8-isopropyl-6-pentafluorphenylmethyl-hexahydro- pyrazino[1,2-a]pyrimidin-4,7-dion. Man erhält das gewünschte Produkt mit MG = 620.12 (berechnet, monoisotop); Meßwert (M+H)⁺: 621.09

### Beispiel 178

### 3-Azetidin-1-yl-1-(2,4-dichlor-benzolsulfonyl)-8-isopropyl-6-pentafluorphenylmethyl-hexahydro-pyrazino[1,2-a]pyrimidin-4,7-dion

### Struktur:

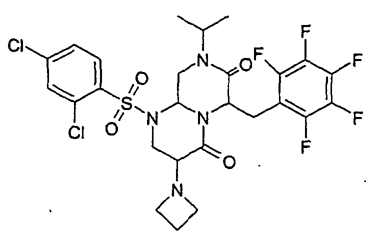

Die Synthese erfolgt analog zu Beispiel 73 ausgehend von 3-Amino-1-(2,4-dichlor-benzolsulfonyl)-8-isopropyl-6-pentafluorphenylmethyl-hexahydro- pyrazino[1,2-a]pyrimidin-4,7-dion. Man erhält das gewünschte Produkt mit MG = 654.09 (berechnet, monoisotop); Meßwert (M+H)⁺: 655.1

### Beispiel 179

### 1-(2,4-Dichlor-benzolsulfonyl)-8-isopropyl-6-pentafluorphenylmethyl-3-pyrrolidin-1-yl-hexahydro-pyrazino[1,2-a]pyrimidin-4,7-dion

### Struktur:

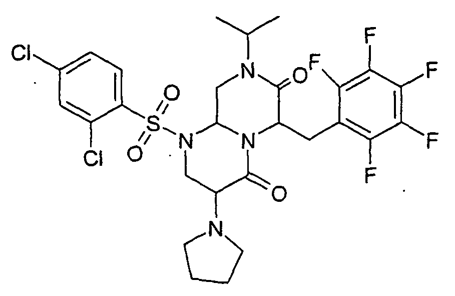

Die Synthese erfolgt analog zu Beispiel 74 ausgehend von 3-Amino-1-(2,4-dichlor-benzolsulfonyl)-8-isopropyl-6-pentafluorphenylmethyl-hexahydro- pyrazino[1,2-a]pyrimidin-4,7-dion. Man erhält das gewünschte Produkt mit MG = 668.11 (berechnet, monoisotop); Meßwert (M+H)⁺: 669.11.

### Beispiel 180

### 1-(2,4-Dichlor-benzolsulfonyl)-8-isopropyl-6-pentafluorphenylmethyl-3-piperidin-1-yl-hexahydro-pyrazino [1,2-a]pyrimidin-4,7-dion

### Struktur:

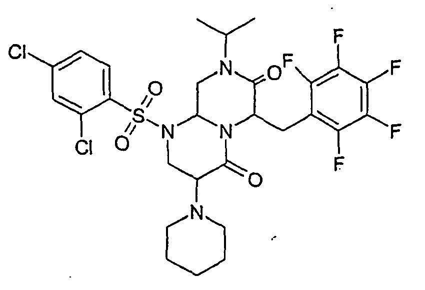

Die Synthese erfolgt analog zu Beispiel 59 ausgehend von 3-Amino-1-(2,4-dichlor-benzolsulfonyl)-8-isopropyl-6-pentafluorphenylmethyl-hexahydro- pyrazino[1,2-a]pyrimidin-4,7-dion. Man erhält das gewünschte Produkt mit MG = 682.12 (berechnet, monoisotop); Meßwert (M+H)⁺: 683.09

### Beispiel 181

### 1-(2,4-Dichlor-benzolsulfonyl)-8-isopropyl-3-morpholin-4-yl-6-pentafluorphenylmethyl-hexahydro-pyrazino[1,2-a]pyrimidin-4,7-dion

### Struktur:

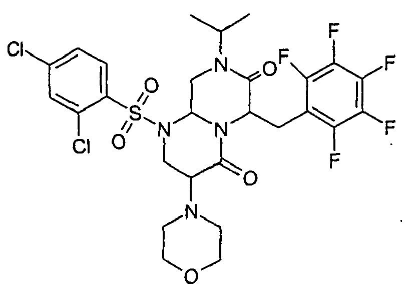

Die Synthese erfolgt analog zu Beispiel 60 ausgehend von 3-Amino-1-(2,4-dichlor-benzolsulfonyl)-8-isopropyl-6-pentafluorphenylmethyl-hexahydro- pyrazino[1,2-a]pyrimidin-4,7-dion. Man erhält das gewünschte Produkt mit MG = 684.10 (berechnet, monoisotop); Meßwert (M+H)⁺: 685.01

### Beispiel 182

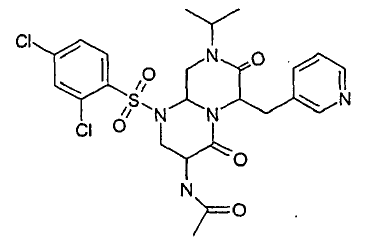

a) {1-[(2,2-Diethoxy-ethyl)-isopropyl-carbamoyl]-2-pyridin-3-yl-ethyl}-carbaminsäure 9H-fluoren-9-ylmethylester
   Die Synthese erfolgt analog zu Beispiel 29a) (Methode B) ausgehend von Fmoc-3-pyridyl-alanin. Man erhält das gewünschte Produkt mit MG = 545.29 (berechnet, monoisotop); Meßwert (M+H)⁺: 546.24
b) 2-Amino-N-(2,2-diethoxy-ethyl)-N-isopropyl-3-pyridin-3-yl-propionamid
   Die Synthese erfolgt analog zu Beispiel 29b) (Methode B) ausgehend von {1-[(2,2-Diethoxyethyl)-isopropyl-carbarnoyl]-2-pyridin-3-yl-ethyl}-carbaminsäure 9H-fluoren-9-ylmethylester. Man erhält das gewünschte Produkt mit MG = 323.22 (berechnet, monoisotop); Meßwert (M+H)⁺: 324.22
c) [1-[(2,2-Diethoxy-ethyl)-isopropyl-carbamoyl]-2-pyridin-3-yl-ethylcarbamoyl}-2-(9H-fluoren-9-ylmethoxycarbonylamino)-ethyl]-carbaminsäurebenzylester
   Die Synthese erfolgte analog zu Beispiel 29c) (Methode B) ausgehend von 2-Amino-N-(2,2-diethoxy-ethyl)-N-isopropyl-3-pyridin-3-yl-propionamid . Man erhält das gewünschte Produkt mit MG = 765.37 (berechnet, monoisotop); Meßwert (M+H)⁺: 766.31
d) (2-Amino-{1-[(2,2-diethoxy-ethyl)-isopropyl-carbamoyl]-2-pyridin-3-yl-ethylcarbamoyl}-ethyl)-carbaminsäurebenzylester
   Die Synthese erfolgte analog zu Beispiel 29d) (Methode B) ausgehend von [1-[(2,2-Diethoxyethyl)-isopropyl-carbamoyl]-2-pyridin-3-yl-ethylcarbamoyl}-2-(9H-fluoren-9-ylmethoxycarbonylamino)-ethyl]-carbaminsäurebenzylester. Man erhält das gewünschte Produkt mit MG = 543.31 (berechnet, monoisotop); Meßwert (M+H)⁺: 544.4
e) (2-(2,4-Dichlor-benzolsulfonylamino)-1-{1-[(2,2-diethoxy-ethyl)-isopropyl-carbamoyl]-2-pyridin-3-yl-ethylcarbamoyl}-ethyl)-carbaminsäurebenzylester
   Die Synthese erfolgte analog zu Beispiel 29e) (Methode B) ausgehend von (2-Amino-{1-[(2,2-diethoxy-ethyl)-isopropyl-carbamoyl]-2-pyridin-3-yl-ethylcarbamoyl}-ethyl)-carbaminsäurebenzylester. Man erhält das gewünschte Produkt mit MG = 751.22 (berechnet, monoisotop); Meßwert (M+H)⁺: 752.19.
f) [1-(2,4-Dichlor-benzolsulfonyl)-8-isopropyl-4,7-dioxo-6-pyridin-3-ylmethyl-octahydro-pyrazino[1,2-a]pyrimidin-3-yl]-carbaminsäurebenzylester
   Die Synthese erfolgt analog zu Beispiel 29f) ausgehend von (2-(2,4-Dichlor-benzolsulfonylamino)-1-{1-[(2,2-diethoxy-ethyl)-isopropyl-carbamoyl]-2- pyridin-3-yl-ethylcarbamoyl}-ethyl)-carbaminsäurebenzylester. Man erhält das gewünschte Produkt mit MG = 659.14 (berechnet, monoisotop); Meßwert (M+H)⁺: 660.10
g) 3-Amino-1-(2,4-dichlor-benzolsulfonyl)-8-isopropyl-6-pyridin-3-ylmethyl-hexahydro-pyrazino[1,2-a]pyrimidin-4,7-dion
   Die Synthese erfolgt analog zu Beispiel 29g) ausgehend von [1-(2,4-Dichlor-benzolsulfonyl)-8-isopropyl-4,7-dioxo-6-pyridin-3-ylmethyl-octahydro-pyrazino[1,2-a]pyrimidin-3-yl]-carbaminsäurebenzylester. Man erhält das gewünschte Produkt mit MG = 525.10 (berechnet, monoisotop); Meßwert (M+H)⁺: 526.1
h) N-[1-(2,4-Dichlor-benzolsulfonyl)-8-isopropyl-4,7-dioxo-6-pyridin-3-ylmethyl- octahydro-pyrazino[1,2-a]pyrimidin-3-yl]-acetamid
   Die Synthese erfolgt analog zu Beispiel 21h) ausgehend von 3-Amino-1-(2,4-dichlor-benzolsulfonyl)-8-isopropyl-6-pyridin-3-ylmethyl-hexahydro- pyrazino[1,2-a]pyrimidin-4,7-dion. Man erhält das gewünschte Produkt mit MG = 567.11 (berechnet, monoisotop); Meßwert (M+H)⁺: 568.11

### Beispiel 183

### Cyclopropancarbonsäure[1-(2,4-dichlor-benzolsulfonyl)-8-isopropyl-4,7-dioxo-6-pyridin-3-ylmethyl-octahydro-pyrazino[1,2-a]pyrimidin-3-yl]-amid

### Struktur:

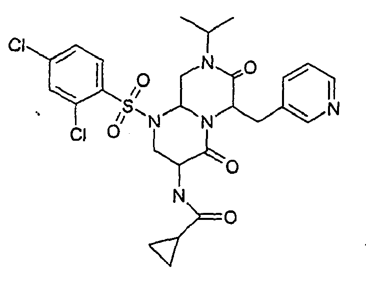

Die Synthese erfolgt analog zu Beispiel 22 ausgehend von 3-Amino-1-(2,4-dichlor-benzolsulfonyl)-8-isopropyl-6-pyridin-3-ylmethyl-hexahydro- pyrazino[1,2-a]pyrimidin-4,7-dion. Man erhält das gewünschte Produkt mit MG = 593.13 (berechnet, monoisotop); Meßwert (M+H)⁺: 594.13

### Beispiel 184

### Cyclobutancarbonsäure [1-(2,4-dichlor-benzolsulfonyl)-8-isopropyl-4,7-dioxo-6- pyridin-3-ylmethyl-octahydro-pyrazino[1,2-a]pyrimidin-3-yl]-amid

### Struktur:

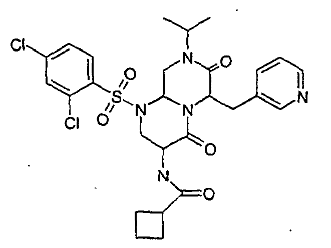

Die Synthese erfolgt analog zu Beispiel 30 ausgehend von 3-Amino-1-(2,4-dichlor-benzolsulfonyl)-8-isopropyl-6-pyridin-3-ylmethyl-hexahydro- pyrazino[1,2-a]pyrimidin-4,7-dion. Man erhält das gewünschte Produkt mit MG = 607.14 (berechnet, monoisotop); Meßwert (M+H)⁺: 608.15

### Beispiel 185

### Cyclopentancarbonsäure [1-(2,4-dichlor-benzolsulfonyl)-8-isopropyl-4,7-dioxo-6- pyridin-3-ylmethyl-octahydro-pyrazino[1,2-a]pyrimidin-3-yl]-amid

### Struktur:

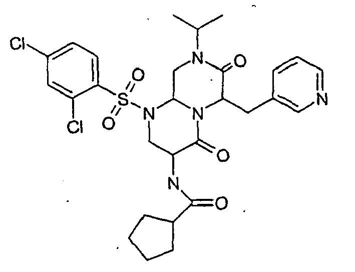

Die Synthese erfolgt analog zu Beispiel 31 ausgehend von 3-Amino-1-(2,4-dichlor-benzolsulfonyl)-8-isopropyl-6-pyridin-3-ylmethyl-hexahydro- pyrazino[1,2-a]pyrimidin-4,7-dion. Man erhält das gewünschte Produkt mit MG = 621.16 (berechnet, monoisotop); Meßwert (M+H)⁺: 622.17

### Beispiel 186

### Pyrrolidin-2-carbonsäure [1-(2,4-dichlor-benzolsulfonyl)-8-isopropyl-4,7-dioxo-6- pyridin-3-ylmethyl-octahydro-pyrazino[1,2-a]pyrimidin-3-yl]-amid

### Struktur:

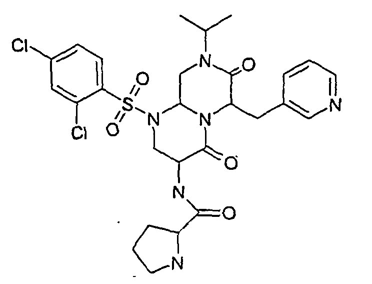

Die Synthese erfolgt analog zu Beispiel 40 ausgehend von 3-Amino-1-(2,4-dichlor-benzolsulfonyl)-8-isopropyl-6-pyridin-3-ylmethyl-hexahydro- pyrazino[1,2-a]pyrimidin-4,7-dion. Man erhält das gewünschte Produkt mit MG = 622.15 (berechnet, monoisotop); Meßwert (M+H)⁺: 623.14

### Beispiel 187

### N-[1-(2,4-Dichlor-benzolsulfonyl)-8-isopropyl-4,7-dioxo-6-pyridin-3-ylmethyl-octahydro-pyrazino[1,2-a]pyrimidin-3-yl]-4-dimethylamino-benzamid

### Struktur:

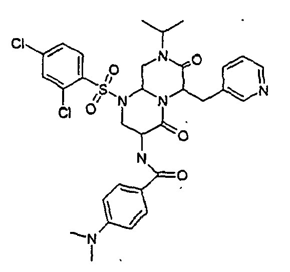

Die Synthese erfolgt analog zu Beispiel 25 ausgehend von 3-Amino-1-(2,4-dichlor-benzolsulfonyl)-8-isopropyl-6-pyridin-3-ylinethyl-hexahydro- pyrazino[1,2-a]pyrimidin-4,7-dion. Man erhält das gewünschte Produkt mit MG = 672.17 (berechnet, monoisotop); Meßwert (M+H)⁺: 673.18

### Beispiel 188

### N-[1-(2,4-Dichlor-benzolsulfonyl)-8-isopropyl-4,7-dioxo-6-pyridin-3-ylmethyl- octahydro-pyrazino[1,2-a]pyrimidin-3-yl]-nicotinamid

### Struktur:

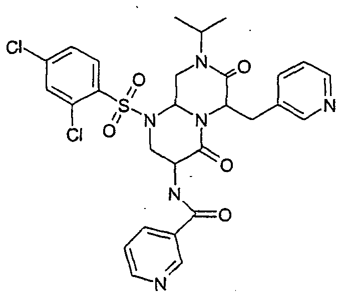

Die Synthese erfolgt analog zu Beispiel 50 ausgehend von 3-Amino-1-(2,4-dichlor-benzolsulfonyl)-8-isopropyl-6-pyridin-3-ylmethyl-hexahydro- pyrazino[1,2-a]pyrimidin-4,7-dion. Man erhält das gewünschte Produkt mit MG = 630.12 (berechnet, monoisotop); Meßwert (M+H)⁺: 631.16

### Beispiel 189

### 1-tert-Butyl-3-[1-(2,4-dichlor-benzolsulfonyl)-8-isopropyl-4,7-dioxo-6-pyridin-3-ylmethyl-octahydro-pyrazino[1,2-a]pyrimidin-3-yl]-harnstoff

### Struktur:

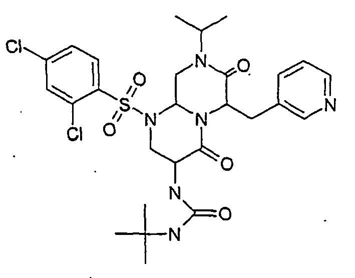

Die Synthese erfolgt analog zu Beispiel 28 ausgehend von 3-Amino-1-(2,4-dichlor-benzolsulfonyl)-8-isopropyl-6-pyridin-3-ylmethyl-hexahydro- pyrazino[1,2-a]pyrimidin-4,7-dion. Man erhält das gewünschte Produkt mit MG = 624.17 (berechnet, monoisotop); Meßwert (M+H)⁺: 625.1

### Beispiel 190

### N-[1-(2,4-Dichlor-benzolsulfonyl)-8-isopropyl-4,7-dioxo-6-pyridin-3-ylmethyl-octahydro-pyrazino [1,2-a]pyrimidin-3-yl]-methansulfonamid

### Struktur:

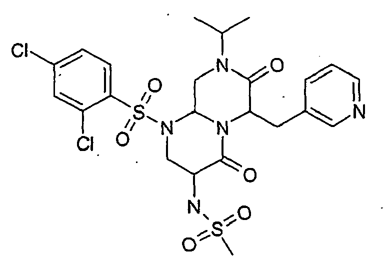

Die Synthese erfolgt analog zu Beispiel 26 ausgehend von 3-Amino-1-(2,4-dichlor-benzolsulfonyl)-8-isopropyl-6-pyridin-3-ylmethyl-hexahydro- pyrazino[1,2-a]pyrimidin-4,7-dion. Man erhält das gewünschte Produkt mit MG = 603.08 (berechnet, monoisotop); Meßwert (M+H)⁺: 604.06

### Beispiel 191

### Cyclopropansulfonsäure [1-(2,4-dichlor-benzolsulfonyl)-8-isopropyl-4,7-dioxo-6-pyridin-3-ylmethyl-octahydro-pyrazino[1,2-a]pyrimidin-3-yl]-amid

### Struktur:

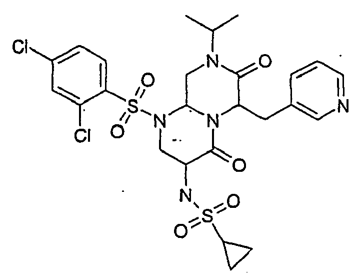

Die Synthese erfolgt analog zu Beispiel 27 ausgehend von 3-Amino-1-(2,4-dichlor-benzolsulfonyl)-8-isopropyl-6-pyridin-3-ylmethyl-hexahydro- pyrazino[1,2-a]pyrimidin-4,7-dion. Man erhält das gewünschte Produkt mit MG = 629.09 (berechnet, monoisotop); Meßwert (M+H)⁺: 630.09

### Beispiel 192

### 1-(2,4-Dichlor-benzolsulfonyl)-3-dimethylamino-8-isopropyl-6-pyridin-3-ylmethyl- hexahydro-pyrazino[1,2-a]pyrimidin-4,7-dion

### Struktur:

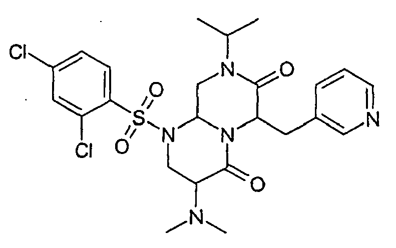

Die Synthese erfolgt analog zu Beispiel 72 ausgehend von 3-Amino-1-(2,4-dichlor-benzolsulfonyl)-8-isopropyl-6-pyridin-3-ylmethyl-hexahydro-pyrazino[1,2-a]pyrimidin-4,7-dion. Man erhält das gewünschte Produkt mit MG = 553.13 (berechnet, monoisotop); Meßwert (M+H)⁺: 554.08

### Beispiel 193

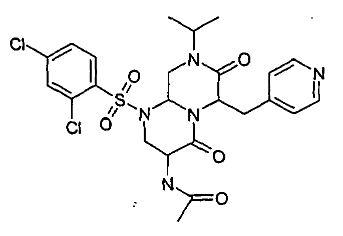

a) {1-[(2,2-Diethoxy-ethyl)-isopropyl-carbamoyl]-2-pyridin-4-yl-ethyl}-carbaminsäure 9H-fluoren-9-ylmethylester
   Die Synthese erfolgt analog zu Beispiel 29a) (Methode B) ausgehend von Fmoc-4-pyridyl-alanin. Man erhält das gewünschte Produkt mit MG = 545.29 (berechnet, monoisotop); Meßwert (M+H)⁺: 546.26
b) 2-Amino-N-(2,2-diethoxy-ethyl)-N-isopropyl-3-pyridin-4-yl-propionamid
   Die Synthese erfolgt analog zu Beispiel 29b) (Methode B) ausgehend von {1-[(2,2-Diethoxyethyl)-isopropyl-carbamoyl]-2-pyridin-4-yl-ethyl}-carbaminsäure 9H-fluoren-9-ylmethylester. Man erhält das gewünschte Produkt mit MG = 323.22 (berechnet, monoisotop); Meßwert (M+H)⁺: 324.22
c) [1-[(2,2-Diethoxy-ethyl)-isopropyl-carbamoyl]-2-pyridin-4-yl-ethylcarbamoyl}-2-(9H-fluoren-9-ylmethoxycarbonylamino)-ethyl]-carbaminsäurebenzylester
   Die Synthese erfolgte analog zu Beispiel 29c) (Methode B) ausgehend von 2-Amino-N-(2,2-diethoxy-ethyl)-N-isopropyl-3-pyridin-4-yl-propionamid . Man erhält das gewünschte Produkt mit MG = 809.36 (berechnet, monoisotop); Meßwert (M+Na)⁺: 832.3
d) (2-Amino-{1-[(2,2-diethoxy-ethyl)-isopropyl-carbamoyl]-2-pyridin-4-yl-ethylcarbamoyl}-ethyl)-carbaminsäurebenzyl ester
   Die Synthese erfolgte analog zu Beispiel 29d) (Methode B) ausgehend von [1-[(2,2-Diethoxyethyl)-isopropyl-carbamoyl]-2-pyridin-4-yl-ethylcarbamoyl}-2-(9H-fluoren-9-ylmethoxycarbonylamino)-ethyl]-carbaminsäurebenzylester. Man erhält das gewünschte Produkt mit MG = 543.31 (berechnet, monoisotop); Meßwert (M+H)⁺: 544.4
e) (2-(2,4-Dichlor-benzolsulfonylamino)-1-{1-[(2,2-diethoxy-ethyl)-isopropyl-carbamoyl]-2-pyridin-4-yl-ethylcarbamoyl}-ethyl)-carbaminsäurebenzylester
   Die Synthese erfolgte analog zu Beispiel 29e) (Methode B) ausgehend von (2-Amino-{1-[(2,2-diethoxy-ethyl)-isopropyl-carbamoyl]-2-pyridin-4-yl-ethylcarbamoyl} -ethyl)-carbaminsäure benzylester. Man erhält das gewünschte Produkt mit MG = 751.22 (berechnet, monoisotop); Meßwert (M+H)⁺: 752.19.
f) [1-(2,4-Dichlor-benzolsulfonyl)-8-isopropyl-4,7-dioxo-6-pyridin-4-ylmethyl-octahydro-pyrazino[1,2-a]pyrimidin-3-yl]-carbaminsäurebenzylester
   Die Synthese erfolgt analog zu Beispiel 29f) (Methode B) ausgehend von (2-(2,4-Dichlor-benzolsulfonylamino)-1-{1-[(2,2-diethoxy-ethyl)-isopropyl-carbamoyl]-2- pyridin-4-yl-ethylcarbamoyl}-ethyl)-carbaminsäurebenzylester. Man erhält das gewünschte Produkt mit MG = 659.14 (berechnet, monoisotop); Meßwert (M+H)⁺: 660.17
g) 3-Amino-1-(2,4-dichlor-benzolsulfonyl)-8-isopropyl-6-pyridin-4-ylmethyl-hexahydro-pyrazino[1,2-a]pyrimidin-4,7-dion
   Die Synthese erfolgt analog zu Beispiel 29g) (Methode B) ausgehend von [1-(2,4-Dichlor-benzolsulfonyl)-8-isopropyl-4,7-dioxo-6-pyridin-4-ylmethyl-octahydro- pyrazino[1,2-a]pyrimidin-3-yl]-carbaminsäurebenzylester. Man erhält das gewünschte Produkt mit MG = 525.10 (berechnet, monoisotop); Meßwert (M+H)⁺: 526.10
h) N-[1-(2,4-Dichlor-benzolsulfonyl)-8-isopropyl-4,7-dioxo-6-pyridin-4-ylmethyl- octahydro-pyrazino[1,2-a]pyrimidin-3-yl]-acetamid
   Die Synthese erfolgt analog zu Beispiel 21h) ausgehend von 3-Amino-1-(2,4-dichlor-benzolsulfonyl)-8-isopropyl-6-pyridin-4-ylmethyl-hexahydro- pyrazino[1,2-a]pyrimidin-4,7-dion. Man erhält das gewünschte Produkt mit MG = 567.11 (berechnet, monoisotop); Meßwert (M+H)⁺: 568.13

### Beispiel 194

### Cyclopropancarbonsäure [1-(2,4-dichlor-benzolsulfonyl)-8-isopropyl-4,7-dioxo-6- pyridin-4-ylmethyl-octahydro-pyrazino[1,2-a]pyrimidin-3-yl]-amid

### Struktur:

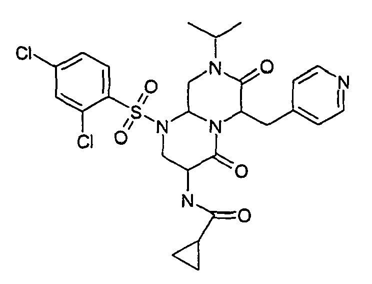

Die Synthese erfolgt analog zu Beispiel 22 ausgehend von 3-Amino-1-(2,4-dichlor-benzolsulfonyl)-8-isopropyl-6-pyridin-4-ylmethyl-hexahydro- pyrazino[1,2-a]pyrimidin-4,7-dion. Man erhält das gewünschte Produkt mit MG = 593.13 (berechnet, monoisotop); Meßwert (M+H)⁺: 594.13

### Beispiel 195

### Cyclopentancarbonsäure [1-(2,4-dichlor-benzolsulfonyl)-8-isopropyl-4,7-dioxo-6-pyridin-4-ylmethyl-octahydro-pyrazino[1,2-a]pyrimidin-3-yl]-amid

### Struktur:

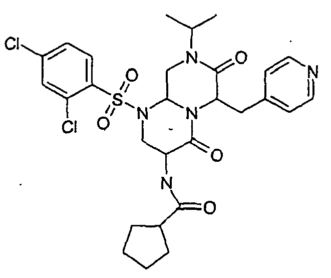

Die Synthese erfolgt analog zu Beispiel 31 ausgehend von 3-Amino-1-(2,4-dichlor-benzolsulfonyl)-8-isopropyl-6-pyridin-4-ylmethyl-hexahydro- pyrazino[1,2-a]pyrimidin-4,7-dion. Man erhält das gewünschte Produkt mit MG = 621.16 (berechnet, monoisotop); Meßwert (M+H)⁺: 622.17

### Beispiel 196

### N-[1-(2,4-Dichlor-benzolsulfonyl)-8-isopropyl-4,7-dioxo-6-pyridin-4-ylmethyl-octahydro-pyrazino[1,2-a]pyrimidin-3-yl]-4-dimethylamino-benzamid

### Struktur:

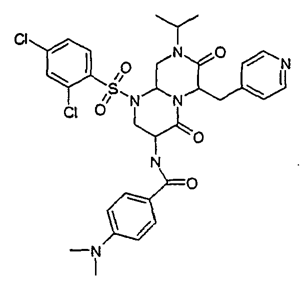

Die Synthese erfolgt analog zu Beispiel 25 ausgehend von 3-Amino-1-(2,4-dichlor-benzolsulfonyl)-8-isopropyl-6-pyridin-4-ylmethyl-hexahydro-pyrazino[1,2-a]pyrimidin-4,7-dion. Man erhält das gewünschte Produkt mit MG = 672.17 (berechnet, monoisotop); Meßwert (M+H)⁺: 673.2

### Beispiel 197

### 1-tert-Butyl-3-[1-(2,4-dichlor-benzolsulfonyl)-8-isopropyl-4,7-dioxo-6-pyridin-4- ylmethyloctahydro-pyrazino[1,2-a]pyrimidin-3-yl]-harnstoff

### Struktur:

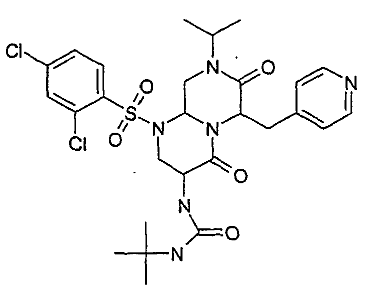

Die Synthese erfolgt analog zu Beispiel 28 ausgehend von 3-Amino-1-(2,4-dichlor-benzolsulfonyl)-8-isopropyl-6-pyridin-4-ylmethyl-hexahydro- pyrazino[1,2-a]pyrimidin-4,7-dion. Man erhält das gewünschte Produkt mit MG = 624.17 (berechnet, monoisotop); Meßwert (M+H)⁺: 625.2

### Beispiel 198

### N-[1-(2,4-Dichlor-benzolsulfonyl)-8-isopropyl-4,7-dioxo-6-pyridin-4-ylmethyl-octahydro-pyrazino[1,2-a]pyrimidin-3-yl]-methansulfonamid

### Struktur:

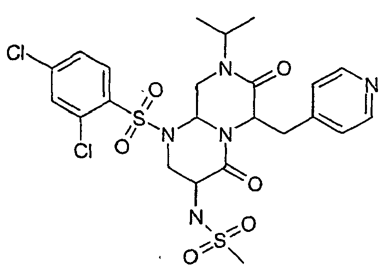

Die Synthese erfolgt analog zu Beispiel 26 ausgehend von 3-Amino-1-(2,4-dichlor-benzolsulfonyl)-8-isopropyl-6-pyridin-4-ylmethyl-hexahydro- pyrazino[1,2-a]pyrimidin-4,7-dion. Man erhält das gewünschte Produkt mit MG = 603.08 (berechnet, monoisotop); Meßwert (M+H)⁺: 604.08

### Beispiel 199

### Cyclopropansulfonsäure [1-(2,4-dichlor-benzolsulfonyl)-8-isopropyl-4,7-dioxo-6- pyridin-4-ylmethyl-octahydro-pyrazino[1,2-a]pyrimidin-3-yl]-amid

### Struktur:

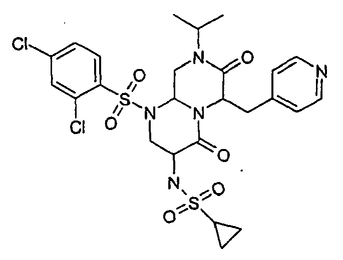

Die Synthese erfolgt analog zu Beispiel 27 ausgehend von 3-Amino-1-(2,4-dichlor-benzolsulfonyl)-8-isopropyl-6-pyridin-4-ylmethyl-hexahydro- pyrazino[1,2-a]pyrimidin-4,7-dion. Man erhält das gewünschte Produkt mit MG = 629.09 (berechnet, monoisotop); Meßwert (M+H)⁺: 630.06

### Beispiel 200

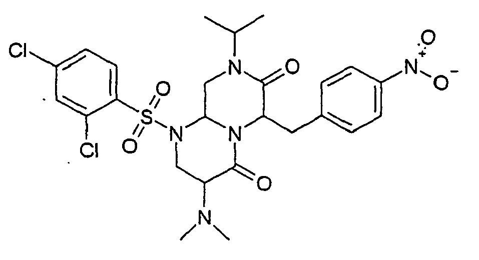

a) [1-[(2,2-Diethoxy-ethyl)-isopropyl-carbamoyl]-2-(4-nitro-phenyl)-ethyl]-carbaminsäure 9H-fluoren-9-ylmethylester
   Die Synthese erfolgt analog zu Beispiel 29a) (Methode B) ausgehend von N-Fmoc-4-nitro-Phe-OH. Man erhält das gewünschte Produkt mit MG = 589.28 (berechnet, monoisotop); Meßwert (M+H)⁺: 590.3
b) 2-Amino-N-(2,2-diethoxy-ethyl)-N-isopropyl-3-(4-nitro-phenyl)-propionamid
   Die Synthese erfolgt analog zu Beispiel 29b) (Methode B) ausgehend von [1-[(2,2-Diethoxyethyl)-isopropyl-carbamoyl]-2-(4-nitro-phenyl)-ethyl]-carbaminsäure 9H-fluoren-9-ylmethylester.
c) [1-[1-[(2,2-Diethoxy-ethyl)-isopropyl-carbamoyl]-2-(4-intro-phenyl)-ethylcarbamoyl]-2-(9H-fluoren-9-ylmethoxycarbonylamino)-ethyl]-carbaminsäurebenzylester
   Die Synthese erfolgte analog zu Beispiel 29c) (Methode B) ausgehend von 2-Amino-N-(2,2-diethoxy-ethyl)-N-isopropyl-3-(4-nitro-phenyl)-propionamid. Man erhält das gewünschte Produkt mit MG = 765.37 (berechnet, monoisotop); Meßwert (M+H)⁺: 766.31
d) {2-Amino-1-[1-[(2,2-diethoxy-ethyl)-isopropyl-carbamoyl]-2-(4-nitro-phenyl)-ethylcarbamoyl]-ethyl}-carbaminsäurebenzylester
   Die Synthese erfolgte analog zu Beispiel 29d) (Methode B) ausgehend von 1-[(2,2-Diethoxyethyl)-isopropyl-carbamoyl]-2-pyridin-4-yl-ethylcarbamoyl}-2-(9H-fluoren-9-ylmethoxycarbonylamino)-ethyl]-carbaminsäurebenzylester. Man erhält das gewünschte Produkt mit MG = 587.68 (berechnet, monoisotop); Meßwert (M-OEt): 542
e) {2-(2,4-Dichlor-benzolsulfonylamino)-1-[1-[(2,2-diethoxy-ethyl)-isopropyl-carbamoyl]-2-(4-nitro-phenyl)-ethylcarbamoyl]-ethyl}-carbaminsäurebenzylester
   Die Synthese erfolgte analog zu Beispiel 29e) (Methode B) ausgehend von {2-Amino-1-[1-[(2,2-diethoxy-ethyl)-isopropyl-carbamoyl]-2-(4-nitro-phenyl)-ethylcarbamoyl]-ethyl}-carbaminsäurebenzylester. Man erhält das gewünschte Produkt mit MG = 795.21 (berechnet, monoisotop); Meßwert (M+Na)⁺: 818.21.
f) [1-(2,4-Dichlor-benzolsulfonyl)-8-isopropyl-6-(4-nitro-benzyl)-4,7-dioxo-octahydro-pyrazino[1,2-a]pyrimidin-3-yl]-carbaminsäurebenzylester
   Die Synthese erfolgt analog zu Beispiel 29f) (Methode B) ausgehend von {2-(2,4-Dichlor-benzolsulfonylamino)-1-[1-[(2,2-diethoxy-ethyl)-isopropyl-carbamoyl]-2- (4-nitro-phenyl)-ethylcarbamoyl]-ethyl}-carbaminsäurebenzylester. Man erhält das gewünschte Produkt mit MG = 703.13 (berechnet, monoisotop); Meßwert (M+H)⁺: 704.1
g) 3-Amino-1-(2,4-dichlor-benzolsulfonyl)-8-isopropyl-6-(4-nitro-benzyl)-hexahydro-pyrazino[1,2-a]pyrimidin-4,7-dion
   Die Synthese erfolgt analog zu Beispiel 29g) (Methode B) ausgehend von [1-(2,4-Dichlor-benzolsulfonyl)-8-isopropyl-6-(4-nitro-benzyl)-4,7-dioxo-octahydro-pyrazino[1,2-a]pyrimidin-3-yl]-carbaminsäurebenzylester. Man erhält das gewünschte Produkt mit MG = 525.10 (berechnet, monoisotop); Meßwert (M+H)⁺: 526.10
h) 1-(2,4-Dichlor-benzolsulfonyl)-3-dimethylamino-8-isopropyl-6-(4-nitro-benzyl)-hexahydro-pyrazino[1,2-a]pyrimidin-4,7-dion
   Einer Lösung von 100 mg (0,17 mmol) 3-Amino-1-(2,4-dichlorbenzolsulfonyl)-8-isopropyl-6-(4-nitrobenzyl)-hexahydropyrazino[1,2-a]pyrimidin-4,7-dion in 5 mL Methanol wurden 0,15 mL Essigsäure und anschließend 0,15 mL Formaldehyd (37 % in H₂O) und 1 mL Natriumcyanoborhydrid (1M in THF) zugesetzt. Das Gemisch wurde 1 Stunde lang bei Raumtemperatur gerührt, im Vakuum eingeengt, mit Essigsäureethylester verdünnt und mit Wasser und Kochsalzlösung gewaschen. Dann wurde es getrocknet (MgSO₄) und im Vakuum eingeengt. Der Rückstand wurde an 12 g SiO₂ chromatographiert (Elution mit Essigsäureethylester/Methanol, Gradient 0 - 5 % Methanol). Man erhielt 25 mg des Dimethylamins. Das Amin wurde mit 1 mL 1M-HCl in Ether behandelt. Das Gemisch wurde im Vakuum eingeengt. Man erhielt 35 mg Dimethylamin-HCl-Salz als weißen Feststoff.
   LC/MS MG = 597,12 (berechnet, monoisotop) Meßwert (M⁺H) = 598,12

### Beispiel 201

### Cyclopentancarbonsäure [6-(4-amino-benzyl)-1-(2,4-dichlor-benzolsulfonyl)-8- isopropyl-4,7-dioxo-octahydro-pyrazino[1,2-a]pyrimidin-3-yl]-amid

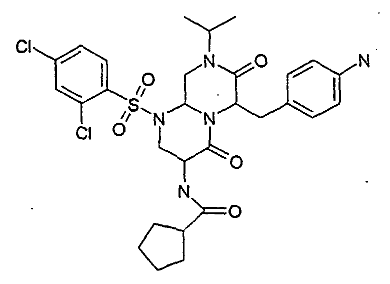

3-Amino-1-(2,4-dichlor-benzolsulfonyl)-8-isopropyl-6-(4-nitro-benzyl)-hexahydro-pyrazino[1,2-a]pyrimidin-4,7-dion wurde analog Beispiel 31 zum Cyclopentancarbonsäureamid umgesetzt. Diese wurde folgendermaßen weiter umgesetzt:

Einer Lösung von 50 mg (0,07 mmol) Cyclopentancarbonsäure-[1-(2,4-dichlorbenzolsulfonyl)-8-isopropyl-6-(4-nitrobenzyl)-4,7-dioxooctahydropyrazino[1,2-a]pyrimidin-3-yl]amid (Beispiel 200) in 3 mL Ethanol wurden 71 mg (0,3 mmol) Zinn(II)-chlorid zugesetzt. Das Gemisch wurde im Mikrowellenofen 5 Minuten lang auf 100°C erwärmt und im Vakuum eingeengt. Der Rückstand wurde mit Essigsäureethylester verdünnt, durch Kieselgur filtriert und im Vakuum eingeengt. Dann wurde er an 4 g SiO₂ chromatographiert (Elution mit Essigsäureethylester/Heptan, Gradient 0 - 100 % Essigsäureethylester). Man erhielt 25 mg des gewünschten Anilins als Öl. LC/MS MG (berechnet, monoisotop) = 635,17 und der Meßwert (M⁺H) = 636,14.

### Beispiel 202

### 6-(4-Amino-benzyl)-1-(2,4-dichlor-benzolsulfonyl)-3-dimethylamino-8-isopropyl-hexahydro-pyrazino[1,2-a]pyrimidin-4,7-dion

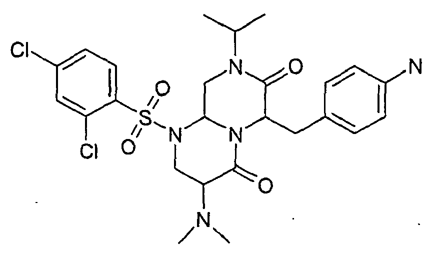

Beispiel 202 wurde analog zu Beispiel 201 reduziert ausgehend von Verbindung 1-(2,4-Dichlor-benzolsulfonyl)-3-dimethylamino-8-isopropyl-6-(4-nitro-benzyl)-hexahydro-pyrazino[1,2-a]pyrimidin-4,7-dion. Man erhält das gewünschte Produkt mit MG = 567.15 (berechnet, monoisotop); Meßwert (M+H)⁺: 568.1

### Beispiel 203

### 6-(4-Amino-benzyl)-1-(2,4-dichlor-benzolsulfonyl)-8-isopropyl-3-piperidin-1-yl-hexahydro-pyrazino[1,2-a]pyrimidin-4,7-dion

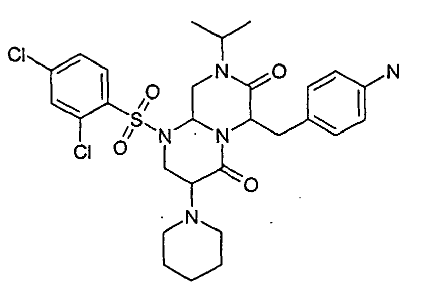

Die Synthese erfolgt analog zu Beispiel 59 ausgehend von 3-Amino-1-(2,4-dichlor-benzolsulfonyl)-8-isopropyl-6-(4-nitro-benzyl)-hexahydro- pyrazino[1,2-a]pyrimidin-4,7-dion zum entsprechenden Piperidinderivat. Die anschließende Reduktion der Nitrogruppe erfolgte wie in Beispiel 201 beschrieben. Man erhält das gewünschte Produkt mit MG = 607.18 (berechnet, monoisotop); Meßwert (M+H)⁺: 608.2

### Beispiel 204

### 4-Chlor-N-{4-[3-(cyclopentancarbonyl-amino)-1-(2,4-dichlor-benzolsulfonyl)-8-isopropyl-4,7-dioxo-octahydro-pyrazino[1,2-a]pyrimidin-6-ylmethyl]-phenyl}-benzamid

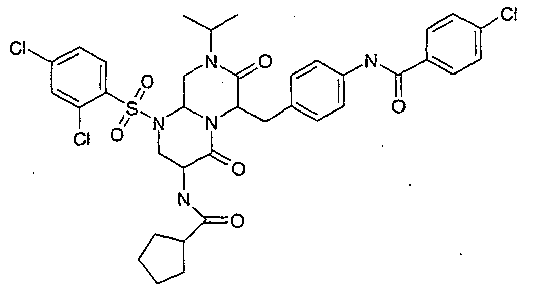

Einer Lösung von 25 mg (0,03 mmol) [6-(4-Aminobenzyl)-1-(2,4-dichlorbenzolsulfonyl)-8-isopropyl-4,7-dioxooctahydropyrazino[1,2-a]pyrimidin-3-yl]-carbaminsäurebenzylester in 1 mL Dichlormethan wurden 5 mg (0,04 mmol) Diisopropylethylamin und anschließend 7 mg (0,04 mmol) 4-Chlorbenzoylchlorid zugesetzt. Das Gemisch wurde über Nacht bei Raumtemperatur gerührt, mit Dichlormethan verdünnt und mit Kochsalzlösung gewaschen. Die organische Phase wurde isoliert, getrocknet (MgSO₄) und im Vakuum eingeengt. Der Rückstand wurde an 8 g SiO₂ chromatographiert (Elution mit Essigsäureethylester/Heptan, Gradient 0 - 100 % Essigsäureethylester). Man erhielt 28,5 mg Amid als Öl. Diese Verbindung wurde im nächsten Schritt direkt verwendet.

Eine Lösung von 28,5 mg (0,03 mmol) des obengenannten Cbz-carbamats in 2 mL Acetonitril wurde bei 0°C mit 28 mg (0,14 mmol) TMSI versetzt. Das Gemisch wurde über Nacht bei Raumtemperatur stehengelassen, im Vakuum eingeengt, mit Methanol verdünnt und durch eine SCX (5 g)-Patrone filtriert (Elution mit 5 mL Methanol und anschließend mit 15 mL 7N-Ammoniak in Methanol). Man erhielt 18 mg des gewünschten Amins als braunes Öl. Diese Verbindung wurde ohne weitere Reinigung im nächsten Schritt verwendet.

Einer Lösung von 18 mg (0,02 mmol) der deblockierten Verbindung in 1 mL DCM wurden 3 mg (0,02 mmol) DIEA und anschließend 2,7 mg (0,02 mmol) Cyclopentancarbonylchlorid zugesetzt. Das Gemisch wurde über Nacht bei Raumtemperatur gerührt und im Vakuum eingeengt. Der Rückstand wurde in 2 mL Essigsäureethylester gelöst, mit Wasser gewaschen, getrocknet (MgSO₄) und im Vakuum eingeengt. Dann wurde dieser an 4 g SiO₂ chromatographiert (Elution mit Essigsäureethylester/Heptan, Gradient 0 - 100 % Essigsäureethylester). Man erhielt 11 mg des gewünschten Amids als Öl.LC/MS MG = 773,16 (berechnet, monoisotop); Meßwert (M⁺H) = 774,12

### Beispiel 205

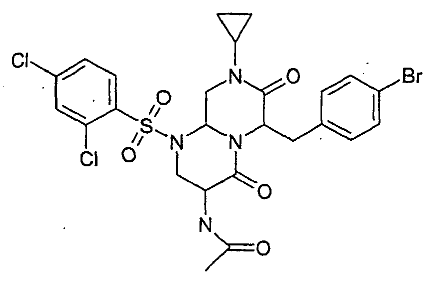

a) Cyclopropyl-(2,2-diethoxy-ethyl)-amin
   Eine Lösung von 2.36g (12 mmol) 2-Bromacetaldehyd-diethylacetal und 4.94 g (86.6 mmol) Cyclopropylamin wurde in einem geschlossenen Gefäß für 2 h auf 120°C erhitzt. Die Reaktionsmischung wurde auf Raumtemperatur abgekühlt und mit 75 mL Ether verdünnt und mit 5% aq NaOH gewaschen, gefolgt von Wasser und gesättigter Natriumchloridlösung. Die organische Phase wurde getrocknet (MgSO₄) und im Vakuum aufkonzentriert. Der Rückstand wurde im Vakuum destilliert (130°C-140°C). Es wurden 2.01 g der gewünschten Verbindung erhalten. LC/MS M+H = 173.
b) [1-[Cyclopropyl- (2,2-diethoxy-ethyl)- carbamoyl]-2-(4-brom-phenyl)-ethyl]- carbaminsäure 9H-fluoren-9-ylmethylester
   Die Synthese erfolgt analog zu Beispiel 29a) (Methode B) ausgehend von N-Fmoc-4-Br-Phe-OH und Cyclopropyl-(2,2-diethoxy-ethyl)-amin. Man erhält das gewünschte Produkt mit MG = 629.19 (berechnet, monoisotop); Meßwert (M+Na)⁺: 643.16
c) 2-Amino-3-(4-brom-phenyl)-N-cyclopropyl-N-(2,2-diethoxy-ethyl)-propionamid
   Die Synthese erfolgt analog zu Beispiel 29b) (Methode B) ausgehend von [1-[Cyclopropyl-(2,2-diethoxy-ethyl)- carbamoyl]-2-(4-brom-phenyl)-ethyl]- carbaminsäure 9H-fluoren-9-ylmethylester. Man erhält das gewünschte Produkt mit MG = 398.12 (berechnet, monoisotop); Meßwert (M+H)⁺: 399.11.
d) [1-{2-(4-Brom-phenyl)-1-[cyclopropyl-(2,2-diethoxy-ethyl)-carbamoyl]-ethylcarbamoyl}-2-(9H-fluoren-9-ylmethoxycarbonylamino)-ethyl]-carbaminsäure-benzylester
   Die Synthese erfolgte analog zu Beispiel 29c) (Methode B) ausgehend von 2-Amino-3-(4-brom-phenyl)-N-cyclopropyl-N-(2,2-diethoxy-ethyl)-propionamid. Man erhält das gewünschte Produkt mit MG = 840.27 (berechnet, monoisotop); Meßwert (M+Na)⁺: 863.23
e) (2-Amino-1-{2-(4-brom-phenyl)-1-[cyclopropyl-(2,2-diethoxy-ethyl)-carbamoyl]-ethylcarbamoyl}-ethyl)-carbaminsäurebenzylester Die Synthese erfolgte analog zu Beispiel 29d) (Methode B) ausgehend von [1-{2-(4-Brom-phenyl)-1-[cyclopropyl-(2,2-diethoxy-ethyl)-carbamoyl]-ethylcarbamoyl}-2-(9H-fluoren-9-ylmethoxycarbonylamino)-ethyl]-carbaminsäure-benzylester . Man erhält das gewünschte Produkt mit MG = 618.21 (berechnet, monoisotop); Meßwert (M+H)⁺: 619.2
f) [1-{2-(4-Brom-phenyl)-1-[cyclopropyl-(2,2-diethoxy-ethyl)-carbamoyl]-ethylcarbamoyl}-2-(2,4-dichlor-benzolsulfonylamino)-ethyl]-carbaminsäurebenzylester
   Die Synthese erfolgte analog zu Beispiel 29e) (Methode B) ausgehend von (2-Amino-1-{2-(4-brom-phenyl)-1-[cyclopropyl-(2,2-diethoxy-ethyl)-carbamoyl]- ethylcarbamoyl}-ethyl)-carbaminsäurebenzylester. Man erhält das gewünschte Produkt mit MG = 826.12 (berechnet, monoisotop); Meßwert (M+Na)⁺: 849.14.
g) [6-(4-Brom-benzyl)-8-cyclopropyl-1-(2,4-dichlor-benzolsulfonyl)-4,7-dioxo-octahydro-pyrazino[1,2-a]pyrimidin-3-yl]-carbaminsäurebenzyl ester Die Synthese erfolgte analog zu Beispiel 29f) (Methode B) ausgehend von [1-{2-(4-Brom-phenyl)-1-[cyclopropyl-(2,2-diethoxy-ethyl)-carbamoyl]-ethylcarbamoyl}-2- (2,4-dichlor-benzolsulfonylamino)-ethyl]-carbaminsäurebenzylester. Man erhält das gewünschte Produkt mit MG = 734.04 (berechnet, monoisotop); Meßwert (M+H)⁺: 735.02
h) 3-Amino-6-(4-brom-benzyl)-8-cyclopropyl-1-(2,4-dichlor-benzolsulfonyl)-hexahydro-pyrazino[1,2-a]pyrimidin-4,7-dion
   Die Synthese erfolgt analog zu Beispiel 29g) (Methode B) ausgehend von [6-(4-Brom-benzyl)-8-cyclopropyl-1-(2,4-dichlor-benzolsulfonyl)-4,7-dioxo-octahydro- pyrazino[1,2-a]pyrimidin-3-yl]-carbaminsäurebenzylester . Man erhält das gewünschte Produkt mit MG = 600.0 (berechnet, monoisotop); Meßwert (M+H)⁺: 600.99
i) N-[6-(4-Brom-benzyl)-8-cyclopropyl-1-(2,4-dichlor-benzolsulfonyl)-4,7-dioxo- octahydro-pyrazino[1,2-a]pyrimidin-3-yl]-acetamid
   Die Synthese erfolgt analog zu Beispiel 21h) ausgehend von 3-Amino-6-(4-brom-benzyl)-8-cyclopropyl-1-(2,4-dichlor-benzolsulfonyl)-hexahydro-pyrazino[1,2-a]pyrimidin-4,7-dion. Man erhält das gewünschte Produkt mit MG = 642.01 (berechnet, monoisotop); Meßwert (M+H)⁺: 643.02

### Beispiel 206

### Cyclopropancarbonsäure [6-(4-brom-benzyl)-8-cyclopropyl-1-(2,4-dichlor- benzolsulfonyl)-4,7-dioxo-octahydro-pyrazino[1,2-a]pyrimidin-3-yl]-amid

### Struktur:

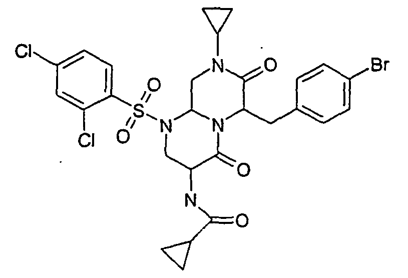

Die Synthese erfolgt analog zu Beispiel 22 ausgehend von 3-Amino-6-(4-brom-benzyl)-8-cyclopropyl-1-(2,4-dichlor-benzolsulfonyl)-hexahydro- pyrazino[1,2-a]pyrimidin-4,7-dion. Man erhält das gewünschte Produkt mit MG = 668.03 (berechnet, monoisotop); Meßwert (M+H)⁺: 669.04

### Beispiel 207

### Cyclobutancarbonsäure [6-(4-brom-benzyl)-8-cyclopropyl-1-(2,4-dichlor-benzolsulfonyl)-4,7-dioxo-octahydro-pyrazino [1,2-a]pyrimidin-3-yl]-amid

### Struktur:

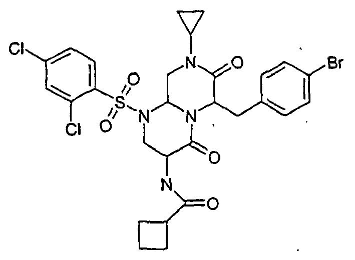

Die Synthese erfolgt analog zu Beispiel 30 ausgehend von 3-Amino-6-(4-brom-benzyl)-8-cyclopropyl-1-(2,4-dichlor-benzolsulfonyl)-hexahydro- pyrazino[1,2-a]pyrimidin-4,7-dion. Man erhält das gewünschte Produkt mit MG = 682.04 (berechnet, monoisotop); Meßwert (M+H)⁺: 683.06

### Beispiel 208

### Cyclopentancarbonsäure [6-(4-brom-benzyl)-8-cyclopropyl-1-(2,4-dichlor- benzolsulfonyl)-4,7-dioxo-octahydro-pyrazino[1,2-a]pyrimidin-3-yl]-amid

### Struktur:

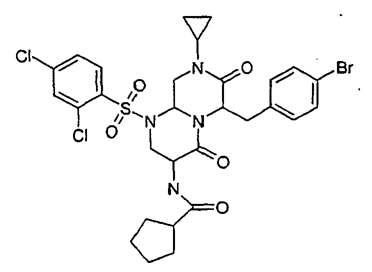

Die Synthese erfolgt analog zu Beispiel 31 ausgehend von 3-Amino-6-(4-brom-benzyl)-8-cyclopropyl-1-(2,4-dichlor-benzolsulfonyl)-hexahydro-pyrazino[1,2-a]pyrimidin-4,7-dion. Man erhält das gewünschte Produkt mit MG = 696.06 (berechnet, monoisotop); Meßwert (M+H)⁺: 697.08

### Beispiel 209

### Pyrrolidin-2-carbonsäure [6-(4-brom-benzyl)-8-cyclopropyl-1-(2,4-dichlor- benzolsulfonyl)-4,7-dioxo-octahydro-pyrazino[1,2-a]pyrimidin-3-yl]-amid

### Struktur:

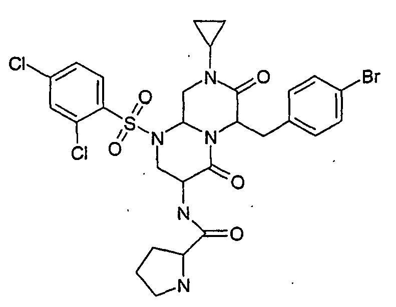

Die Synthese erfolgt analog zu Beispiel 40 ausgehend von 3-Amino-6-(4-brom-benzyl)-8-cyclopropyl-1-(2,4-dichlor-benzolsulfonyl)-hexahydro- pyrazino[1,2-a]pyrimidin-4,7-dion. Man erhält das gewünschte Produkt mit MG = 697.05 (berechnet, monoisotop); Meßwert (M+H)⁺: 698.02

### Beispiel 210

### N-[6-(4-Brom-benzyl)-8-cyclopropyl-1-(2,4-dichlor-benzolsulfonyl)-4,7-dioxo- octahydro-pyrazino[1,2-a]pyrimidin-3-yl]-4-dimethylamino-benzamid

### Struktur:

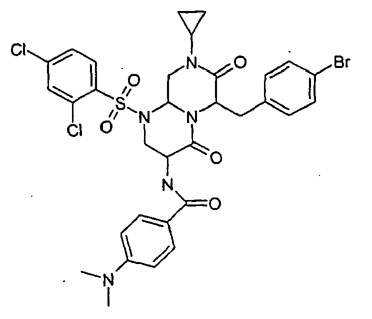

Die Synthese erfolgt analog zu Beispiel 47 ausgehend von 3-Amino-6-(4-brom-benzyl)-8-cyclopropyl-1-(2,4-dichlor-benzolsulfonyl)-hexahydro- pyrazino[1,2-a]pyrimidin-4,7-dion. Man erhält das gewünschte Produkt mit MG = 747.07 (berechnet, monoisotop); Meßwert (M+H)⁺: 748.08

### Beispiel 211

### N-[6-(4-Brom-benzyl)-8-cyclopropyl-1-(2,4-dichlor-benzolsulfonyl)-4,7-dioxo-octahydro-pyrazino[1,2-a]pyrimidin-3-yl]-nicotinamid

### Struktur:

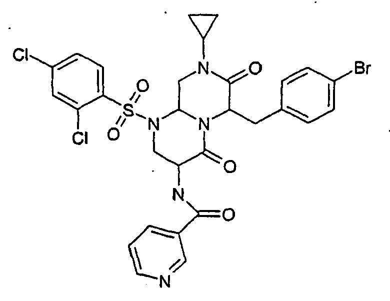

Die Synthese erfolgt analog zu Beispiel 50 ausgehend von 3-Amino-6-(4-brom-benzyl)-8-cyclopropyl-1-(2,4-dichlor-benzolsulfonyl)-hexahydro- pyrazino[1,2-a]pyrimidin-4,7-dion. Man erhält das gewünschte Produkt mit MG = 705.02 (berechnet, monoisotop); Meßwert (M+H)⁺: 706.06

### Beispiel 212

### 1-[6-(4-Brom-benzyl)-8-cyclopropyl-1-(2,4-dichlor-benzolsulfonyl)-4,7-dioxo-octahydro-pyrazino[1,2-a]pyrimidin-3-yl]-3-tert-butyl-harnstoff

### Struktur:

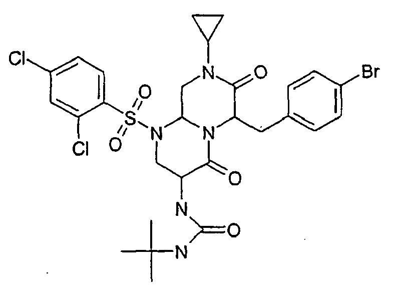

Die Synthese erfolgt analog zu Beispiel 28 ausgehend von 3-Amino-6-(4-brom-benzyl)-8-cyclopropyl-1-(2,4-dichlor-benzolsulfonyl)-hexahydro- pyrazino[1,2-a]pyrimidin-4,7-dion. Man erhält das gewünschte Produkt mit MG = 699.07 (berechnet, monoisotop); Meßwert (M+H)⁺: 700.09

### Beispiel 213

### N-[6-(4-Brom-benzyl)-8-cyclopropyl-1-(2,4-dichlor-benzolsulfonyl)-4,7-dioxo- octahydro-pyrazino[1,2-a]pyrimidin-3-yl]-methansulfonamid

### Struktur:

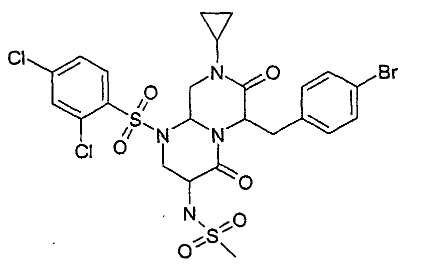

Die Synthese erfolgt analog zu Beispiel 26 ausgehend von 3-Amino-6-(4-brom-benzyl)-8-cyclopropyl-1-(2,4-dichlor-benzolsulfonyl)-hexahydro- pyrazino[1,2-a]pyrimidin-4,7-dion. Man erhält das gewünschte Produkt mit MG = 677.98 (berechnet, monoisotop); Meßwert (M+H)⁺: 679.0

### Beispiel 214

### Cyclopropansulfonsäure [6-(4-brom-benzyl)-8-cyclopropyl-1-(2,4-dichlor- benzolsulfonyl)-4,7-dioxo-octahydro-pyrazino[1,2-a]pyrimidin-3-yl]-amid

### Struktur:

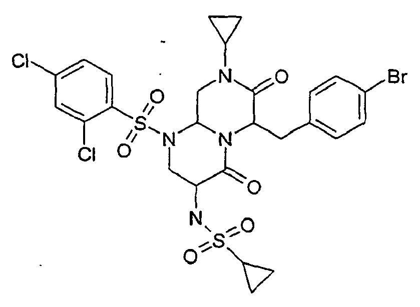

Die Synthese erfolgt analog zu Beispiel 27 ausgehend von 3-Amino-6-(4-brom-benzyl)-8-cyclopropyl-1-(2,4-dichlor-benzolsulfonyl)-hexahydro-pyrazino[1,2-a]pyrimidin-4,7-dion. Man erhält das gewünschte Produkt mit MG = 703.99 (berechnet, monoisotop); Meßwert (M+H)⁺: 705.01

### Beispiel 215

### 6-(4-Brom-benzyl)-8-cyclopropyl-1-(2,4-dichlor-benzolsulfonyl)-3-dimethylamino-hexahydro-pyrazino[1,2-a]pyrimidin-4,7-dion

### Struktur:

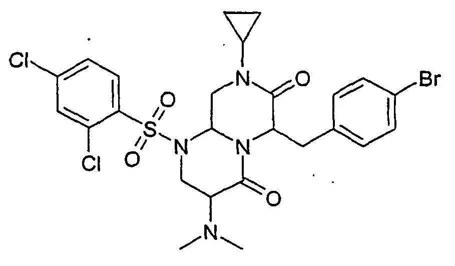

Die Synthese erfolgt analog zu Beispiel 72 ausgehend von 3-Amino-6-(4-brom-benzyl)-8-cyclopropyl-1-(2,4-dichlor-benzolsulfonyl)-hexahydro- pyrazino[1,2-a]pyrimidin-4,7-dion. Man erhält das gewünschte Produkt mit MG = 628.03 (berechnet, monoisotop); Meßwert (M+H)⁺: 629.44

### Beispiel 216

### 3-Azetidin-1-yl-6-(4-brom-benzyl)-8-cyclopropyl-1-(2,4-dichlor-benzolsulfonyl)-hexahydro-pyrazino[1,2-a]pyrimidin-4,7-dion

### Struktur:

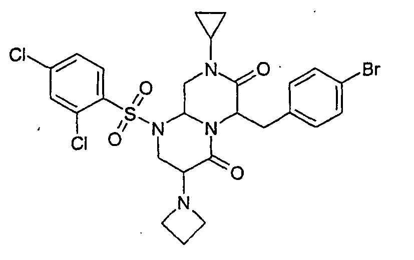

Die Synthese erfolgt analog zu Beispiel 73 ausgehend von 3-Amino-6-(4-brom-benzyl)-8-cyclopropyl-1-(2,4-dichlor-benzolsulfonyl)-hexahydro-pyrazino[1,2-a]pyrimidin-4,7-dion. Man erhält das gewünschte Produkt mit MG = 636.08 (berechnet, monoisotop); Meßwert (M+H)⁺: 637.1

### Beispiel 217

### 6-(4-Brom-benzyl)-8-cyclopropyl-1-(2,4-dichlor-benzolsulfonyl)-3-pyrrolidin-1-yl-hexahydro-pyrazino[1,2-a]pyrimidin-4,7-dion

### Struktur:

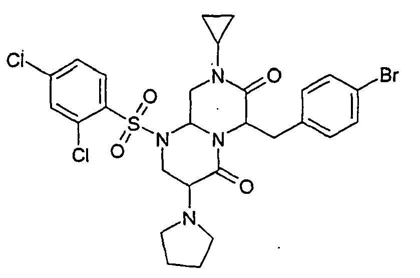

Die Synthese erfolgt analog zu Beispiel 74 ausgehend von 3-Amino-6-(4-brom-benzyl)-8-cyclopropyl-1-(2,4-dichlor-benzolsulfonyl)-hexahydro- pyrazino[1,2-a]pyrimidin-4,7-dion. Man erhält das gewünschte Produkt mit MG = 654.05 (berechnet, monoisotop); Meßwert (M+H)⁺: 655.04

### Beispiel 218

### 6-(4-Brom-benzyl)-8-cyclopropyl-1-(2,4-dichlor-benzolsulfonyl)-3-piperidin-1-yl-hexahydro-pyrazino[1,2-a]pyrimidin-4,7-dion

### Struktur:

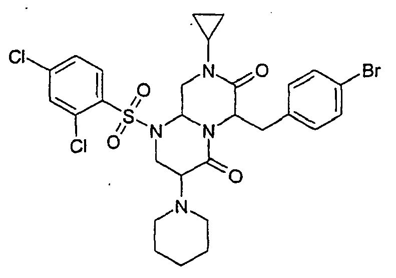

Die Synthese erfolgt analog zu Beispiel 59 ausgehend von 3-Amino-6-(4-brom-benzyl)-8-cyclopropyl-1-(2,4-dichlor-benzolsulfonyl)-hexahydro-pyrazino[1,2-a]pyrimidin-4,7-dion. Man erhält das gewünschte Produkt mit MG = 668.06 (berechnet, monoisotop); Meßwert (M+H)⁺: 669.08

### Beispiel 219

### 6-(4-Brom-benzyl)-8-cyclopropyl-1-(2,4-dichlor-benzolsulfonyl)-3-morpholin-4-yl-hexahydro-pyrazino[1,2-a]pyrimidin-4,7-dion

### Struktur:

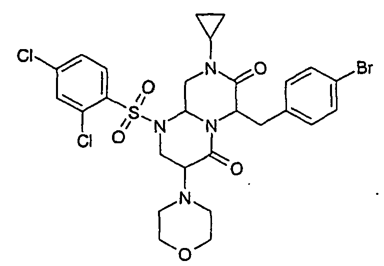

Die Synthese erfolgt analog zu Beispiel 60 ausgehend von 3-Amino-6-(4-brom-benzyl)-8-cyclopropyl-1-(2,4-dichlor-benzolsulfonyl)-hexahydro-pyrazino[1,2-a]pyrimidin-4,7-dion. Man erhält das gewünschte Produkt mit MG = 670.04 (berechnet, monoisotop); Meßwert (M+H)⁺: 670.97

### Beispiel 220

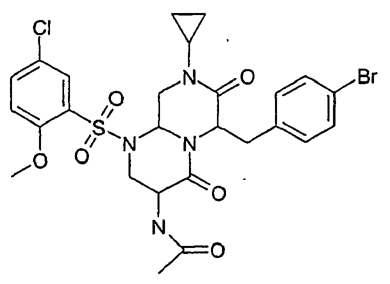

a) [1-{2-(4-Brom-phenyl)-1-[cyclopropyl-(2,2-diethoxy-ethyl)-carbamoyl]-ethylcarbamoyl}-2-(5-chlor-2-methoxy-benzolsulfonylamino)-ethyl]-carbaminsäurebenzylester
   Die Synthese erfolgt analog zu Beispiel 29e) (Methode B) ausgehend von (2-Amino-1-{2-(4-brom-phenyl)-1-[cyclopropyl-(2,2-diethoxy-ethyl)-carbamoyl]-ethylcarbamoyl}-ethyl)-carbaminsäure-benzylester. Man erhält das gewünschte Produkt mit MG = 822.17 (berechnet, monoisotop); Meßwert (M+Na)⁺: 845.19
b) [6-(4-Brom-benzyl)-1-(5-chlor-2-methoxy-benzolsulfonyl)-8-cyclopropyl-4,7-dioxo-octahydro- pyrazino[1,2-a]pyrimidin-3-yl]-carbaminsäurebenzylester
   Die Synthese erfolgt analog zu Beispiel 29f) (Methode B) ausgehend von [1-{2-(4-Bromphenyl)-1-[cyclopropyl-(2,2-diethoxy-ethyl)-carbamoyl]-ethylcarbamoyl}-2-(5-chlor-2-methoxy-benzolsulfonylamino)-ethyl]-carbaminsäurebenzylester. Man erhält das gewünschte Produkt mit MG = 730.09 (berechnet, monoisotop); Meßwert (M+H)⁺: 731.09
c) 3-Amino-6-(4-brom-benzyl)-1-(5-chlor-2-methoxy-benzolsulfonyl)-8-cyclopropyl-hexahydro- pyrazino[1,2-a]pyrimidin-4,7-dion
   Die Synthese erfolgt analog zu Beispiel 29g) (Methode B) ausgehend von [6-(4-Brom-benzyl)-1-(5-chlor-2-methoxy-benzolsulfonyl)-8-cyclopropyl-4,7-dioxo-octahydro- pyrazino[1,2-a]pyrimidin-3-yl]-carbaminsäurebenzylester . Man erhält das gewünschte Produkt mit MG = 596.05 (berechnet, monoisotop); Meßwert (M+H)⁺: 597.04
d) N-[6-(4-Brom-benzyl)-1-(5-chlor-2-methoxy-benzolsulfonyl)- 8-cyclopropyl-4,7-dioxo-octahydro-pyrazino[1,2-a]pyrimidin-3-yl]-acetamid
   Die Synthese erfolgt analog zu Beispiel 21h) ausgehend von 3-Amino-6-(4-brom-benzyl)-1-(5-chlor-2-methoxy-benzolsulfonyl)-8-cyclopropyl-hexahydro- pyrazino[1,2-a]pyrimidin-4,7-dion. Man erhält das gewünschte Produkt mit MG = 638.06 (berechnet, monoisotop); Meßwert (M+H)⁺: 639.11.

### Beispiel 221

### Cyclopropancarbonsäure [6-(4-brom-benzyl)-1-(5-chlor-2-methoxy-benzolsulfonyl)-8-cyclopropyl-4,7-dioxo-octahydro-pyrazino[1,2-a]pyrimidin-3-yl]-amid

### Struktur:

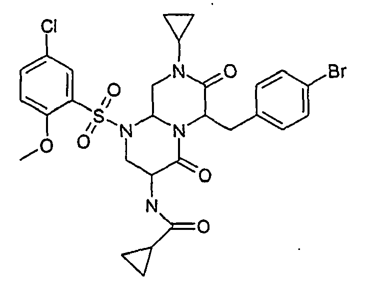

Die Synthese erfolgt analog zu Beispiel 22 ausgehend von 3-Amino-6-(4-brom-benzyl)-1-(5-chlor-2-methoxy-benzolsulfonyl)-8-cyclopropyl-hexahydro-pyrazino[1,2-a]pyrimidin-4,7-dion. Man erhält das gewünschte Produkt mit MG = 664.08 (berechnet, monoisotop); Meßwert (M+H)⁺: 665.13

### Beispiel 222

### Cyclobutancarbonsäure [6-(4-brom-benzyl)-1-(5-chlor-2-methoxy-benzolsulfonyl)-8-cyclopropyl-4,7-dioxo-octahydro-pyrazino[1,2-a]pyrimidin-3-yl]-amid -

### Struktur:

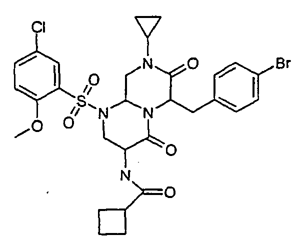

Die Synthese erfolgt analog zu Beispiel 30 ausgehend von 3-Amino-6-(4-brom-benzyl)-1-(5-chlor-2-methoxy-benzolsulfonyl)-8-cyclopropyl-hexahydro-pyrazino[1,2-a]pyrimidin-4,7-dion. Man erhält das gewünschte Produkt mit MG = 678.09 (berechnet, monoisotop); Meßwert (M+H)⁺: 679.15

### Beispiel 223

### Cyclopentancarbonsäure [6-(4-brom-benzyl)-1-(5-chlor-2-methoxy-benzolsulfonyl)-8-cyclopropyl-4,7-dioxo-octahydro-pyrazino[1,2-a]pyrimidin-3-yl]-amid

### Struktur:

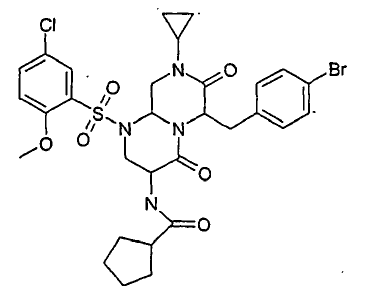

Die Synthese erfolgt analog zu Beispiel 31 ausgehend von 3-Amino-6-(4-brom-benzyl)-1-(5-chlor-2-methoxy-benzolsulfonyl)-8-cyclopropyl-hexahydro-pyrazino[1,2-a]pyrimidin-4,7-dion. Man erhält das gewünschte Produkt mit MG = 692.11 (berechnet, monoisotop); Meßwert (M+H)⁺: 693.16

### Beispiel 224

### Pyrrolidin-2-carbonsäure [6-(4-brom-benzyl)-1-(5-chlor-2-methoxy-benzolsulfonyl)- 8-cyclopropyl-4,7-dioxo-octahydro-pyrazino[1,2-a]pyrimidin-3-yl]-amid

### Struktur:

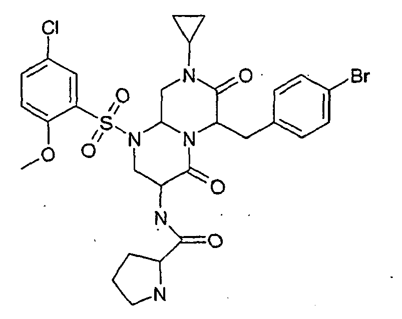

Die Synthese erfolgt analog zu Beispiel 40 ausgehend von 3-Amino-6-(4-brom-benzyl)-1-(5-chlor-2-methoxy-benzolsulfonyl)-8-cyclopropyl-hexahydro-pyrazino[1,2-a]pyrimidin-4,7-dion. Man erhält das gewünschte Produkt mit MG = 693.10 (berechnet, monoisotop); Meßwert (M+H)⁺: 694.06

### Beispiel 225

### N-[6-(4-Brom-benzyl)-1-(5-chlor-2-methoxy-benzolsulfonyl)-8-cyclopropyl-4,7-dioxo-octahydro-pyrazino[1,2-a]pyrimidin-3-yl]-4-dimethylamino-benzamid

### Struktur:

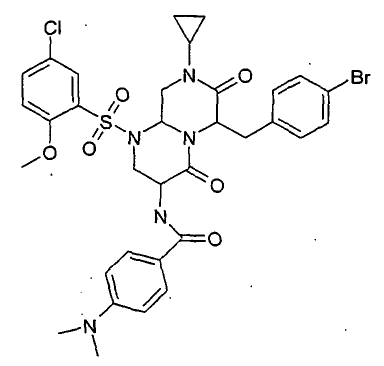

Die Synthese erfolgt analog zu Beispiel 25 ausgehend von 3-Amino-6-(4-brom-benzyl)-1-(5-chlor-2-methoxy-benzolsulfonyl)-8-cyclopropyl-hexahydro- pyrazino[1,2-a]pyrimidin-4,7-dion. Man erhält das gewünschte Produkt mit MG = 743.12 (berechnet, monoisotop); Meßwert (M+H)⁺: 744.17

### Beispiel 226

### N-[6-(4-Brom-benzyl)-1-(5-chlor-2-methoxy-benzolsulfonyl)-8-cyclopropyl-4,7-dioxo-octahydro-pyrazino[1,2-a]pyrimidin-3-yl]-nicotinamid

### Struktur:

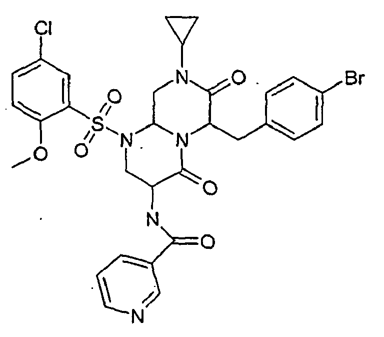

Die Synthese erfolgt analog zu Beispiel 50 ausgehend von 3-Amino-6-(4-brom-benzyl)-1-(5-chlor-2-methoxy-benzolsulfonyl)-8-cyclopropyl-hexahydro-pyrazino[1,2-a]pyrimidin-4,7-dion. Man erhält das gewünschte Produkt mit MG = 701.07 (berechnet, monoisotop); Meßwert (M+H)⁺: 702.08

### Beispiel 227

### 1-[6-(4-Brom-benzyl)-1-(5-chlor-2-methoxy-benzolsulfonyl)-8-cyclopropyl-4,7-dioxo-octahydro-pyrazino[1,2-a]pyrimidin-3-yl]-3-tert-butyl-harnstoff

### Struktur:

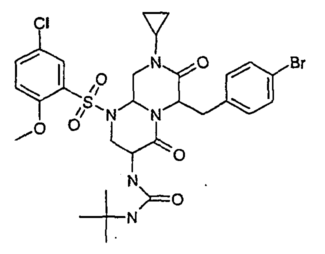

Die Synthese erfolgt analog zu Beispiel 28 ausgehend von 3-Amino-6-(4-brom-benzyl)-1-(5-chlor-2-methoxy-benzolsulfonyl)-8-cyclopropyl-hexahydro-pyrazino[1,2-a]pyrimidin-4,7-dion. Man erhält das gewünschte Produkt mit MG = 695.12 (berechnet, monoisotop); Meßwert (M+H)⁺: 696.17

### Beispiel 228

### N-[6-(4-Brom-benzyl)-1-(5-chlor-2-methoxy-benzolsulfonyl)-8-cyclopropyl-4,7-dioxo-octahydro-pyrazino[1,2-a]pyrimidin-3-yl]-methansulfonamid

### Struktur:

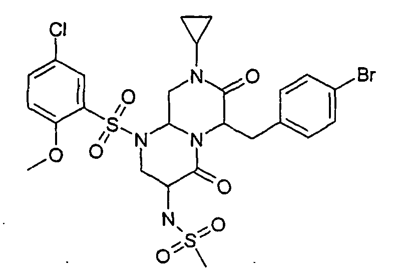

Die Synthese erfolgt analog zu Beispiel 26 ausgehend von 3-Amino-6-(4-brom-benzyl)-1-(5-chlor-2-methoxy-benzolsulfonyl)-8-cyclopropyl-hexahydro-pyrazino[1,2-a]pyrimidin-4,7-dion. Man erhält das gewünschte Produkt mit MG = 674.03 (berechnet, monoisotop); Meßwert (M+H)⁺: 675.08

### Beispiel 229

### Cyclopropansulfonsäure [6-(4-brom-benzyl)-1-(5-chlor-2-methoxy-benzolsulfonyl)-8-cyclopropyl-4,7-dioxo-octahydro-pyrazino[1,2-a]pyrimidin-3-yl]-amid

### Struktur:

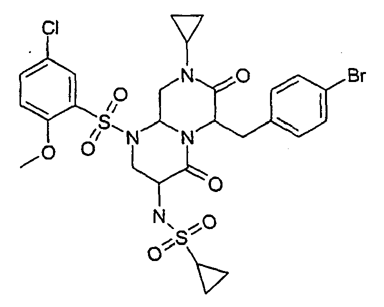

Die Synthese erfolgt analog zu Beispiel 27 ausgehend von 3-Amino-6-(4-brom-benzyl)-1-(5-chlor-2-methoxy-benzolsulfonyl)-8-cyclopropyl-hexahydro-pyrazino[1,2-a]pyrimidin-4,7-dion. Man erhält das gewünschte Produkt mit MG = 700.04 (berechnet, monoisotop); Meßwert (M+H)⁺: 701.1

### Beispiel 230

### 6-(4-Brom-benzyl)-1-(5-chlor-2-methoxy-benzolsulfonyl)-8-cyclopropyl-3-dimethylamino-hexahydro-pyrazino[1,2-a]pyrimidin-4,7-dion

### Struktur:

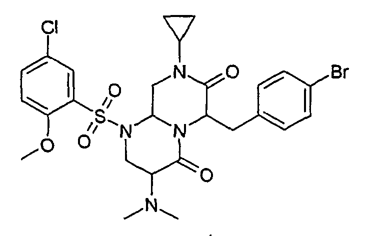

Die Synthese erfolgt analog zu Beispiel 72 ausgehend von 3-Amino-6-(4-brom-benzyl)-1-(5-chlor-2-methoxy-benzolsulfonyl)-8-cyclopropyl-hexahydro- pyrazino[1,2-a]pyrimidin-4,7-dion. Man erhält das gewünschte Produkt mit MG = 743.12 (berechnet, monoisotop); Meßwert (M+H)⁺: 744.17

### Beispiel 231

### 3-Azetidin-1-yl-6-(4-brom-benzyl)-1-(5-chlor-2-methoxy-benzolsulfonyl)-8-cyclopropyl-hexahydro-pyrazino[1,2-a]pyrimidin-4,7-dion

### Struktur:

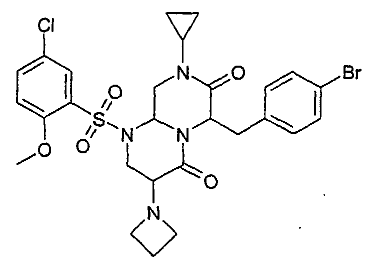

Die Synthese erfolgt analog zu Beispiel 73 ausgehend von 3-Amino-6-(4-brom-benzyl)-1-(5-chlor-2-methoxy-benzolsulfonyl)-8-cyclopropyl-hexahydro- pyrazino[1,2-a]pyrimidin-4,7-dion. Man erhält das gewünschte Produkt mit MG = 640.03 (berechnet, monoisotop); Meßwert (M+H)⁺: 641.0

### Beispiel 232

### 6-(4-Brom-benzyl)-1-(5-chlor-2-methoxy-benzolsulfonyl)-8-cyclopropyl-3-pyrrolidin-1-yl-hexahydro-pyrazino[1,2-a]pyrimidin-4,7-dion

### Struktur:

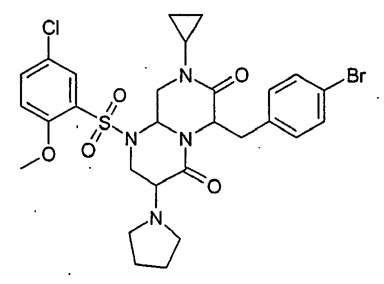

Die Synthese erfolgt analog zu Beispiel 74 ausgehend von 3-Amino-6-(4-brom-benzyl)-1-(5-chlor-2-methoxy-benzolsulfonyl)-8-cyclopropyl-hexahydro-pyrazino[1,2-a]pyrimidin-4,7-dion. Man erhält das gewünschte Produkt mit MG = 650.10 (berechnet, monoisotop); Meßwert (M+H)⁺: 651.07

### Beispiel 233

### 6-(4-Brom-benzyl)-1-(5-chlor-2-methoxy-benzolsulfonyl)-8-cyclopropyl-3-piperidin-1-yl-hexahydro-pyrazino[1,2-a]pyrimidin-4,7-dion

### Struktur:

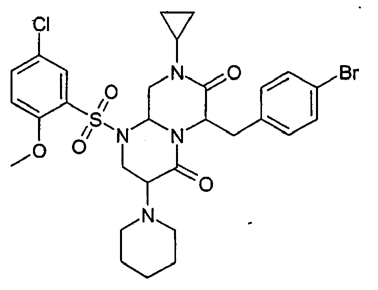

Die Synthese erfolgt analog zu Beispiel 59 ausgehend von 3-Amino-6-(4-brom-benzyl)-1-(5-chlor-2-methoxy-benzolsulfonyl)-8-cyclopropyl-hexahydro- pyrazino[1,2-a]pyrimidin-4,7-dion. Man erhält das gewünschte Produkt mit MG = 664.11 (berechnet, monoisotop); Meßwert (M+H)⁺: 665.12

### Beispiel 234

### 6-(4-Brom-benzyl)-1-(5-chlor-2-methoxy-benzolsulfonyl)-8-cyclopropyl-3-morpholin-4-yl-hexahydro-pyrazino[1,2-a]pyrimidin-4,7-dion

### Struktur:

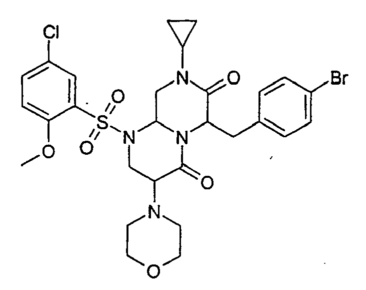

Die Synthese erfolgt analog zu Beispiel 60 ausgehend von 3-Amino-6-(4-brom-benzyl)-1-(5-chlor-2-methoxy-benzolsulfonyl)-8-cyclopropyl-hexahydro-pyrazino[1,2-a]pyrimidin-4,7-dion. Man erhält das gewünschte Produkt mit MG = 666.09 (berechnet, monoisotop); Meßwert (M+H)⁺: 667.02

### Beispiel 235

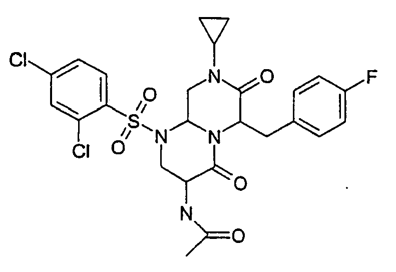

a) [6-(4-Fluor-benzyl)-8-cyclopropyl-1-(2,4-dichlor-benzolsulfonyl)-4,7-dioxo-octahydro-pyrazino [1,2-a]pyrimidin-3-yl]-carbaminsäurebenzylester
   Die Synthese erfolgt analog zu Beispiel 66a. Man erhält das gewünschte Produkt mit MG = 689.16 (berechnet, monoisotop); Meßwert (M+H)⁺: 690.10
b) 3-Amino-6-(4-fluor-benzyl)-8-cyclopropyl-1-(2,4-dichlor-benzolsulfonyl)-hexahydro-pyrazino[1,2-a]pyrimidin-4,7-dion
   Die Synthese erfolgt analog zu Beispiel 66b) ausgehend von [6-(4-Fluor-benzyl)-8-cyclopropyl-1-(2,4-dichlor-benzolsulfonyl)-4,7-dioxo-octahydro-pyrazino[1,2-a]pyrimidin-3-yl]-carbaminsäurebenzylester . Man erhält das gewünschte Produkt mit MG = 540.08 (berechnet, monoisotop); Meßwert (M+H)⁺: 541.06
c) N-[6-(4-Fluor-benzyl)-8-cyclopropyl-1-(2,4-dichlor-benzolsulfonyl)-4,7-dioxo-octahydro-pyrazino[1,2-a]pyrimidin-3-yl]-acetamid
   Die Synthese erfolgt analog zu Beispiel 21h) ausgehend von 3-Amino-6-(4-fluor-benzyl)-8-cyclopropyl-1-(2,4-dichlor-benzolsulfonyl)-hexahydro- pyrazino[1,2-a]pyrimidin-4,7-dion. Man erhält das gewünschte Produkt mit MG = 582.09 (berechnet, monoisotop); Meßwert (M+H)⁺: 583.10

### Beispiel 236

### Cyclopropancarbonsäure[8-cyclopropyl-1-(2,4-dichlor-benzolsulfonyl)-6-(4-fluor-benzyl)-4,7-dioxo-octahydro-pyrazino[1,2-a]pyrimidin-3-yl]-amid

### Struktur:

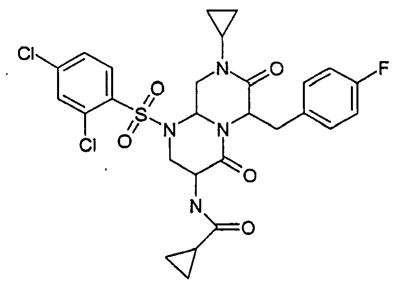

Die Synthese erfolgt analog zu Beispiel 22 ausgehend von 3-Amino-6-(4-fluor-benzyl)-8-cyclopropyl-1-(2,4-dichlor-benzolsulfonyl)-hexahydro-pyrazino[1,2-a]pyrimidin-4,7-dion. Man erhält das gewünschte Produkt mit MG = 608.11 (berechnet, monoisotop); Meßwert (M+H)⁺:609.11

### Beispiel 237

### Cyclobutancarbonsäure [8-cyclopropyl-1-(2,4-dichlor-benzolsulfonyl)-6-(4-fluor- benzyl)-4,7-dioxo-octahydro-pyrazino[1,2-a]pyrimidin-3-yl]-amid

### Struktur:

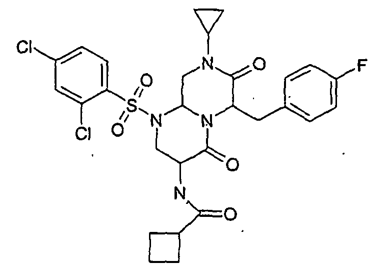

Die Synthese erfolgt analog zu Beispiel 30 ausgehend von 3-Amino-6-(4-fluor-benzyl)-8-cyclopropyl-1 -(2,4-dichlor-benzolsulfonyl)-hexahydro- pyrazino[1,2-a]pyrimidin-4,7-dion. Man erhält das gewünschte Produkt mit MG = 622.12 (berechnet, monoisotop); Meßwert (M+H)⁺: 623.13

### Beispiel 238

### Cyclopentancarbonsäure [8-cyclopropyl-1-(2,4-dichlor-benzolsulfonyl)-6-(4-fluor-benzyl)-4,7-dioxo-octahydro-pyrazino[1,2-a]pyrimidin-3-yl]-amid

### Struktur:

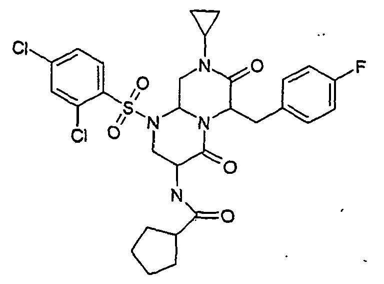

Die Synthese erfolgt analog zu Beispiel 31 ausgehend von 3-Amino-6-(4-fluor-benzyl)-8-cyclopropyl-1-(2,4-dichlor-benzolsulfonyl)-hexahydro-pyrazino[1,2-a]pyrimidin-4,7-dion. Man erhält das gewünschte Produkt mit MG = 636.14 (berechnet, monoisotop); Meßwert (M+H)⁺: 637.14

### Beispiel 239

### N-[8-Cyclopropyl-1-(2,4-dichlor-benzolsulfonyl)-6-(4-fluor-benzyl)-4,7-dioxo- octahydro-pyrazino[1,2-a]pyrimidin-3-yl]-4-dimethylamino-benzamid

### Struktur:

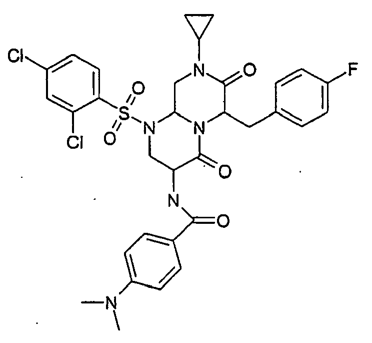

Die Synthese erfolgt analog zu Beispiel 25 ausgehend von 3-Amino-6-(4-fluor-benzyl)-8-cyclopropyl-1-(2,4-dichlor-benzolsulfonyl)-hexahydro-pyrazino[1,2-a]pyrimidin-4,7-dion. Man erhält das gewünschte Produkt mit MG = 687.15 (berechnet, monoisotop); Meßwert (M+H)⁺: 688.15

### Beispiel 240

### 1-tert-Butyl-3-[8-cyclopropyl-1-(2,4-dichlor-benzolsulfonyl)-6-(4-fluor-benzyl)-4,7- dioxo-octahydro-pyrazino[1,2-a]pyrimidin-3-yl]-harnstoff

### Struktur:

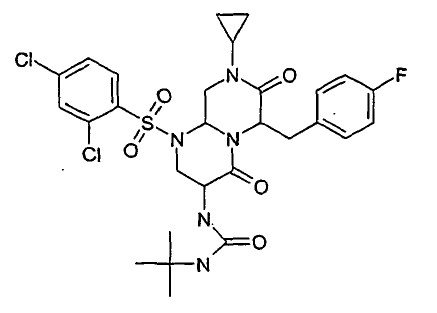

Die Synthese erfolgt analog zu Beispiel 28 ausgehend von 3-Amino-6-(4-fluor-benzyl)-8-cyclopropyl-1-(2,4-dichlor-benzolsulfonyl)-hexahydro-pyrazino[1,2-a]pyrimidin-4,7-dion. Man erhält das gewünschte Produkt mit MG = 639.15 (berechnet, monoisotop); Meßwert (M+H)⁺: 640.18

### Beispiel 241

### N-[8-Cyclopropyl-1-(2,4-dichlor-benzolsulfonyl)-6-(4-fluor-benzyl)-4,7-dioxo-octahydro-pyrazino[1,2-a]pyrimidin-3-yl]-methansulfonamid

### Struktur:

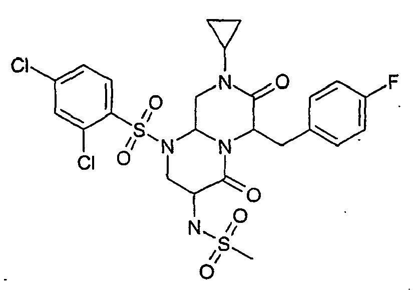

Die Synthese erfolgt analog zu Beispiel 26 ausgehend von 3-Amino-6-(4-fluor-benzyl)-8-cyclopropyl-1-(2,4-dichlor-benzolsulfonyl)-hexahydro-pyrazino[1,2-a]pyrimidin-4,7-dion. Man erhält das gewünschte Produkt mit MG = 618.06 (berechnet, monoisotop); Meßwert (M+H)⁺: 619.08

### Beispiel 242

### 8-Cylopropyl-1-(2,4-dichlor-benzolsulfonyl)-3-dimethylamino-6-(4-fluor-benzyl)-hexahydro-pyrazino[1,2-a]pyrimidin-4,7-dion

### Struktur:

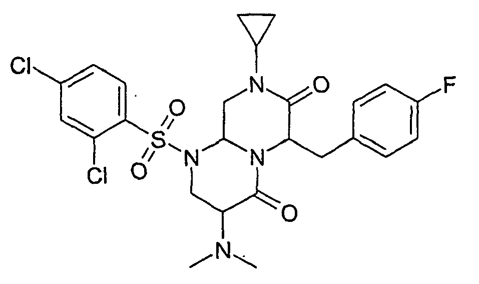

Die Synthese erfolgt analog zu Beispiel 72 ausgehend von 3-Amino-6-(4-fluor-benzyl)-8-cyclopropyl-1-(2,4-dichlor-benzolsulfonyl)-hexahydro-pyrazino[1,2-a]pyrimidin-4,7-dion. Man erhält das gewünschte Produkt mit MG = 568.11 (berechnet, monoisotop); Meßwert (M+H)⁺: 569.12

### Beispiel 243

### 8-Cyclopropyl-1-(2,4-dichlor-benzolsulfonyl)-6-(4-fluor-benzyl)-3-piperidin-1-yl-hexahydro-pyrazino[1,2-a]pyrimidin-4,7-dion

### Struktur:

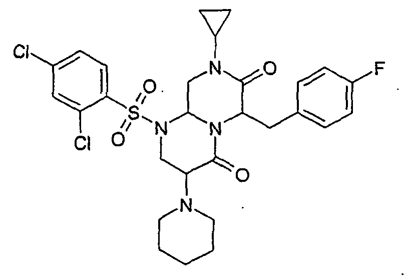

Die Synthese erfolgt analog zu Beispiel 59 ausgehend von 3-Amino-6-(4-fluor-benzyl)-8-cyclopropyl-1-(2,4-dichlor-benzolsulfonyl)-hexahydro-pyrazino[1,2-a]pyrimidin-4,7-dion. Man erhält das gewünschte Produkt mit MG = 608.14 (berechnet, monoisotop); Meßwert (M+H)⁺: 609.14

### Beispiel 244

### 8-Cyclopropyl-1-(2,4-dichlor-benzolsulfonyl)-6-(4-fluor-benzyl)-3-morpholin-4-yl- hexahydropyrazino[1,2-a]pyrimidin-4,7-dion

### Struktur:

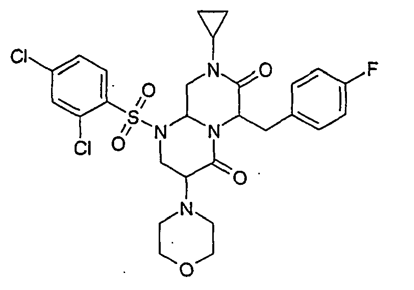

Die Synthese erfolgt analog zu Beispiel 60 ausgehend von 3-Amino-6-(4-fluor-benzyl)-8-cyclopropyl-1-(2,4-dichlor-benzolsulfonyl)-hexahydro-pyrazino[1,2-a]pyrimidin-4,7-dion. Man erhält das gewünschte Produkt mit MG = 610.12 (berechnet, monoisotop); Meßwert (M+H)⁺: 611.1

### Beispiel 245

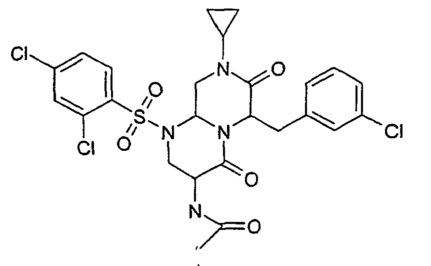

a) [6-(3-Chlor-benzyl)-8-cyclopropyl-1-(2,4-dichlor-benzolsulfonyl)-4,7-dioxo-octahydro-pyrazino[1,2-a]pyrimidin-3-yl]-carbaminsäurebenzylester
   Die Synthese erfolgt analog zu Beispiel 66a). Man erhält das gewünschte Produkt mit MG = 690.09 (berechnet, monoisotop); Meßwert (M+H)⁺: 691.0
b) 3-Amino-6-(3-chlor-benzyl)-8-cyclopropyl-1-(2,4-dichlor-benzolsulfonyl)-hexahydro-pyrazino[1,2-a]pyrimidin-4,7-dion
   Die Synthese erfolgt analog zu Beispiel 66b) ausgehend von [6-(3-Chlor-benzyl)-8-cyclopropyl-1-(2,4-dichlor-benzolsulfonyl)-4,7-dioxo-octahydro- pyrazino[1,2-a]pyrimidin-3-yl]-carbaminsäurebenzylester. Man erhält das gewünschte Produkt mit MG = 556.05 (berechnet, monoisotop); Meßwert (M+H)⁺: 557.04
c) N-[6-(3-Chlor-benzyl)-8-cyclopropyl-1-(2,4-dichlor-benzolsulfonyl)-4,7-dioxo-octahydro-pyrazino[1,2-a]pyrimidin-3-yl]-acetamid
   Die Synthese erfolgt analog zu Beispiel 21h) ausgehend von 3-Amino-6-(3-chlor-benzyl)-8-cyclopropyl-1-(2,4-dichlor-benzolsulfonyl)-hexahydro-pyrazino[1,2-a]pyrimidin-4,7-dion. Man erhält das gewünschte Produkt mit MG = 598.06 (berechnet, monoisotop); Meßwert (M+H)⁺: 599.0

### Beispiel 246

### Cyclopropancarbonsäure [6-(3-chlor-benzyl)-8-cyclopropyl-1-(2,4-dichlor-benzolsulfonyl)-4,7-dioxo-octahydro-pyrazino[1,2-a]pyrimidin-3-yl]-amid

### Struktur:

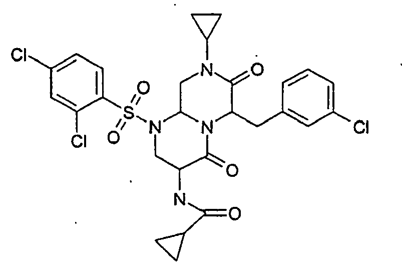

Die Synthese erfolgt analog zu Beispiel 22 ausgehend von 3-Amino-6-(3-chlor-benzyl)-8-cyclopropyl-1-(2,4-dichlor-benzolsulfonyl)-hexahydro-pyrazino[1,2-a]pyrimidin-4,7-dion. Man erhält das gewünschte Produkt mit MG = 624.08 (berechnet, monoisotop); Meßwert (M+H)⁺: 625.0

### Beispiel 247

### Cyclobutancarbonsäure[6-(3-chlor-benzyl)-8-cyclopropyl-1-(2,4-dichlor-benzolsulfonyl)-4,7-dioxo-octahydro-pyrazino[1,2-a]pyrimidin-3-yl]-amid

### Struktur:

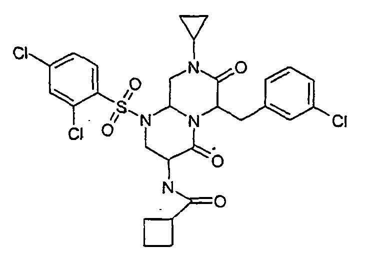

Die Synthese erfolgt analog zu Beispiel 30 ausgehend von 3-Amino-6-(3-chlor-benzyl)-8-cyclopropyl-1-(2,4-dichlor-benzolsulfonyl)-hexahydro-pyrazino[1,2-a]pyrimidin-4,7-dion. Man erhält das gewünschte Produkt mit MG = 638.09 (berechnet, monoisotop); Meßwert (M+H)⁺: 639.08

### Beispiel 248

### Cyclopentancarbonsäure [6-(3-chlor-benzyl)-8-cyclopropyl-1-(2,4-dichlor- benzolsulfonyl)-4,7-dioxo-octahydro-pyrazino[1,2-a]pyrimidin-3-yl]-amid

### Struktur:

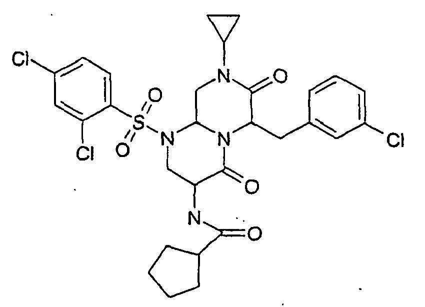

Die Synthese erfolgt analog zu Beispiel 31 ausgehend von 3-Amino-6-(3-chlor-benzyl)-8-cyclopropyl-1-(2,4-dichlor-benzolsulfonyl)-hexahydro-pyrazino[1,2-a]pyrimidin-4,7-dion. Man erhält das gewünschte Produkt mit MG = 652.11 (berechnet, monoisotop); Meßwert (M+H)⁺: 653.0

### Beispiel 249

### N-[6-(3-Chlor-benzyl)-8-cyclopropyl-1-(2,4-dichlor-benzolsulfonyl)-4,7-dioxo- octahydro-pyrazino[1,2-a]pyrimidin-3-yl]-4-dimethylamino-benzamid

### Struktur:

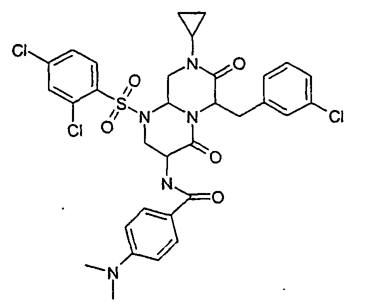

Die Synthese erfolgt analog zu Beispiel 25 ausgehend von 3-Amino-6-(3-chlor-benzyl)-8-cyclopropyl-1-(2,4-dichlor-benzolsulfonyl)-hexahydro-pyrazino[1,2-a]pyrimidin-4,7-dion. Man erhält das gewünschte Produkt mit MG = 703.12 (berechnet, monoisotop); Meßwert (M+H)⁺: 704.0

### Beispiel 250

### 1-tert-Butyl-3-[6-(3-chlor-benzyl)-8-cyclopropyl-1-(2,4-dichlor-benzolsulfonyl)-4,7-dioxo-octahydro-pyrazino[1,2-a]pyrimidin-3-yl]-harnstoff

### Struktur:

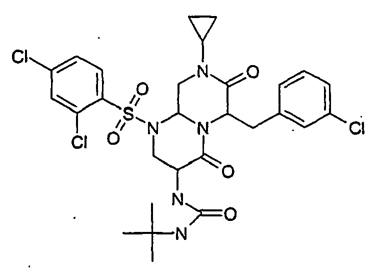

Die Synthese erfolgt analog zu Beispiel 28 ausgehend von 3-Amino-6-(3-chlor-benzyl)-8-cyclopropyl-1-(2,4-dichlor-benzolsulfonyl)-hexahydro-pyrazino[1,2-a]pyrimidin-4,7-dion. Man erhält das gewünschte Produkt mit MG = 655.12 (berechnet, monoisotop); Meßwert (M+H)⁺: 656.11

### Beispiel 251

### N-[6-(3-Chlor-benzyl)-8-cyclopropyl-1-(2,4-dichlor-benzolsulfonyl)-4,7-dioxo- octahydro-pyrazino[1,2-a]pyrimidin-3-yl]-methansulfonamid

### Struktur:

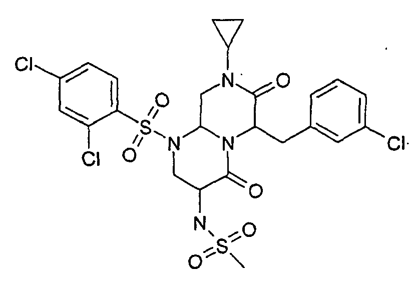

Die Synthese erfolgt analog zu Beispiel 26 ausgehend von 3-Amino-6-(3-chlor-benzyl)-8-cyclopropyl-1-(2,4-dichlor-benzolsulfonyl)-hexahydro-pyrazino[1,2-a]pyrimidin-4,7-dion. Man erhält das gewünschte Produkt mit MG = 634.03 (berechnet, monoisotop); Meßwert (M+H)⁺: 635.0

### Beispiel 252

### 6-(3-Chlor-benzyl)-8-cyclopropyl-1-(2,4-dichlor-benzolsulfonyl)-3-dimethylamino-hexahydro-pyrazino[1,2-a]pyrimidin-4,7-dion

### Struktur:

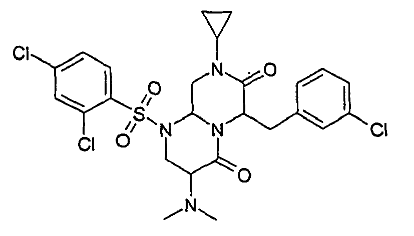

Die Synthese erfolgt analog zu Beispiel 72 ausgehend von 3-Amino-6-(3-chlor-benzyl)-8-cyclopropyl-1-(2,4-dichlor-benzolsulfonyl)-hexahydro- pyrazino[1,2-a]pyrimidin-4,7-dion. Man erhält das gewünschte Produkt mit MG = 584.08 (berechnet, monoisotop); Meßwert (M+H)⁺: 585.1

### Beispiel 253

### 3-Azetidin-1-yl-6-(3-chlor-benzyl)-8-cyclopropyl-1-(2,4-dichlor-benzolsulfonyl)- hexahydropyrazino[1,2-a]pyrimidin-4,7-dion

### Struktur:

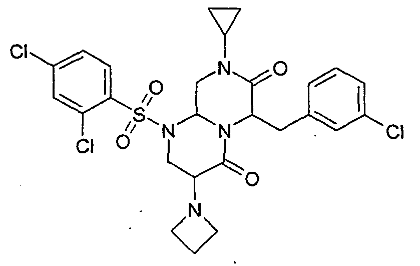

Die Synthese erfolgt analog zu Beispiel 73 ausgehend von 3-Amino-6-(3-chlor-benzyl)-8-cyclopropyl-1-(2,4-dichlor-benzolsulfonyl)-hexahydro-pyrazino[1,2-a]pyrimidin-4,7-dion. Man erhält das gewünschte Produkt mit MG = 596.08 (berechnet, monoisotop); Meßwert (M+H)⁺: 597.07

### Beispiel 254

### 6-(3-Chlor-benzyl)-8-cyclopropyl-1-(2,4-dichlor-benzolsulfonyl)-3-pyrrolidin-1-yl-hexahydro-pyrazino[1,2-a]pyrimidin-4,7-dion

### Struktur:

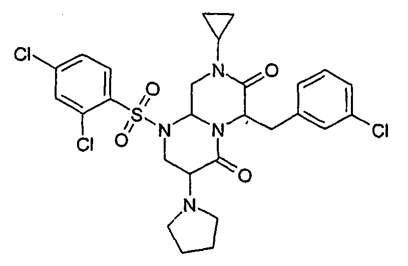

Die Synthese erfolgt analog zu Beispiel 74 ausgehend von 3-Amino-6-(3-chlor-benzyl)-8-cyclopropyl-1-(2,4-dichlor-benzolsulfonyl)-hexahydro-pyrazino[1,2-a]pyrimidin-4,7-dion. Man erhält das gewünschte Produkt mit MG =610.10 (berechnet, monoisotop); Meßwert (M+H)⁺: 611.09

### Beispiel 255

### 6-(3-Chlor-benzyl)-8-cyclopropyl-1-(2,4-dichlor-benzolsulfonyl)-3-piperidin-1-yl- hexahydropyrazino[1,2-a]pyrimidin-4,7-dion

### Struktur:

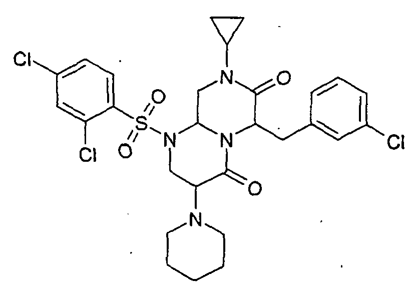

Die Synthese erfolgt analog zu Beispiel 59 ausgehend von 3-Amino-6-(3-chlor-benzyl)-8-cyclopropyl-1-(2,4-dichlor-benzolsulfonyl)-hexahydro-pyrazino[1,2-a]pyrimidin-4,7-dion. Man erhält das gewünschte Produkt mit MG = 624.11 (berechnet, monoisotop); Meßwert (M+H)⁺: 625.11

### Beispiel 256

### 6-(3-Chlor-benzyl)-8-cyclopropyl-1-(2,4-dichlor-benzolsulfonyl)-3-morpholin-4-yl- hexahydropyrazino[1,2-a]pyrimidin-4,7-dion

### Struktur:

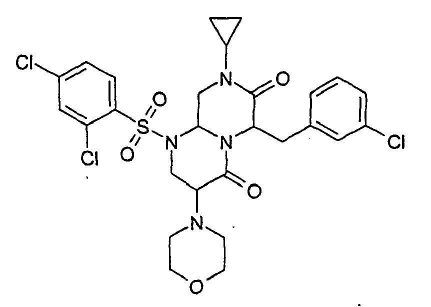

Die Synthese erfolgt analog zu Beispiel 60 ausgehend von 3-Amino-6-(3-chlor-benzyl)-8-cyclopropyl-1-(2,4-dichlor-benzolsulfonyl)-hexahydro- pyrazino[1,2-a]pyrimidin-4,7-dion. Man erhält das gewünschte Produkt mit MG = 626.09 (berechnet, monoisotop); Meßwert (M+H)⁺: 627.09

### Beispiel 257

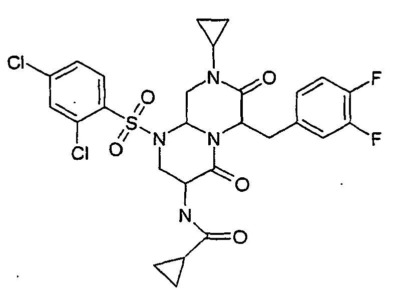

a) [1-[Cyclopropyl- (2,2-diethoxy-ethyl)- carbamoyl]-2-(3,4-difluor-phenyl)-ethyl]-carbaminsäure 9H-fluoren-9-ylmethylester
   Die Synthese erfolgt analog zu Beispiel 29a) (Methode B) ausgehend von N-Fmoc-3,4-F₂-Phe-OH und Cyclopropyl-(2,2-diethoxy-ethyl)-amin. Man erhält das gewünschte Produkt mit MG = 578.26 (berechnet, monoisotop); Meßwert (M+H)⁺: 579.3
b) 2-Amino-3-(3,4-difluor-phenyl)-N-cyclopropyl-N-(2,2-diethoxy-ethyl)-propionamid
   Die Synthese erfolgt analog zu Beispiel 29b) (Methode B) ausgehend von [1-[Cyclopropyl-(2,2-diethoxy-ethyl)-carbamoyl]-2-(3,4-difluor-phenyl)-ethyl]- carbaminsäure 9H-fluoren-9-ylmethylester. Man erhält das gewünschte Produkt mit MG = 356.19 (berechnet, monoisotop); Meßwert (M+H)⁺: 357.23
c) [1-{2-(3,4-Difluor-phenyl)-1-[cyclopropyl-(2,2-diethoxy-ethyl)-carbamoyl]-ethylcarbamoyl}-2- (9H-fluoren-9-ylmethoxycarbonylamino)-ethyl]-carbaminsäure-benzylester
   Die Synthese erfolgte analog zu Beispiel 29c) (Methode B) ausgehend von 2-Amino-3-(3,4-difluor-phenyl)-N-cyclopropyl-N-(2,2-diethoxy-ethyl)-propionamid. Man erhält das gewünschte Produkt mit MG = 798.34 (berechnet, monoisotop); Meßwert (M+Na)⁺: 821.35
d) (2-Amino-1-{2-(3,4-difluor-phenyl)-1-[cyclopropyl-(2,2-diethoxy-ethyl)-carbamoyl]-ethylcarbamoyl}-ethyl)-carbaminsäurebenzylester Die Synthese erfolgte analog zu Beispiel 29d) (Methode B) ausgehend von [1-{2-(3,4-Difluor-phenyl)-1-[cyclopropyl-(2,2-diethoxyethyl)-carbamoyl]-ethylcarbamoyl}-2- (9H-fluoren-9-ylmethoxycarbonylamino)-ethyl]-carbaminsäure-benzylester. Man erhält das gewünschte Produkt mit MG = 576.28 (berechnet, monoisotop); Meßwert (M+Na)⁺: 599.27
f) [1-{2-(3,4-Difluor-phenyl)-1-[cyclopropyl-(2,2-diethoxy-ethyl)-carbamoyl]-ethylcarbamoyl}-2- (2,4-dichlor-benzolsulfonylamino)-ethyl]-carbaminsäurebenzylester
   Die Synthese erfolgte analog zu Beispiel 29e) (Methode B) ausgehend von (2-Amino-1-{2-(3,4-difluor-phenyl)-1-[cyclopropyl-(2,2-diethoxy-ethyl)-carbamoyl]- ethylcarbamoyl}-ethyl)-carbaminsäurebenzylester. Man erhält das gewünschte Produkt mit MG = 784.19 (berechnet, monoisotop); Meßwert (M+Na)⁺: 807.18.
e) [6-(3,4-Difluor-benzyl)-8-cyclopropyl-1-(2,4-dichlor-benzolsulfonyl)-4,7-dioxo-octahydro-pyrazino[1,2-a]pyrimidin-3-yl]-carbaminsäurebenzylester
   Die Synthese erfolgt analog zu Beispiel 29f) (Methode B) ausgehend von [1-{2-(3,4-Difluorphenyl)-1-[cyclopropyl-(2,2-diethoxy-ethyl)-carbamoyl]-ethylcarbamoyl}-2-(2,4-dichlor-benzolsulfonylamino)-ethyl]-carbaminsäurebenzylester. Man erhält das gewünschte Produkt mit MG = 692.11 (berechnet, monoisotop); Meßwert (M+H)⁺: 693.0
f) 3-Amino-6-(3,4-difluor-benzyl)-8-cyclopropyl-1-(2,4-dichlor-benzolsulfonyl)-hexahydro-pyrazino[1,2-a]pyrimidin-4,7-dion
   Die Synthese erfolgt analog zu Beispiel 29g) (Methode B) ausgehend von [6-(3,4-Difluor-benzyl)-8-cyclopropyl-1-(2,4-dichlor-benzolsulfonyl)-4,7-dioxo-octahydro-pyrazino[1,2-a]pyrimidin-3-yl]-carbaminsäurebenzylester. Man erhält das gewünschte Produkt mit MG = 558.07 (berechnet, monoisotop); Meßwert (M+H)⁺: 559.05
g) Cyclopropancarbonsäure [8-cyclopropyl-1-(2,4-dichlor-benzolsulfonyl)-6-(3,4-difluor-benzyl)-4,7-dioxo-octahydro-pyrazino[1,2-a]pyrimidin-3-yl]-amid
   Die Synthese erfolgt analog zu Beispiel 22 ausgehend von 3-Amino-6-(3,4-difluor-benzyl)-8-cyclopropyl-1-(2,4-dichlor-benzolsulfonyl)-hexahydro-pyrazino[1,2-a]pyrimidin-4,7-dion. Man erhält das gewünschte Produkt mit MG = 626.10 (berechnet, monoisotop); Meßwert (M+H)⁺: 627.18

### Beispiel 258

### Cyclobutancarbonsäure [8-cyclopropyl-1-(2,4-dichlor-benzolsulfonyl)-6-(3,4- difluor-benzyl)-4,7-dioxo-octahydro-pyrazino[1,2-a]pyrimidin-3-yl]-amid

### Struktur:

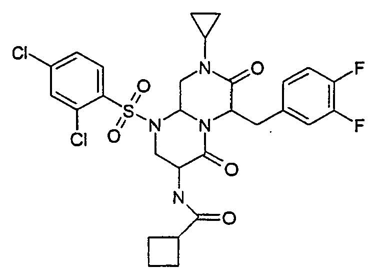

Die Synthese erfolgt analog zu Beispiel 30 ausgehend von 3-Amino-6-(3,4-difluor-benzyl)-8-cyclopropyl-1-(2,4-dichlor-benzolsulfonyl)-hexahydro-pyrazino[1,2-a]pyrimidin-4,7-dion. Man erhält das gewünschte Produkt mit MG = 640.11 (berechnet, monoisotop); Meßwert (M+H)⁺: 641.1

### Beispiel 259

### Cyclopentancarbonsäure [8-cyclopropyl-1-(2,4-dichlor-benzolsulfonyl)-6-(3,4- difluor-benzyl)-4,7-dioxo-octahydro-pyrazino[1,2-a]pyrimidin-3-yl]-amid

### Struktur:

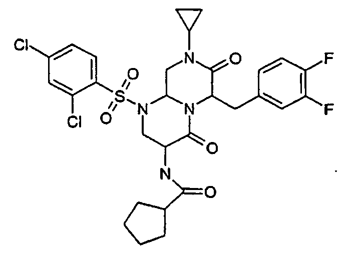

Die Synthese erfolgt analog zu Beispiel 31 ausgehend von 3-Amino-6-(3,4-difluor-benzyl)-8-cyclopropyl-1-(2,4-dichlor-benzolsulfonyl)-hexahydro-pyrazino[1,2-a]pyrimidin-4,7-dion. Man erhält das gewünschte Produkt mit MG = 654.13 (berechnet, monoisotop); Meßwert (M+H)⁺: 655.21

### Beispiel 260

### Cyclohexancarbonsäure [8-cyclopropyl-1-(2,4-dichlor-benzolsulfonyl)-6-(3,4- difluor-benzyl)-4,7-dioxo-octahydro-pyrazino[1,2-a]pyrimidin-3-yl]-amid

### Struktur:

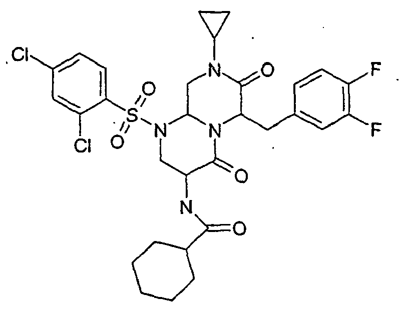

Die Synthese erfolgt analog zu Beispiel 32 ausgehend von 3-Amino-6-(3,4-difluor-benzyl)-8-cyclopropyl-1-(2,4-dichlor-benzolsulfonyl)-hexahydro- pyrazino[1,2-a]pyrimidin-4,7-dion. Man erhält das gewünschte Produkt mit MG = 668.14 (berechnet, monoisotop); Meßwert (M+H)⁺: 669.22

### Beispiel 261

### N-[8-Cyclopropyl-1-(2,4-dichlor-benzolsulfonyl)-6-(3,4-difluor-benzyl)-4,7-dioxo-octahydro-pyrazino[1,2-a]pyrimidin-3-yl]-methansulfonamid

### Struktur:

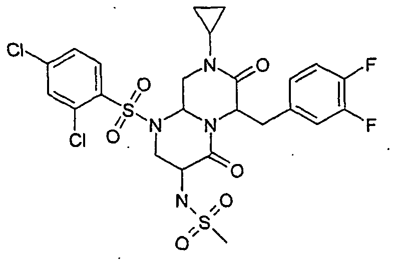

Die Synthese erfolgt analog zu Beispiel 26 ausgehend von 3-Amino-6-(3,4-difluor-benzyl)-8-cyclopropyl-1-(2,4-dichlor-benzolsulfonyl)-hexahydro- pyrazino[1,2-a]pyrimidin-4,7-dion. Man erhält das gewünschte Produkt mit MG = 636.05 (berechnet, monoisotop); Meßwert (M+H)⁺: 637.07

### Beispiel 262

### 8-Cyclopropyl-1-(2,4-dichlor-benzolsulfonyl)-6-(3,4-difluor-benzyl)-3-dimethylamino-hexahydro-pyrazino[1,2-a]pyrimidin-4,7-dion

### Struktur:

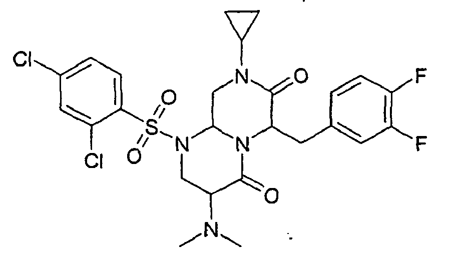

Die Synthese erfolgt analog zu Beispiel 72 ausgehend von 3-Amino-6-(3,4-difluor-benzyl)-8-cyclopropyl-1-(2,4-dichlor-benzolsulfonyl)-hexahydro- pyrazino[1,2-a]pyrimidin-4,7-dion. Man erhält das gewünschte Produkt mit MG=586.10 (berechnet, monoisotop); Meßwert (M+H)⁺: 587.10

### Beispiel 263

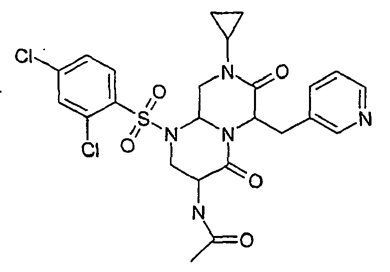

a) {1-[Cyclopropyl-(2,2-diethoxy-ethyl)-carbamoyl]-2-pyridin-3-yl-ethyl}-carbaminsäure 9H-fluoren-9-ylmethylester
   Die Synthese erfolgt analog zu Beispiel 29a) (Methode B) ausgehend von Fmoc-PAL-OH und Cyclopropyl-(2,2-diethoxy-ethyl)-amin. Man erhält das gewünschte Produkt mit MG = 543.27 (berechnet, monoisotop); Meßwert (M+H)⁺: 544.21
b) 2-Amino-N-cyclopropyl-N-(2,2-diethoxy-ethyl)-3-pyridin-3-yl-propionamid
   Die Synthese erfolgt analog zu Beispiel 29b) (Methode B) ausgehend von {1-[Cyclopropyl-(2,2-diethoxy-ethyl)-carbamoyl]-2-pyridin-3-yl-ethyl}-carbaminsäure 9H- fluoren-9-ylmethylester. Man erhält das gewünschte Produkt mit MG = 321.21 (berechnet, monoisotop); Meßwert (M+H)⁺: 322.20
c) [1-{1-[Cyclopropyl-(2,2-diethoxy-ethyl)-carbamoyl]-2-pyridin-3-yl-ethylcarbamoyl}-2-(9H-fluoren-9-ylmethoxycarbonylamino)-ethyl]-carbaminsäurebenzylester
   Die Synthese erfolgte analog zu Beispiel 29c) (Methode B) ausgehend von 2-Amino-N-cyclopropyl-N-(2,2-diethoxy-ethyl)-3-pyridin-3-yl-propionamid. Man erhält das gewünschte Produkt mit MG = 798.34 (berechnet, monoisotop); Meßwert (M+Na)⁺: 821.35
d) (2-Amino-1-{1-[cyclopropyl-(2,2-diethoxy-ethyl)-carbamoyl]-2-pyridin-3-yl-ethylcarbamoyl}- ethyl)-carbaminsäurebenzylester. Die Synthese erfolgte analog zu Beispiel 29d) (Methode B) ausgehend von [1-{1-[Cyclopropyl-(2,2-diethoxy-ethyl)-carbamoyl]-2-pyridin-3-yl-ethylcarbamoyl}-2-(9H-fluoren-9-ylmethoxycarbonylamino)-ethyl]-carbaminsäurebenzylester. Man erhält das gewünschte Produkt mit MG = 541.29 (berechnet, monoisotop); Meßwert (M+H)⁺: 542.30
e) [1-{1-[Cyclopropyl-(2,2-diethoxy-ethyl)-carbamoyl]-2-pyridin-3-yl-ethylcarbamoyl}-2-(2,4-dichlor-benzolsulfonylamino)-ethyl]-carbaminsäurebenzylester
   Die Synthese erfolgte analog zu Beispiel 29e) (Methode B) ausgehend von (2-Amino-1-{1-[cyclopropyl-(2,2-diethoxy-ethyl)-carbamoyl]-2-pyridin-3-yl-ethylcarbamoyl}- ethyl)-carbaminsäurebenzylester. Man erhält das gewünschte Produkt mit MG = 826.12 (berechnet, monoisotop); Meßwert (M+Na)⁺: 849.14.
f) [8-Cyclopropyl-1-(2,4-dichlor-benzolsulfonyl)-4,7-dioxo-6-pyridin-3-ylmethyl-octahydro-pyrazino[1,2-a]pyrimidin-3-yl]-carbaminsäurebenzylester
   Die Synthese erfolgt analog zu Beispiel 29f) (Methode B) ausgehend von [1-{1-[Cyclopropyl-(2,2-diethoxy-ethyl)-carbamoyl]-2-pyridin-3-yl-ethylcarbamoyl}-2-(2,4- dichlor-benzolsulfonylamino)-ethyl]-carbaminsäurebenzylester. Man erhält das gewünschte Produkt mit MG = 657.12 (berechnet, monoisotop); Meßwert (M+H)⁺: 658.11
g) 3-Amino-8-cyclopropyl-1-(2,4-dichlor-benzolsulfonyl)-6-pyridin-3-ylmethyl-hexahydro-pyrazino[1,2-a]pyrimidin-4,7-dion
   Die Synthese erfolgt analog zu Beispiel 29g) (Methode B) ausgehend von [8-Cyclopropyl-1-(2,4-dichlor-benzolsulfonyl)-4,7-dioxo-6-pyridin-3-ylmethyl-octahydro- pyrazino[1,2-a]pyrimidin-3-yl]-carbaminsäurebenzylester. Man erhält das gewünschte Produkt mit MG = 523.08 (berechnet, monoisotop); Meßwert (M+H)⁺: 524.09
h) N-[8-Cyclopropyl-1-(2,4-dichlor-benzolsulfonyl)-4,7-dioxo-6-pyridin-3-ylmethyl-octahydro-pyrazino[1,2-a]pyrimidin-3-yl]-acetamid
   Die Synthese erfolgt analog zu Beispiel 21h) ausgehend von 3-Amino-8-cyclopropyl-1-(2,4-dichlor-benzolsulfonyl)-6-pyridin-3-ylmethyl-hexahydro- pyrazino[1,2-a]pyrimidin-4,7-dion. Man erhält das gewünschte Produkt mit MG = 565.10 (berechnet, monoisotop); Meßwert (M+H)⁺: 566.10

### Beispiel 264

### Cyclopropancarbonsäure [8-cyclopropyl-1-(2,4-dichlor-benzolsulfonyl)-4,7-dioxo-6- pyridin-3-ylmethyl-octahydro-pyrazino[1,2-a]pyrimidin-3-yl]-amid

### Struktur:

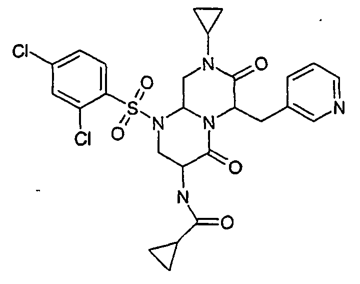

Die Synthese erfolgt analog zu Beispiel 22 ausgehend von 3-Amino-8-cyclopropyl-1-(2,4-dichlor-benzolsulfonyl)-6-pyridin-3-ylmethyl-hexahydro- pyrazino[1,2-a]pyrimidin-4,7-dion. Man erhält das gewünschte Produkt mit MG = 591.11 (berechnet, monoisotop); Meßwert (M+H)⁺: 592.09

### Beispiel 265

### Cyclobutancarbonsäure [8-cyclopropyl-1-(2,4-dichlor-benzolsulfonyl)-4,7-dioxo-6- pyridin-3-ylmethyl-octahydro-pyrazino[1,2-a]pyrimidin-3-yl]-amid

### Struktur:

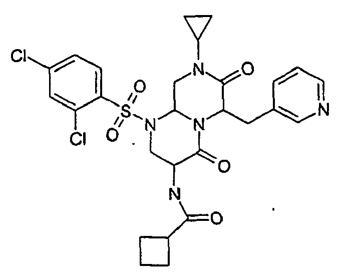

Die Synthese erfolgt analog zu Beispiel 30 ausgehend von 3-Amino-8-cyclopropyl-1-(2,4-dichlor-benzolsulfonyl)-6-pyridin-3-ylmethyl-hexahydro- pyrazino[1,2-a]pyrimidin-4,7-dion. Man erhält das gewünschte Produkt mit MG = 605.13 (berechnet, monoisotop); Meßwert (M+H)⁺: 606.12

### Beispiel 266

### Cyclopentancarbonsäure [8-cyclopropyl-1-(2,4-dichlor-benzolsulfonyl)-4,7-dioxo-6-pyridin-3-ylmethyl-octahydro-pyrazino[1,2-a]pyrimidin-3-yl]-amid

### Struktur:

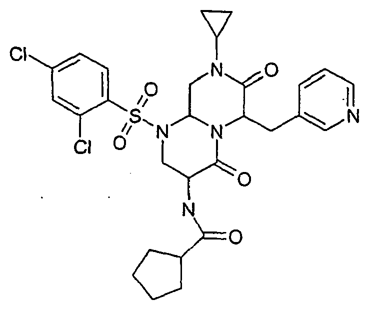

Die Synthese erfolgt analog zu Beispiel 31 ausgehend von 3-Amino-8-cyclopropyl-1-(2,4-dichlor-benzolsulfonyl)-6-pyridin-3-ylmethyl-hexahydro- pyrazino[1,2-a]pyrimidin-4,7-dion. Man erhält das gewünschte Produkt mit MG = 619.14 (berechnet, monoisotop); Meßwert (M+H)⁺: 620.14

### Beispiel 267

### Pyrrolidin-2-carbonsäure[8-cyclopropyl-1-(2,4-dichlor-benzolsulfonyl)-4,7-dioxo-6- pyridin-3-ylmethyl-octahydro-pyrazino[1,2-a]pyrimidin-3-yl]-amid

### Struktur:

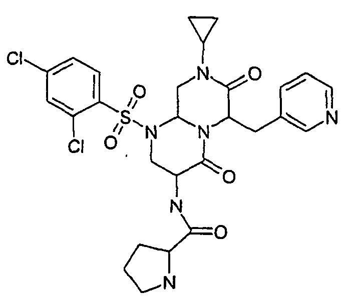

Die Synthese erfolgt analog zu Beispiel 40 ausgehend von 3-Amino-8-cyclopropyl-1-(2,4-dichlor-benzolsulfonyl)-6-pyridin-3-ylmethyl-hexahydro-pyrazino[1,2-a]pyrimidin-4,7-dion. Man erhält das gewünschte Produkt mit MG = 620.14 (berechnet, monoisotop); Meßwert (M+H)⁺: 621.12

### Beispiel 268

### N-[8-Cyclopropyl-1-(2,4-dichlor-benzolsulfonyl)-4,7-dioxo-6-pyridin-3-ylmethyl- octahydro-pyrazino[1,2-a]pyrimidin-3-yl]-4-dimethylamino-benzamid

### Struktur:

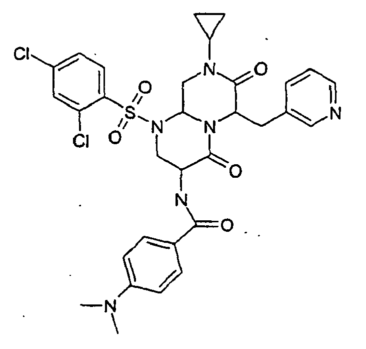

Die Synthese erfolgt analog zu Beispiel 25 ausgehend von 3-Amino-8-cyclopropyl-1-(2,4-dichlor-benzolsulfonyl)-6-pyridin-3-ylmethyl-hexahydro- pyrazino[1,2-a]pyrimidin-4,7-dion. Man erhält das gewünschte Produkt mit MG = 670.15 (berechnet, monoisotop); Meßwert (M+H)⁺: 671.16

### Beispiel 269

### Pyridine-2-carbonsäure [8-cylopropyl-1-(2,4-dichlor-benzolsulfonyl)-4,7-dioxo-6-pyridin-3-ylmethyl-octahydro-pyrazino[1,2-a]pyrimidin-3-yl]-amid

### Struktur:

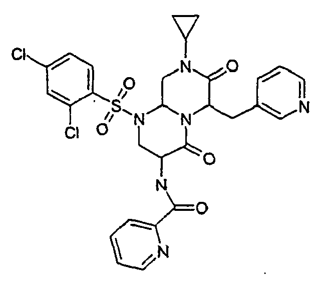

Die Synthese erfolgt analog zu Beispiel 49 ausgehend von 3-Amino-8-cyclopropyl-1-(2,4-dichlor-benzolsulfonyl)-6-pyridin-3-ylmethyl-hexahydro- pyrazino[1,2-a]pyrimidin-4,7-dion. Man erhält das gewünschte Produkt mit MG = 628.11 (berechnet, monoisotop); Meßwert (M+H)⁺: 629.14

### Beispiel 270

### 1-tert-Butyl-3-[8-cyclopropyl-1-(2,4-dichlor-benzolsulfonyl)-4,7-dioxo-6-pyridin-3-ylmethyl-octahydro-pyrazino[1,2-a]pyrimidin-3-yl]-harnstoff

### Struktur:

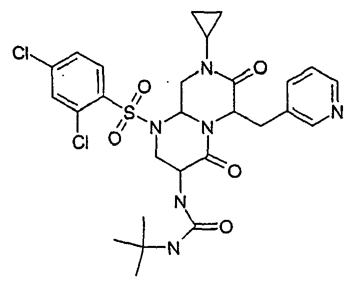

Die Synthese erfolgt analog zu Beispiel 28 ausgehend von 3-Amino-8-cyclopropyl-1-(2,4-dichlor-benzolsulfonyl)-6-pyridin-3-ylmethyl-hexahydro-pyrazino[1,2-a]pyrimidin-4,7-dion. Man erhält das gewünschte Produkt mit MG = 622.15 (berechnet, monoisotop); Meßwert (M+H)⁺: 623.16

### Beispiel 271

### N-[8-Cyclopropyl-1-(2,4-dichlor-benzolsulfonyl)-4,7-dioxo-6-pyridin-3-ylmethyl-octahydro-pyrazino[1,2-a]pyrimidin-3-yl]-methansulfonamid

### Struktur:

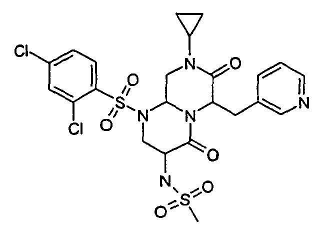

Die Synthese erfolgt analog zu Beispiel 26 ausgehend von 3-Amino-8-cyclopropyl-1-(2,4-dichlor-benzolsulfonyl)-6-pyridin-3-ylmethyl-hexahydro- pyrazino[1,2-a]pyrimidin-4,7-dion. Man erhält das gewünschte Produkt mit MG = 601.06 (berechnet, monoisotop); Meßwert (M+H)⁺: 602.06

### Beispiel 272

### Cyclopropansulfonsäure [8-cyclopropyl-1-(2,4-dichlor-benzolsulfonyl)-4,7-dioxo-6- pyridin-3-ylmethyl-octahydro-pyrazino[1,2-a]pyrimidin-3-yl]-amid

### Struktur:

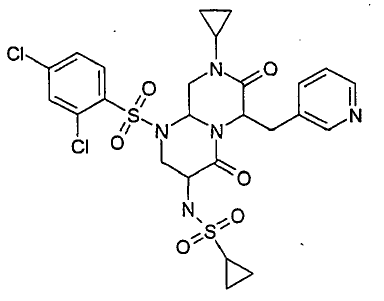

Die Synthese erfolgt analog zu Beispiel 27 ausgehend von 3-Amino-8-cyclopropyl-1-(2,4-dichlor-benzolsulfonyl)-6-pyridin-3-ylmethyl-hexahydro- pyrazino[1,2-a]pyrimidin-4,7-dion. Man erhält das gewünschte Produkt mit MG = 627.08 (berechnet, monoisotop); Meßwert (M+H)⁺: 628.09

### Beispiel 273

### 8-Cyclopropyl-1-(2,4-dichlor-benzolsulfonyl)-3-dimethylamino-6-pyridin-3-ylmethyl-hexahydro-pyrazino[1,2-a]pyrimidin-4,7-dion

### Struktur:

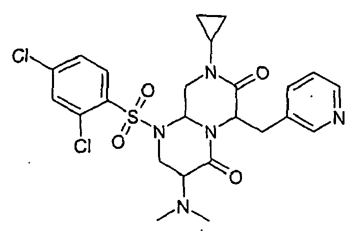

Die Synthese erfolgt analog zu Beispiel 72 ausgehend von 3-Amino-8-cyclopropyl-1-(2,4-dichlor-benzolsulfonyl)-6-pyridin-3-ylmethyl-hexahydro-pyrazino[1,2-a]pyrimidin-4,7-dion. Man erhält das gewünschte Produkt mit MG = 551.12 (berechnet, monoisotop); Meßwert (M+H)⁺: 552.14

### Beispiel 274

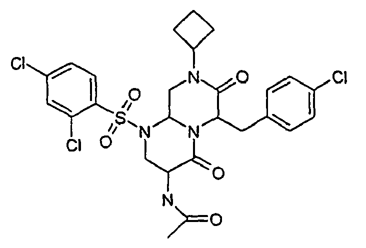

a) Cyclobutyl-(2,2-diethoxyethyl)-amin
   Eine Lösung von 1,75 mL (11,65 mmol) 1-Brom-2,2-diethoxyethan und 2 mL (23,3 mmol) Cyclobutylamin wurde in einem verschlossenen Gefäß über Nacht auf 80°C erwärmt. Das Reaktionsgemisch wurde mit 40 % NaOH (in H₂O) versetzt und mit Ether extrahiert. Die organische Phase wurde isoliert, getrocknet (MgSO₄) und im Vakuum eingeengt. Man erhielt 2,39 g Substanz als orange Flüssigkeit.
b) 2-Amino-3-(4-chlorphenyl)-N-cyclobutyl-N-(2,2-diethoxyethyl)-propionamid
   Eine Lösung von 1,0 g (2,37 mmol) Fmoc-Phe(4-Cl)-OH und 489 mg (2,61 mmol) des Amins Cyclobutyl-(2,2-diethoxyethyl)-amin wurde in 9,5 mL Dimethylformamid gelöst. Zu dieser Lösung wurden 722 mg (2,61 mmol) DMTMM zugegeben. Das Gemisch wurde über Nacht bei Raumtemperatur gerührt, mit Essigsäureethylester verdünnt und mit Wasser und Kochsalzlösung gewaschen. Die organische Phase wurde isoliert, getrocknet (MgSO₄) und im Vakuum eingeengt. Der Rückstand wurde an 40 g SiO₂ chromatographiert (Elution mit EtOAc/Heptan, Gradient. 10 - 70 %). Man erhielt 890 mg der Substanz als weißen Schaum. LC/MS M⁺ = 591, Meßwert (M⁺Na) = 613 und M-OEt = 545 stimmten mit der gewünschten Substanz überein. Eine Lösung von 890 mg (1,5 mmol) der genannten Substanz wurde in 7,5 mL Dimethylformamid gelöst und mit 0,8 mL Diethylamin versetzt. Das Reaktionsgemisch wurde 10 Minuten lang bei Raumtemperatur gerührt und im Vakuum eingeengt. Der Rückstand wurde einer Flash-Chromatographie an 12 g SiO₂ unterzogen (Elution mit MeOH in DCM, Gradient 1 - 10 %). Man erhielt 470 mg des gewünschten Amins 2-Amino-3-(4-chlorphenyl)-N-cyclobutyl-N-(2,2-diethoxyethyl)-propionamid als farbloses Öl. LC/MS M⁺ = 368, Meßwert (M⁺Na) = 391 und M-OEt = 323 stimmen mit der Struktur überein.
c) [1-{2-(4-Chlorphenyl)-1-[cyclobutyl-(2,2-diethoxyethyl)-carbamoyl]-ethylcarbamoyl}-2-(2,4-dichlorbenzolsulfonylamino)-ethyl]-carbaminsäurebenzylester
   Eine Lösung von 2,5 g (10,5 mmol) Z-Dap-OH in 21 mL 1N-NaOH wurde bis zur Homogenität gerührt. Der Z-Dap-OH-Lösung wurde eine Lösung von 2,83 g (11,5 mmol) 2,4-Dichlorphenylsulfonylchlorid in 29 mL Dioxan langsam zugesetzt. Dann wurde 2 Stunden lang gerührt. Das Reaktionsgemisch wurde mit Zitronensäure angesäuert und mit DCM extrahiert. Die organische Phase wurde getrocknet (MgSO₄) und im Vakuum eingeengt. Man erhielt 4,08 g des gewünschten Sulfonamids, das bei dem nachstehend beschriebenen Verfahren ohne weitere Reinigung eingesetzt wurde. Eine Lösung von 840 mg (2,28 mmol) 2-Amino-3-(4-chlorphenyl)-N-cyclobutyl-N-(2,2-diethoxyethyl)-propionamid und 1,22 g (2,73 mmol) des genannten Sulfonamid in 9 mL DMF wurden mit 755 mg (2,73 mmol) DMTMM versetzt. Das Gemisch wurde 2 Tage lang bei Raumtemperatur gerührt, mit Essigsäureethylester verdünnt und mit Wasser und Kochsalzlösung gewaschen. Die organische Phase wurde isoliert, getrocknet (MgSO₄) und im Vakuum eingeengt. Man erhielt 1,9 g Rohsubstanz als weißen Schaum. Der Rückstand wurde einer Flash-Chromatographie an 40 g SiO₂ unterzogen (Elution mit EtOAc/Heptan, Gradient 10 - 80 %). Man erhielt 1,09 g der gewünschten Substanz [1-{2-(4-Chlorphenyl)-1-[cyclobutyl-(2,2-diethoxyethyl)-carbamoyl]-ethylcarbamoyl}-1-(2,4-dichlorbenzolsulfonylamino)-ethyl]-carbaminsäurebenzylester als weißen Feststoff. LC/MS M⁺ = 798, Meßwert (M⁺Na) = 819 und M-OEt = 753 stimmen mit der Struktur überein.
d) [6-(4-Chlor-benzyl)-8-cyclobutyl-1-(2,4-dichlor-benzolsulfonyl)-4,7-dioxo-octahydro-pyrazino[1,2-a]pyrimidin-3-yl]-carbaminsäurebenzylester
   Die Synthese erfolgt analog zu Beispiel 29f) (Methode B) ausgehend von [1-{2-(4-Chlorphenyl)-1-[cyclobutyl-(2,2-diethoxy-ethyl)-carbamoyl]-ethylcarbamoyl}-2-(2,4-dichlor-benzolsulfonylamino)-ethyl]-carbaminsäurebenzylester. Man erhält das gewünschte Produkt mit MG = 704.10 (berechnet, monoisotop); Meßwert (M+H)⁺: 705.1
e) 3-Amino-6-(4-chlor-benzyl)-8-cyclobutyl-1-(2,4-dichlor-benzolsulfonyl)-hexahydro-pyrazino[1,2-a]pyrimidin-4,7-dion
   Die Synthese erfolgt analog zu Beispiel 29g) (Methode B) ausgehend von [6-(4-Chlor-benzyl)-8-cyclobutyl-1-(2,4-dichlor-benzolsulfonyl)-4,7-dioxo-octahydro-pyrazino[1,2-a]pyrimidin-3-yl]-carbaminsäurebenzylester Man erhält das gewünschte Produkt mit MG = 570.07 (berechnet, monoisotop); Meßwert (M+H)⁺: 570.99
f) N-[6-(4-Chlor-benzyl)-8-cyclobutyl-1-(2,4-dichlor-benzolsulfonyl)-4,7-dioxo-octahydro-pyrazino[1,2-a]pyrimidin-3-yl]-acetamid
   Die Synthese erfolgt analog zu Beispiel 21h) ausgehend von 3-Amino-6-(4-chlor-benzyl)-8-cyclobutyl-1-(2,4-dichlor-benzolsulfonyl)-hexahydro- pyrazino[1,2-a]pyrimidin-4,7-dion. Man erhält das gewünschte Produkt mit MG = 612.08 (berechnet, monoisotop); Meßwert (M+H)⁺: 613.07

### Beispiel 275

### Cyclopropancarbonsäure [6-(4-chlor-benzyl)-8-cyclobutyl-1-(2,4-dichlor- benzolsulfonyl)-4,7-dioxo-octahydro-pyrazino[1,2-a]pyrimidin-3-yl]-amid

### Struktur:

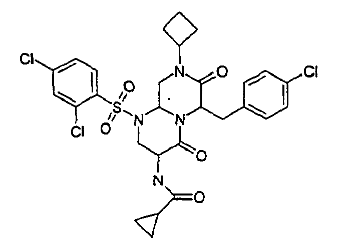

Die Synthese erfolgt analog zu Beispiel 22 ausgehend von 3-Amino-6-(4-chlor-benzyl)-8-cyclobutyl-1-(2,4-dichlor-benzolsulfonyl)-hexahydro- pyrazino[1,2-a]pyrimidin-4,7-dion. Man erhält das gewünschte Produkt mit MG = 638.09 (berechnet, monoisotop); Meßwert (M+H)⁺: 639.09

### Beispiel 276

### 1-[6-(4-Chlor-benzyl)-8-cyclobutyl-1-(2,4-dichlor-benzolsulfonyl)-4,7-dioxo-octahydro-pyrazino[1,2-a]pyrimidin-3-yl]-3-ethyl-harnstoff

### Struktur:

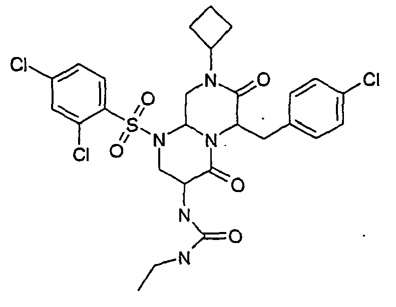

Die Synthese erfolgt analog zu Beispiel 57 ausgehend von 3-Amino-6-(4-chlor-benzyl)-8-cyclobutyl-1-(2,4-dichlor-benzolsulfonyl)-hexahydro- pyrazino[1,2-a]pyrimidin-4,7-dion. Man erhält das gewünschte Produkts mit MG = 641.10 (berechnet, monoisotop); Meßwert (M+H)⁺: 642.09

### Beispiel 277

### N-[6-(4-Chlor-benzyl)-8-cyclobutyl-1-(2,4-dichlor-benzolsulfonyl)-4,7-dioxo- octahydro-pyrazino[1,2-a]pyrimidin-3-yl]-methansulfonamid

### Struktur:

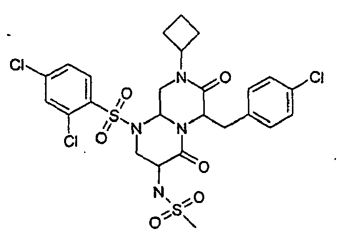

Die Synthese erfolgt analog zu Beispiel 26 ausgehend von 3-Amino-6-(4-chlor-benzyl)-8-cyclobutyl-1-(2,4-dichlor-benzolsulfonyl)-hexahydro- pyrazino[1,2-a]pyrimidin-4,7-dion. Man erhält das gewünschte Produkt mit MG = 648.04 (berechnet, monoisotop); Meßwert (M+H)⁺: 649.15

### Beispiel 278

### 6-(4-Chlor-benzyl)-8-cyclobutyl-1-(2,4-dichlor-benzolsulfonyl)-3-dimethylamino- hexahydropyrazino[1,2-a]pyrimidin-4,7-dion

### Struktur:

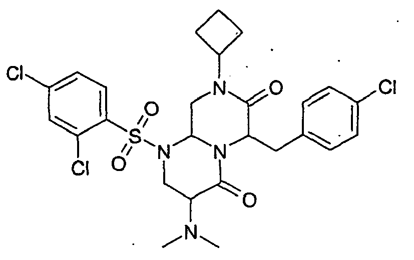

Die Synthese erfolgt analog zu Beispiel 72 ausgehend von 3-Amino-6-(4-chlor-benzyl)-8-cyclobutyl-1-(2,4-dichlor-benzolsulfonyl)-hexahydro- pyrazino[1,2-a]pyrimidin-4,7-dion. Man erhält das gewünschte Produkt mit MG = 598.10 (berechnet, monoisotop); Meßwert (M+H)⁺: 599.07

### Beispiel 279

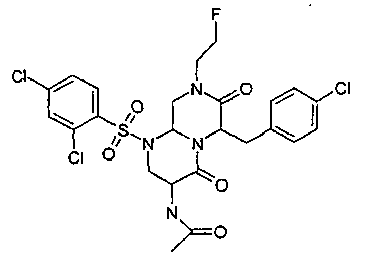

a) (2,2-Diethoxyethyl)-(2-fluorethyl)-amin
   Eine Lösung von 3,1 mL (21,5 mmol) 1-Amino-2,2-diethoxyethan, 3,0 g (23,6 mmol) 1-Brom-2-fluorethan und 5,56 g (43 mmol) Diisopropylethylamin wurde 6 Stunden lang in einem verschlossenen Gefäß auf 100°C erwärmt. Das Reaktionsgemisch wurde mit Ether verdünnt und mit 1N-NaOH und Wasser gewaschen. Die organische Phase wurde getrocknet (MgSO₄) und im Vakuum eingeengt. Der Rückstand wurde im Vakuum destilliert. Man erhielt 1,3 g Substanz als klare Flüssigkeit.
b) 2-Amino-3-(4-chlor-phenyl)-N-(2,2-diethoxy-ethyl)-N-(2-fluor-ethyl)-propionamid
   Die Synthese erfolgt analog zu Beispiel 274b). Man erhält das gewünschte Produkt mit MG = 360.16 (berechnet, monoisotop); Meßwert (M+H)⁺: 361.2
c) [1-{2-(4-Chlor-phenyl)-1-[(2,2-diethoxy-ethyl)-(2-fluor-ethyl)-carbamoyl]-ethylcarbamoyl}-2-(2,4-dichlor-benzolsulfonylamino)-ethyl]-carbaminsäurebenzyl ester
   Die Synthese erfolgt analog zu Beispiel 274c). Man erhält das gewünschte Produkt mit MG = 788.16 (berechnet, monoisotop); Meßwert (M+Na)⁺: 811.19
d) [6-(4-Chlor-benzyl)-1-(2,4-dichlor-benzolsulfonyl)-8-(2-fluor-ethyl)-4,7-dioxo- octahydro-pyrazino[1,2-a]pyrimidin-3-yl]-carbaminsäurebenzylester
   Die Synthese erfolgt analog zu Beispiel 29f) (Methode B) ausgehend von [1-{2-(4-Chlorphenyl)-1-[(2,2-diethoxy-ethyl)-(2-fluor-ethyl)-carbamoyl]-ethylcarbamoyl}-2-(2,4-dichlor-benzolsulfonylamino)-ethyl]-carbaminsäurebenzyl ester. Man erhält das gewünschte Produkt mit MG = 696.08 (berechnet, monoisotop); Meßwert (M+H)⁺: 697.04
e) 3-Amino-6-(4-chlor-benzyl)-1-(2,4-dichlor-benzolsulfonyl)-8-(2-fluor-ethyl)-hexahydro-pyrazino[1,2-a]pyrimidin-4,7-dion
   Die Synthese erfolgt analog zu Beispiel 29e) (Methode B) ausgehend von [6-(4-Chlor-benzyl)-1-(2,4-dichlor-benzolsulfonyl)-8-(2-fluor-ethyl)-4,7-dioxo-octahydro-pyrazino[1,2-a]pyrimidin-3-yl]-carbaminsäurebenzylester. Man erhält das gewünschte Produkt mit MG = 562.04 (berechnet, monoisotop); Meßwert (M+H)⁺: 563.03
f) N-[6-(4-Chlor-benzyl)-1-(2,4-dichlor-benzolsulfonyl)-8-(2-fluor-ethyl)-4,7-dioxo- octahydro-pyrazino[1,2-a]pyrimidin-3-yl]-acetamid
   Die Synthese erfolgt analog zu Beispiel 21h) ausgehend von 3-Amino-6-(4-chlor-benzyl)-1-(2,4-dichlor-benzolsulfonyl)-8-(2-fluor-ethyl)-hexahydro- pyrazino[1,2-a]pyrimidin-4,7-dion. Man erhält das gewünschte Produkt mit MG = 604.05 (berechnet, monoisotop); Meßwert (M+H)⁺: 605.07

### Beispiel 280

### Cyclopropancarbonsäure [6-(4-chlor-benzyl)-1-(2,4-dichlor-benzolsulfonyl)-8-(2- fluor-ethyl)-4,7-dioxo-octahydro-pyrazino[1,2-a]pyrimidin-3-yl]-amid

### Struktur:

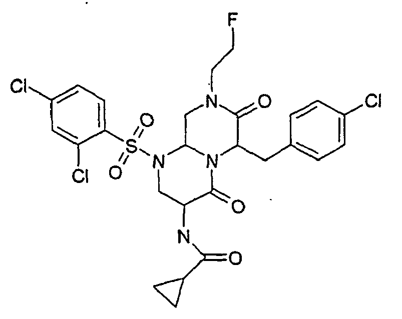

Die Synthese erfolgt analog zu Beispiel 22 ausgehend von 3-Amino-6-(4-chlor-benzyl)-1-(2,4-dichlor-benzolsulfonyl)-8-(2-fluor-ethyl)-hexahydro- pyrazino[1,2-a]pyrimidin-4,7-dion. Man erhält das gewünschte Produkt mit MG = 630.07 (berechnet, monoisotop); Meßwert (M+H)⁺: 631.06

### Beispiel 281

### Cyclobutancarbonsäure [6-(4-chlor-benzyl)-1-(2,4-dichlor-benzolsulfonyl)-8-(2- fluor-ethyl)-4,7-dioxo-octahydro-pyrazino[1,2-a]pyrimidin-3-yl]-amid

### Struktur:

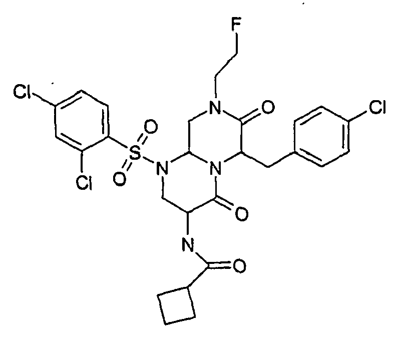

Die Synthese erfolgt analog zu Beispiel 30 ausgehend von 3-Amino-6-(4-chlor-benzyl)-1-(2,4-dichlor-benzolsulfonyl)-8-(2-fluor-ethyl)-hexahydro- pyrazino[1,2-a]pyrimidin-4,7-dion. Man erhält das gewünschte Produkt mit MG = 644.08 (berechnet, monoisotop); Meßwert (M+H)⁺: 645.11

### Beispiel 282

### 1-tert-Butyl-3-[6-(4-chlor-benzyl)-1-(2,4-dichlor-benzolsulfonyl)-8-(2-fluor-ethyl)- 4,7-dioxo-octahydro-pyrazino[1,2-a]pyrimidin-3-yl]-harnstoff

### Struktur:

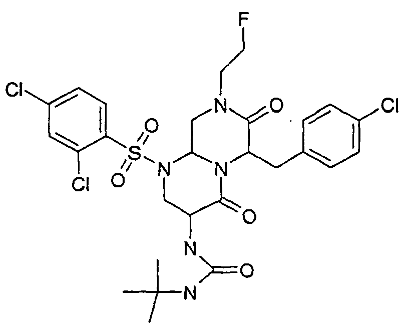

Die Synthese erfolgt analog zu Beispiel 28 ausgehend von 3-Amino-6-(4-chlor-benzyl)-1-(2,4-dichlor-benzolsulfonyl)-8-(2-fluor-ethyl)-hexahydro- pyrazino[1,2-a]pyrimidin-4,7-dion. Man erhält das gewünschte Produkt mit MG = 661.11 (berechnet, monoisotop); Meßwert (M+H)⁺: 662.1

### Beispiel 283

### N-[6-(4-Chlor-benzyl)-1-(2,4-dichlor-benzolsulfonyl)-8-(2-fluor-ethyl)-4,7-dioxo- octahydro-pyrazino[1,2-a]pyrimidin-3-yl]-methansulfonamid

### Struktur:

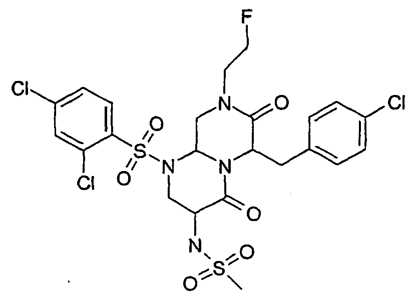

Die Synthese erfolgt analog zu Beispiel 26 ausgehend von 3-Amino-6-(4-chlor-benzyl)-1-(2,4-dichlor-benzolsulfonyl)-8-(2-fluor-ethyl)-hexahydro- pyrazino[1,2-a]pyrimidin-4,7-dion. Man erhält das gewünschte Produkt mit MG = 640.02 (berechnet, monoisotop); Meßwert (M+H)⁺: 641.03

### Beispiel 284

### 6-(4-Chlor-benzyl)-1-(2,4-dichlor-benzolsulfonyl)-3-dimethylamino-8-(2-fluor-ethyl)-hexahydro-pyrazino[1,2-a]pyrimidin-4,7-dion

### Struktur:

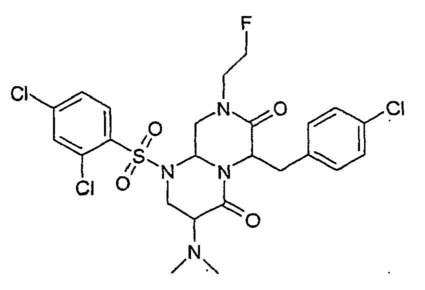

Die Synthese erfolgt analog zu Beispiel 72 ausgehend von 3-Amino-6-(4-chlor-benzyl)-1-(2,4-dichlor-benzolsulfonyl)-8-(2-fluor-ethyl)-hexahydro- pyrazino[1,2-a]pyrimidin-4,7-dion. Man erhält das gewünschte Produkt mit MG = 590.07 (berechnet, monoisotop); Meßwert, (M+H)⁺: 591.04

### Beispiel 285

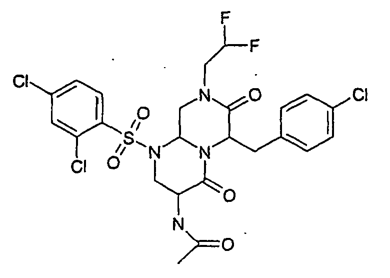

a) (2,2-Diethoxyethyl)-(2,2-difluorethyl)-amin
   Eine Lösung von 3 g (22,5 mmol) 1-Amino-2,2-diethoxyethan, 3,1 g (24,8 mmol) Difluoracetaldehydethylhemiacetal und 1 Pellet NaOH-Feststoff in 44 mL Toluol wurde 1,5 Stunden lang in einem Dean-Stark-Abscheider auf 120°C erwärmt. Das Gemisch wurde stehengelassen, bis es auf Raumtemperatur abgekühlt war und wurde im Vakuum eingeengt. Der Rückstand wurde mit 80 mL Methanol verdünnt und mit 3,4 g (90 mmol) Natriumborhydrid in kleinen Mengen versetzt. Dann wurde das Reaktionsgemisch über Nacht gerührt, im Vakuum eingeengt und zwischen Essigsäureethylester und Wasser aufgeteilt. Die organische Phase wurde isoliert, getrocknet (MgSO₄) und im Vakuum eingeengt. Man erhielt 3,7 g Rohsubstanz als farbloses Öl. Der Rückstand wurde einer Flash-Chromatographie an 40 g SiO₂ unterzogen (Elution mit DCM/MeOH, Gradient 1 - 8 %). Man erhielt 3,1 g des gewünschten Amins als farbloses Öl.
b) 2-Amino-3-(4-chlor-phenyl)-N-(2,2-diethoxy-ethyl)-N-(2,2-difluor-ethyl)-propionamid
   Die Synthese erfolgt analog zu Beispiel 274b). Man erhält das gewünschte Produkt mit MG = 378.15 (berechnet, monoisotop); Meßwert (M+H)⁺: 379.18
c) [1-{2-(4-Chlor-phenyl)-1-[(2,2-diethoxy-ethyl)-(2,2-difluor-ethyl)-carbamoyl]-ethylcarbamoyl]-2-(2,4-dichlor-benzolsulfonylamino)-ethyl]-carbaminsäurebenzyl ester
   Die Synthese erfolgt analog zu Beispiel 274c). Man erhält das gewünschte Produkt mit MG = 806.15 (berechnet, monoisotop); Meßwert (M+Na)⁺: 829.11
d) [6-(4-Chlor-benzyl)-1-(2,4-dichlor-benzolsulfonyl)-8-(2,2-difluor-ethyl)-4,7-dioxo-octahydro-pyrazino[1,2-a]pyrimidin-3-yl]-carbaminsäurebenzylester
   Die Synthese erfolgt analog zu Beispiel 29f) (Methode B) ausgehend von [1-{2-(4-Chlorphenyl)-1-[(2,2-diethoxy-ethyl)-(2,2-difluor-ethyl)-carbamoyl]- ethylcarbamoyl}-2-(2,4-dichlor-benzolsulfonylamino)-ethyl]-carbaminsäurebenzyl ester. Man erhält das gewünschte Produkt mit MG = 714.07 (berechnet, monoisotop); Meßwert (M+H)⁺: 715.02
e) 3-Amino-6-(4-chlor-benzyl)-1-(2,4-dichlor-benzolsulfonyl)-8-(2,2-difluor-ethyl)-hexahydro-pyrazino[1,2-a]pyrimidin-4,7-dion
   Die Synthese erfolgt analog zu Beispiel 29e) (Methode B) ausgehend von [6-(4-Chlor-benzyl)-1-(2,4-dichlor-benzolsulfonyl)-8-(2,2-difluor-ethyl)-4,7-dioxo- octahydro-pyrazino[1,2-a]pyrimidin-3-yl]-carbaminsäurebenzylester. Man erhält das gewünschte Produkt mit MG = 580.03 (berechnet, monoisotop); Meßwert (M+H)⁺: 580.99
f) N-[6-(4-Chlor-benzyl)-1-(2,4-dichlor-benzolsulfonyl)-8-(2,2-diffuor-ethyl)-4,7-dioxo-octahydro-pyrazino[1,2-a]pyrimidin-3-yl]-acetamid
   Die Synthese erfolgt analog zu Beispiel 21h) ausgehend von 3-Amino-6-(4-chlor-benzyl)-1-(2,4-dichlor-benzolsulfonyl)-8-(2,2-difluor-ethyl)-hexahydro- pyrazino[1,2-a]pyrimidin-4,7-dion. Man erhält das gewünschte Produkt mit MG = 622.04 (berechnet, monoisotop); Meßwert (M+H)⁺: 623.03

### Beispiel 286

### Cyclopropancarbonsäure [6-(4-chlor-benzyl)-1-(2,4-dichlor-benzolsulfonyl)-8-(2,2-difluor-ethyl)-4,7-dioxo-octahydro-pyrazino[1,2-a]pyrimidin-3-yl]-amid

### Struktur:

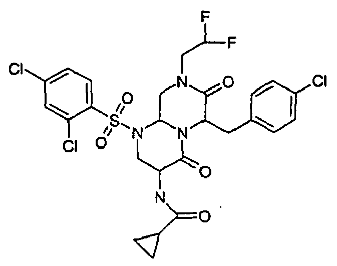

Die Synthese erfolgt analog zu Beispiel 22 ausgehend von 3-Amino-6-(4-chlor-benzyl)-1-(2,4-dichlor-benzolsulfonyl)-8-(2,2-difluor-ethyl)-hexahydro- pyrazino[1,2-a]pyrimidin-4,7-dion. Man erhält das gewünschte Produkt mit MG = 648.06 (berechnet, monoisotop); Meßwert (M+H)⁺: 649.04

### Beispiel 287

### Cyclobutancarbonsäure [6-(4-chlor-benzyl)-1-(2,4-dichlor-benzolsulfonyl)-8-(2,2-difluor-ethyl)-4,7-dioxo-octahydro-pyrazino[1,2-a]pyrimidin-3-yl]-amid

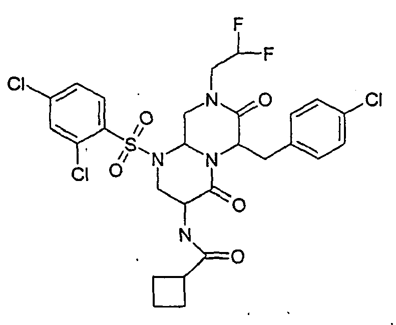

Die Synthese erfolgt analog zu Beispiel 30 ausgehend von 3-Amino-6-(4-chlor-benzyl)-1-(2,4-dichlor-benzolsulfonyl)-8-(2,2-difluor-ethyl)-hexahydro- pyrazino[1,2-a]pyrimidin-4,7-dion. Man erhält das gewünschte Produkt mit MG = 662.07 (berechnet, monoisotop); Meßwert (M+H)⁺: 663.06

### Beispiel 288

### Cyclopentancarbonsäure [6-(4-chlor-benzyl)-1-(2,4-dichlor-benzolsulfonyl)-8-(2,2-difluor-ethyl)-4,7-dioxo-octahydro-pyrazino[1,2-a]pyrimidin-3-yl]-amid

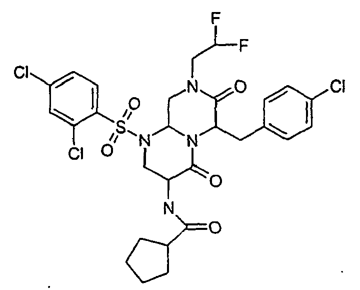

Die Synthese erfolgt analog zu Beispiel 31 ausgehend von 3-Amino-6-(4-chlor-benzyl)-1-(2,4-dichlor-benzolsulfonyl)-8-(2,2-difluor-ethyl)-hexahydro- pyrazino[1,2-a]pyrimidin-4,7-dion. Man erhält das gewünschte Produkt mit MG = 676.09 (berechnet, monoisotop); Meßwert (M+H)⁺: 677.07

### Beispiel 289

### Pyrrolidin-2-carbonsäure [6-(4-chlor-benzyl)-1-(2,4-dichlor-benzolsulfonyl)-8-(2,2-difluor-ethyl)-4,7-dioxo-octahydro-pyrazino[1,2-a]pyrimidin-3-yl]-amid

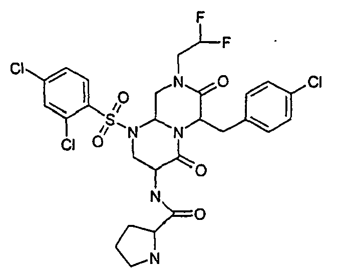

Die Synthese erfolgt analog zu Beispiel 40 ausgehend von 3-Amino-6-(4-chlor-benzyl)-1-(2,4-dichlor-benzolsulfonyl)-8-(2,2-difluor-ethyl)-hexahydro- pyrazino[1,2-a]pyrimidin-4,7-dion. Man erhält das gewünschte Produkt mit MG = 677.08 (berechnet, monoisotop); Meßwert (M+H)⁺: 678.08

### Beispiel 290

### 6-(4-Chlor-benzyl)-1-(2,4-dichlor-benzolsulfonyl)-8-(2,2-difluor-ethyl)-3-dimethylaminohexahydro-pyrazino[1,2-a]pyrimidin-4,7-dion

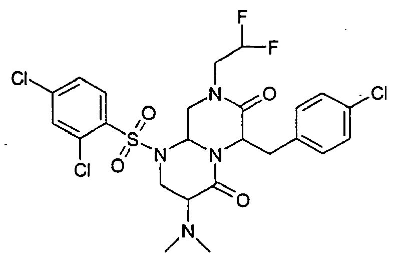

Die Synthese erfolgt analog zu Beispiel 72 ausgehend von 3-Amino-6-(4-chlor-benzyl)-1-(2,4-dichlor-benzolsulfonyl)-8-(2,2-difluor-ethyl)-hexahydro- pyrazino[1,2-a]pyrimidin-4,7-dion. Man erhält das gewünschte Produkt mit MG = 608.06 (berechnet, monoisotop); Meßwert (M+H)⁺: 609.05

### Beispiel 291

### 6-(4-Chlor-benzyl)-1-(2,4-dichlor-benzolsulfonyl)-8-(2,2-difluor-ethyl)-3-morpholin-4-yl-hexahydro-pyrazino[1,2-a]pyrimidin-4,7-dion

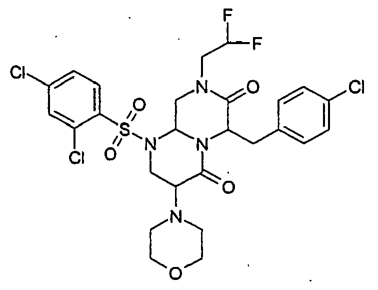

Die Synthese erfolgt analog zu Beispiel 60 ausgehend von 3-Amino-6-(4-chlor-benzyl)-1-(2,4-dichlor-benzolsulfonyl)-8-(2,2-difluor-ethyl)-hexahydro- pyrazino[1,2-a]pyrimidin-4,7-dion. Man erhält das gewünschte Produkt mit MG = 650.07 (berechnet, monoisotop); Meßwert (M+H)⁺: 651.07

### Beispiel 292

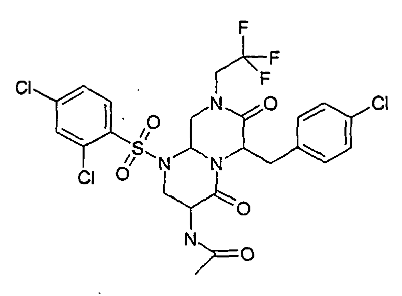

a) (2,2-Diethoxyethyl)-(2,2,2-trifluorethyl)-amin
   Eine Lösung von 1 g (7,5 mmol) 1-Amino-2,2-diethoxyethan, 1,26 g (7,9 mmol) Trifluoracetaldehydethylhemiacetal und 1 Pellet NaOH in 15 mL Toluol wurde 3 Stunden lang in einem Dean-Stark-Abscheider auf 110°C erwärmt. Das Gemisch wurde weitere 3 Stunden lang auf 125°C erwärmt. Das Reaktionsgemisch wurde stehengelassen, bis es auf Raumtemperatur abgekühlt war und wurde im Vakuum eingeengt. Der Rückstand wurde mit 25 mL Methanol verdünnt und mit 1,13 g (30 mmol) Natriumborhydrid versetzt. Dann wurde das Reaktionsgemisch auf 70°C erwärmt und über Nacht gerührt, im Vakuum eingeengt und zwischen Wasser und Essigsäureethylester aufgeteilt. Die organische Phase wurde isoliert, getrocknet (MgSO₄) und im Vakuum eingeengt. Man erhielt 740 mg des gewünschten Amins als klares Öl.
b) 2-Amino-3-(4-chlor-phenyl)-N-(2,2-diethoxy-ethyl)-N-(2,2,2-trifluor-ethyl)-propionamid
   Die Synthese erfolgt analog zu Beispiel 274b). Man erhält das gewünschte Produkt mit MG = 378.15 (berechnet, monoisotop); Meßwert (M+H)⁺: 379.18
c) [1-{2-(4-Chlor-phenyl)-1-[(2,2-diethoxy-ethyl)-(2,2,2-trifluor-ethyl)-carbamoyl]-ethylcarbamoyl}-2-(2,4-dichlor-benzolsulfonylamino)-ethyl]-carbaminsäurebenzyl ester
   Die Synthese erfolgt analog zu Beispiel 274c). Man erhält das gewünschte Produkt mit MG = 806.15 (berechnet, monoisotop); Meßwert (M+Na)⁺: 829.11
d) [6-(4-Chlor-benzyl)-1-(2,4-dichlor-benzolsulfonyl)-8-(2,2,2-trifluor-ethyl)-4,7-dioxo-octahydro-pyrazino[1,2-a]pyrimidin-3-yl]-carbaminsäurebenzylester
   Die Synthese erfolgt analog zu Beispiel 29f) (Methode B) ausgehend von [1-{2-(4-Chlorphenyl)-1-[(2,2-diethoxy-ethyl)-(2,2,2-trifluor-ethyl)-carbamoyl]- ethylcarbamoyl}-2-(2,4-dichlor-benzolsulfonylamino)-ethyl]-carbaminsäurebenzyl ester. Man erhält das gewünschte Produkt mit MG = 732.06 (berechnet, monoisotop); Meßwert (M+H)⁺: 732.12
e) 3-Amino-6-(4-chlor-benzyl)-1-(2,4-dichlor-benzolsulfonyl)-8-(2,2,2-trifluor-ethyl)-hexahydro- pyrazino[1,2-a]pyrimidin-4,7-dion
   Die Synthese erfolgt analog zu Beispiel 29e) (Methode B) ausgehend von [6-(4-Chlor-benzyl)-1-(2,4-dichlor-benzolsulfonyl)-8-(2,2,2-trifluor-ethyl)-4,7-dioxo- octahydro-pyrazino[1,2-a]pyrimidin-3-yl]-carbaminsäurebenzylester. Man erhält das gewünschte Produkt mit MG = 598.02 (berechnet, monoisotop); Meßwert (M+H)⁺: 599.06
f) N-[6-(4-Chlor-benzyl)-1-(2,4-dichlor-benzolsulfonyl)-8-(2,2,2-trifluor-ethyl)-4,7-dioxo-octahydro-pyrazino[1,2-a]pyrimidin-3-yl]-acetamid
   Die Synthese erfolgt analog zu Beispiel 21h) ausgehend von 3-Amino-6-(4-chlor-benzyl)-1-(2,4-dichlor-benzolsulfonyl)-8-(2,2,2-trifluor-ethyl)-hexahydro- pyrazino[1,2-a]pyrimidin-4,7-dion. Man erhält das gewünschte Produkt mit MG = 640.03 (berechnet, monoisotop); Meßwert (M+H)⁺: 641.06

### Beispiel 293

### Cyclopropancarbonsäure [6-(4-chlor-benzyl)-1-(2,4-dichlor-benzolsulfonyl)-4,7- dioxo-8-(2,2,2-trifluor-ethyl)-octahydro-pyrazino[1,2-a]pyrimidin-3-yl]-amid

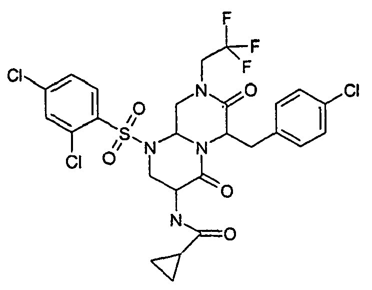

Die Synthese erfolgt analog zu Beispiel 22 ausgehend von 3-Amino-6-(4-chlor-benzyl)-1-(2,4-dichlor-benzolsulfonyl)-8-(2,2,2-trifluor-ethyl)-hexahydro- pyrazino[1,2-a]pyrimidin-4,7-dion. Man erhält das gewünschte Produkt mit MG = 666.05 (berechnet, monoisotop); Meßwert (M+H)⁺: 667.07

### Beispiel 294

### Cyclobutancarbonsäure [6-(4-chlor-benzyl)-1-(2,4-dichlor-benzolsulfonyl)-4,7- dioxo-8-(2,2,2-trifluor-ethyl)-octahydro-pyrazino[1,2-a]pyrimidin-3-yl]-amid

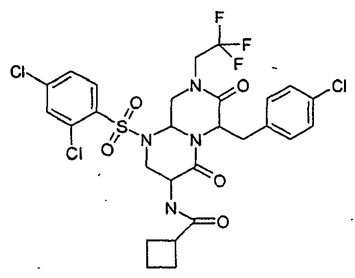

Die Synthese erfolgt analog zu Beispiel 30 ausgehend von 3-Amino-6-(4-chlor-benzyl)-1-(2,4-dichlor-benzolsulfonyl)-8-(2,2,2-trifluor-ethyl)-hexahydro- pyrazino[1,2-a]pyrimidin-4,7-dion. Man erhält das gewünschte Produkt mit MG = 680.06 (berechnet, monoisotop); Meßwert (M+H)⁺: 681.02

### Beispiel 295

### Cyclopentancarbonsäure [6-(4-chlor-benzyl)-1-(2,4-dichlor-benzolsulfonyl)-4,7- dioxo-8-(2,2,2-trifluor-ethyl)-octahydro-pyrazino[1,2-a]pyrimidin-3-yl]-amid

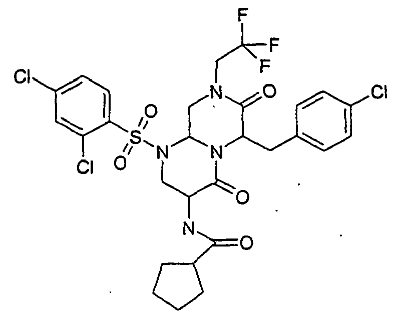

Die Synthese erfolgt analog zu Beispiel 31 ausgehend von 3-Amino-6-(4-chlor-benzyl)-1-(2,4-dichlor-benzolsulfonyl)-8-(2,2,2-trifluor-ethyl)-hexahydro- pyrazino[1,2-a]pyrimidin-4,7-dion. Man erhält das gewünschte Produkt mit MG = 694.08 (berechnet, monoisotop); Meßwert (M+H)⁺: 695.10

### Beispiel 296

### Piperidin-4-carbonsäure [6-(4-chlor-benzyl)-1-(2,4-dichlor-benzolsulfonyl)-4,7- dioxo-8-(2,2,2-trifluor-ethyl)-octahydro-pyrazino[1,2-a]pyimidin-3-yl]-amid

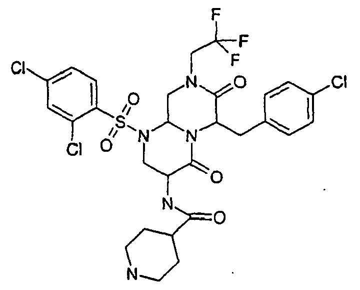

Die Synthese erfolgt analog zu Beispiel 37 ausgehend von 3-Amino-6-(4-chlor-benzyl)-1-(2,4-dichlor-benzolsulfonyl)-8-(2,2,2-trifluor-ethyl)-hexahydro-pyrazino[1,2-a]pyrimidin-4,7-dion. Man erhält das gewünschte Produkt mit MG = 709.09 (berechnet, monoisotop); Meßwert (M+H)⁺: 710.09

### Beispiel 297

### 6-(4-Chlor-benzyl)-1-(2,4-dichlor-benzolsulfonyl)-3-dimethylamino-8-(2,2,2-trifluor- ethyl)-hexahydro-pyrazino[1,2-a]pyrimidin-4,7-dion

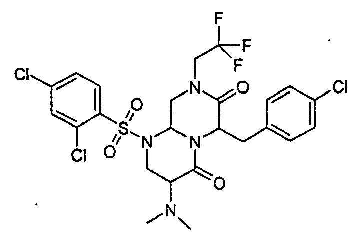

Die Synthese erfolgt analog zu Beispiel 72 ausgehend von 3-Amino-6-(4-chlor-benzyl)-1-(2,4-dichlor-benzolsulfonyl)-8-(2,2,2-trifluor-ethyl)-hexahydro- pyrazino[1,2-a]pyrimidin-4,7-dion. Man erhält das gewünschte Produkt mit MG = 626.05 (berechnet, monoisotop); Meßwert (M+H)⁺: 627.05

### Beispiel 298

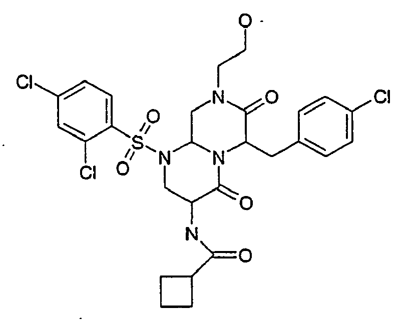

a) [6-(4-Chlor-benzyl)-1-(2,4-dichlor-benzolsulfonyl)-8-(2-hydroxy-ethyl)-4,7-dioxo-octahydro-pyrazino[1,2-a]pyrimidin-3-yl]-carbaminsäurebenzylester
   Eine Lösung von 3,13 g (3,96 mmol) [1-{2-(4-Chlorphenyl)-1-[(2,2-diethoxyethyl)-(2-fluorethyl)-carbamoyl]-ethylcarbamoyl}-2-(2,4-dichlorbenzolsulfonylamino)-ethyl]-carbaminsäurebenzylester in 20 mL Ameisensäure wurde auf 60°C erwärmt und 6 Stunden lang gerührt. Das Reaktionsgemisch wurde im Vakuum eingeengt, mit EtOAc verdünnt und mit 1N-NaHCO₃ und Kochsalzlösung gewaschen. Die organische Phase wurde isoliert, getrocknet (MgSO₄) und im Vakuum eingeengt. Diese Rohsubstanz wurde einer Flash-Chromatographie an 120 g SiO₂ unterzogen (Elution mit EtOAc/Heptan, Gradient 50 - 100 %). Man erhielt 350 mg der Substanz [6-(4-Chlorbenzyl)-1-(2,4-dichlorbenzolsulfonyl)-8-(2-fluorethyl)-4,7-dioxooctahydropyrazino[1,2-a]pyrimidin-3-yl]-carbaminsäurebenzylester Bei einer weiteren Elution der Säule erhielt man 980 mg der dem [6-(4-Chlorbenzyl)-1-(2,4-dichlorbenzolsulfonyl)-8-(2-hydroxyethyl)-4,7-dioxooctahydropyrazino[1,2₋a]pyrimidin-3-yl]-carbaminsäurebenzylester-Nebenprodukt entsprechenden Verbindung. Das bei der LC/MS erwartete, monoisotope MG = 694 und der Meßwert (M⁺H) = 695 stimmen mit der Struktur überein.
b) 3-Amino-6-(4-chlor-benzyl)-1-(2,4-dichlor-benzolsulfonyl)-8-(2-hydroxy-ethyl)-hexahydro-pyrazino[1,2-a]pyrimidin-4,7-dion
   Die Synthese erfolgt analog zu Beispiel 29g) (Methode B) ausgehend von [6-(4-Chlor-benzyl)-1-(2,4-dichlor-benzolsulfonyl)-8-(2-hydroxy-ethyl)-4,7-dioxo-octahydro-pyrazino[1,2-a]pyrimidin-3-yl]-carbaminsäurebenzylester. Man erhält das gewünschte Produkt mit MG = 560.04 (berechnet, monoisotop); Meßwert (M+H)⁺: 561.03
c) Cyclobutancarbonsäure [6-(4-chlor-benzyl)-1-(2,4-dichlor-benzolsulfonyl)-8-(2- hydroxyethyl)-4,7-dioxo-octahydro-pyrazino[1,2-a]pyrimidin-3-yl]-amid
   Die Synthese erfolgt analog zu Beispiel 30 ausgehend von 3-Amino-6-(4-chlor-benzyl)-1-(2,4-dichlor-benzolsulfonyl)-8-(2-hydroxy-ethyl)-hexahydro- pyrazino[1,2-a]pyrimidin-4,7-dion. Man erhält das gewünschte Produkt mit MG = 642.09 (berechnet, monoisotop); Meßwert (M+H)⁺: 643.11

### Beispiel 299

### Cyclopentancarbonsäure [6-(4-chlor-benzyl)-1-(2,4-dichlor-benzolsulfonyl)-8-(2- hydroxyethyl)-4,7-dioxo-octahydro-pyrazino[1,2-a]pyrimidin-3-yl]-amid

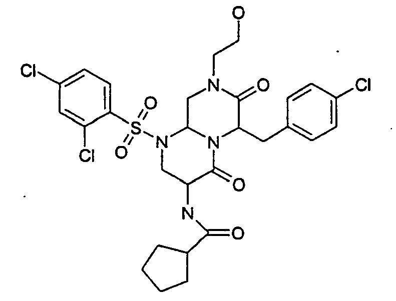

Die Synthese erfolgt analog zu Beispiel 31 ausgehend von 3-Amino-6-(4-chlor-benzyl)-1-(2,4-dichlor-benzolsulfonyl)-8-(2-hydroxy-ethyl)-hexahydro-pyrazino[1,2-a]pyrimidin-4,7-dion. Man erhält das gewünschte Produkt mit MG = 656.10 (berechnet, monoisotop); Meßwert (M+H)⁺: 657.12

### Beispiel 300

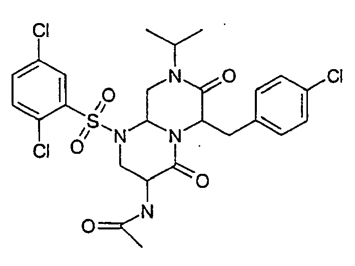

a) 2-Benzyloxycarbonylamino-3-(2,5-dichlor-benzolsulfonylamino)-propionsäure
   Die Synthese erfolgt analog zu Beispiel 21d) ausgehend von 2,5-Dichlor-benzolsulfonyl chlorid. Man erhält das gewünschte Produkt mit MG = 446,01 (berechnet, monoisotop); Meßwert (M+H-CO₂)⁺: 403,00.

a) N-{2-(4-Chlor-phenyl)-1-[(2,2-diethoxy-ethyl)-isopropyl-carbamoyl]-ethyl}-3-(2,5-dichlor-benzolsulfonylamino)-2-methansulfonylamino-propionamid
   Die Synthese erfolgt analog zu Beispiel 21e) ausgehend von 2-Benzyloxycarbonylamino-3-(2,6-dichlor-benzolsulfonylamino)-propionsäure. Man erhält das gewünschte Produkt mit MG = 784,186 (berechnet, monoisotop); Meßwert (M-CO₂+H)⁺: 741,10
b) [6-(4-Chlor-benzyl)-1-(2,5-dichlor-benzolsulfonyl)-8-isopropyl-4,7-dioxo-octahydro-pyrazino[1,2-a]pyrimidin-3-yl]-carbaminsäurebenzylester
   Die Synthese erfolgt analog zu Beispiel 21f) ausgehend von N-{2-(4-Chlor-phenyl)-1-[(2,2-diethoxy-ethyl)-isopropyl-carbamoyl]-ethyl}-3-(2,5 -dichlor-benzolsulfonylamino)-2-methansulfonylamino-propionamid. Man erhält das gewünschte Produkt mit MG = 692,10 (berechnet, monoisotop); Meßwert (M+H)⁺: 693,05
d) 3-Amino-6-(4-chlor-benzyl)-1-(2,5-dichlor-benzolsulfonyl)-8-isopropyl-hexahydro-pyrazino[1,2-a]pyrimidin-4,7-dion
   Die Synthese erfolgt analog Bsp. 21g) ausgehend von [6-(4-Chlor-benzyl)-1-(2,5-dichlor-benzolsulfonyl)-8-isopropyl-4,7-dioxo-octahydro- pyrazino[1,2-a]pyrimidin-3-yl]-carbaminsäurebenzylester. Man erhält das gewünschte Produkt mit MG = 558,07 (berechnet, monoisotop); Meßwert (M+H)⁺: 559,10
e) N-[6-(4-Chlor-benzyl)-1-(2,5-dichlor-benzolsulfonyl)-8-isopropyl-4,7-dioxo-octahydro-pyrazino[1,2-a]pyrimidin-3-yl]-acetamid
   Die Synthese erfolgt analog Bsp. 21h) ausgehend von 3-Amino-6-(4-chlor-benzyl)-1-(2,5-dichlor-benzolsulfonyl)-8-isopropyl-hexahydro- pyrazino[1,2-a]pyrimidin-4,7-dion. Man erhält das gewünschte Produkt mit MG = 600.08 (berechnet, monoisotop); Meßwert (M+H)⁺: 601.13.

### Beispiel 301

### N-[6-(4-Chlor-benzyl)-1-(2,5-dichlor-benzolsulfonyl)-8-isopropyl-4,7-dioxo-octahydro-pyrazino[1,2-a]pyrimidin-3-yl]-methansulfonamid

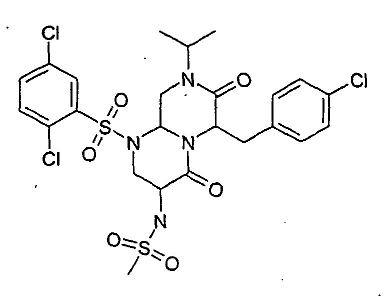

Die Synthese erfolgt analog Bsp. 26 ausgehend von 3-Amino-6-(4-chlor-benzyl)-1-(2,5-dichlor-benzolsulfonyl)-8-isopropyl-hexahydro- pyrazino[1,2-a]pyrinmidin-4,7-dion. Man erhält das gewünschte Produkt mit MG = 636.04 (berechnet, monoisotop); Meßwert (M+H)⁺: 637.02.

### Beispiel 302

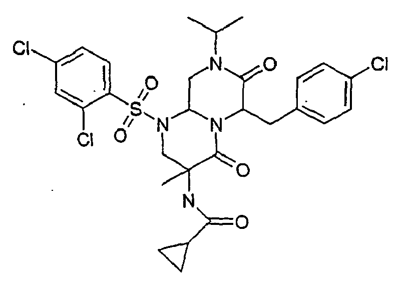

a) 2-Benzyloxycarbonylamino-3-hydroxy-2-methyl-propionsäure- methylester:
   250 mg(2.1 mmol) 2-Amino-3-methylhydroxypropansäure werden in 9 mL wasserfreiem MeOH gelöst. Die Reaktionslösung wird im Eisbad gekühlt und es werden langsam 0.153 mL (2.1 mmol) SOCl₂ zugetropft. Man läßt die Lösung langsam über Nacht auf Raumtemperatur kommen. Die Reaktionslösung wird im Vakuum aufkonzentriert und anschließend mit 1 mL EtOAc und 5 mL gesättigter NaHCO₃ (wässrig) versetzt, gefolgt von 0.285 mL (1.995 mmol) Benzylchlorformiat. Die Lösung wird über Nacht bei Raumtemperatur gerührt. Dann werden 5 mL EtOAc zugegeben, die organische Phase abgetrennt und 2-mal mit je 1mL 1N HCl (aq) und 2-mal mit je 2 mL einer gesättigten NaHCO₃ (aq) gewaschen, über MgSO₄ getrocknet und im Vakuum aufkonzentriert. Die Aufreinigung erfolgte mittels Chromatographie (40g SiO₂, Eluent MeOH/DCM (Gradient 0-10%). Man erhält das gewünschte Produkt als farbloses Öl (0.130 g) mit MW=267 (berechnet, monoisotop), Meßwert:(M+ Na)⁺290.
b) 2-Benzyloxycarbonylamino-2-methyl-3-oxo-propionsäure methylester:
   Zu einer Lösung von 56 mg (0.21 mmol) 2-Benzyloxycarbonylamino-3-hydroxy-2-methyl-propionsäuremethylester in 1 mL DCM, wird langsam eine Lösung aus 97.7 mg (0.23 mmol) Dess Martin Periodinan Reagenz (Aldrich) in 1 mL DCM zugetropft, anschließend läßt man 30 min bei Raumtemperatur rühren. Danach wird die Lösung mit 5 mL Diethylether und einer Mischung aus 4 mL gesättigter NaHCO₃ und 0.36 g Na₂S₂O₃.5H₂O versetzt. Es wird 15 min gerührt bis sich der Feststoff aufgelöst hat, dann wird die Etherphase mit gesättigter NaHCO₃ und Wasser gewaschen, über MgSO₄ getrocknet und im Vakuum eingeengt. Das Rohprodukt wird chromatographisch an 4 g SiO₂ mit EtOAc/Heptan (Gradient 0-50%) gereinigt. Es werden 40 mg des gewünschten Aldehyds erhalten.
c) 3-Allylamino-2-benzyloxycarbonylamino-2-methyl-propionsäuremethyle ster:
   Zu einer Lösung von 0.874g (3.3 mmol) 2-Benzyloxycarbonylamino-2-methyl-3-oxo-propionsäuremethylester in 15 mL DCM, werden 1.237 mL (16.5 mmol) Allylamin und 0.9g (7.48 mmol) MgSO4 zugegeben. Es wird über Nacht bei Raumtemperatur gerührt. Dann wurde die Reaktionsmischung abfiltriert und im Vakuum aufkonzentriert. Zu dem Rohprodukt werden 50 mL wasserfreies MeOH, 6mL NaCNBH₃ (1.0M in THF) und 1.8 mL Essigsäure gegeben. Diese Mischung wird 2h bei RT gerührt. Die Reaktionslösung wird im Vakuum aufkonzentriert, anschließend wird sie mit EtOAc und Wasser versetzt. Die organische Phase wird abgetrennt und über MgSO₄ getrocknet und das Lösungsmittel im Vakuum entfernt. Die Aufreinigung erfolgt chromatographisch (25 g SiO₂, Eluent MeOH/DCM (Gradient 0-10%)). Es werden 1.035 g des gewünschten Produktes als Öl erhalten. MW= 306 (berechnet, monoisotop), Meßwert: (M+ H)⁺ 307.
d) 3-Amino-2-benzyloxycarbonylamino-2-methyl-propionsäuremethylester:
   Eine Lösung aus 0.22 g (1.42 mmol) N,N-Dimethylbarbitursäure, 0.02g (0.017 mmol) Pd(PPh₃)₄, 1.5 mL DCM and 0.145g (0.47mmol) 3-Allylamino-2-benzyloxycarbonylamino-2-methyl-propionsäuremethylester wird für 2 h auf 35ºC erwärmt. Anschließend wird die Reaktionslösung im Vakuum aufkonzentriert, dann mit 20 mL Diethylether versetzt und 3 mal mit je 20 mL gesättigter Na₂CO₃ gewaschen. Durch tropfenweise Zugabe von 4N HCl wird ein pH-Wert von 2 eingestellt. Die wässrige Phase wird abgetrennt und mit 2 mL EtOAc extrahiert und das Lösungsmittel im Vakuum entfernt. Es werden 0.105g des gewünschten Produktes erhalten. MW (berechnet, monoisotop)= 266, Meßwert: (M+ H) ⁺ 267.
e) 2-Benzyloxycarbonylamino-3-(2,4-dichlor-benzolsulfonylamino)-2-methy 1-propionsäuremethylester:
   0.103 g (0.341 mmol) 3-Amino-2-benzyloxycarbonylamino-2-methyl-propionsäuremethylester, 0.109g (0.443 mmol) 2,4 Dichlorbenzolsulfonylchlorid and 0.237 mL(1.36 mmol) DIEA werden in 2 mL DCM gelöst. Es wird über Nacht bei Raumtemperatur gerührt. 2 mL werden zur Reaktionslösung gegeben, anschließend die organische Phase abgetrennt und über MgSO₄ getrocknet und das Lösungsmittel im Vakuum entfernt. Das Rohprodukt wird chromatographisch an 4 g SiO₂, EtOAc/DCM als Eluent (Gradient 0-40%) gereinigt. Es werden 0.081 g des gewünschten Produktes als Öl erhalten. MW= 474 (berechnet, monoisotop), Meßwert: (M+ H)⁺475.
f) 2-Benzyloxycarbonylamino-3-(2,4-dichlor-benzolsulfonylamino)-2-methy 1-propionsäure:
   0.08 g (0.168 mmol) 2-Benzyloxycarbonylamino-3-(2,4-dichlor-benzolsulfonyl amino) -2-methyl-propionsäuremethylester, 0.093 g (0.674 mmol) Kaliumcarbonat werden in einer Mischung aus 4.5 mL MeOH und 0.5 mL Wasser 90 min gerührt. Anschließend werden 3 mL gesättigte NaHCO₃-Lösung zugegeben und 2h bei 60°C gerührt. Es werden 0.5 mL Wasser, gefolgt von 4N HCl (aq) zugegeben bis ein pH-Wert von 2 erreicht wird. Die Lösung wird 3 mal mit je 10 mL EtOAc extrahiert, die vereinigten organischen Phasen werden über MgSO₄ getrocknet und das Lösungsmittel im Vakuum entfernt. Es werden 68 mg des gewünschten Produktes erhalten. MW= 460 (berechnet, monoisotop), Meßwert: ( M+H)⁺461.
g) [1-{2-(4-Chlor-phenyl)-1-[(2,2-diethoxy-ethyl)-isopropyl-carbamoyl]-ethylcarbamoyl-2-(2,4-dichlor-benzolsulfonylamino)-1-methyl-ethyl]-carbaminsäurebenzylester:
   Zu einer Lösung von 68 mg (0.15mmol)) 2-Benzyloxycarbonylamino-3-(2,4-dichlor-benzolsulfonylamino)-2-methyl-propionsäure in 3 mL DMF werden 52.6 mg (0.15 mol) 2-Amino-3-(4-chlor-phenyl)-N-(2,2-diethoxy-ethyl)-N-isopropyl propionamide und 41 mg 4-(4,6-Dimethoxy[1,3,5] triazin-2-yl)4-methylmorpholiniumchlorid xH₂O (DMTMM) gegeben. Diese Reaktionslösung wird über Nacht bei Raumtemperatur gerührt, 10 mL Diethylether zugegeben, die organische Phase abgetrennt und über MgSO₄ getrocknet. Das Lösungsmittel wird im Vakuum entfernt und das Rohprodukt chromatographisch an 4g SiO₂ (Eluent MeOH/DCM, Gradient 0-1%) gereinigt. Es werden 83 mg des gewünschten Produktes erhalten. MW= 798 (berechnet, monoisotop), Meßwert: (M+ H) ⁺ 799.
h) [6-(4-Chlor-benzyl)-1-(2,4-dichlor-benzolsulfonyl)-8-isopropyl-3-methyl-4,7 dioxo-octahydro-pyrazino[1,2-a]pyrimidin-3-yl]-carbaminsäurebenzylester:
   Eine Lösung von 80 mg (0.1 mmol) [1-{2-(4-Chlor-phenyl)-1-[(2,2-diethoxy-ethyl)-isopropyl-carbamoyl]-ethylcarbamoyl}-2-(2,4-dichlor-benzolsulfonylamino)-1-methyl-ethyl]-carbaminsäurebenzylester in 1.5 mL HCOOH (99%) wird 24 h bei 60 °C gerührt. Die Reaktionslösung wird im Vakuum aufkonzentriert und das Rohprodukt chromatographisch gereinigt (4 g SiO₂, Eluent EtOAc/Heptan (Gradient 0-50%)). Es werden 30 mg des gewünschten Produktes als weißer Feststoff erhalten. MW (berechnet, monoisotop)= 706, Meßwert: (M+H)⁺=707
i) 3-Amino-6-(4-chlor-benzyl)-1-(2,4-dichlor-benzolsulfonyl)-8-isopropyl-3-methyl-hexahydro-pyrazino[1,2-a]pyrimidin-4,7-dion:
   Eine Lösung von [6-(4-Chlor-benzyl)-1-(2,4-dichlor-benzolsulfonyl)-8-isopropyl -3-methyl-4,7-dioxo-octahydro-pyrazino[1,2-a]pyrimidin-3-yl]-carbaminsäurebenzylester in 2 mL Acetonitril wird im Eisbad auf 0 °C gekühlt und mit 0.05 mL (0.35 mmol) TMSI versetzt. Die Lösung wird über Nacht bei Raumtemperatur gerührt und anschließend im Vakuum aufkonzentriert, in 1 mL MeOH gelöst und auf eine SCX 1 g Säule gegeben, Verunreinigungen werden mit 5 mL MeOH heruntergewaschen, gefolgt von 5 mL 2N NH₃ in MeOH zum eluieren des Produktes. Nach Aufkonzentration im Vakuum werden 15 mg des gewünschten Produktes erhalten. MW (berechnet, monoisotop)= 572, Meßwert: ( M+H)⁺573.

Cyclopropancarbonsäure [1-benzolsulfonyl-6-(4-chlor-benzyl)-8-isopropyl-3-methyl-4,7-dioxo-octahydro-pyrazino[1,2-a]pyrimidin-3-yl]-amid:

Die Synthese erfolgt analog zu Beispiel 22 ausgehend von 3-Amino-6-(4-chlor-benzyl)-1-(2,4-dichlor-benzolsulfonyl)-8-isopropyl-3-methyl-hexahydro-pyrazino[1,2-a]pyrimidin-4,7-dion. Man erhält das gewünschte Produkt mit MG = 640 (berechnet, monoisotop); Meßwert (M+H)⁺: 641

## Patentansprüche

1. Verbindungen der Formel I, 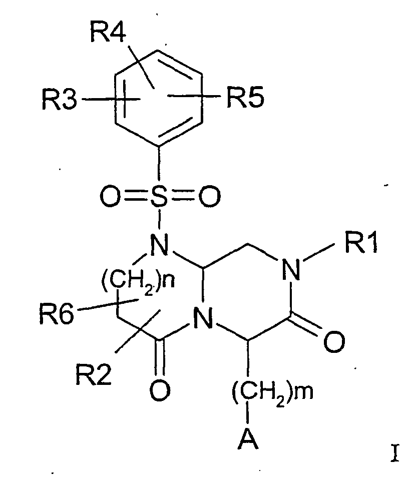 worin bedeuten
A 3-12 gliedriger mono-, bi- oder spirobicyclischer Ring, der ein oder mehrere Heteroatome aus der Gruppe N, O und S enthalten kann und der 3-12 gliedrige Ring weitere Substituenten wie F, Cl, Br, NO₂, CF₃, OCF₃, CN, (C₁-C₆)-Alkyl, Aryl, CON(R11)(R12), N(R13)(R14), OH, O-(C₁-C₆)-Alkyl, S-(C₁-C₆)-Alkyl, N(R15)CO(C₁-C₆)-Alkyl oder COO-(C₁-C₆)-Alkyl tragen kann;
R11, R12, R13, R14, R15 unabhängig voneinander H, (C₁-C₆)-Alkyl, Heterocyclus;
n 0, 1;
m 0, 1, 2, 3, 4, 5, 6;
R1 R8, (C₁-C₆)-Alkylen-R8, (C₂-C₆)-Alkenylen-R9, (SO₂)-R8, (SO₂)-(C₁-C₆)-Alkylen-R8, (SO₂)-(C₂-C₆)-Alkenylen-R9, (C=O)-R8, (C=O)-(C₁-C₆)-Alkylen-R8, (C=O)NH-R8, (C=O)-(C₂-C₆)-Alkenylen-R9, (C=O)-NH- (C₁-C₆)-Alkylen-R8, (C=O)-NH- (C₂-C₆)-Alkenylen-R9, COO-R8, COO-(C₁-C₆)-Alkylen-R8, COO-(C₂-C₆)-Alkenylen-R9, Alkinylen-R9, (C₁-C₄-Alkyl)-Heterocyclus, wobei die Alkylengruppen mit F substituiert sein könne;
R8, R9 unabhängig voneinander H, F, Cl, Br, I, OH, CF₃, Aryl, Heterocyclus, (C₃-C₈)-Cycloalkyl, wobei die Ringe oder Ringssysteme bis zu 3-fach substituiert sein können mit F, Cl, Br, I, OH, CF₃, NO₂, CN, OCF₃, O-(C₁-C₆)-Alkyl, (C₁-C₆)-Alkyl, -NH₂, CON(R11)(R12), N(R13)(R14), SO₂-CH₃, COOH, COO-(C₁-C₆)-Alkyl, CONH₂;
R2 NH₂, NO₂, N(R13)(R14), NH-SO₂-CH₃, NH-SO₂-R12, NR11-SO₂-R12, N(CO)R11, ein Stickstoffhaltiger Heterocyclus, wobei der Heterocyclus über ein Stickstoffatom gebunden ist, NHCONR11, N(C₁-C₆-Alkyl)N⁺(C₁-C₄-Alkyl)₃;
R3, R4, R5 unabhängig voneinander H, F, Cl, Br, J, OH, CF₃, NO₂, CN, OCF₃, O-(C₁-C₆)-Alkyl, O-(C₁-C₄)-Alkoxy-(C₁-C₄)-alkyl, S-(C₁-C₆)-Alkyl, (C₁-C₆)-Alkyl, (C₂-C₆)-Alkenyl, (C₃-C₈)-Cycloalkyl, O-(C₃-C₈)-Cycloalkyl, (C₃-C₈)-Cycloalkenyl, O-(C₃-C₈)-Cycloalkenyl, (C₂-C₆)-Alkinyl, Aryl, O-Aryl (C₁-C₈)-Alkylen-Aryl, O-(C₁-C₈)-Alkylen-Aryl, S-Aryl, N((C₁-C₆)-Alkyl)₂, SO₂-CH₃, COOH, COO-(C₁-C₆)-Alkyl, CO-N((C₁-C₆)-Alkyl)₂;
R6 H, F, Cl, Br, J, OH, CF₃, NO₂, CN, OCF₃, O-(C₁-C₆)-Alkyl, O-(C₁-C₄)-Alkoxy-(C₁-C₄)-alkyl, S-(C₁-C₆)-Alkyl, (C₁-C₆)-Alkyl, (C₂-C₆)-Alkenyl, (C₃-C₈)-Cycloalkyl, O-(C₃-C₈)-Cycloalkyl, (C₃-C₈)-Cycloalkenyl, O-(C₃-C₈)-Cycloalkenyl, (C₂-C₆)-Alkinyl, (C₀-C₈)-Alkylen-Aryl, O-(C₀-C₈)-Alkylen-Aryl, S-Aryl, N((C₁-C₆)-Alkyl)₂, SO₂-CH₃, COOH, COO-(C₁-C₆)-Alkyl, CO-N((C₁-C₆)-Alkyl)₂;
sowie deren physiologisch verträglichen Salze.

2. Verbindungen der Formel I, gemäß Anspruch 1, **dadurch gekennzeichnet, daß** darin I die Struktur Ia besitzt 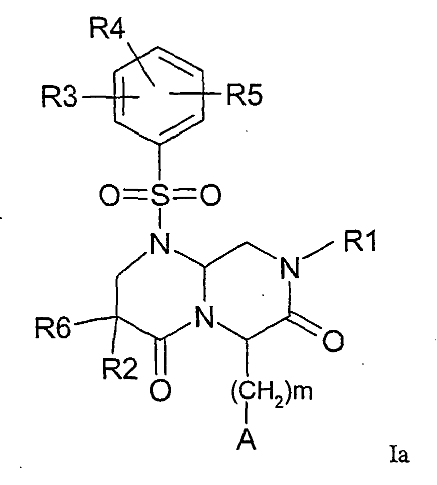 worin bedeuten
A 3-12 gliedriger mono-, bi- oder spirobicyclischer Ring, der ein oder mehrere Heteroatome aus der Gruppe N, O und S enthalten kann und der 3-12 gliedrige Ring weitere Substituenten wie F, Cl, Br, NO₂, CF₃, OCF₃, CN, (C₁-C₆)-Alkyl, Aryl, CON(R11)(R12), N(R13)(R14), OH, O-(C₁-C₆)-Alkyl, S-(C₁-C₆)-Alkyl, N(R15)CO(C₁-C₆)-Alkyl oder COO-(C₁-C₆)-Alkyl tragen kann;
R11, R12, R13, R14, R15 unabhängig voneinander H, (C₁-C₆)-Alkyl, Heterocyclus;
m 0, 1, 2, 3, 4, 5, 6;
R1 R8, (C₁-C₆)-Alkylen-R8, (C₂-C₆)-Alkenylen-R9, (SO₂)-R8, (SO₂)-(C₁-C₆)-Alkylen-R8, (SO₂)-(C₂-C₆)-Alkenylen-R9, (C=O)-R8, (C=O)-(C₁-C₆)-Alkylen-R8, (C=O)NH-R8, (C=O)-(C₂-C₆)-Alkenylen-R9, (C=O)-NH- (C₁-C₆)-Alkylen-R8, (C=O)-NH- (C₂-C₆)-Akenylen-R9, COO-R8, COO-(C₁-C₆)-Alkylen-R8, COO-(C₂-C₆)-Alkenylen-R9, Alkinylen-R9, (C₁-C₄-Alkyl)-Heterocyclus;
R8, R9 unabhängig voneinander H, F, Cl, Br, I, OH, CF₃, Aryl, Heterocyclus, (C₃-C₈)-Cycloalkyl, wobei die Ringe oder Ringssysteme bis zu 3-fach substituiert sein können mit F, Cl, Br, I, OH, CF₃, NO₂, CN, OCF₃, O-(C₁-C₆)-Alkyl, (C₁-C₆)-Alkyl, NH₂, CON(R11)(R12), N(R13)(R14), SO₂-CH₃, COOH, COO-(C₁-C₆)-Alkyl, CONH₂;
R2 NH₂,NO₂, N(R13)(R14), NH-SO₂-CH₃, NH-SO₂-R12, NR11-SO₂-R12, N(CO)R11, ein Stickstoffhaltiger Heterocyclus, wobei der Heterocyclus über ein Stickstoffatom gebunden ist, NHCONR11, N(C₁-C₆-Alkyl)N⁺(C₁-C₄-Alkyl)₃;
R3, R4, R5 unabhängig voneinander H, F, Cl, Br, J, OH, CF₃, NO₂, CN, OCF₃, O-(C₁-C₆)-Alkyl, O-(C₁-C₄)-Alkoxy-(C₁-C₄)-alkyl, S-(C₁-C₆)-Alkyl, (C₁-C₆)-Alkyl, (C₂-C₆)-Alkenyl, (C₃-C₈)-Cycloalkyl, O-(C₃-C₈)-Cycloalkyl, (C₃-C₈)-Cycloalkenyl, O-(C₃-C₈)-Cycloalkenyl, (C₂-C₆)-Alkinyl, Aryl, O-Aryl (C₀-C₈)-Alkylen-Aryl, O-(C₀-C₈)-Alkylen-Aryl, S-Aryl, N((C₁-C₆)-Alkyl)₂, SO₂-CH₃, COOH, COO-(C₁-C₆)-Alkyl, CO-N((C₁-C₆)-Alkyl)₂;
R6 H, F, Cl, Br, J, OH, CF₃, NO₂, CN, OCF₃, O-(C₁-C₆)-Alkyl, O-(C₁-C₄)-Alkoxy-(C₁-C₄)-alkyl, S-(C₁-C₆)-Alkyl, (C₁-C₆)-Alkyl, (C₂-C₆)-Alkenyl, (C₃-C₈)-Cycloalkyl, O-(C₃-C₈)-Cycloalkyl, (C₃-C₈)-Cycloalkenyl, O-(C₃-C₈)-Cycloalkenyl, (C₂-C₆)-Alkinyl, Aryl, O-Aryl, (C₁-C₈)-Alkylen-Aryl, O-(C₁-C₈)-Alkylen-Aryl, S-Aryl, N((C₁-C₆)-Alkyl)₂, SO₂-CH₃, COOH, COO-(C₁-C₆)-Alkyl, CO-N((C₁-C₆)-Alkyl)₂;
sowie deren physiologisch verträglichen Salze.

3. Verbindungen der Formel I, gemäß Anspruch 1 oder 2, **dadurch gekennzeichnet, daß** darin bedeuten
A Aryl, wobei der Arylring substituiert sein kann mit F, Cl, Br, NO₂, CF₃, OCF₃, CN, (C₁-C₆)-Alkyl, Aryl, CON(R11)(R12), N(R13)(R14), OH, O-(C₁-C₆)-Alkyl, S-(C₁-C₆)-Alkyl, N(R15)CO(C₁-C₆)-Alkyl oder COO-(C₁-C₆)-Alkyl;
R11, R12, R13, R14, R15 unabhängig voneinander H, (C₁-C₆)-Alkyl, Heterocyclus;
m 1;
R1 R8, (C₁-C₆)-Alkylen-R8, (C₂-C₆)-Alkenylen-R9, (SO₂)-R8, (SO₂)-(C₁-C₆)-Alkylen-R8, (SO₂)-(C₂-C₆)-Alkenylen-R9, (C=O)-R8, (C=O)-(C₁-C₆)-Alkylen-R8, (C=O)NH-R8, (C=O)-(C₂-C₆)-Alkenylen-R9, (C=O)-NH- (C₁-C₆)-Alkylen-R8, (C=O)-NH- (C₂-C₆)-Alkenylen-R9, COO-R8, COO-(C₁-C₆)-Alkylen-R8, COO-(C₂-C₆)-Alkenylen-R9, Alkinylen-R9, (C₁-C₄-Alkyl)-Heterocyclus;
R8, R9 unabhängig voneinander H, F, Cl, Br, I, OH, CF₃, Aryl, Heterocyclus, (C₃-C₈)-Cycloalkyl, wobei die Ringe oder Ringssysteme bis zu 3-fach substituiert sein können mit F, Cl, Br, I, OH, CF₃, NO₂, CN, OCF₃, O-(C₁-C₆)-Alkyl, (C₁-C₆)-Alkyl, NH₂, CON(R11)(R12), N(R13)(R14), SO₂-CH₃, COOH, COO-(C₁-C₆)-Alkyl, CONH₂;
R2 NH₂, NO₂, N(R13)(R14), NH-SO₂-CH₃, NH-SO₂-R12, NR11-SO₂-R12, N(CO)R11, ein Stickstoffhaltiger Heterocyclus, wobei der Heterocyclus über ein Stickstoffatom gebunden ist, NHCONR11, N(C₁-C₆-Alkyl)N⁺(C₁-C₄-Alkyl)₃;
R3 H
R4, R5 unabhängig voneinander H, F, Cl, Br, OH, CF₃, OCF₃, O-(C₁-C₆)-Alkyl, (C₁-C₆)-Alkyl;
R6 H;
sowie deren physiologisch verträglichen Salze.

4. Verbindungen gemäß einem oder mehreren der Ansprüche 1 bis 3, **dadurch gekennzeichnet, daß** darin bedeuten
A Aryl, wobei der Arylring substituiert sein kann mit F, Cl, Br, NO₂, CF₃, OCF₃, CN, (C₁-C₆)-Alkyl, Aryl, CON(R11)(R12), N(R13)(R14), OH, O-(C₁-C₆)-Alkyl, S-(C₁-C₆)-Alkyl, N(R15)CO(C₁-C₆)-Alkyl oder COO-(C₁-C₆)-Alkyl;
R11, R12, R13, R14, R15 unabhängig voneinander H, (C₁-C₆)-Alkyl, Heterocyclus;
m 1;
R1 (C₁-C₆)-Alkyl, (C₁-C₆)-Alkylen-R8;
R8, R9 unabhängig voneinander F, Cl, Br, I, OH, CF₃;
R2 NH₂, NO₂, CN, N(R13)(R14), NH-SO₂-CH₃, NH-SO₂-R12, NR11-SO₂-R12, N(CO)R11, ein Stickstoffhaltiger Heterocyclus, wobei der Heterocyclus über ein Stickstoffatom gebunden ist, NHCONR11, N(C₁-C₆-Alkyl)N⁺(C₁-C₄-Alkyl)₃;
R3 H
R4 F, Cl, Br, OH, CF₃, OCF₃, O-(C₁-C₆)-Alkyl, (C₁-C₆)-Alkyl;
R5 H, F, Cl, Br, OH, CF₃, OCF₃, O-(C₁-C₆)-Alkyl, (C₁-C₆)-Alkyl;
R6 H;
sowie deren physiologisch verträglichen Salze.

5. Verbindungen gemäß einem oder mehreren der Ansprüche 1 bis 4 zur Anwendung als Arzneimittel.

6. Arzneimittel enthaltend eine oder mehrere der Verbindungen gemäß einem oder mehreren der Ansprüche 1 bis 4.

7. Arzneimittel enthaltend eine oder mehrere der Verbindungen gemäß einem oder mehreren der Ansprüche 1 bis 4 und ein oder mehrere anorektische Wirkstoffe.

8. Arzneimittel enthaltend eine oder mehrere der Verbindungen gemäß einem oder mehreren der Ansprüche 1 bis 4 und ein oder mehrere Statine.

9. Arzneimittel, gemäß Anspruch 6, **dadurch gekennzeichnet, daß** es als weiteren Wirkstoff eine oder mehrere Antidiabetika, hypbglykämischen Wirkstoffe, HMGCoA-Reduktase Inhibitoren, Cholesterinresorptionsinhibitoren, PPAR gamma Agonisten, PPAR alpha Agonisten, PPAR alpha/gamma Agonisten, Fibrate, MTP-Inhibitoren, Gallensäureresorptionsinhibitoren, CETP-Inhibitoren, polymere Gallensäureadsorber, LDL-Rezeptorinducer, ACAT-Inhibitoren, Antioxidantien, Lipoprotein-Lipase Inhibitoren, ATP-Citrat-Lyase Inhibitoren, Squalen synthetase inhibitoren, Lipoprotein(a) antagonisten, Lipase Inhibitoren, Insuline, Sulphonylharnstoffe, Biguanide, Meglitinide, Thiazolidindione, α-Glukosidase-Inhibitoren, auf den ATP-abhängigen Kaliumkanal der Betazellen wirkende Wirkstoffe, CART-Agonisten, NPY-Agonisten, MC4-Agonisten, Orexin-Agonisten, H3-Agonisten, TNF-Agonisten, CRF-Agonisten, CRF BP-Antagonisten, Urocortin-Agonisten, β3-Agonisten, MSH (Melanocyt-stimulierendes Hormon)-Agonisten, CCK-Agonisten, Serotonin-Wiederaufnahme-Inhibitoren, gemischte Serotonin- und noradrenerge Verbindungen, 5HT-Agonisten, Bombesin-Agonisten, Galanin-Antagonisten, Wachstumshormone, Wachstumshormon freisetzende Verbindungen, TRH-Agonisten, entkoppelnde Protein 2- oder 3-Modulatoren, Leptinagonisten, DA-Agonisten (Bromocriptin, Doprexin), Lipase/Amylase-Inhibitoren, PPAR-Modulatoren, RXR-Modulatoren oder TR-β-Agonisten oder Amphetamine enthält.

10. Verbindungen gemäß einem oder mehreren der Ansprüche 1 bis 4 in Kombination mit mindestens einem weiteren anorektischen Wirkstoff zur Anwendung als Medikament zur Prophylaxe oder Behandlung der Adipositas.

11. Verbindungen gemäß einem oder mehreren der Ansprüche 1 bis 4 in Kombination mit mindestens einem weiteren anorektischen Wirkstoff zur Anwendung als Medikament zur Prophylaxe oder Behandlung des Typ II Diabetes.

12. Verfahren zur Herstellung eines Arzneimittels enthaltend eine oder mehrere der Verbindungen gemäß einem oder mehreren der Ansprüche 1 bis 4, **dadurch gekennzeichnet, dass** der Wirkstoff mit einem pharmazeutisch geeigneten Träger vermischt wird und diese Mischung in eine für die Verabreichung geeignete Form gebracht wird.

13. Verwendung der Verbindungen gemäß einem oder mehreren der Ansprüche 1 bis 4 zur Herstellung eines Medikaments zur Gewichtsreduktion bei Säugetieren.

14. Verwendung der Verbindungen gemäß einem oder mehreren der Ansprüche 1 bis 4 zur Herstellung eines Medikaments zur Prophylaxe oder Behandlung der Adipositas.

15. Verwendung der Verbindungen gemäß einem oder mehreren der Ansprüche 1 bis 4 zur Herstellung eines Medikaments zur Prophylaxe oder Behandlung des Typ II Diabetes.

16. Verwendung der Verbindungen gemäß einem oder mehreren der Ansprüche 1 bis 4 zur Herstellung eines Medikaments zur Prophylaxe oder Behandlung des Metabolischen Syndroms.

17. Verwendung der Verbindungen gemäß einem oder mehreren der Ansprüche 1 bis 4 zur Herstellung eines Medikaments zur Behandlung von weiblichen und männlichen Sexualstörungen.

## Claims

1. A compound of the formula I, 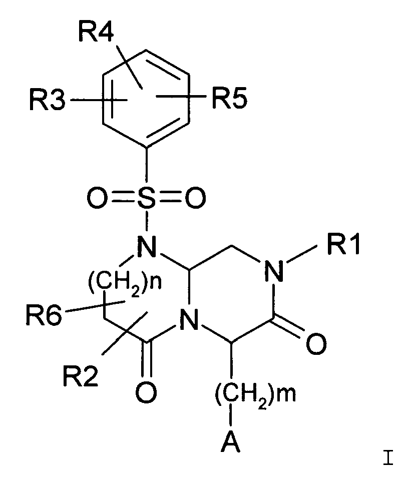 in which the meanings are
A 3-12 membered mono-, bi- or spirobicyclic ring which may comprise one or more heteroatoms from the group of N, O and S and which 3-12 membered ring may have further substituents such as F, Cl, Br, NO₂, CF₃, OCF₃, CN, (C₁-C₆)-alkyl, aryl, CON(R11)(R12), N(R13)(R14), OH, O-(C₁-C₆)-alkyl, S-(C₁-C₆)-alkyl, N(R15)CO(C₁-C₆)-alkyl or COO-(C₁-C₆)-alkyl;
R11, R12, R13, R14, R15 independently of one another H, (C₁-C₆)-alkyl, heterocycle;
n 0, 1;
m 0, 1, 2, 3, 4, 5, 6;
R1 R8, (C₁-C₆)-alkylene-R8, (C₂-C₆)-alkenylene-R9, (SO₂)-R8, (SO₂)-(C₁-C₆)-alkylene-R8, (SO₂)-(C₂-C₆)-alkenylene-R9, (C=O)-R8, (C=O)-(C₁-C₆)-alkylene-R8, (C=O)NH-R8, (C=O)-(C₂-C₆)-alkenylene-R9, (C=O)-NH-(C₁-C₆)-alkylene-R8, (C=O)-NH-(C₂-C₆)-alkenylene-R9, COO-R8, COO-(C₁-C₆)-alkylene-R8, COO-(C₂-C₆)-alkenylene-R9, alkynylene-R9, (C₁-C₄-alkyl)-heterocycle, where the alkylene groups may be substituted by F;
R8, R9 independently of one another H, F, Cl, Br, I, OH, CF₃, aryl, heterocycle, (C₃-C₈)-cycloalkyl, where the rings or ring systems may be substituted up to 3 times by F, Cl, Br, I, OH, CF₃, NO₂, CN, OCF₃, O-(C₁-C₆)-alkyl, (C₁-C₆)-alkyl, NH₂, CON(R11)(R12), N(R13)(R14), SO₂-CH₃, COOH, COO-(C₁-C₆)-alkyl, CONH₂;
R2 NH₂, NO₂ N(R13)(R14), NH-SO₂-CH₃, NH-SO₂-R12, NR11-SO₂-R12, N(CO)R11, a nitrogen-containing heterocycle, where the heterocycle is bonded via a nitrogen atom, NHCONR11, N(C₁-C₆-alkyl)N⁺(C₁-C₄-alkyl)₃;
R3, R4, R5 independently of one another H, F, Cl, Br, I, OH, CF₃, NO₂, CN, OCF₃, O-(C₁-C₆)-alkyl, O-(C₁-C₄)-alkoxy-(C₁-C₄)-alkyl, S-(C₁-C₆)-alkyl, (C₁-C₆)-alkyl, (C₂-C₆)-alkenyl, (C₃-C₈)-cycloalkyl, O-(C₃-C₈)-cycloalkyl, (C₃-C₈)-cycloalkenyl, O- (C₃-C₈)-cycloalkenyl, (C₂-C₆)-alkynyl, aryl, O-aryl (C₁-C₈)-alkylene-aryl, O-(C₁-C₈)-alkylene-aryl, S-aryl, N ((C₁-C₆)-alkyl)₂, SO₂-CH₃, COOH, COO-(C₁-C₆)-alkyl, CO-N ((C₁-C₆)-alkyl) ₂;
R6 H, F, Cl, Br, I, OH, CF₃, NO₂, CN, OCF₃, O-(C₁-C₆)-alkyl, O-(C₁-C₄)-alkoxy-(C₁-C₄)-alkyl, S-(C₁-C₆)-alkyl, (C₁-C₆)-alkyl, (C₂-C₆)-alkenyl, (C₃-C₈)-cycloalkyl, O-(C₃-C₈)-cycloalkyl, (C₃-C₈)-cycloalkenyl, O-(C₃-C₈)-cycloalkenyl, (C₂-C₆)-alkynyl, (C₀-C₈)-alkylene-aryl, O-(C₀-C₈)-alkylene-aryl, S-aryl, N((C₁-C₆)-alkyl)₂, SO₂-CH₃, COOH, COO- (C₁-C₆)-alkyl, CO-N ((C₁-C₆)-alkyl)₂;
and the physiologically tolerated salts thereof.

2. A compound of the formula I as claimed in claim 1, wherein I has the structure Ia 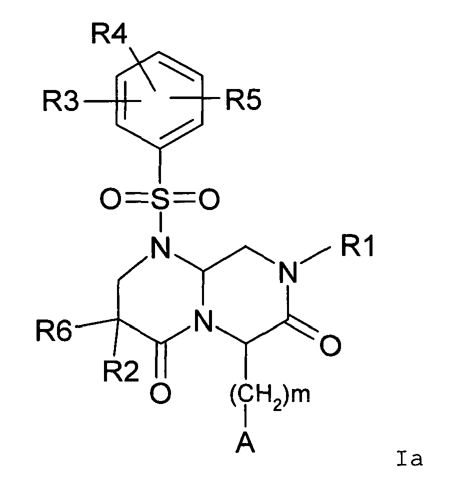 in which the meanings are
A 3-12 membered mono-, bi- or spirobicyclic ring which may comprise one or more heteroatoms from the group of N, O and S and which 3-12 membered ring may have further substituents such as F, Cl, Br, NO₂, CF₃, OCF₃, CN, (C₁-C₆)-alkyl, aryl, CON(R11)(R12), N(R13)(R14), OH, O-(C₁-C₆)-alkyl, S-(C₁-C₆)-alkyl, N(R15)CO(C₁-C₆)-alkyl or COO-(C₁-C₆)-alkyl;
R11, R12, R13, R14, R15 independently of one another H, (C₁-C₆)-alkyl, heterocycle;
m 0, 1, 2, 3, 4, 5, 6;
R1 R8, (C₁-C₆)-alkylene-R8, (C₂-C₆)-alkenylene-R9, (SO₂)-R8, (SO₂)-(C₁-C₆)-alkylene-R8, (SO₂)-(C₂-C₆)-alkenylene-R9, (C=O)-R8, (C=O)-(C₁-C₆)-alkylene-R8, (C=O)NH-R8, (C=O)-(C₂-C₆)-alkenylene-R9, (C=O)-NH-(C₁-C₆)-alkylene-R8, (C=O)-NH-(C₂-C₆)-alkenylene-R9, COO-R8, COO-(C₁-C₆)-alkylene-R8, COO-(C₂-C₆)-alkenylene-R9, alkynylene-R9, (C₁-C₄-alkyl)-heterocycle;
R8, R9 independently of one another H, F, Cl, Br, I, OH, CF₃, aryl, heterocycle, (C₃-C₈)-cycloalkyl, where the rings or ring systems may be substituted up to 3 times by F, Cl, Br, I, OH, CF₃, NO₂, CN, OCF₃, O-(C₁-C₆)-alkyl, (C₁-C₆)-alkyl, NH₂, CON(R11)(R12), N(R13)(R14), SO₂-CH₃, COOH, COO-(C₁-C₆)-alkyl, CONH₂;
R2 NH₂, NO₂, N(R13)(R14), NH-SO₂-CH₃, NH-SO₂-R12, NR11-SO₂-R12, N(CO)R11, a nitrogen-containing heterocycle, where the heterocycle is bonded via a nitrogen atom, NHCONR11, N(C₁-C₆-alkyl) N⁺ (C₁-C₄-alkyl)₃;
R3, R4, R5 independently of one another H, F, Cl, Br, I, OH, CF₃, NO₂, CN, OCF₃, O-(C₁-C₆)-alkyl, O-(C₁-C₄)-alkoxy- (C₁-C₄)-alkyl, S- (C₁-C₆)-alkyl, (C₁-C₆)-alkyl, (C₂-C₆)-alkenyl, (C₃-C₈)-cycloalkyl, O-(C₃-C₈)-cycloalkyl, (C₃-C₈)-cycloalkenyl, O-(C₃-C₈)-cycloalkenyl, (C₂-C₆)-alkynyl, aryl, O-aryl (C₀-C₈)-alkylene-aryl, O-(C₀-C₈)-alkylene-aryl, S-aryl, N ((C₁-C₆)-alkyl)₂, SO₂-CH₃, COOH, COO-(C₁-C₆)-alkyl, CO-N ((C₁-C₆)-alkyl)₂;
R6 H, F, Cl, Br, I, OH, CF₃, NO₂, CN, OCF₃, O-(C₁-C₆)-alkyl, O-(C₁-C₄) -alkoxy-(C₁-C₄)-alkyl, S-(C₁-C₆)-alkyl, (C₁-C₆)-alkyl, (C₂-C₆)-alkenyl, (C₃-C₈)-cycloalkyl, O-(C₃-C₈)-cycloalkyl, (C₃-C₈)-cycloalkenyl, O-(C₃-C₈)-cycloalkenyl, (C₂-C₆)-alkynyl, aryl, O-aryl, (C₁-C₈)-alkylene-aryl, O-(C₁-C₈)-alkylene-aryl, S-aryl, N((C₁-C₆)-alkyl)₂, SO₂-CH₃, COOH, COO-(C₁-C₆)-alkyl, CO-N ((C₁-C₆)-alkyl)₂;
and physiologically tolerated salts thereof.

3. A compound of the formula I as claimed in claim 1 or 2, wherein the meanings are
A aryl, where the aryl ring may be substituted by F, Cl, Br, NO₂, CF₃, OCF₃, CN, (C₁-C₆)-alkyl, aryl, CON(R11)(R12), N(R13)(R14), OH, O-(C₁-C₆)-alkyl, S-(C₁-C₆)-alkyl, N(R15)CO(C₁-C₆)-alkyl or COO-(C₁-C₆)-alkyl;
R11, R12, R13, R14, R15 independently of one another H, (C₁-C₆)-alkyl, heterocycle;
m 1;
R1 R8, (C₁-C₆) -alkylene-R8, (C₂-C₆) -alkenylene-R9, (SO₂)-R8, (SO₂)-(C₁-C₆)-alkylene-R8, (SO₂)-(C₂-C₆)-alkenylene-R9, (C=O)-R8, (C=O)-(C₁-C₆)-alkylene-R8, (C=O)NH-R8, (C=O)-(C₂-C₆)-alkenylene-R9, (C=O)-NH-(C₁-C₆)-alkylene-R8, (C=O)-NH-(C₂-C₆)-alkenylene-R9, COO-R8, COO-(C₁-C₆)-alkylene-R8, COO-(C₂-C₆)-alkenylene-R9, alkynylene-R9, (C₁-C₄-alkyl) -heterocycle;
R8, R9 independently of one another H, F, Cl, Br, I, OH, CF₃, aryl, heterocycle, (C₃-C₈)-cycloalkyl, where the rings or ring systems may be substituted up to 3 times by F, Cl, Br, I, OH, CF₃, NO₂, CN, OCF₃, O-(C₁-C₆)-alkyl, (C₁-C₆)-alkyl, NH₂, CON(R11) (R12), N(R13) (R14), SO₂-CH₃, COOH, COO-(C₁-C₆)-alkyl, CONH₂;
R2 NH₂, NO₂, N (R13) (R14), NH-SO₂-CH₃, NH-SO₂-R12, NR11-SO₂-R12, N(CO)R11, a nitrogen-containing heterocycle, where the heterocycle is bonded via a nitrogen atom, NHCONR11, N(C₁-C₆-alkyl)N₊(C₁-C₄-alkyl)₃;
R3 H
R4, R5 independently of one another H, F, Cl, Br, OH, CF₃, OCF₃, O- (C₁-C₆) -alkyl, (C₁-C₆)-alkyl;
R6 H;
and the physiologically tolerated salts thereof.

4. A compound as claimed in one or more of claims 1 to 3, wherein the meanings are
A aryl, where the aryl ring may be substituted by F, Cl, Br, NO₂, CF₃, OCF₃, CN, (C₁-C₆)-alkyl, aryl, CON(R11)(R12), N(R13)(R14), OH, O-(C₁-C₆)-alkyl, S-(C₁-C₆)-alkyl, N(R15)CO(C₁-C₆)-alkyl or COO-(C₁-C₆)-alkyl;
R11, R12, R13, R14, R15 independently of one another H, (C₁-C₆)-alkyl, heterocycle;
m 1;
R1 (C₁-C₆)-alkyl, (C₁-C₆)-alkylene-R8;
R8, R9 independently of one another F, Cl, Br, I, OH, CF₃;
R2 NH₂, NO₂, CN, N (R13) (R14), NH-SO₂-CH₃, NH-SO₂-R12, NR11-SO₂-R12, N(CO)R11, a nitrogen-containing heterocycle, where the heterocycle is bonded via a nitrogen atom, NHCONR11, N(C₁-C₆-alkyl)N⁺(C₁-C₄-alkyl)₃;
R3 H
R4 F, Cl, Br, OH, CF₃, OCF₃, O-(C₁-C₆)-alkyl, (C₁-C₆)-alkyl;
R5 H, F, Cl, Br, OH, CF₃, OCF₃, O-(C₁-C₆)-alkyl, (C₁-C₆)-alkyl;
R6 H;
and the physiologically tolerated salts thereof.

5. A compound as claimed in one or more of claims 1 to 4 for use as medicament.

6. A medicament comprising one or more of the compounds as claimed in one or more of claims 1 to 4.

7. A medicament comprising one or more of the compounds as claimed in one or more of claims 1 to 4 and one or more anorectic active ingredients.

8. A medicament comprising one or more of the compounds as claimed in one or more of claims 1 to 4 and one or more statins.

9. A medicament as claimed in claim 6, which comprises as further active ingredient one or more antidiabetics, hypoglycemic active ingredients, HMGCoA reductase inhibitors, cholesterol absorption inhibitors, PPAR gamma agonists, PPAR alpha agonists, PPAR alpha/gamma agonists, fibrates, MTP inhibitors, bile acid adsorption inhibitors, CETP inhibitors, polymeric bile acid adsorbents, LDL receptor inducers, ACAT inhibitors, antioxidants, lipoprotein lipase inhibitors, ATP-citrate lyase inhibitors, squalene synthetase inhibitors, lipoprotein(a) antagonists, lipase inhibitors, insulins, sulfonylureas, biguanides, meglitinides, thiazolidinediones, α-glucosidase inhibitors, active ingredients acting on the ATP-dependent potassium channel of beta cells, CART agonists, NPY agonists, MC4 agonists, orexin agonists, H3 agonists, TNF agonists, CRF agonists, CRF BP-antagonists, urocortin agonists, β3 agonists, MSH (melanocyte-stimulating hormone) agonists, CCK agonists, serotonin reuptake inhibitors, mixed serotoninergic and noradrenergic compounds, 5HT agonists, bombesin agonists, galanin antagonists, growth hormones, growth hormone-releasing compounds, TRH agonists, uncoupling protein 2 or 3 modulators, leptin agonists, DA agonists (bromocriptine, Doprexin), lipase/amylase inhibitors, PPAR modulators, RXR modulators or TR-β agonists or amphetamines.

10. A compound as claimed in one or more of claims 1 to 4 in combination with at least one further anorectic active ingredient for use as medicament for the prophylaxis or treatment of obesity.

11. A compound as claimed in one or more of claims 1 to 4 in combination with at least one further anorectic active ingredient for use as medicament for the prophylaxis or treatment of type II diabetes.

12. A process for producing a medicament comprising one or more of the compounds as claimed in one or more of claims 1 to 4, which comprises mixing the active ingredient with a pharmaceutically suitable carrier and converting this mixture into a form suitable for administration.

13. The use of the compound as claimed in one or more of claims 1 to 4 for producing a medicament for weight reduction in mammals.

14. The use of the compound as claimed in one or more of claims 1 to 4 for producing a medicament for the prophylaxis or treatment of obesity.

15. The use of the compound as claimed in one or more of claims 1 to 4 for producing a medicament for the prophylaxis or treatment of type II diabetes.

16. The use of the compound as claimed in one or more of claims 1 to 4 for producing a medicament for the prophylaxis or treatment of metabolic syndrome.

17. The use of the compounds as claimed in one or more of claims 1 to 4 for producing a medicament for the treatment of female and male sexual disorders.

## Revendications

1. Composés de formule I, 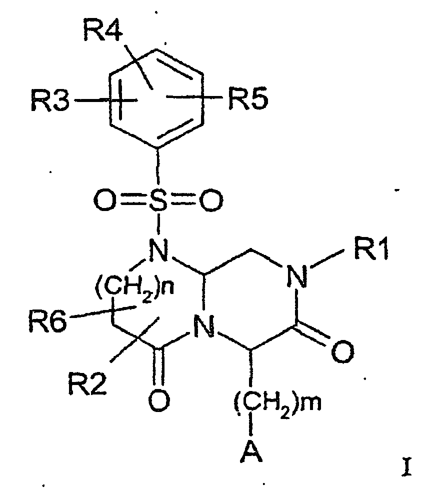 dans laquelle
A représente un cycle mono-, bi- ou spiro-bicyclique à 3-12 chaînons, qui peut contenir un ou plusieurs hétéroatomes choisis parmi N, O et S et le cycle à 3-12 chaînons peut porter d'autres substituants tels que F, Cl, Br, NO₂, CF₃, OCF₃, CN, alkyle en C₁-C₆, aryle, CON(R11)(R12), N(R13)(R14), OH, O-alkyle(C₁-C₆), S-alkyle(C₁-C₆), N(R15)CO-alkyle(C₁-C₆) ou COO-alkyle(C₁-C₆);
R11, R12, R13, R14, R15 représentent, indépendamment les uns des autres, H, un groupe alkyle en C₁-C₆, un hétérocycle;
n est 0, 1 ;
m est 0, 1, 2, 3, 4, 5, 6;
R1 représente R8, un groupe alkylène(C₁-C₆)-R8, alcénylène(C₂-C₆)-R9, (SO₂)-R8, (SO₂)-alkylène (C₁-C₆)-R8, (SO₂)-alcénylène(C₂-C₆)-R9, (C=O)-R8, (C=O)-alkylène(C₁-C₆)₋R8, (C=O)NH-R8, (C=O)-alcénylène(C₂-C₆)₋R9, (C=O)-NH-alkylène(C₁-C₆)-R8, (C=O)-NH-alcénylène(C₂-C₆)-R9, COO-R8, COO-alkylène(C₁-C₆)-R8, COO-alcénylène(C₂-C₆)-R9, alcynylène-R9, [alkyl(C₁-C₄)]-hétérocycle, les groupes alkylène pouvant être substitués par F ;
R8, R9 représentent, indépendamment l'un de l'autre, H, F, Cl, Br, I, OH, CF₃, un groupe aryle, un hétérocycle, un groupe cycloalkyle en C₃-C₈, les cycles ou systèmes cycliques pouvant être jusqu'à trois fois substitués par F, Cl, Br, I, OH, CF₃, NO₂, CN, OCF₃, O-alkyle (C₁-C₆), alkyle en C₁-C₆, NH₂, CON(R11)(R12), N(R13)(R14), SO₂-CH₃, COOH, COO-alkyle(C₁-C₆), CONH₂;
R2 représente NH₂, NO₂, N(R13)(R14), NH-SO₂-CH₃, NH-SO₂-R12, NR11-SO₂-R12, N(CO)R11, un hétérocycle azoté, l'hétérocycle étant lié par un atome d'azote, NHCONR11, N[alkyle(C₁-C₆)]N⁺[alkyle(C₁-C₄)]₃ ;
R3, R4, R5 représentent, chacun indépendamment, H, F, Cl, Br, I, OH, CF₃, NO₂, CN, OCF₃, un groupe O-alkyle(C₁-C₆), O-alcoxy(C₁-C₄)-alkyle (C₁-C₄), S-alkyle (C₁-C₆), alkyle en C₁-C₆, alcényle en C₂-C₆, cycloalkyle en C₃-C₈, O-cycloalkyle(C₃-C₈), cycloalcényle en C₃-C₈, O-cycloalcényle(C₃₋C₈₎, alcynyle en C₂-C₆, aryle, O-aryle, alkylène(C₁-C₈)-aryle, O-alkylène (C₁-C₈)-aryle, S-aryle, N[alkyle(C₁-C₆)]₂, SO₂-CH₃, COOH, COO-alkyle (C₁-C₆), CO-N[alkyle(C₁-C₆)]₂;
R6 représente H, F, Cl, Br, I, OH, CF₃, NO₂, CN, OCF₃, un groupe O-alkyle(C₁-C₆), O-alcoxy (C₁-C₄) -alkyle (C₁-C₄), S-alkyle (C₁-C₆), alkyle en C₁-C₆, alcényle en C₂-C₆, cycloalkyle en C₃-C₈, O-cycloalkyle(C₃-C₈), cycloalcényle en C₃-C₈, O-cycloalcényle (C₃-C₈) alcynyle en C₂-C₆, alkylène(C₀-C₈)-aryle, O-alkylène(C₀-C₈)-aryle, S-aryle, N[alkyle(C₁-C₆)]₂, SO₂-CH₃, COOH, COO-alkyle(C₁-C₆), CO-N[alkyle(C₁-C₆₎]₂ ;
ainsi que leurs sels physiologiquement acceptables.

2. Composés de formule I selon la revendication 1, **caractérisés en ce que** dans ces composés I présente la structure Ia 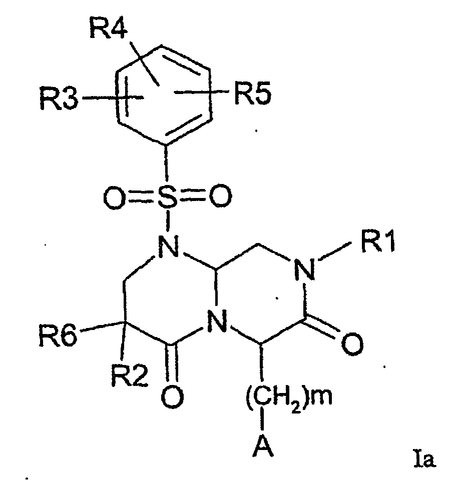 dans laquelle
A représente un cycle mono-, bi- ou spiro-bicyclique à 3-12 chaînons, qui peut contenir un ou plusieurs hétéroatomes choisis parmi N, O et S et le cycle à 3-12 chaînons peut porter d'autres substituants tels que F, Cl, Br, NO₂, CF₃, OCF₃, CN, alkyle en C₁-C₆, aryle, CON(R11)(R12), N(R13)(R14), OH, O-alkyle (C₁-C₆), S-alkyle(C₁-C₆), N(R15)CO-alkyle(C₁-C₆) ou COO-alkyle(C₁-C₆);
R11, R12, R13, R14, R15 représentent, indépendamment les uns des autres, H, un groupe alkyle en C₁-C₆, un hétérocycle;
m est 0, 1, 2, 3, 4, 5, 6 ;
R1 représente R8, un groupe alkylène(C₁-C₆)-R8, alcénylène(C₂-C₆)-R9, (SO₂)-R8, (SO₂)-alkylène(C₁-C₆)-R8, (SO₂)-alcénylène(C₂-C₆)-R9, (C=O)-R8, (C=O)-alkylène(C₁-C₆)-R8, (C=O)NH-R8, (C=O)-alcénylène(C₂-C₆)-R9, (C=O)-NH-alkylène(C₁-C₆)-R8, (C=O)-NH-alcénylène(C₂-C₆)-R9, COO-R8, COO-alkylène(C₁-C₆)-R8, COO-alcénylène(C₂-C₆)-R9, alcynylène-R9, [alkyl(C₁-C₄)]-hétérocycle;
R8, R9 représentent, indépendamment l'un de l'autre, H, F, Cl, Br, I, OH, CF₃, un groupe aryle, un hétérocycle, un groupe cycloalkyle en C₃-C₈, les cycles ou systèmes cycliques pouvant être jusqu'à trois fois substitués par F, Cl, Br, I, OH, CF₃, NO₂, CN, OCF₃, O-alkyle (C₁-C₆), alkyle en C₁-C₆, NH₂, CON(R11)(R12), N(R13)(R14), SO₂-CH₃, COOH, COO-alkyle(C₁-C₆), CONH₂;
R2 représente NH₂, NO₂, N(R13)(R14), NH-SO₂-CH₃, NH-SO₂-R12, NR11-SO₂-R12, N(CO)R11, un hétérocycle azoté, l'hétérocycle étant lié par un atome d'azote, NHCONR11, N[alkyle(C₁-C₆)]N⁺[alkyle(C₁-C₄)]_{3;}
R3, R4, R5 représentent, chacun indépendamment, H, F, Cl, Br, I, OH, CF₃, NO₂, CN, OCF₃, un groupe O-alkyle(C₁-C₆), O-alcoxy(C₁-C₄)-alkyle(C₁-C₄), S-alkyle(C₁-C₆), alkyle en C₁-C₆, alcényle en C₂-C₆, cycloalkyle en C₃-C₈, O-cycloalkyle(C₃-C₈), cycloalcényle en C₃-C₈, O-cycloalcényle(C₃-C₈), alcynyle en C₂-C₆, aryle, O-aryle, alkylène(C₀-C₈)-aryle, O-alkylène(C₀-C₈)-aryle, S-aryle, N [alkyle (C₁-C₆)]₂, SO₂-CH₃, COOH, COO-alkyle(C₁-C₆), CO-N[alkyle(C₁-C₆)]₂ ;
R6 représente H, F, Cl, Br, I, OH, CF₃, NO₂, CN, OCF₃, un groupe O-alkyle(C₁-C₆), O-alcoxy (C₁-C₄) -alkyle (C₁-C₄), S-alkyle (C₁-C₆), alkyle en C₁-C₆, alcényle en C₂-C₆, cycloalkyle en C₃-Cₑ, O-cycloalkyle(C₃-C₈), cycloalcényle en C₃-C₈, O-cycloalcényle (C₃-C₈), alcynyle en C₂-C₆, aryle, O-aryle, alkylène(C₁-C₈)-aryle, O-alkylène(C₁-C₈)-aryle, S-aryle, N [alkyle (C₁-C₆)]₂, SO₂-CH₃, COOH, COO-alkyle (C₁-C₆), CO-N[alkyle(C₁-C₆)]₂;
ainsi que leurs sels physiologiquement acceptables.

3. Composés de formule I selon la revendication 1 ou 2, **caractérisés en ce que** dans ces composés
A représente un cycle aryle, le cycle aryle pouvant être substitué par F, Cl, Br, NO₂, CF₃, OCF₃, CN, alkyle en C₁-C₆, aryle, CON(R11)(R12), N(R13)(R14), OH, O-alkyle (C₁-C₆), S-alkyle(C₁-C₆), N(R15)CO-alkyle(C₁-C₆) ou COO-alkyle(C₁-C₆);
R11, R12, R13, R14, R15 représentent, indépendamment les uns des autres, H, un groupe alkyle en C₁-C₆, un hétérocycle;
m est 1;
R1 représente R8, un groupe alkylène(C₁-C₆)-R8, alcénylène(C₂-C₆)-R9, (SO₂)-R8, (SO₂)-alkylène(C₁-C₆)-R8, (SO₂)-alcénylène(C₂-C₆)-R9, (C=O)-R8, (C=O)-alkylène(C₁-C₆)-R8, (C=O)NH-R8, (C=O)-alcénylène(C₂-C₆)-R9, (C=O)-NH-alkylène(C₁-C₆)-R8, (C=O)-NH-alcénylène(C₂-C₆)-R9, COO-R8, COO-alkylène(C₁-C₆)-R8, COO-alcénylène(C₂-C₆)-R9, alcynylène-R9, [alkyl(C₁-C₄)]-hétérocycle;
R8, R9 représentent, indépendamment l'un de l'autre, H, F, Cl, Br, I, OH, CF₃, un groupe aryle, un hétérocycle, un groupe cycloalkyle en C₃-C₈, les cycles ou systèmes cycliques pouvant être jusqu'à trois fois substitués par F, Cl, Br, I, OH, CF₃, NO₂, CN, OCF₃, O-alkyle (C₁-C₆), alkyle en C₁-C₆, NH₂, CON(R11)(R12), N(R13)(R14), SO₂-CH₃, COOH, COO-alkyle(C₁-C₆), CONH₂;
R2 représente NH₂, NO₂, N(R13)(R14), NH-SO₂-CH₃, NH-SO₂-R12, NR11-SO₂-R12, N(CO)R11, un hétérocycle azoté, l'hétérocycle étant lié par un atome d'azote, NHCONR11, N[alkyle(C₁-C₆)]N⁺[alkyle(C₁-C₄)]₃;
R3 représente H ;
R4, R5 représentent, chacun indépendamment, H, F, Cl, Br, OH, CF₃, OCF₃, un groupe O-alkyle(C₁-C₆), alkyle en C₁-C₆;
R6 représente H ;
ainsi que leurs sels physiologiquement acceptables.

4. Composés selon une ou plusieurs des revendications 1 à 3, **caractérisés en ce que** dans ces composés
A représente un cycle aryle, le cycle aryle pouvant être substitué par F, Cl, Br, NO₂, CF₃, OCF₃, CN, alkyle en C₁-C₆, aryle, CON(R11)(R12), N(R13)(R14), OH, O-alkyle(C₁-C₆), S-alkyle(C₁-C₆), N(R15)CO-alkyle(C₁-C₆) ou COO-alkyle(C₁-C₆):
R11, R12, R13, R14, R15 représentent, indépendamment les uns des autres, H, un groupe alkyle en C₁-C₆, un hétérocycle;
m est 1 ;
R1 représente un groupe alkyle en C₁-C₆, alkylène(C₁-C₆)-R8;
R8, R9 représentent, indépendamment l'un de l'autre, F, Cl, Br, I, OH, CF₃ ;
R2 représente NH₂, NO₂, CN, N(R13)(R14), NH-SO₂-CH₃, NH-SO₂-R12, NR11-SO₂-R12, N(CO)R11, un hétérocycle azoté, l'hétérocycle étant lié par un atome d'azote, l'hétérocycle étant lié par un atome d'azote, NHCONR11, N[alkyle(C₁-C₆)]N⁺[alkyle (C₁-C₄)]₃;
R3 représente H ;
R4 représente F, Cl, Br, OH, CF₃, OCF₃, un groupe O-alkyle(C₁-C₆), alkyle en C₁-C₆;
R5 représente H, F, Cl, Br, OH, CF₃, OCF₃, un groupe O-alkyle(C₁-C₆), alkyle en C₁-C₆;
R6 représente H ;
ainsi que leurs sels physiologiquement acceptables.

5. Composés selon une ou plusieurs des revendications 1 à 4, pour utilisation en tant que médicament.

6. Médicament contenant un ou plusieurs des composés selon une ou plusieurs des revendications 1 à 4.

7. Médicament contenant un ou plusieurs des composés selon une ou plusieurs des revendications 1 à 4 et un ou plusieurs principes actifs anorexigènes.

8. Médicament contenant un ou plusieurs des composés selon une ou plusieurs des revendications 1 à 4 et une ou plusieurs statines.

9. Médicament selon la revendication 6, **caractérisé en ce qu'**il contient comme autre principe actif un(e) ou plusieurs antidiabétiques, principes actifs hypoglycémiants, inhibiteurs de HMGCoA-réductase, inhibiteurs de la résorption du cholestérol, agonistes de PPAR gamma, agonistes de PPAR alpha, agonistes de PPAR alpha/gamma, fibrates, inhibiteurs de MTP, inhibiteurs de la résorption d'acides biliaires, inhibiteurs de CETP, adsorbeurs polymères d'acides biliaires, inducteurs de récepteurs de LDL, inhibiteurs d'ACAT, antioxydants, inhibiteurs de lipoprotéine lipase, inhibiteurs d'ATP-citrate lyase, inhibiteurs de squalène synthétase, antagonistes de lipoprotéine (a), inhibiteurs de lipase, insulines, sulfonylurées, biguanides, méglitinides, thiazolidinediones, inhibiteurs d'α-glucosidase, principes actifs agissant sur le canal potassique ATP-dépendant des cellules bêta, agonistes de CART, agonistes de NPY, agonistes de MC4, agonistes d'orexine, agonistes de H3, agonistes de TNF, agonistes de CRF, antagonistes de CRF BP, agonistes d'urocortine, agonistes de β3, agonistes de MSH (hormone stimulant les mélanocytes), agonistes de CCK, inhibiteurs de la recapture de sérotonine, composés sérotoninergiques et noradrénergiques mixtes, agonistes de 5HT, agonistes de bombésine, antagonistes de galanine, hormones de croissance, composés libérant l'hormone de croissance, agonistes de TRH, modulateurs de protéine 2 ou 3 découpleurs, agonistes de leptine, agonistes de DA (bromocriptine, doprexine), inhibiteurs de lipase/amylase, modulateurs de PPAR, modulateurs de RXR ou agonistes de TR-β ou amphétamines.

10. Composés selon une ou plusieurs des revendications 1 à 4, en association avec au moins un autre principe actif anorexigène, pour utilisation en tant que médicament destiné à la prophylaxie ou au traitement de l'adiposité.

11. Composés selon une ou plusieurs des revendications 1 à 4, en association avec au moins un autre principe actif anorexigène, pour utilisation en tant que médicament destiné à la prophylaxie ou au traitement du diabète de type II.

12. Procédé pour la fabrication d'un médicament contenant un ou plusieurs des composés selon une ou plusieurs des revendications 1 à 4, **caractérisé en ce qu'**on mélange le principe actif avec un véhicule pharmaceutiquement convenable et on met ce mélange sous une forme appropriée à l'administration.

13. Utilisation des composés selon une ou plusieurs des revendications 1 à 4, pour la fabrication d'un médicament destiné à la réduction du poids chez les mammifères.

14. Utilisation des composés selon une ou plusieurs des revendications 1 à 4, pour la fabrication d'un médicament destiné à la prophylaxie ou au traitement de l'adiposité.

15. Utilisation des composés selon une ou plusieurs des revendications 1 à 4, pour la fabrication d'un médicament destiné à la prophylaxie ou au traitement du diabète de type II.

16. Utilisation des composés selon une ou plusieurs des revendications 1 à 4, pour la fabrication d'un médicament destiné à la prophylaxie ou au traitement du syndrome métallique.

17. Utilisation des composés selon une ou plusieurs des revendications 1 à 4, pour la fabrication d'un médicament destiné au traitement de troubles sexuels masculins et féminins.
